# EUROPEAN PATENT APPLICATION

(11) **EP 4 703 357 A1**
(43) Date of publication of application: **04.03.2026**
(21) Application number: 24796271.5
(22) Date of filing: 26.04.2024
(51) Int. Cl.: C07D 401/14, A61K 31/4196, A61K 31/5377, A61P 35/00

(54) **TRIAZOLYL-CONTAINING COMPOUNDS**

(30) Priority: 28.04.2023 CN 202310493340
(71) Applicant: Chia Tai Tianqing Pharmaceutical Group Co., Ltd., Lianyungang, Jiangsu 222062 (CN)
(72) Inventor: PENG, Yan, Lianyungang, Jiangsu 222062 (CN); ZHOU, Jiawei, Lianyungang, Jiangsu 222062 (CN); ZHAO, Qingzhen, Lianyungang, Jiangsu 222062 (CN); ZHANG, Shibo, Lianyungang, Jiangsu 222062 (CN); JIANG, Shuaishuai, Lianyungang, Jiangsu 222062 (CN)
(74) Representative: Grünecker Patent- und Rechtsanwälte PartG mbB
(86) International application number: PCT/CN2024/090155
(87) International publication number: WO 2024/222900

(57) **Abstract**

The present application belongs to the field of pharmaceutical chemistry, and provides triazolyl-containing compounds and a preparation method therefor. Particularly, the present application relates to crystals or solvates of compounds of formula I, and relates to the use thereof in preparation of drugs for treating tumors.

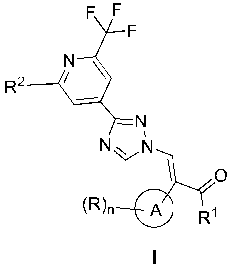

## Description

### CROSS-REFERENCE TO RELATED APPLICATIONS

The present application claims priority and benefit to the Chinese Patent Application No. 202310493340.1 filed with China National Intellectual Property Administration on April 28, 2023, the disclosure of which is incorporated herein by reference in its entirety.

### TECHNICAL FIELD

The present disclosure pertains to the field of pharmaceutical chemistry, and provides a triazolyl-containing compound and a preparation method therefor. Particularly, the present disclosure relates to a crystalline form of the compound, a solvate of the compound, and the like, and relates to use thereof in preparing a medicament for treating tumors.

### BACKGROUND

Nuclear export protein-1 (exportin-1, also known as CRM-1 or XPO-1) has become a key "carrier" protein for transporting some crucial growth-regulatory proteins and tumor suppressor factors from the nucleus to the cytoplasm of eukaryotic cells. When efflux of the exportin-1 becomes abnormally high (e.g., due to the production of overexpressed XPO-1), depletion of these nuclear regulator factors can trigger a wide variety of diseases.

XPO1 is the sole nuclear export factor transporting tumor suppressor factors (e.g., p53, p27, FOXO1, and IkB), and it is overexpressed in various solid tumors and hematological malignant tumors (e.g., GBM, ovarian cancer, pancreatic cancer, cervical cancer, AML, MM, CLL, and NHL). Here, the primary oncogenic mechanism of XPO1 is overexpression of the XPO1 protein in multiple types of cancer cells, and its association with the proliferated cell cycle, depleting tumor suppressor proteins (e.g., p53, p27, FOXO1, and IkB) in the cell nucleus, which allows growth of cancer cells (e.g., M. L. Crochiere et al., Oncotarget v.7, pages 1863-1877 (2015)). Generally, selective inhibitor of nuclear export (SINE) compounds are a class of small molecules that inhibit nuclear export of cargo proteins by covalently binding to cysteine at position 528 (Cys528) in the cargo-binding pocket of exportin 1 (XPO1, also known as chromosome maintenance protein 1 (CRM1)) and exert anti-proliferative effects. The interaction between XPO1 and the activated small G-protein Ran (Ran-GTP) in the cell nucleus facilitates the binding to cargo proteins that comprise short amino acid sequences of hydrophobic residues known as a nuclear export signal (NES).

According to preclinical results, many inflammatory, neurodegenerative and autoimmune diseases also involve similar pathologies. Thus, for example, glucocorticoids are widely used anti-inflammatory and immunomodulatory drugs, the efficacy/mechanism of action of which is based primarily on the restoration of sufficient steroid-activated glucocorticoid receptors (GRs) to interfere with the overactivity of transcription factors such as NF-κB in the cell nucleus. Nowadays, although drugs that inhibit diseases caused by excessive efflux of XPO-1 have been demonstrated to have clinical efficacy by the U.S. FDA, new drug therapies are still urgently needed for many diseases.

### SUMMARY

In one aspect, the present disclosure provides a compound of formula I, a stereoisomer thereof, a pharmaceutically acceptable salt thereof, or a solvate thereof: 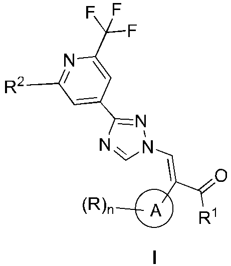 wherein,
R¹ is selected from the group consisting of -NH₂, C₁₋₆ alkyl-O-, C₁₋₆ alkyl-NH-, and (C₁₋₆ alkyl)₂N-; ring A is selected from the group consisting of a C₆₋₁₀ aromatic ring group and a 5- to 10-membered heteroaromatic ring group;
R is selected from the group consisting of halogen, CN, OH, NH₂, C₁₋₆ alkyl, C₁₋₆ alkyl-O-, C₁₋₆ alkyl-S-, C₁₋₆ haloalkyl-O-, C₁₋₆ haloalkyl-S-, C₁₋₆ haloalkyl, C₁₋₆ alkyl substituted with 3- to 10-membered heterocycloalkyl, R^{a}NH-, and (R^{a})₂N-;
R^{a} is selected from the group consisting of C₁₋₆ alkyl, C₃₋₁₀ cycloalkyl, and 3- to 10-membered heterocycloalkyl, and R^{a} is optionally substituted with one or more groups selected from the group consisting of halogen, CN, OH, NH₂, C₁₋₆ alkyl, C₁₋₆ alkyl-O-, and 5- to 6-membered heterocycloalkyl;
n is selected from the group consisting of 0, 1, 2, 3, and 4;
R² is selected from the group consisting of cyclopropyl and trifluoromethyl.

In another aspect, the present disclosure provides a crystalline form or an amorphous form of the compound of formula I, or a crystalline form or an amorphous form of a stereoisomer thereof, or a pharmaceutically acceptable salt of the compound of formula I, or a solvate of the compound of formula I.

In another aspect, the present disclosure provides a crystalline form or an amorphous form of the compound of formula I, or a crystalline form or an amorphous form of a stereoisomer thereof, or a crystalline form or an amorphous form of a pharmaceutically acceptable salt of the compound of formula I, or a solvate of the compound of formula I.

In another aspect, the present disclosure provides a crystalline form or an amorphous form of the compound of formula I, or a crystalline form or an amorphous form of a stereoisomer thereof, or a crystalline form or an amorphous form of a pharmaceutically acceptable salt of the compound of formula I, or a crystalline form or an amorphous form of a solvate of the compound of formula I.

In another aspect, the present disclosure provides a crystalline form of the compound of formula I, or a crystalline form of a stereoisomer thereof, or a crystalline form of a pharmaceutically acceptable salt of the compound of formula I, or a solvate of the compound of formula I.

In another aspect, the present disclosure provides a crystalline form of the compound of formula I, or a crystalline form of a stereoisomer thereof, or a crystalline form or an amorphous form of a pharmaceutically acceptable salt of the compound of formula I, or a crystalline form or an amorphous form of a solvate of the compound of formula I.

In another aspect, the present disclosure provides a crystalline form of the compound of formula I, or a crystalline form of a stereoisomer thereof, or a crystalline form of a pharmaceutically acceptable salt of the compound of formula I, or a crystalline form of a solvate of the compound of formula I.

In another aspect, the present disclosure provides a crystalline form or an amorphous form of the compound of formula I, or a solvate of the compound of formula I.

In another aspect, the present disclosure provides a crystalline form of the compound of formula I or a solvate of the compound of formula I.

In another aspect, the present disclosure provides a crystalline form of the compound of formula I, or a crystalline form or an amorphous form of a solvate of the compound of formula I.

In another aspect, the present disclosure provides a crystalline form of the compound of formula I.

In another aspect, the present disclosure provides a pharmaceutically acceptable salt of the compound of formula I.

In another aspect, the present disclosure provides a solvate of the compound of formula I.

In another aspect, the present disclosure provides a crystalline form or an amorphous form of a solvate of the compound of formula I.

In some embodiments, the compound of formula I is a crystalline form or an amorphous form of the compound of formula I.

In some embodiments, the compound of formula I is a crystalline form of the compound of formula I.

In some embodiments, the compound of formula I is an amorphous form of the compound of formula I.

In some embodiments, the stereoisomer of the compound of formula I is a crystalline form or an amorphous form of the stereoisomer of the compound of formula I.

In some embodiments, the pharmaceutically acceptable salt of the compound of formula I is a crystalline form or an amorphous form of the pharmaceutically acceptable salt of the compound of formula I.

In some embodiments, the solvate of the compound of formula I is a crystalline form or an amorphous form of the solvate of the compound of formula I.

In some embodiments, the compound of formula I, the stereoisomer thereof, the pharmaceutically acceptable salt thereof, or the solvate thereof is selected from the group consisting of a crystalline form of the compound of formula I, a crystalline form or an amorphous form of the stereoisomer thereof, a crystalline form or an amorphous form of the pharmaceutically acceptable salt of the compound of formula I, and a solvate of the compound of formula I.

In some embodiments, the compound of formula I, the stereoisomer thereof, the pharmaceutically acceptable salt thereof, or the solvate thereof is selected from the group consisting of a crystalline form of the compound of formula I, a crystalline form or an amorphous form of the stereoisomer thereof, a crystalline form or an amorphous form of the pharmaceutically acceptable salt of the compound of formula I, and a crystalline form or an amorphous form of the solvate of the compound of formula I.

In some embodiments, the solvate of the compound of formula I has a solvent content of: about 0.5%-50% wt, about 0.5%-40% wt, about 0.5%-30% wt, about 0.5%-20% wt, about 1%-15% wt, about 1.5%-12% wt, about 2%-10% wt, or about 2%-7% wt. In some embodiments, the solvate of the compound of formula I has a solvent content of: about 1% wt, about 2% wt, about 3% wt, about 4% wt, about 5% wt, about 6% wt, about 7% wt, about 8% wt, about 9% wt, about 10% wt, about 11% wt, about 12% wt, about 13% wt, about 14% wt, about 15% wt, about 16% wt, about 17% wt, about 18% wt, about 19% wt, or about 20% wt, or a range formed by any of the above values. In some embodiments, the solvate of the compound of formula I has a solvent content of: about 2% wt, about 4% wt, about 6% wt, about 7% wt, about 8% wt, or about 10% wt, or a range formed by any of the above values. In some embodiments, the solvate of the compound of formula I has a solvent content of: about 2% wt, about 4% wt, about 6% wt, about 7% wt, or about 10% wt, or a range formed by any of the above values. In some embodiments, the solvate of the compound of formula I has a solvent content of: about 2% wt, about 4% wt, or about 7% wt, or a range formed by any of the above values. In some embodiments, the solvate of the compound of formula I has a solvent content of: about 4% wt, about 6% wt, or about 10% wt, or a range formed by any of the above values.

In some embodiments, in the solvate of the compound of formula I, the molar ratio of the compound of formula I to the solvent is selected from the group consisting of 1:0.1-50, 1:0.1-30, 1:0.1-20, 1:0.1-15, 1:0.1-10, 1:0.15-10, 1:0.2-10, 1:0.2-8, 1:0.2-8, 1:0.2-6, 1:0.2-5, 1:0.2-4, 1:0.2-3, 1:0.2-2, 1:0.2-1, and 1:0.3-1.

In some embodiments, in the solvate of the compound of formula I, the molar ratio of the compound of formula I to the solvent is about 1:0.2, about 1:0.3, about 1:0.4, about 1:0.5, about 1:1, about 1:2, about 1:3, about 1:4, about 1:5, about 1:6, about 1:7, about 1:8, about 1:9, or about 1:10, or a range formed by any of the above values.

In some embodiments, in the solvate of the compound of formula I, the molar ratio of the compound of formula I to the solvent is about 1:0.5 or about 1:1, or a range formed by any of the above values. In some embodiments, in the solvate of the compound of formula I, the molar ratio of the compound of formula I to the solvent is about 1:0.5. In some embodiments, in the solvate of the compound of formula I, the molar ratio of the compound of formula I to the solvent is about 1:1.

In some embodiments, the solvent in the solvate of the compound of formula I is selected from one or more of the group consisting of water, hydrocarbons, alcohols, ethers, esters, amides, sulfoxides, ketones, and nitriles.

In some embodiments, the solvent in the solvate of the compound of formula I is selected from one or more of the group consisting of water, hydrocarbons, alcohols, ethers, esters, amides, sulfoxides, and ketones. In some embodiments, the solvent in the solvate of the compound of formula I is selected from one or more of the group consisting of water, alcohols, ethers, esters, and ketones. In some embodiments, the solvent in the solvate of the compound of formula I is selected from one or more of the group consisting of water, alcohols, ethers, esters, ketones, and nitriles.

In some embodiments, the solvent in the solvate of the compound of formula I is selected from one or more of the group consisting of water, C₅₋₁₀ hydrocarbons, C₁₋₁₀ alcohols, C₂₋₁₀ ethers, C₂₋₁₀ esters, C₂₋₁₀ amides, C₂₋₁₀ sulfoxides, C₂₋₁₀ ketones, and C₂₋₁₀ nitriles.

In some embodiments, the solvent in the solvate of the compound of formula I is selected from one or more of the group consisting of water, C₅₋₁₀ hydrocarbons, C₁₋₁₀ alcohols, C₂₋₁₀ ethers, C₂₋₁₀ esters, C₂₋₁₀ amides, C₂₋₁₀ sulfoxides, and C₂₋₁₀ ketones.

In some embodiments, the solvent in the solvate of the compound of formula I is selected from one or more of the group consisting of water, C₅₋₁₀ hydrocarbons, C₁₋₆ alcohols, C₂₋₆ ethers, C₂₋₆ esters, C₂₋₆ amides, C₂₋₆ sulfoxides, C₂₋₆ ketones, and C₂₋₆ nitriles.

In some embodiments, the solvent in the solvate of the compound of formula I is selected from one or more of the group consisting of water, C₅₋₁₀ hydrocarbons, C₁₋₆ alcohols, C₂₋₆ ethers, C₂₋₆ esters, C₂₋₆ amides, C₂₋₆ sulfoxides, and C₂₋₆ ketones.

In some embodiments, the solvent in the solvate of the compound of formula I is selected from one or more of the group consisting of water, C₁₋₁₀ alcohols, C₂₋₁₀ ethers, C₂₋₁₀ esters, C₂₋₁₀ ketones, and C₂₋₁₀ nitriles.

In some embodiments, the solvent in the solvate of the compound of formula I is selected from one or more of the group consisting of water, C₁₋₁₀ alcohols, C₂₋₁₀ ethers, C₂₋₁₀ esters, and C₂₋₁₀ ketones. In some embodiments, the solvent in the solvate of the compound of formula I is selected from one or more of the group consisting of water, C₁₋₆ alcohols, C₂₋₆ ethers, C₂₋₆ esters, C₂₋₆ ketones, and C₂₋₆ nitriles.

In some embodiments, the solvent in the solvate of the compound of formula I is selected from one or more of the group consisting of water, C₁₋₆ alcohols, C₂₋₆ ethers, C₂₋₆ esters, and C₂₋₆ ketones.

In some embodiments, the solvent in the solvate of the compound of formula I is selected from one or more of the group consisting of water, C₁₋₃ alcohols, C₂₋₆ ethers, C₄₋₆ esters, and C₃₋₄ ketones.

In some embodiments, the solvent in the solvate of the compound of formula I is selected from one or more of the group consisting of water, C₁₋₃ alcohols, C₄₋₅ esters, and C₃ ketones.

In some embodiments, the solvent in the solvate of the compound of formula I is selected from one or more of the group consisting of water, C₁₋₆ alcohols, and C₂₋₆ esters.

In some embodiments, the solvent in the solvate of the compound of formula I is selected from one or more of the group consisting of water, C₁₋₆ alcohols, C₂₋₆ ethers, C₂₋₆ esters, C₃₋₄ ketones, and C₂₋₄ nitriles.

In some embodiments, the solvent in the solvate of the compound of formula I is selected from one or more of the group consisting of water, C₁₋₃ alcohols, C₅₋₆ ethers, C₄₋₅ esters, C₃ ketones, and C₂ nitriles.

In some embodiments, the solvent in the solvate of the compound of formula I is selected from one or more of the group consisting of water, methanol, ethanol, isopropanol, 1,4-dioxane, tetrahydrofuran, ethyl acetate, isopropyl acetate, acetone, and acetonitrile.

In some embodiments, the solvent in the solvate of the compound of formula I is selected from one or more of the group consisting of water, methanol, ethanol, isopropanol, ethyl acetate, isopropyl acetate, and acetone.

In some embodiments, the solvent in the solvate of the compound of formula I is selected from one or more of the group consisting of water, a mixture of water and ethyl acetate, methanol, a mixture of methanol and isopropyl acetate, a mixture of methanol and isopropyl acetate and water, ethanol, isopropanol, isopropyl acetate, and acetone.

In some embodiments, the solvent in the solvate of the compound of formula I is selected from one or more of the group consisting of water, a mixture of water and ethyl acetate, methanol, ethanol, isopropanol, isopropyl acetate, and acetone.

In some embodiments, the solvent in the solvate of the compound of formula I is selected from one or more of the group consisting of water, methanol, ethanol, isopropanol, and ethyl acetate.

In some embodiments, the solvent in the solvate of the compound of formula I comprises water, or a mixture of water and ethyl acetate. In some embodiments, the solvent in the solvate of the compound of formula I comprises water. In some embodiments, the solvent in the solvate of the compound of formula I comprises a mixture of water and ethyl acetate.

In some embodiments, the solvent in the solvate of the compound of formula I comprises one or more of methanol, ethanol, or isopropanol.

In some embodiments, the solvent in the solvate of the compound of formula I comprises ethyl acetate or isopropyl acetate. In some embodiments, the solvent in the solvate of the compound of formula I comprises ethyl acetate.

In some embodiments, the solvent in the solvate of the compound of formula I comprises acetone. In some embodiments, the solvent in the solvate of the compound of formula I comprises acetonitrile.

In some embodiments, the solvent in the solvate of the compound of formula I comprises tetrahydrofuran.

In some embodiments, the solvent in the solvate of the compound of formula I comprises 1,4-dioxane.

In some embodiments, the groups in the compound of formula I are as defined herein.

In some embodiments, R¹ is selected from the group consisting of -NH₂, C₁₋₄ alkyl-O-, C₁₋₄ alkyl-NH-, and (C₁₋₄ alkyl)₂N-.

In some embodiments, R¹ is selected from the group consisting of -NH₂, C₁₋₃ alkyl-O-, C₁₋₃ alkyl-NH-, and (C₁₋₃ alkyl)₂N-.

In some embodiments, R¹ is selected from the group consisting of -NH₂, isopropyl-O-, and methyl-NH-. In some embodiments, R¹ is selected from the group consisting of -NH₂ and isopropyl-O-. In some embodiments, R¹ is selected from -NH₂.

In some embodiments, ring A is selected from the group consisting of phenyl and a 5- to 10-membered heteroaromatic ring group. In some embodiments, ring A is selected from the group consisting of 5-, 6-, 7-, 8-, 9-, and 10-membered heteroaromatic ring groups. In some embodiments, ring A is selected from the group consisting of 5- to 6-membered and 9- to 10-membered heteroaromatic ring groups. In some embodiments, ring A is selected from the group consisting of 6- and 10-membered heteroaromatic ring groups. In some embodiments, ring A is selected from a 10-membered heteroaromatic ring group. In some embodiments, ring A is selected from the group consisting of 5- to 6-membered heteroaromatic ring groups. In some embodiments, ring A is selected from a 6-membered heteroaromatic ring group.

In some embodiments, ring A is selected from the group consisting of pyrimidinyl, pyridinyl, pyrazolyl, isoxazolyl, oxazolyl, quinolyl, indazolyl, pyridazinyl, thiazolyl, furanyl, pyranyl, thienyl, pyrrolyl, pyrazinyl, isothiazolyl, oxazolyl, indolyl, naphthyridinyl, isoquinolyl, quinazolinyl, and benzofuranyl.

In some embodiments, ring A is selected from the group consisting of pyrimidinyl, pyridinyl, pyrazolyl, isoxazolyl, quinolyl, indazolyl, naphthyridinyl, and isoquinolyl. In some embodiments, ring A is selected from the group consisting of pyrimidinyl, pyridinyl, and naphthyridinyl. In some specific embodiments, ring A is selected from the group consisting of pyrimidinyl and pyridinyl; in some specific embodiments, ring A is pyrimidinyl; in some specific embodiments, ring A is pyridinyl; in some specific embodiments, ring A is selected from the group consisting of pyrazolyl and isoxazolyl; in some specific embodiments, ring A is selected from the group consisting of quinolyl, indazolyl, naphthyridinyl, and isoquinolyl; in some specific embodiments, ring A is selected from the group consisting of quinolyl and naphthyridinyl.

In some embodiments, ring A is selected from the group consisting of

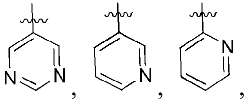

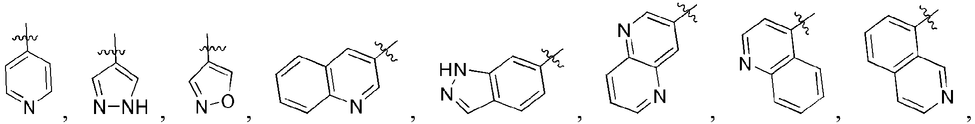

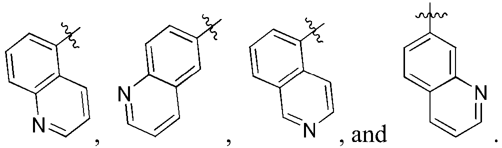

In some embodiments, ring A is selected from the group consisting of

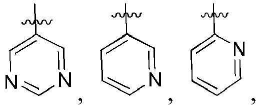

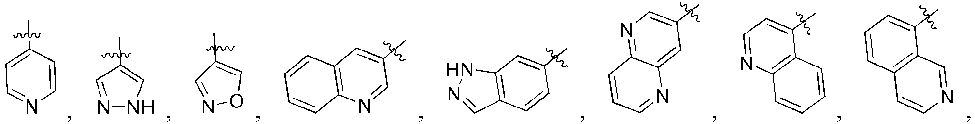

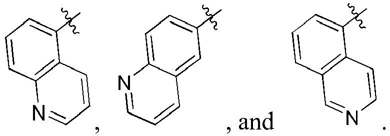

In some embodiments, R is selected from the group consisting of halogen, CN, OH, NH₂, C₁₋₄ alkyl, C₁₋₄ alkyl-O-, C₁₋₄ alkyl-S-, C₁₋₄ haloalkyl-O-, C₁₋₄ haloalkyl-S-, C₁₋₄ haloalkyl, R^{a}NH-, and (R^{a})₂N-. In some embodiments, R is selected from the group consisting of halogen, CN, OH, NH₂, C₁₋₃ alkyl, C₁₋₃ alkyl-O-, C₁₋₃ alkyl-S-, C₁₋₃ haloalkyl-O-, C₁₋₃ haloalkyl-S-, C₁₋₃ haloalkyl, R^{a}NH-, and (R^{a})₂N-. In some embodiments, R is selected from the group consisting of fluorine, chlorine, CN, OH, NH₂, C₁₋₃ alkyl, C₁₋₃ alkyl-O-, C₁₋₃ haloalkyl, and R^{a}NH-.

In some embodiments, R is selected from the group consisting of fluorine, CN, NH₂, methyl, methoxy, trifluoromethyl, and R^{a}NH-.

In some embodiments, R^{a} is selected from the group consisting of C₁₋₄ alkyl, C₃₋₆ cycloalkyl, and 3-to 6-membered heterocycloalkyl, and R^{a} is optionally substituted with one or more groups selected from the group consisting of halogen, CN, OH, NH₂, and 5- to 6-membered heterocycloalkyl.

In some embodiments, R^{a} is selected from the group consisting of C₁₋₃ alkyl, C₅₋₆ cycloalkyl, and 5-to 6-membered heterocycloalkyl, and R^{a} is optionally substituted with one or more groups selected from the group consisting of halogen, CN, OH, NH₂, and 5- to 6-membered heterocycloalkyl.

In some embodiments, R^{a} is selected from the group consisting of C₁₋₃ alkyl and 5- to 6-membered heterocycloalkyl, and R^{a} is optionally substituted with one or more groups selected from the group consisting of halogen, CN, OH, NH₂, and 5- to 6-membered heterocycloalkyl.

In some embodiments, R^{a} is selected from the group consisting of C₁₋₃ alkyl and 6-membered heterocycloalkyl, and R^{a} is optionally substituted with one or more groups selected from the group consisting of fluorine, chlorine, bromine, CN, OH, NH₂, and 6-membered heterocycloalkyl.

In some embodiments, R^{a} is selected from the group consisting of methyl, ethyl, propyl, and tetrahydropyranyl, and R^{a} is optionally substituted with one or more fluorine or dioxane.

In some embodiments, R^{a} is selected from the group consisting of FCH₂CH₂-, F₂CHCH₂-, F₃CCH₂-, CF₃CH(CH₃)-, CH₃CF₂CH₂-, tetrahydropyranyl, and dioxane-CH₂-.

In some embodiments, R is selected from the group consisting of fluorine, CN, NH₂, methyl, methoxy, trifluoromethyl,

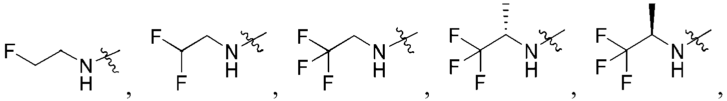

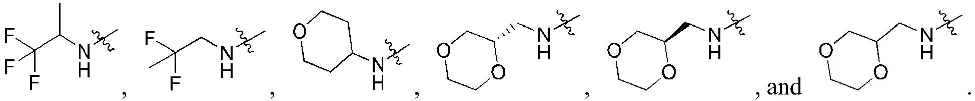

In some embodiments, R is selected from the group consisting of methyl and 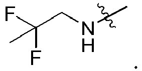 In some embodiments, R is selected from

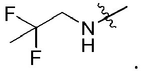

In some embodiments, n is selected from the group consisting of 0, 1, 2, and 3.

In some embodiments, n is selected from the group consisting of 0, 1, and 2.

In some embodiments, n is selected from the group consisting of 0 and 1. In some embodiments, n is selected from 0. In some embodiments, n is selected from 1.

In some embodiments, R² is selected from cyclopropyl.

In some embodiments, structural unit 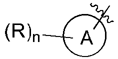 is selected from the group consisting of

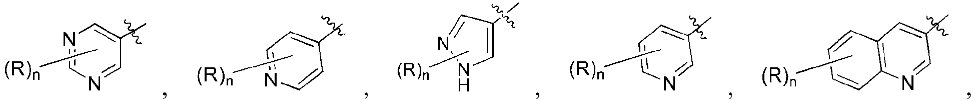

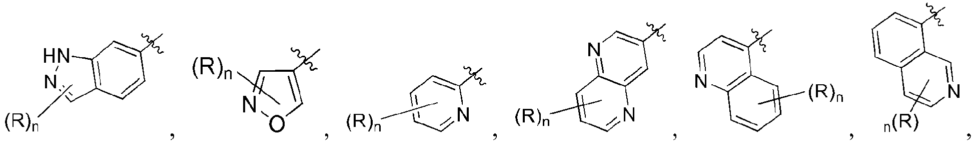

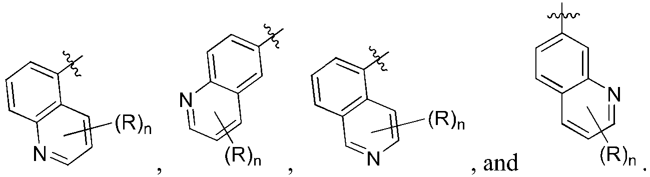

In some embodiments, structural unit 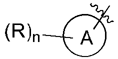 is selected from the group consisting of

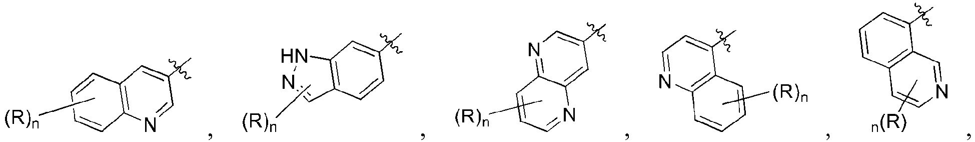

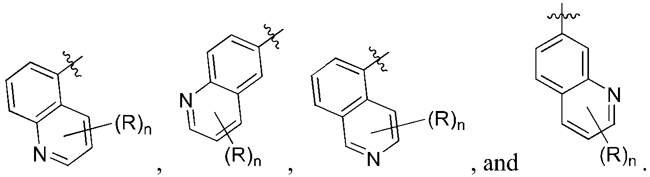

In some embodiments, structural unit 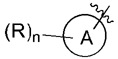 is selected from the group consisting of 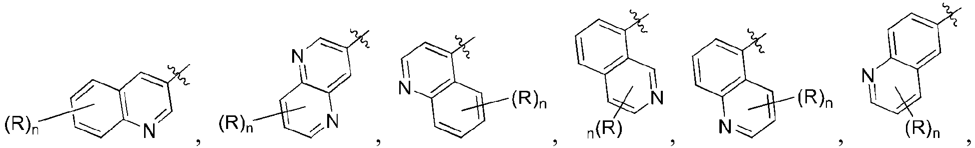 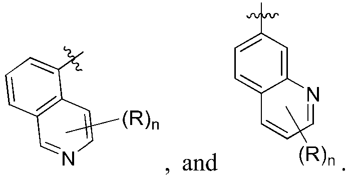 In some embodiments, structural unit 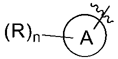 is selected from the group consisting of 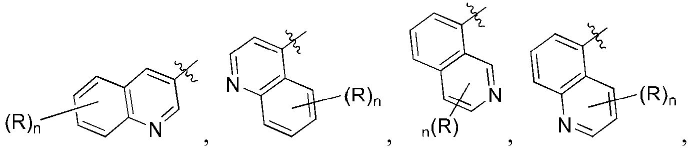 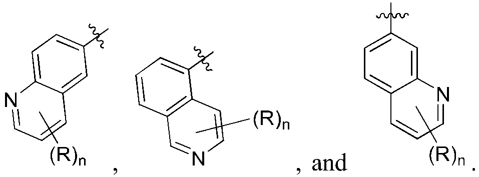 In some embodiments, structural unit 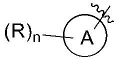 is selected from

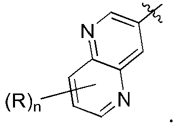

In some embodiments, structural unit 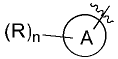 is selected from the group consisting of

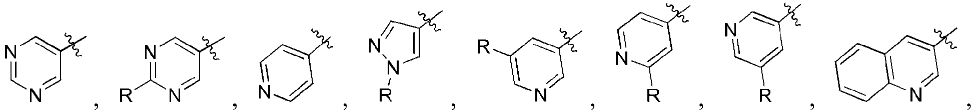

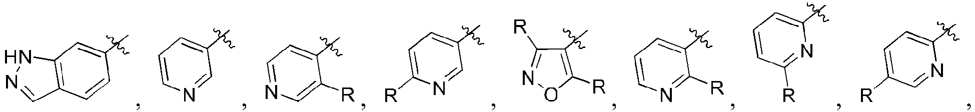

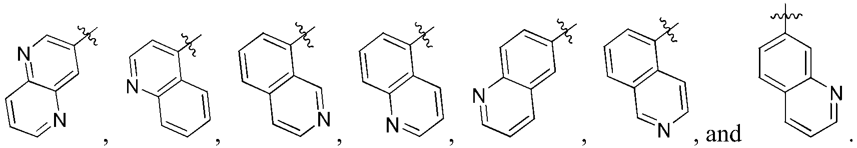

In some embodiments, structural unit 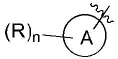 is selected from the group consisting of

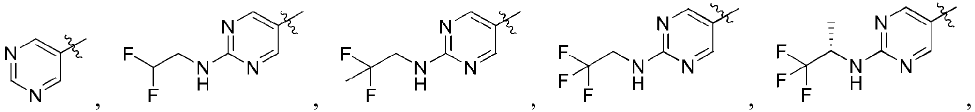

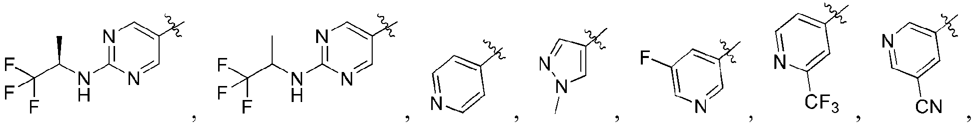

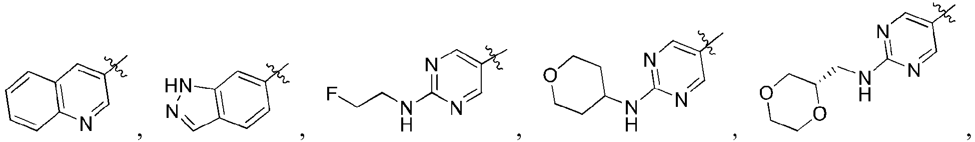

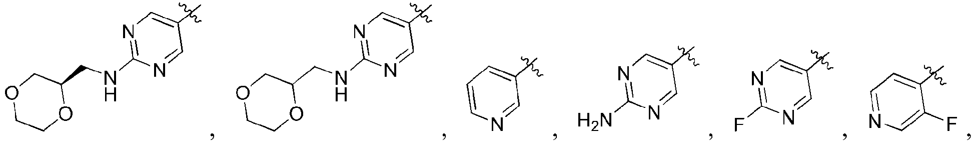

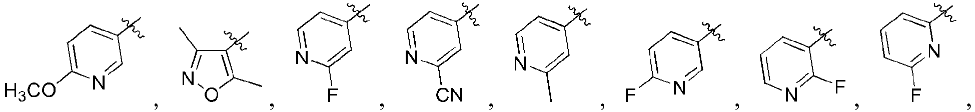

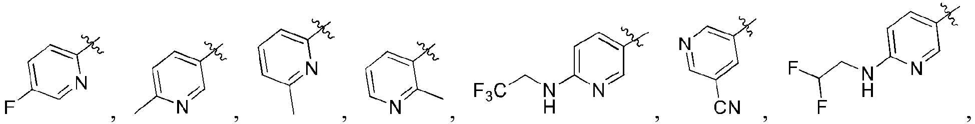

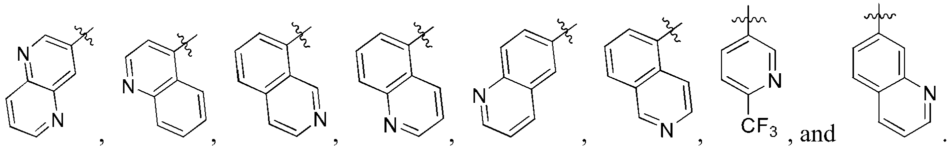

In some embodiments, structural unit 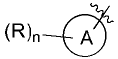 is selected from the group consisting of

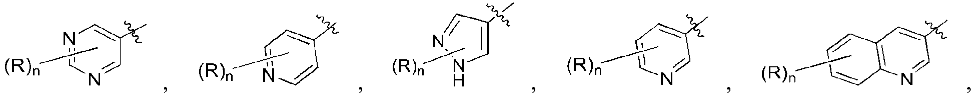

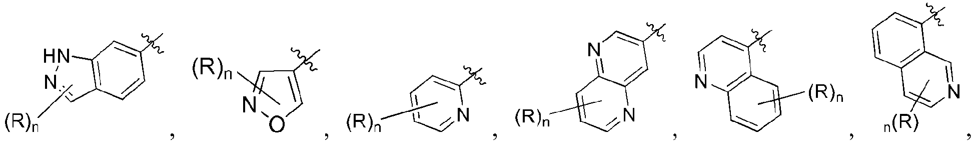

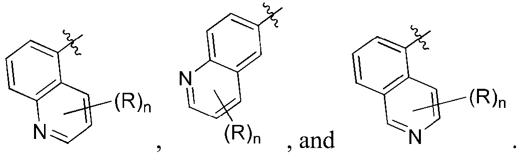

In some embodiments, structural unit 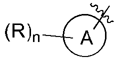 is selected from the group consisting of 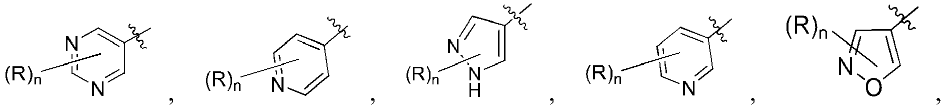 and

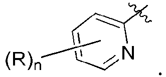

In some embodiments, structural unit 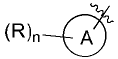 is selected from the group consisting of

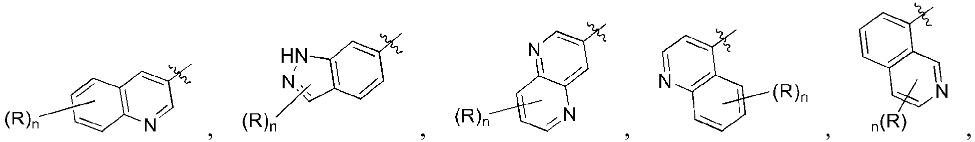

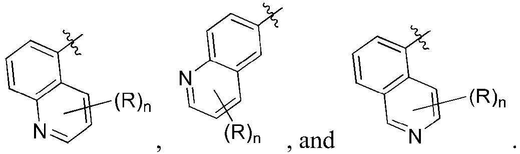

In some embodiments, structural unit 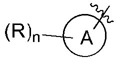 is selected from the group consisting of

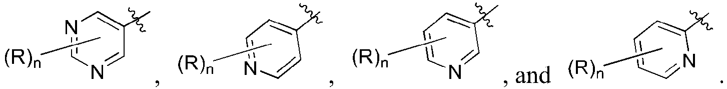

In some embodiments, structural unit 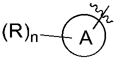 is selected from 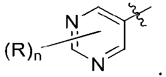 In some embodiments, structural unit 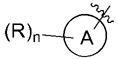 is selected from

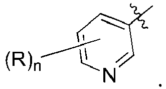

In some embodiments, structural unit 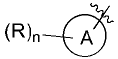 is selected from the group consisting of

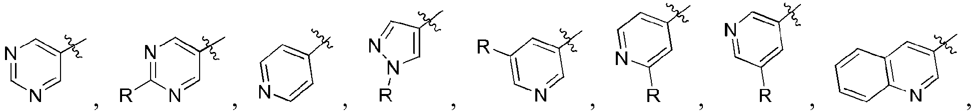

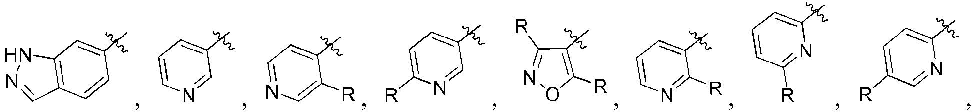

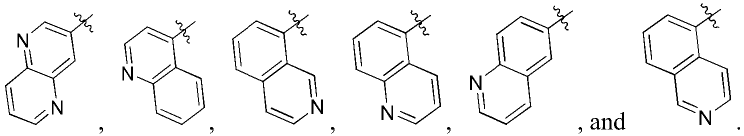

In some embodiments, structural unit 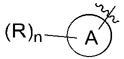 is selected from the group consisting of

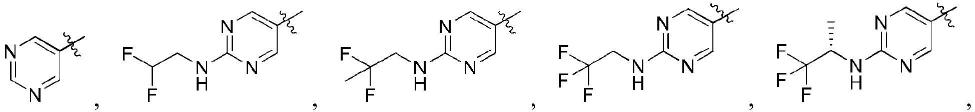

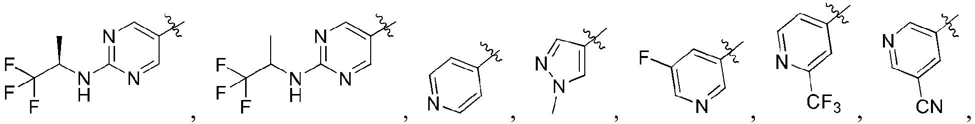

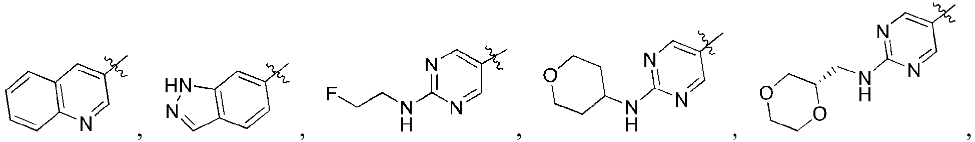

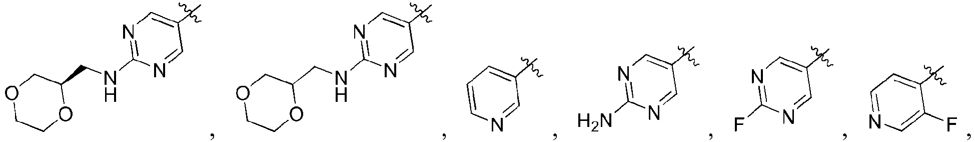

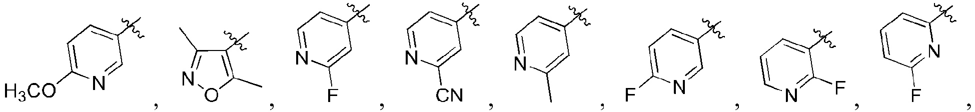

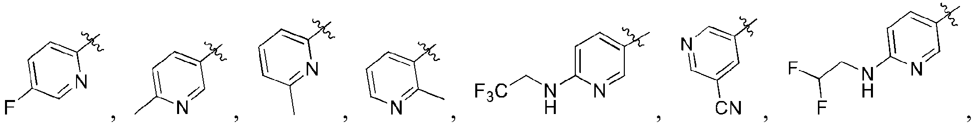

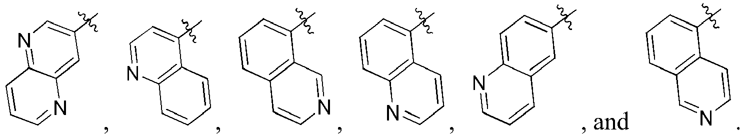

In some embodiments, the compound of formula I is selected from the group consisting of compounds of formula I-1 and I-2, 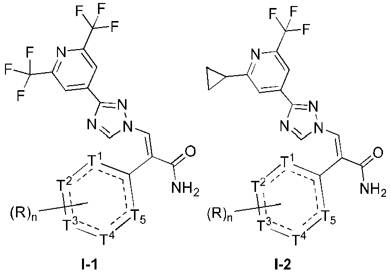
wherein R and n are as described herein;
T¹, T², T³, T⁴, and T⁵ are each independently selected from the group consisting of a bond, O, S, N, and CH, no more than one of which is selected from a bond and at least one of which is selected from N;
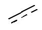 represents a single bond or double bond.

In some embodiments, T¹, T², T³, T⁴, and T⁵ are each independently selected from the group consisting of N and CH, at least one of which is selected from N.

In some embodiments, T² and T⁴ are selected from N, and T¹, T³, and T⁵ are selected from CH.

In some embodiments, T³ is selected from N, and T¹, T², T⁴, and T⁵ are selected from CH.

In some embodiments, T² is selected from N, and T¹, T³, T⁴, and T⁵ are selected from CH.

In some embodiments, structural unit 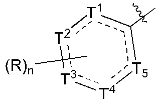 is selected from

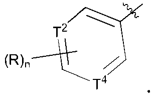

In some embodiments, structural unit 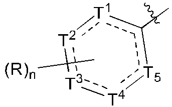 is selected from

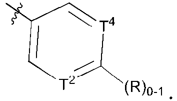

In some embodiments, structural unit 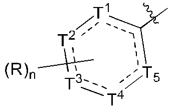 is selected from the group consisting of 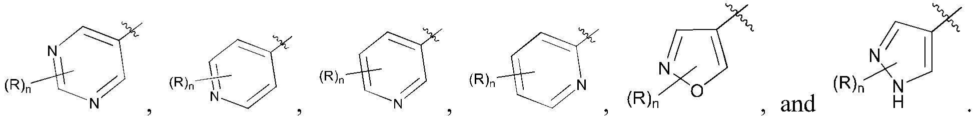 In some embodiments, structural unit 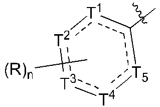 is selected from the group consisting of 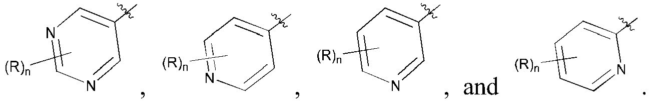 In some embodiments, structural unit 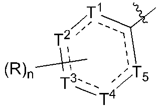 is selected from 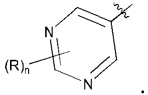 In some embodiments, structural unit 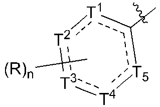 is selected from

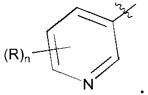

In some embodiments, the present disclosure encompasses the variables defined above and embodiments thereof, as well as any combination thereof.

The heteroatom in the heterocycloalkyl or heteroaromatic ring group described above is selected from the group consisting of nitrogen, oxygen, and sulfur, and the remaining ring atoms are selected from carbon. The heteroatom in the heterocycloalkyl or heteroaromatic ring group described above is selected from the group consisting of nitrogen and oxygen, and the remaining ring atoms are selected from carbon. The heteroatom in the heterocycloalkyl or heteroaromatic ring group described above is selected from nitrogen, and the remaining ring atoms are selected from carbon.

In some embodiments, the number of the heteroatom is selected from the group consisting of 1, 2, 3, and 4. In some embodiments, the number of the heteroatom is selected from the group consisting of 1, 2, and 3. In some embodiments, the number of the heteroatom is selected from the group consisting of 1 and 2.

In some embodiments, the compound of formula I is selected from the group consisting of the following compounds: 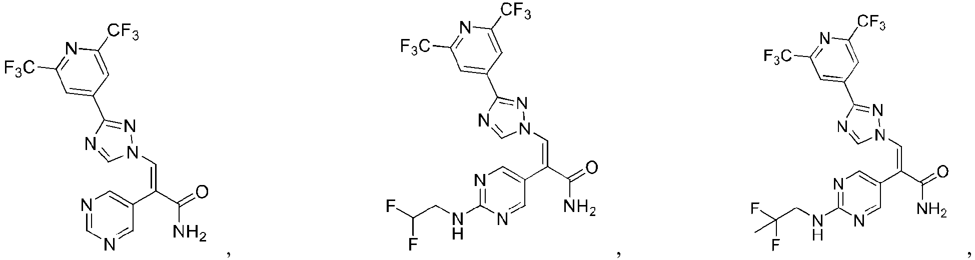 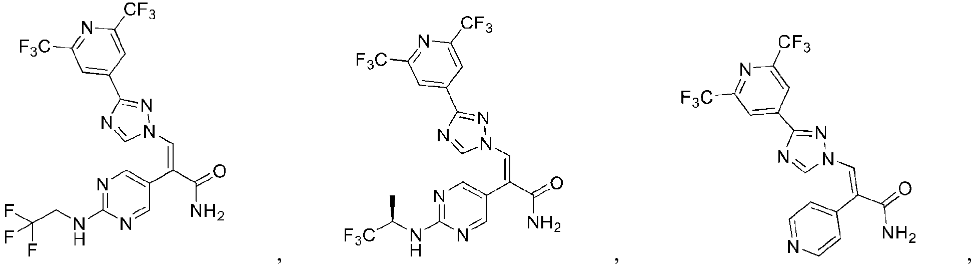 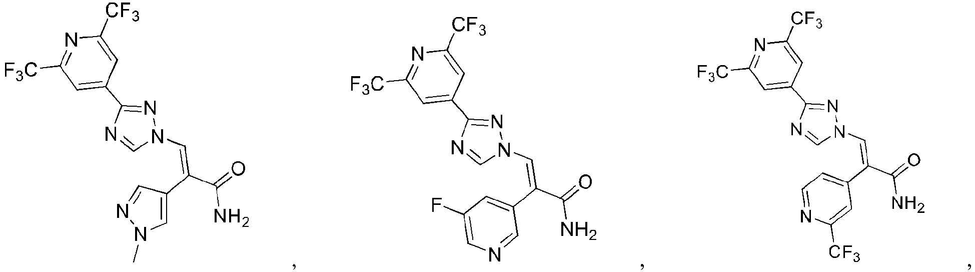 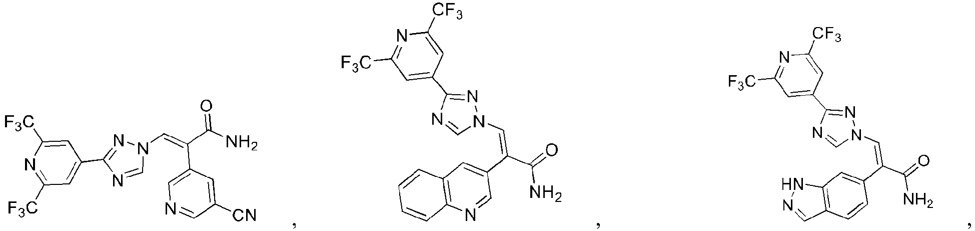 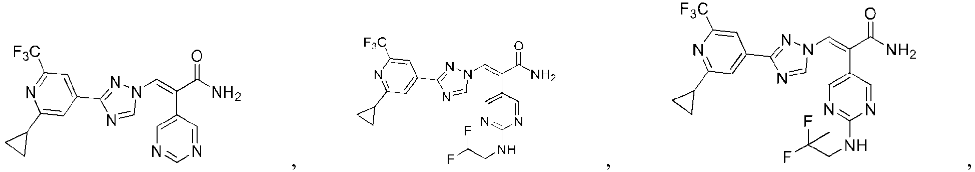 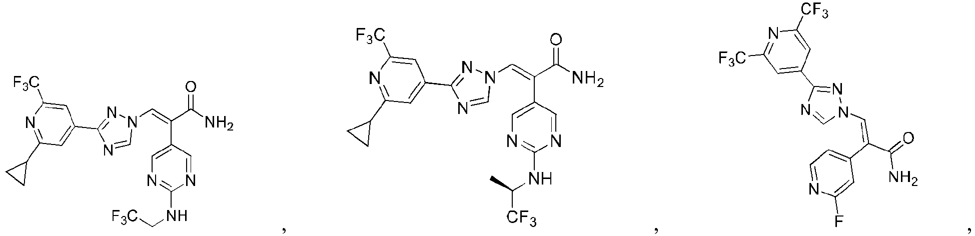 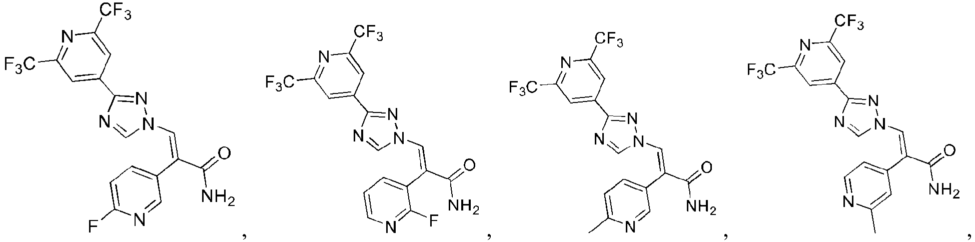 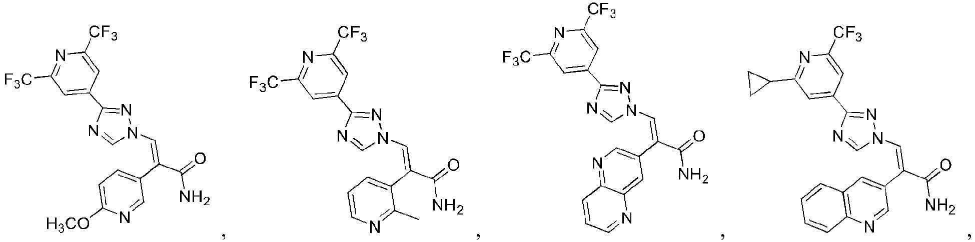 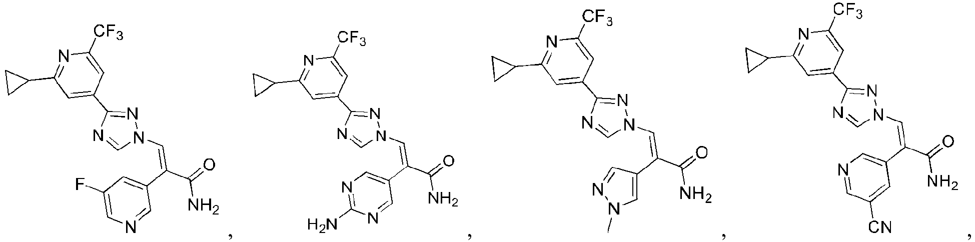 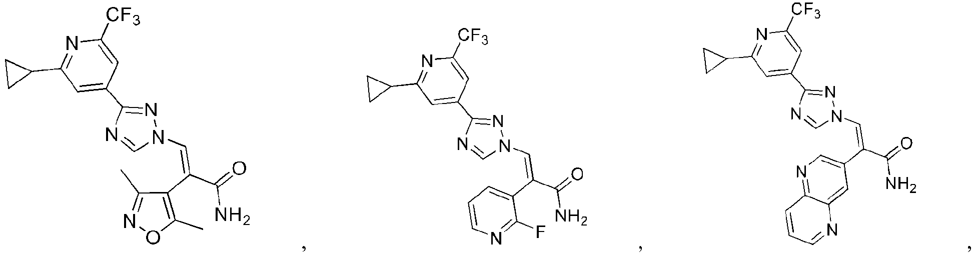 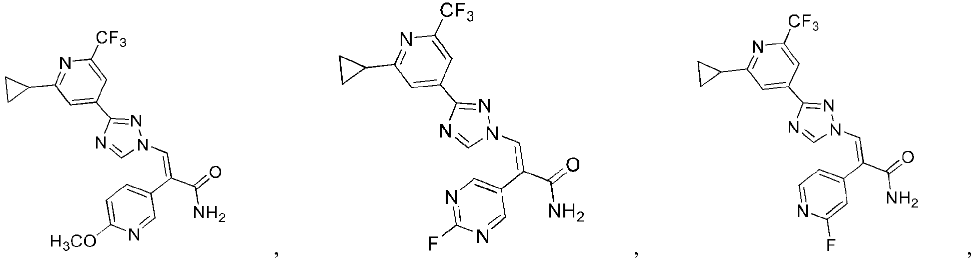 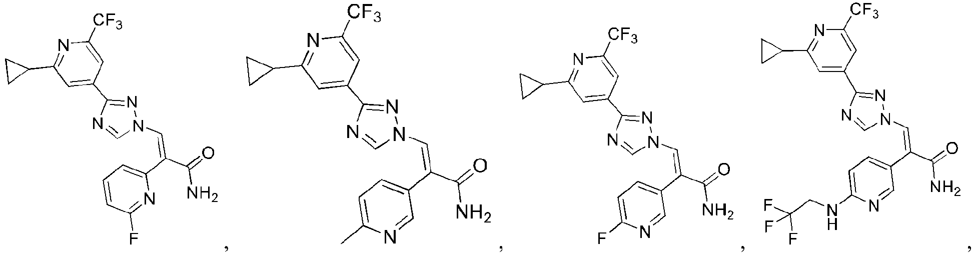 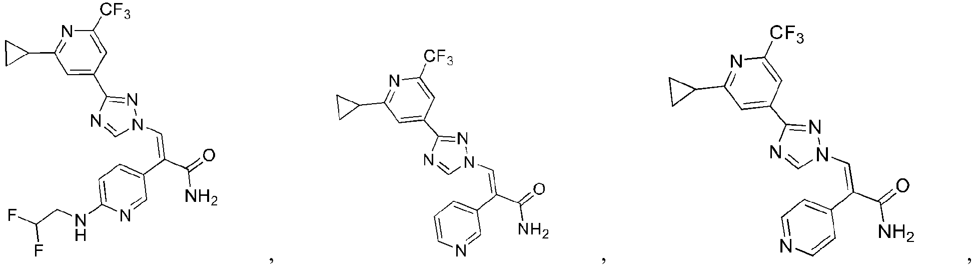 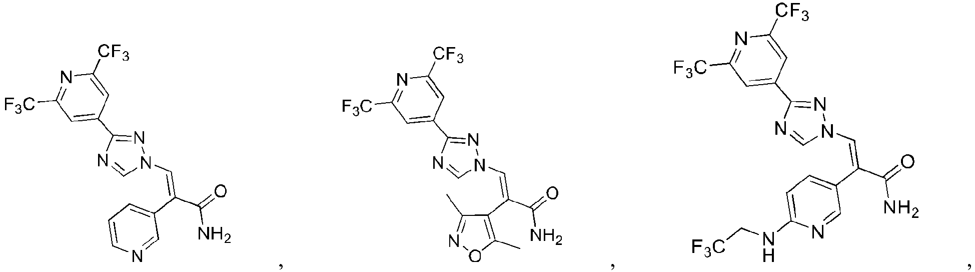 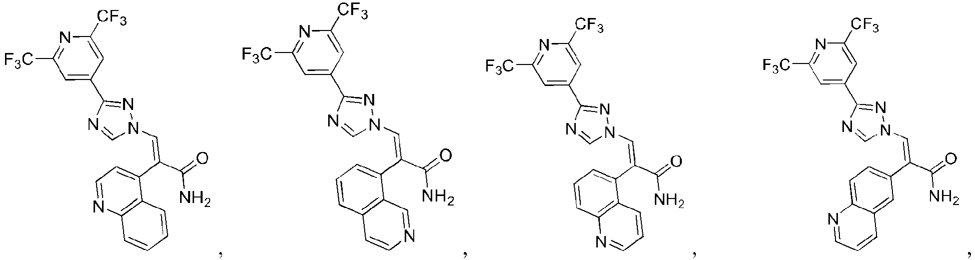 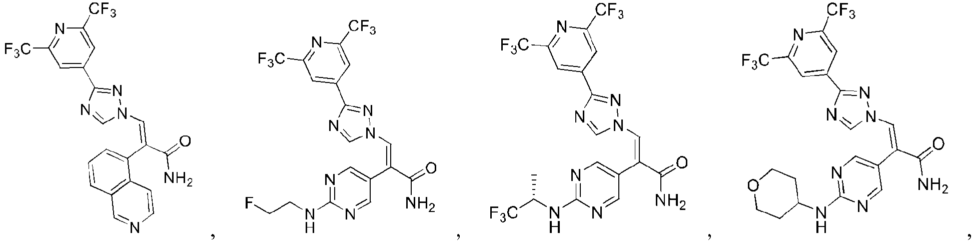 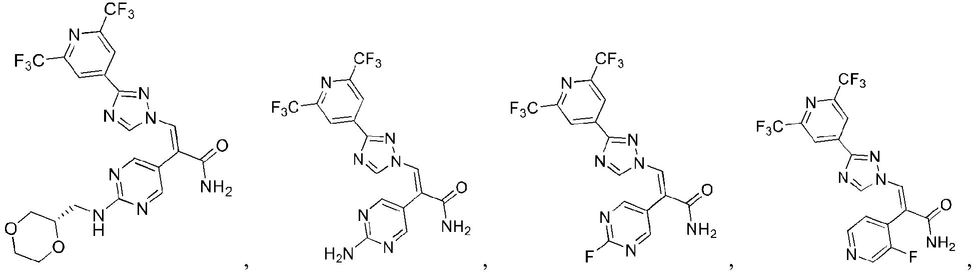 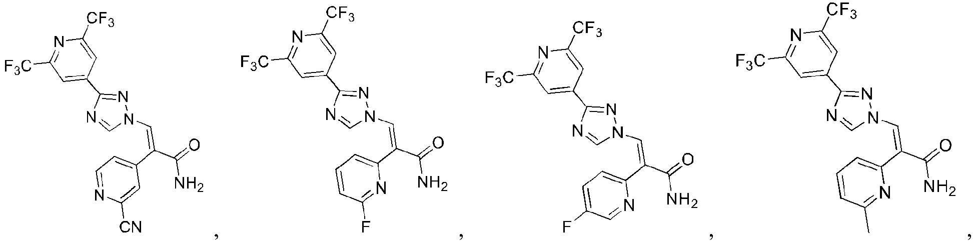 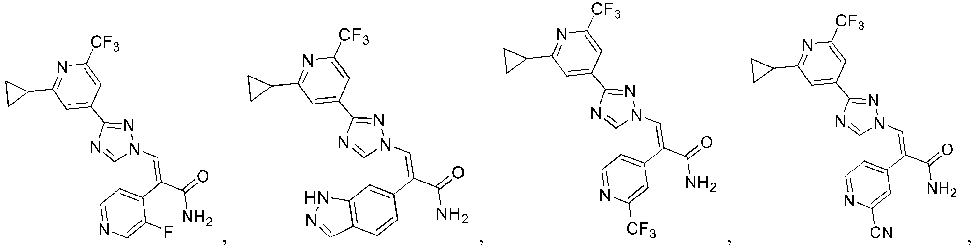 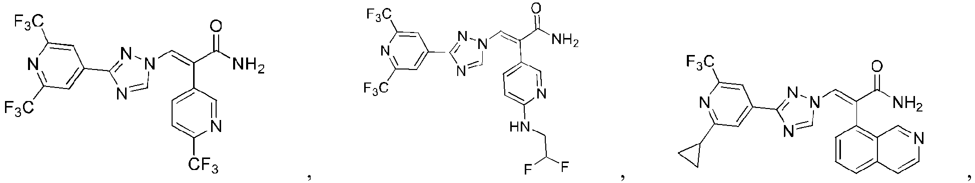 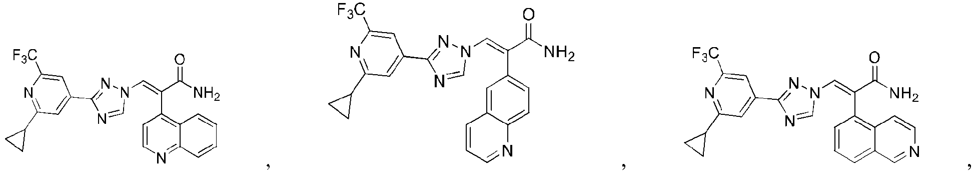 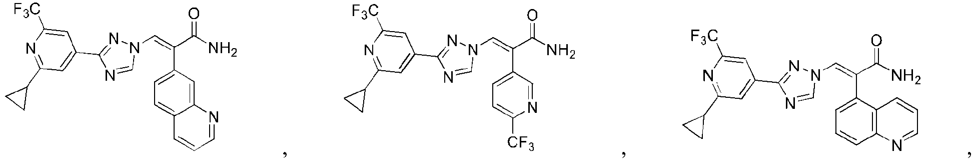 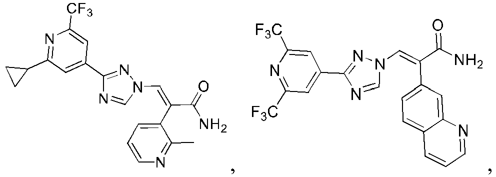 and .

In some embodiments, the compound of formula I is selected from the group consisting of compounds 2-A, 3-A, 11-A, 15-A, 21-A, 25-A, 38-A, and 59-A, as follows:

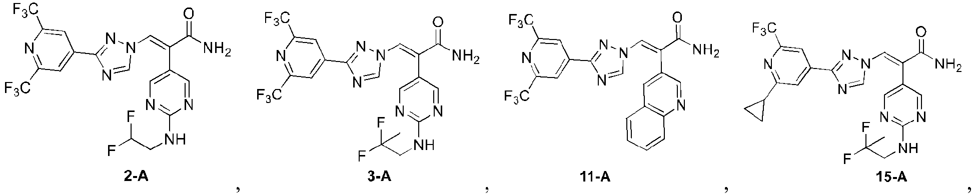

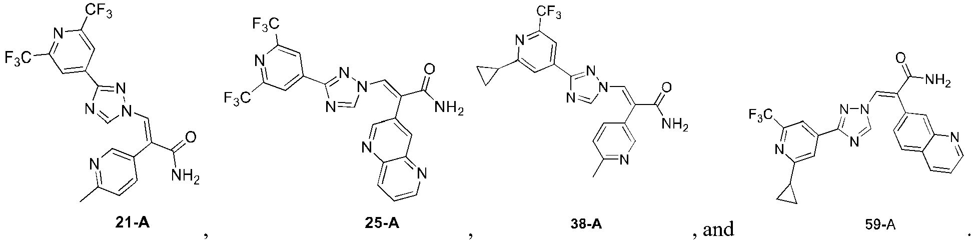

In some embodiments, the compound of formula I is selected from the group consisting of compounds 3-A, 15-A, 21-A, and 25-A, as follows:

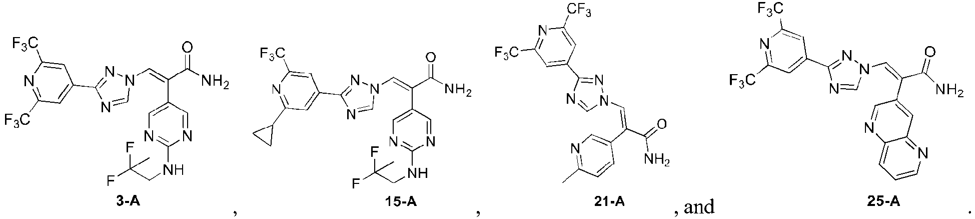

In some embodiments, the compound of formula I is selected from compound 2-A, as follows:

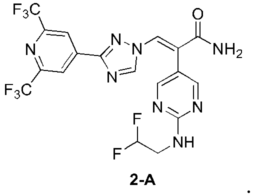

In some embodiments, the compound of formula I is selected from compound 3-A, as follows:

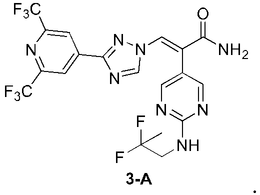

In some embodiments, the compound of formula I is selected from compound 11-A, as follows:

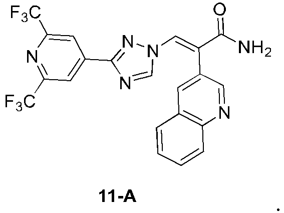

In some embodiments, the compound of formula I is selected from compound 15-A, as follows:

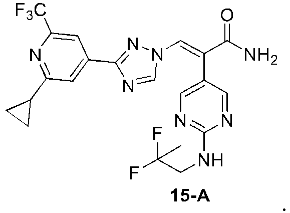

In some embodiments, the compound of formula I is selected from compound 21-A, as follows:

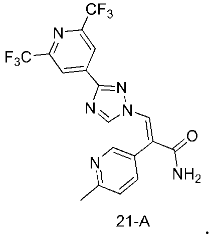

In some embodiments, the compound of formula I is selected from compound 25-A, as follows:

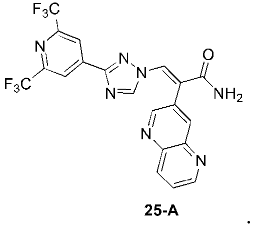

In some embodiments, the compound of formula I is selected from compound 38-A, as follows:

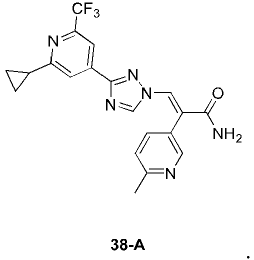

In some embodiments, the compound of formula I is selected from compound 59-A, as follows:

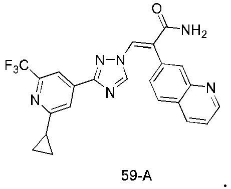

In another aspect, the present disclosure provides compound 21-A, 2-A, 3-A, 11-A, 15-A, 25-A, 38-A, or 59-A, a stereoisomer thereof, a pharmaceutically acceptable salt thereof, or a solvate thereof.

In another aspect, the present disclosure provides compound 21-A, 3-A, 15-A, or 25-A, a stereoisomer thereof, a pharmaceutically acceptable salt thereof, or a solvate thereof.

In another aspect, the present disclosure provides a crystalline form or an amorphous form of compound 21-A, 2-A, 3-A, 11-A, 15-A, 25-A, 38-A, or 59-A, a crystalline form or an amorphous form of a stereoisomer thereof, a crystalline form or an amorphous form of a pharmaceutically acceptable salt of compound 21-A, 2-A, 3-A, 11-A, 15-A, 25-A, 38-A, or 59-A, or a solvate of compound 21-A, 2-A, 3-A, 11-A, 15-A, 25-A, 38-A, or 59-A.

In another aspect, the present disclosure provides a crystalline form or an amorphous form of compound 21-A, 3-A, 15-A, or 25-A, a crystalline form or an amorphous form of a stereoisomer thereof, a crystalline form or an amorphous form of a pharmaceutically acceptable salt of compound 21-A, 3-A, 15-A, or 25-A, or a solvate of compound 21-A, 3-A, 15-A, or 25-A.

In another aspect, the present disclosure provides a crystalline form of compound 21-A, 2-A, 3-A, 11-A, 15-A, 25-A, 38-A, or 59-A, a crystalline form of a stereoisomer thereof, a crystalline form of a pharmaceutically acceptable salt of compound 21-A, 2-A, 3-A, 11-A, 15-A, 25-A, 38-A, or 59-A, or a solvate of compound 21-A, 2-A, 3-A, 11-A, 15-A, 25-A, 38-A, or 59-A.

In another aspect, the present disclosure provides a crystalline form of compound 21-A, 3-A, 15-A, or 25-A, a crystalline form of a stereoisomer thereof, a crystalline form of a pharmaceutically acceptable salt of compound 21-A, 3-A, 15-A, or 25-A, or a solvate of compound 21-A, 3-A, 15-A, or 25-A.

In another aspect, the present disclosure provides a crystalline form of compound 21-A, 2-A, 3-A, 11-A, 15-A, 25-A, 38-A, or 59-A, a pharmaceutically acceptable salt of compound 21-A, 2-A, 3-A, 11-A, 15-A, 25-A, 38-A, or 59-A, or a solvate of compound 21-A, 2-A, 3-A, 11-A, 15-A, 25-A, 38-A, or 59-A.

In another aspect, the present disclosure provides a crystalline form of compound 21-A, 3-A, 15-A, or 25-A, a pharmaceutically acceptable salt of compound 21-A, 3-A, 15-A, or 25-A, or a solvate of compound 21-A, 3-A, 15-A, or 25-A.

In another aspect, the present disclosure provides a crystalline form or an amorphous form of compound 21-A, 2-A, 3-A, 11-A, 15-A, 25-A, 38-A, or 59-A, a crystalline form or an amorphous form of a pharmaceutically acceptable salt of compound 21-A, 2-A, 3-A, 11-A, 15-A, 25-A, 38-A, or 59-A, or a solvate of compound 21-A, 2-A, 3-A, 11-A, 15-A, 25-A, 38-A, or 59-A.

In another aspect, the present disclosure provides a crystalline form or an amorphous form of compound 21-A, 3-A, 15-A, or 25-A, a crystalline form or an amorphous form of a pharmaceutically acceptable salt of compound 21-A, 3-A, 15-A, or 25-A, or a solvate of compound 21-A, 3-A, 15-A, or 25-A.

In another aspect, the present disclosure provides a crystalline form of compound 21-A, 2-A, 3-A, 11-A, 15-A, 25-A, 38-A, or 59-A, a crystalline form or an amorphous form of a pharmaceutically acceptable salt of compound 21-A, 2-A, 3-A, 11-A, 15-A, 25-A, 38-A, or 59-A, or a solvate of compound 21-A, 2-A, 3-A, 11-A, 15-A, 25-A, 38-A, or 59-A.

In another aspect, the present disclosure provides a crystalline form of compound 21-A, 3-A, 15-A, or 25-A, a crystalline form or an amorphous form of a pharmaceutically acceptable salt of compound 21-A, 3-A, 15-A, or 25-A, or a solvate of compound 21-A, 3-A, 15-A, or 25-A.

In some embodiments, the pharmaceutically acceptable salts of compounds 21-A, 2-A, 3-A, 11-A, 15-A, 25-A, 38-A, and 59-A are respectively selected from the group consisting of crystalline forms and amorphous forms of the pharmaceutically acceptable salts of compounds 21-A, 2-A, 3-A, 11-A, 15-A, 25-A, 38-A, and 59-A.

In some embodiments, the solvates of compounds 21-A, 2-A, 3-A, 11-A, 15-A, 25-A, 38-A, and 59-A are respectively selected from the group consisting of crystalline forms and amorphous forms of the solvates of compounds 21-A, 2-A, 3-A, 11-A, 15-A, 25-A, 38-A, and 59-A.

In some embodiments, the solvates of compounds 21-A, 3-A, 15-A, and 25-A are respectively selected from the group consisting of crystalline forms and amorphous forms of the solvates of compounds 21-A, 3-A, 15-A, and 25-A.

In some embodiments, the crystalline forms of compounds 21-A, 2-A, 3-A, 11-A, 15-A, 25-A, 38-A, and 59-A, the pharmaceutically acceptable salts of compounds 21-A, 2-A, 3-A, 11-A, 15-A, 25-A, 38-A, and 59-A, or the solvates of compounds 21-A, 2-A, 3-A, 11-A, 15-A, 25-A, 38-A, and 59-A are respectively selected from the group consisting of crystalline forms of compounds 21-A, 2-A, 3-A, 11-A, 15-A, 25-A, 38-A, and 59-A, crystalline forms or amorphous forms of the pharmaceutically acceptable salts of compound 21-A, 2-A, 3-A, 11-A, 15-A, 25-A, 38-A, and 59-A, or crystalline forms or amorphous forms of the solvates of compounds 21-A, 2-A, 3-A, 11-A, 15-A, 25-A, 38-A, and 59-A.

In some embodiments, the crystalline forms of compounds 21-A, 3-A, 15-A, and 25-A, the pharmaceutically acceptable salts of compounds 21-A, 3-A, 15-A, and 25-A, or the solvates of compounds 21-A, 3-A, 15-A, and 25-A are respectively selected from the group consisting of crystalline forms of compounds 21-A, 3-A, 15-A, and 25-A, crystalline forms or amorphous forms of the pharmaceutically acceptable salts of compounds 21-A, 3-A, 15-A, and 25-A, and crystalline forms or amorphous forms of the solvates of compounds 21-A, 3-A, 15-A, and 25-A.

In yet another aspect, the present application provides a crystalline form or an amorphous form of compound 21-A, 2-A, 3-A, 11-A, 15-A, 25-A, 38-A, or 59-A, or a solvate of compound 21-A, 2-A, 3-A, 11-A, 15-A, 25-A, or 38-A.

In yet another aspect, the present application provides a crystalline form or an amorphous form of compound 21-A, 3-A, 15-A, or 25-A, or a solvate of compound 21-A, 3-A, 15-A, or 25-A.

In yet another aspect, the present application provides a crystalline form of compound 21-A, 2-A, 3-A, 11-A, 15-A, 25-A, 38-A, or 59-A, or a solvate of compound 21-A, 2-A, 3-A, 11-A, 15-A, 25-A, or 38-A.

In yet another aspect, the present application provides a crystalline form of compound 21-A, 3-A, 15-A, or 25-A, or a solvate of compound 21-A, 3-A, 15-A, or 25-A.

In yet another aspect, the present application provides a crystalline form of compound 21-A, 2-A, 3-A, 11-A, 15-A, 25-A, 38-A, or 59-A, or a crystalline form or an amorphous form of a solvate of compound 21-A, 2-A, 3-A, 11-A, 15-A, 25-A, 38-A, or 59-A.

In yet another aspect, the present application provides a crystalline form of compound 21-A, 2-A, 3-A, 11-A, 15-A, 25-A, 38-A, or 59-A.

In yet another aspect, the present application provides a solvate of compound 21-A, 2-A, 3-A, 11-A, 15-A, 25-A, 38-A, or 59-A. In yet another aspect, the present application provides a crystalline form or an amorphous form of a solvate of compound 21-A, 2-A, 3-A, 11-A, 15-A, 25-A, 38-A, or 59-A.

In some embodiments, the solvate of compound 21-A, 2-A, 3-A, 11-A, 15-A, 25-A, 38-A, or 59-A has a solvent content of: about 0.5%-50% wt, about 0.5%-40% wt, about 0.5%-30% wt, about 0.5%-20% wt, about 1%-15% wt, about 1.5%-12% wt, about 2%-10% wt, or about 2%-7% wt. In some embodiments, the solvate of compound 21-A, 2-A, 3-A, 11-A, 15-A, 25-A, 38-A, or 59-A has a solvent content of: about 1% wt, about 2% wt, about 3% wt, about 4% wt, about 5% wt, about 6% wt, about 7% wt, about 8% wt, about 9% wt, about 10% wt, about 11% wt, about 12% wt, about 13% wt, about 14% wt, about 15% wt, about 16% wt, about 17% wt, about 18% wt, about 19% wt, or about 20% wt, or a range formed by any of the above values. In some embodiments, the solvate of compound 21-A, 2-A, 3-A, 11-A, 15-A, 25-A, 38-A, or 59-A has a solvent content of: about 2% wt, about 4% wt, about 6% wt, about 7% wt, about 8% wt, or about 10% wt, or a range formed by any of the above values. In some embodiments, the solvate of compound 21-A, 2-A, 3-A, 11-A, 15-A, 25-A, 38-A, or 59-A has a solvent content of: about 2% wt, about 4% wt, about 6% wt, about 7% wt, or about 10% wt, or a range formed by any of the above values. In some embodiments, the solvate of compound 21-A, 2-A, 3-A, 11-A, 15-A, 25-A, 38-A, or 59-A has a solvent content of: about 2% wt, about 4% wt, or about 7% wt, or a range formed by any of the above values. In some embodiments, the solvate of compound 21-A, 2-A, 3-A, 11-A, 15-A, 25-A, 38-A, or 59-A has a solvent content of: about 4% wt, about 6% wt, about 7% wt, about 8% wt, or about 10% wt, or a range formed by any of the above values. In some embodiments, the solvate of compound 21-A, 2-A, 3-A, 11-A, 15-A, 25-A, 38-A, or 59-A has a solvent content of: about 4% wt, about 6% wt, or about 10% wt, or a range formed by any of the above values.

In some embodiments, in the solvate of compound 21-A, 2-A, 3-A, 11-A, 15-A, 25-A, 38-A, or 59-A, the molar ratio of compound 21-A, 2-A, 3-A, 11-A, 15-A, 25-A, 38-A, or 59-A to the solvent is selected from the group consisting of about 1:0.1-50, about 1:0.1-30, about 1:0.1-20, about 1:0.1-15, about 1:0.1-10, about 1:0.15-10, about 1:0.2-10, about 1:0.2-8, about 1:0.2-8, about 1:0.2-6, about 1:0.2-5, about 1:0.2-4, about 1:0.2-3, about 1:0.2-2, about 1:0.2-1, and about 1:0.3-1. In some embodiments, in the solvate of compound 21-A, 2-A, 3-A, 11-A, 15-A, 25-A, 38-A, or 59-A, the molar ratio of compound 21-A, 2-A, 3-A, 11-A, 15-A, 25-A, 38-A, or 59-A to the solvent is about 1:0.2, about 1:0.3, about 1:0.4, about 1:0.5, about 1:1, about 1:2, about 1:3, about 1:4, about 1:5, about 1:6, about 1:7, about 1:8, about 1:9, or about 1:10, or a range formed by any of the above values. In some embodiments, in the solvate of compound 21-A, 2-A, 3-A, 11-A, 15-A, 25-A, 38-A, or 59-A, the molar ratio of compound 21-A, 2-A, 3-A, 11-A, 15-A, 25-A, 38-A, or 59-A to the solvent is about 1:0.5 or 1:1, or a range formed by any of the above values. In some embodiments, in the solvate of compound 21-A, 2-A, 3-A, 11-A, 15-A, 25-A, 38-A, or 59-A, the molar ratio of compound 21-A, 2-A, 3-A, 11-A, 15-A, 25-A, 38-A, or 59-A to the solvent is about 1:0.5. In some embodiments, in the solvate of compound 21-A, 2-A, 3-A, 11-A, 15-A, 25-A, 38-A, or 59-A, the molar ratio of compound 21-A, 2-A, 3-A, 11-A, 15-A, 25-A, 38-A, or 59-A to the solvent is about 1:1.

In some embodiments, the solvent in the solvate of compound 21-A, 2-A, 3-A, 11-A, 15-A, 25-A, 38-A, or 59-A is selected from one or more of the group consisting of water, hydrocarbons, alcohols, ethers, esters, amides, sulfoxides, ketones, and nitriles.

In some embodiments, the solvent in the solvate of compound 21-A, 2-A, 3-A, 11-A, 15-A, 25-A, 38-A, or 59-A is selected from one or more of the group consisting of water, hydrocarbons, alcohols, ethers, esters, amides, sulfoxides, and ketones.

In some embodiments, the solvent in the solvate of compound 21-A, 2-A, 3-A, 11-A, 15-A, 25-A, 38-A, or 59-A is selected from one or more of the group consisting of water, alcohols, ethers, esters, ketones, and nitriles.

In some embodiments, the solvent in the solvate of compound 21-A, 2-A, 3-A, 11-A, 15-A, 25-A, 38-A, or 59-A is selected from one or more of the group consisting of water, alcohols, ethers, esters, and ketones.

In some embodiments, the solvent in the solvate of compound 21-A, 2-A, 3-A, 11-A, 15-A, 25-A, 38-A, or 59-A is selected from one or more of the group consisting of water, C₅₋₁₀ hydrocarbons, C₁₋₁₀ alcohols, C₂₋₁₀ ethers, C₂₋₁₀ esters, C₂₋₁₀ amides, C₂₋₁₀ sulfoxides, C₂₋₁₀ ketones, and C₂₋₁₀ nitriles.

In some embodiments, the solvent in the solvate of compound 21-A, 2-A, 3-A, 11-A, 15-A, 25-A, 38-A, or 59-A is selected from one or more of the group consisting of water, C₅₋₁₀ hydrocarbons, C₁₋₁₀ alcohols, C₂₋₁₀ ethers, C₂₋₁₀ esters, C₂₋₁₀ amides, C₂₋₁₀ sulfoxides, and C₂₋₁₀ ketones.

In some embodiments, the solvent in the solvate of compound 21-A, 2-A, 3-A, 11-A, 15-A, 25-A, 38-A, or 59-A is selected from one or more of the group consisting of water, C₅₋₁₀ hydrocarbons, C₁₋₆ alcohols, C₂₋₆ ethers, C₂₋₆ esters, C₂₋₆ amides, C₂₋₆ sulfoxides, C₂₋₆ ketones, and C₂₋₆ nitriles.

In some embodiments, the solvent in the solvate of compound 21-A, 2-A, 3-A, 11-A, 15-A, 25-A, 38-A, or 59-A is selected from one or more of the group consisting of water, C₅₋₁₀ hydrocarbons, C₁₋₆ alcohols, C₂₋₆ ethers, C₂₋₆ esters, C₂₋₆ amides, C₂₋₆ sulfoxides, and C₂₋₆ ketones.

In some embodiments, the solvent in the solvate of compound 21-A, 2-A, 3-A, 11-A, 15-A, 25-A, 38-A, or 59-A is selected from one or more of the group consisting of water, C₁₋₁₀ alcohols, C₂₋₁₀ ethers, C₂₋₁₀ esters, C₂₋₁₀ ketones, and C₂₋₁₀ nitriles.

In some embodiments, the solvent in the solvate of compound 21-A, 2-A, 3-A, 11-A, 15-A, 25-A, 38-A, or 59-A is selected from one or more of the group consisting of water, C₁₋₁₀ alcohols, C₂₋₁₀ ethers, C₂₋₁₀ esters, and C₂₋₁₀ ketones.

In some embodiments, the solvent in the solvate of compound 21-A, 2-A, 3-A, 11-A, 15-A, 25-A, 38-A, or 59-A is selected from one or more of the group consisting of water, C₁₋₆ alcohols, C₂₋₆ ethers, C₂₋₆ esters, C₂₋₆ ketones, and C₂₋₆ nitriles.

In some embodiments, the solvent in the solvate of compound 21-A, 2-A, 3-A, 11-A, 15-A, 25-A, 38-A, or 59-A is selected from one or more of the group consisting of water, C₁₋₆ alcohols, C₂₋₆ ethers, C₂₋₆ esters, and C₂₋₆ ketones.

In some embodiments, the solvent in the solvate of compound 21-A, 2-A, 3-A, 11-A, 15-A, 25-A, 38-A, or 59-A is selected from one or more of the group consisting of water, C₁₋₃ alcohols, C₂₋₆ ethers, C₄₋₆ esters, C₃₋₄ ketones, and C₂₋₄ nitriles.

In some embodiments, the solvent in the solvate of compound 21-A, 2-A, 3-A, 11-A, 15-A, 25-A, 38-A, or 59-A is selected from one or more of the group consisting of water, C₁₋₃ alcohols, C₂₋₆ ethers, C₄₋₆ esters, and C₃₋₄ ketones.

In some embodiments, the solvent in the solvate of compound 21-A, 2-A, 3-A, 11-A, 15-A, 25-A, 38-A, or 59-A is selected from one or more of the group consisting of water, C₁₋₃ alcohols, C₄₋₅ esters, C₅₋₆ ethers, C₃ ketones, and C₂ nitriles.

In some embodiments, the solvent in the solvate of compound 21-A, 2-A, 3-A, 11-A, 15-A, 25-A, 38-A, or 59-A is selected from one or more of the group consisting of water, C₁₋₃ alcohols, C₄₋₅ esters, and C₃ ketones.

In some embodiments, the solvent in the solvate of compound 21-A, 2-A, 3-A, 11-A, 15-A, 25-A, 38-A, or 59-A is selected from one or more of the group consisting of water, C₁₋₆ alcohols, C₂₋₆ ethers, C₂₋₆ esters, C₃₋₄ ketones, and C₂₋₄ nitriles.

In some embodiments, the solvent in the solvate of compound 21-A, 2-A, 3-A, 11-A, 15-A, 25-A, 38-A, or 59-A is selected from one or more of the group consisting of water, C₁₋₆ alcohols, and C₂₋₆ esters.

In some embodiments, the solvent in the solvate of compound 21-A, 2-A, 3-A, 11-A, 15-A, 25-A, 38-A, or 59-A is selected from one or more of the group consisting of water, methanol, ethanol, isopropanol, 1,4-dioxane, tetrahydrofuran, ethyl acetate, isopropyl acetate, acetone, and acetonitrile. In some embodiments, the solvent in the solvate of compound 21-A, 2-A, 3-A, 11-A, 15-A, 25-A, 38-A, or 59-A is selected from one or more of the group consisting of water, methanol, ethanol, isopropanol, ethyl acetate, isopropyl acetate, and acetone.

In some embodiments, the solvent in the solvate of compound 21-A, 2-A, 3-A, 11-A, 15-A, 25-A, 38-A, or 59-A is selected from one or more of the group consisting of water, a mixture of water and ethyl acetate, methanol, a mixture of methanol and isopropyl acetate, a mixture of methanol and isopropyl acetate and water, ethanol, isopropanol, isopropyl acetate, acetone, 1,4-dioxane, tetrahydrofuran, and acetonitrile.

In some embodiments, the solvent in the solvate of compound 21-A, 2-A, 3-A, 11-A, 15-A, 25-A, 38-A, or 59-A is selected from one or more of the group consisting of water, a mixture of water and ethyl acetate, methanol, a mixture of methanol and isopropyl acetate, a mixture of methanol and isopropyl acetate and water, ethanol, isopropanol, isopropyl acetate, and acetone.

In some embodiments, the solvent in the solvate of compound 21-A, 2-A, 3-A, 11-A, 15-A, 25-A, 38-A, or 59-A is selected from one or more of the group consisting of water, a mixture of water and ethyl acetate, methanol, ethanol, isopropanol, isopropyl acetate, and acetone.

In some embodiments, the solvent in the solvate of compound 21-A, 2-A, 3-A, 11-A, 15-A, 25-A, 38-A, or 59-A is selected from one or more of the group consisting of water, methanol, ethanol, isopropanol, and ethyl acetate.

In some embodiments, the solvent in the solvate of compound 21-A, 2-A, 3-A, 11-A, 15-A, 25-A, 38-A, or 59-A comprises water, or a mixture of water and ethyl acetate. In some embodiments, the solvent in the solvate of compound 21-A, 2-A, 3-A, 11-A, 15-A, 25-A, 38-A, or 59-A comprises water. In some embodiments, the solvent in the solvate of compound 21-A, 2-A, 3-A, 11-A, 15-A, 25-A, 38-A, or 59-A comprises ethyl acetate. In some embodiments, the solvent in the solvate of compound 21-A, 2-A, 3-A, 11-A, 15-A, 25-A, 38-A, or 59-A comprises a mixture of water and ethyl acetate.

In some embodiments, the solvent in the solvate of compound 21-A comprises water, ethyl acetate, isopropyl acetate, methanol, isopropanol, or acetone.

In some embodiments, the solvent in the solvate of compound 3-A comprises acetonitrile, 1,4-dioxane, or tetrahydrofuran.

In some embodiments, the solvent in the solvate of compound 15-A comprises acetonitrile. In some embodiments, the solvent in the solvate of compound 21-A, 2-A, 3-A, 11-A, 15-A, 25-A, 38-A, or 59-A comprises one or more of methanol, ethanol, or isopropanol.

In some embodiments, the solvent in the solvate of compound 21-A, 2-A, 3-A, 11-A, 15-A, 25-A, 38-A, or 59-A comprises ethyl acetate or isopropyl acetate. In some embodiments, the solvent in the solvate of compound 21-A, 2-A, 3-A, 11-A, 15-A, 25-A, 38-A, or 59-A comprises ethyl acetate.

In some embodiments, the solvent in the solvate of compound 21-A, 2-A, 3-A, 11-A, 15-A, 25-A, 38-A, or 59-A comprises acetone.

In some embodiments, the solvent in the solvate of compound 21-A, 2-A, 3-A, 11-A, 15-A, 25-A, 38-A, or 59-A comprises tetrahydrofuran or 1,4-dioxane.

In some embodiments, the solvent in the solvate of compound 21-A, 2-A, 3-A, 11-A, 15-A, 25-A, 38-A, or 59-A comprises acetonitrile.

In yet another aspect, the present disclosure provides a crystal form of compound 3-A or a solvate of compound 3-A.

In yet another aspect, the present disclosure provides a crystal form of compound 3-A or a crystalline form or an amorphous form of a solvate of compound 3-A.

In yet another aspect, the present disclosure provides a crystal form of compound 3-A or a crystalline form of a solvate of compound 3-A.

In yet another aspect, the present disclosure provides a crystal form of compound 3-A.

In yet another aspect, the present disclosure provides a solvate of compound 3-A.

In yet another aspect, the present disclosure provides a crystalline form or an amorphous form of a solvate of compound 3-A.

In yet another aspect, the present disclosure provides a crystalline form of a solvate of compound 3-A.

In yet another aspect, the present disclosure provides a crystal form of compound 15-A or a solvate of compound 15-A.

In yet another aspect, the present disclosure provides a crystal form of compound 15-A or a crystalline form or an amorphous form of a solvate of compound 15-A.

In yet another aspect, the present disclosure provides a crystal form of compound 15-A or a crystalline form of a solvate of compound 15-A.

In yet another aspect, the present disclosure provides a crystal form of compound 15-A.

In yet another aspect, the present disclosure provides a solvate of compound 15-A.

In yet another aspect, the present disclosure provides a crystalline form or an amorphous form of a solvate of compound 15-A.

In yet another aspect, the present disclosure provides a crystalline form of a solvate of compound 15-A.

In yet another aspect, the present disclosure provides a crystal form of compound 21-A or a solvate of compound 21-A.

In yet another aspect, the present disclosure provides a crystal form of compound 21-A or a crystalline form or an amorphous form of a solvate of compound 21-A.

In yet another aspect, the present disclosure provides a crystal form of compound 21-A or a crystalline form of a solvate of compound 21-A.

In yet another aspect, the present disclosure provides a crystal form of compound 21-A.

In yet another aspect, the present disclosure provides a solvate of compound 21-A.

In yet another aspect, the present disclosure provides a crystalline form or an amorphous form of a solvate of compound 21-A.

In yet another aspect, the present disclosure provides a crystalline form of a solvate of compound 21-A.

In yet another aspect, the present disclosure provides a crystal form of compound 25-A.

In some embodiments, the solvent is selected from the group consisting of water and C₁₋₁₀ alcohols.

In some embodiments, the solvent is selected from the group consisting of water and C₁₋₆ alcohols.

In some embodiments, the solvent is selected from the group consisting of water and C₁₋₃ alcohols.

In some embodiments, the solvent is selected from one or more of the group consisting of water, methanol, ethanol, and isopropanol.

In some embodiments, the solvent is selected from C₁₋₆ alcohols.

In some embodiments, the solvent is selected from C₁₋₃ alcohols.

In some embodiments, the solvent is selected from water.

In some embodiments, the solvent is selected from one or more of the group consisting of methanol, ethanol, and isopropanol.

The present disclosure provides a crystal form A of compound 21-A, in an X-ray powder diffraction (XRPD) pattern using Cu Kα radiation, the crystal form A of compound 21-A has diffraction peaks at 2θ angles selected from about 6.44±0.20°, about 8.19±0.20°, and about 12.84±0.20°. In some embodiments, the above crystal form A has diffraction peaks at about 6.44±0.20°, about 8.19±0.20°, about 12.84±0.20°, about 15.49±0.20°, or about 21.82±0.20°. In some embodiments, the above crystal form A has diffraction peaks at about 6.44±0.20°, about 8.19±0.20°, about 12.84±0.20°, about 15.49±0.20°, about 18.20±0.20°, about 21.82±0.20°, or about 25.86±0.20°. In some embodiments, the above crystal form A has diffraction peaks at about 6.44±0.20°, about 8.19±0.20°, about 8.65±0.20°, about 12.84±0.20°, about 15.49±0.20°, about 18.20±0.20°, about 21.82±0.20°, about 22.75±0.20°, or about 25.86±0.20°. In some embodiments, the above crystal form A has diffraction peaks at about 6.44±0.20°, about 8.19±0.20°, about 8.65±0.20°, about 11.49±0.20°, about 12.84±0.20°, about 15.49±0.20°, about 16.84±0.20°, about 18.20±0.20°, about 21.08±0.20°, about 21.82±0.20°, about 22.75±0.20°, or about 25.86±0.20°. In some embodiments, the above crystal form A has diffraction peaks at about 6.44±0.20°, about 8.19±0.20°, about 8.65±0.20°, about 11.49±0.20°, about 12.84±0.20°, about 15.49±0.20°, about 16.84±0.20°, about 18.20±0.20°, about 21.08±0.20°, about 21.82±0.20°, about 22.75±0.20°, about 23.05±0.20°, or about 25.86±0.20°.

In some embodiments, the above crystal form A has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 6.44+0.20°, about 8.19+0.20°, about 8.65+0.20°, about 1.49±0.20°, about 12.84+0.20°, about 15.49+0.20°, about 16.84+0.20°, about 18.20+0.20°, about 21.08+0.20°, about 21.82+0.20°, about 22.75+0.20°, about 23.05+0.20°, and about 25.86+0.20°.

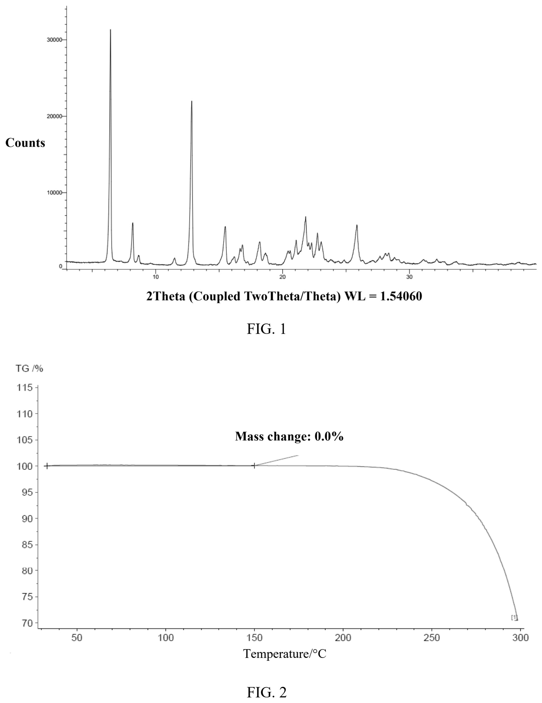

In some embodiments of the present disclosure, the above crystal form A has an XRPD pattern as shown in FIG. 1.

In some embodiments of the present disclosure, the above crystal form A has characteristic diffraction peaks in an XRPD pattern at the following 2θ angles, as shown in Table 1.

**Table 1: XRPD pattern data for crystal form A**

| No. | 2θ(±0.20°) | Relative intensity (%) | No. | 2θ(±0.20°) | Relative intensity (%) |
|---|---|---|---|---|---|
| 1 | 6.44 | 100.00% | 11 | 20.51 | 4.70% |
| 2 | 8.19 | 17.20% | 12 | 21.08 | 10.00% |
| 3 | 8.65 | 3.50% | 13 | 21.82 | 19.80% |
| 4 | 11.49 | 3.00% | 14 | 22.29 | 8.60% |
| 5 | 12.84 | 70.40% | 15 | 22.75 | 12.70% |
| 6 | 15.49 | 16.30% | 16 | 23.05 | 9.00% |
| 7 | 16.20 | 3.30% | 17 | 25.86 | 16.30% |
| 8 | 16.84 | 8.40% | 18 | 28.12 | 4.10% |
| 9 | 18.20 | 9.70% | 19 | 28.36 | 4.30% |
| 10 | 18.65 | 5.00% | | | |

In some embodiments of the present disclosure, the above crystal form A shows (almost) no weight loss change in a thermogravimetric analysis (TGA) curve upon heating to 150 °C.

In some embodiments of the present disclosure, the above crystal form A has a TGA profile as shown in FIG. 2.

In another aspect, the present disclosure provides a preparation method for the above crystal form A, which includes: 1) mixing compound 21-A with isopropyl acetate; and 2) separating a solid to give the crystal form A.

In the preparation method for the above crystal form A, step 1) further includes after mixing, heating the mixture under reflux (e.g., 60-100 °C or 90 °C). Optionally, further, after the heating under refluxing described above, gradient cooling is performed. The gradient cooling includes cooling from 60-100 °C or 90 °C to 0-30 °C or 0-10 °C.

In some embodiments, in the preparation method for the above crystal form A, the solid obtained in step 2) is dried (optionally at a temperature of 50-80 °C or 60 °C).

In some embodiments, the preparation method for the crystal form A includes: 1) adding compound 21-A to isopropyl acetate, heating the mixture under reflux to dissolve completely (optionally at a temperature of 60-100 °C or 90 °C), gradiently cooling the mixture to (0-30 °C or 0-10 °C) and stirring the mixture; and 2) separating a solid, and drying the obtained solid (optionally at a temperature of 50-80 °C or 60 °C) to give the crystal form A.

In some embodiments, in the preparation method for the crystal form A, the mass-to-volume ratio of compound 21-A to isopropyl acetate in step 1) is selected from the group consisting of 1 g: 1-50 mL, 1 g:5-40 mL, 1 g:5-30 mL, 1 g:5-25 mL, 1 g:5-20 mL, 1 g:5-15 mL, 1 g:5-10 mL, and 1 g:8 mL.

In yet another aspect, the present disclosure provides a hydrate of compound 21-A. In yet another aspect, the present disclosure provides a crystalline form or an amorphous form of a hydrate of compound 21-A. In yet another aspect, the present disclosure provides a crystalline form of a hydrate of compound 21-A.

The present disclosure provides a crystal form B of compound 21-A, in an X-ray powder diffraction (XRPD) pattern using Cu Kα radiation, the crystal form B of compound 21-A has diffraction peaks at 2θ angles selected from about 7.15±0.20°, about 9.71±0.20°, and about 21.78±0.20°. In some embodiments, the above crystal form B has diffraction peaks at about 7.15±0.20°, about 9.71±0.20°, about 14.35±0.20°, about 21.78±0.20°, or about 27.99±0.20°. In some embodiments, the above crystal form B has diffraction peaks at about 7.15±0.20°, about 9.71±0.20°, about 14.35±0.20°, about 18.73±0.20°, about 21.78±0.20°, about 25.09±0.20°, or about 27.99±0.20°. In some embodiments, the above crystal form B has diffraction peaks at about 7.15±0.20°, about 9.71±0.20°, about 14.35±0.20°, about 16.55±0.20°, about 18.73±0.20°, about 21.78±0.20°, about 22.93±0.20°, about 25.09±0.20°, or about 27.99±0.20°. In some embodiments, the above crystal form B has diffraction peaks at about 7.15±0.20°, about 9.71±0.20°, about 10.05±0.20°, about 14.35±0.20°, about 16.55±0.20°, about 17.32±0.20°, about 18.73±0.20°, about 21.78±0.20°, about 22.93±0.20°, about 25.09±0.20°, or about 27.99±0.20°. In some embodiments, the above crystal form B has diffraction peaks at about 7.15+0.20°, about 9.71±0.20°, about 10.05+0.20°, about 14.35+0.20°, about 16.55+0.20°, about 17.32+0.20°, about 18.73+0.20°, about 19.74+0.20°, about 21.78+0.20°, about 22.93+0.20°, about 25.09+0.20°, about 27.99+0.20°, or about 28.94+0.20°. In some embodiments, the above crystal form B has diffraction peaks at about 7.15+0.20°, about 9.71+0.20°, about 10.05+0.20°, about 14.35+0.20°, about 16.07+0.20°, about 16.55+0.20°, about 17.32+0.20°, about 18.73±0.20°, about 19.74±0.20°, about 21.78±0.20°, about 22.51±0.20°, about 22.93±0.20°, about 25.09±0.20°, about 27.99±0.20°, or about 28.94±0.20°.

In some embodiments, the above crystal form B has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 7.15±0.20°, about 9.71±0.20°, about 10.05±0.20°, about 14.35±0.20°, about 16.07±0.20°, about 16.55±0.20°, about 17.32±0.20°, about 18.73±0.20°, about 19.74±0.20°, about 21.78±0.20°, about 22.51±0.20°, about 22.93±0.20°, about 25.09±0.20°, about 27.99±0.20°, and about 28.94±0.20°.

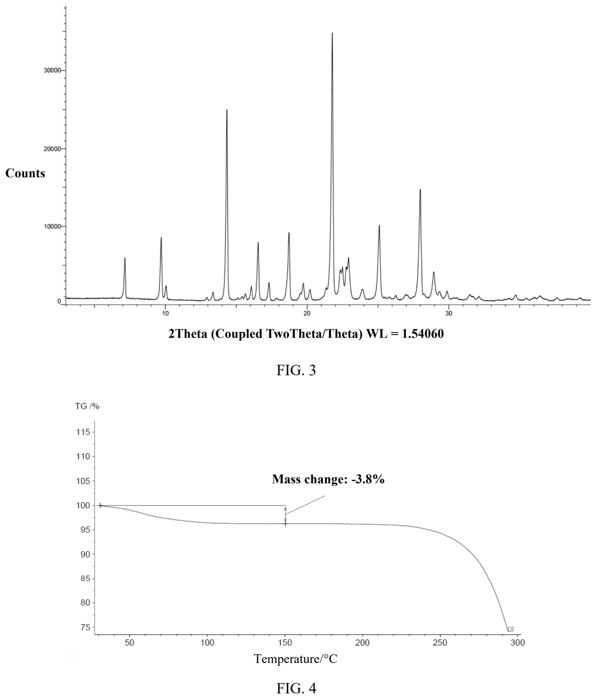

In some embodiments, the above crystal form B has an XRPD pattern as shown in FIG. 3.

In some embodiments, the above crystal form B has characteristic diffraction peaks in an XRPD pattern at the following 2θ angles, as shown in Table 2.

**Table 2: XRPD pattern data for crystal form B**

| No. | 2θ(±0.20°) | Relative intensity (%) | No. | 2θ(±0.20°) | Relative intensity (%) |
|---|---|---|---|---|---|
| 1 | 7.15 | 15.00% | 11 | 20.21 | 4.00% |
| 2 | 9.71 | 23.20% | 12 | 21.78 | 100.00% |
| 3 | 10.05 | 5.20% | 13 | 22.51 | 12.00% |
| 4 | 13.37 | 3.00% | 14 | 22.93 | 15.50% |
| 5 | 14.35 | 71.50% | 15 | 23.89 | 3.60% |
| 6 | 16.07 | 4.90% | 16 | 25.09 | 27.60% |
| 7 | 16.55 | 21.60% | 17 | 27.99 | 41.10% |
| 8 | 17.32 | 6.60% | 18 | 28.94 | 10.20% |
| 9 | 18.73 | 25.20% | 19 | 29.87 | 3.10% |
| 10 | 19.74 | 6.30% | | | |

In some embodiments, the above crystal form B shows a weight loss of about 3.8% in a thermogravimetric analysis (TGA) curve upon heating to 150 °C.

In some embodiments of the present disclosure, the above crystal form B has a TGA profile as shown in FIG. 4.

In some embodiments, the above crystal form B is a hydrate or an anhydrous crystal form of compound 21-A. In some embodiments, the above crystal form B is a crystal form of a hydrate of compound 21-A.

In some embodiments of the present disclosure, in the above crystal form B, the ratio of the number of compound 21-A molecules to the number of water molecules (i.e., the molar ratio) is selected from the group consisting of about 1:0.1-10, about 1:0.5-10, about 1:0.5-8, about 1:0.5-7, about 1:0.5-6, about 1:0.5-5, about 1:0.5-4, about 1:0.5-3, about 1:0.5-2, and about 1:0.5-1. In some embodiments of the present disclosure, in the above crystal form B, the ratio of the number of compound 21-A molecules to the number of water molecules (i.e., the molar ratio) is selected from the group consisting of about 1:0.5, about 1:1, about 1:2, about 1:3, and about 1:4, or a range formed by any of the above values. In some embodiments of the present disclosure, in the above crystal form B, the ratio of the number of molecules of the compound of formula I to the number of water molecules (i.e., the molar ratio) is selected from about 1:1.

In some embodiments, the above crystal form B is a crystal form of a hydrate of compound 21-A, wherein the water content is about 0.5%-20% wt, about 1%-15% wt, about 1%-12% wt, about 1%-10% wt, about 1%-8% wt, about 1%-6% wt, about 1%-4% wt, about 2%-4% wt, or about 3%-4% wt. In some embodiments, the above crystal form B is a crystal form of a hydrate of compound 21-A, wherein the water content is about 1% wt, about 2% wt, about 3% wt, about 4% wt, about 5% wt, about 6% wt, about 7% wt, about 8% wt, about 9% wt, about 10% wt, about 11% wt, about 12% wt, about 13% wt, about 14% wt, or about 15% wt, or a range formed by any of the above values. In some embodiments, the above crystal form B is a crystal form of a hydrate of compound 21-A, wherein the water content is about 4 wt%.

In another aspect, the present disclosure provides a preparation method for the above crystal form B, which includes: mixing compound 21-A with isopropyl acetate and water to give the crystal form B.

In some embodiments, the preparation method for the crystal form B includes: 1) mixing compound 21-A with isopropyl acetate and water; and separating a solid; and 2) adding water to the obtained solid, and separating to give the crystal form B.

In some embodiments, the preparation method for the crystal form B includes: 1) mixing compound 21-A with isopropyl acetate and water, heating the mixture (optionally at a heating temperature of 70-100 °C or 90 °C) to dissolve completely, cooling, and separating a solid; and 2) optionally, dissolving the obtained solid in water, heating (40-100 °C, 40-70 °C or 50 °C) and stirring, and further optionally, after heating and stirring, cooling and separating a solid.

In some embodiments, in the preparation method for the crystal form B, the cooling in step 1) is gradient cooling, and optionally cooling from 70-100 °C or 90 °C to 0-30 °C or 0-10 °C.

In some embodiments, in the preparation method for the crystal form B, the cooling in step 2) is gradient cooling, and optionally cooling from 40-100 °C, 40-70 °C, or 50 °C to 10-30 °C or 20-30 °C. In some embodiments, in the preparation method for the crystal form B, the solid obtained in step 2) is dried.

In some embodiments, the preparation method for the crystal form B includes: 1) adding compound 21-A to a mixed solvent of isopropyl acetate and water in a ratio of 100:1 (v:v), heating the mixture under reflux to dissolve completely (optionally at a temperature of 70-100 °C or 90 °C), gradiently cooling the mixture to (0-30 °C or 0-10 °C) and stirring the mixture, and separating a solid; 2) further, adding the obtained solid to water (the mass-to-volume ratio of the solid to the water is 1 g:5-50 mL, 1 g:5-30 mL, 1 g:5-20 mL, 1 g:5-10 mL, or 1 g:10 mL), stirring the mixture (at a temperature of 40-100 °C, 40-70 °C, or 50 °C), cooling the mixture (to 10-30 °C or 20-30 °C), stirring the mixture, separating a solid, and drying the obtained solid (optionally at a temperature of 30-50 °C or 40 °C) to give the crystal form B.

In some embodiments, in the preparation method for the crystal form B, the mass-to-volume ratio of compound 21-A to isopropyl acetate in step 1) is selected from the group consisting of 1 g:10-50 mL, 1 g:10-40 mL, 1 g:10-30 mL, 1 g:15-25 mL, and 1 g:20 mL.

In some embodiments, in the preparation method for the crystal form B, the mass-to-volume ratio of compound 21-A to water in step 2) is selected from the group consisting of 1 g:5-50 mL, 1 g:5-40 mL, 1 g:5-30 mL, 1 g:5-20 mL, 1 g:5-15 mL, 1 g:8-12 mL, and 1 g:10 mL.

The present disclosure provides a crystal form C of compound 21-A, in an X-ray powder diffraction (XRPD) pattern using Cu Kα radiation, the crystal form C of compound 21-A has diffraction peaks at 2θ angles selected from about 9.51±0.20°, about 18.91±0.20°, and about 19.97±0.20°. In some embodiments, the above crystal form C has diffraction peaks at about 9.51±0.20°, about 13.46±0.20°, about 18.91±0.20°, about 19.97±0.20°, or about 20.55±0.20°. In some embodiments, the above crystal form C has diffraction peaks at about 9.51±0.20°, about 13.46±0.20°, about 15.30±0.20°, about 18.91±0.20°, about 19.97±0.20°, about 20.55±0.20°, or about 22.09±0.20°. In some embodiments, the above crystal form C has diffraction peaks at about 9.16±0.20°, about 9.51±0.20°, about 13.46±0.20°, about 15.30±0.20°, about 17.67±0.20°, about 18.91±0.20°, about 19.97±0.20°, about 20.55±0.20°, or about 22.09±0.20°. In some embodiments, the above crystal form C has diffraction peaks at about 9.16±0.20°, about 9.51±0.20°, about 13.46±0.20°, about 15.30±0.20°, about 17.20±0.20°, about 17.67±0.20°, about 18.91±0.20°, about 19.16±0.20°, about 19.97±0.20°, about 20.55±0.20°, about 22.09±0.20°, or about 28.35±0.20°.

In some embodiments, the above crystal form C has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 9.16±0.20°, about 9.51±0.20°, about 13.46±0.20°, about 15.30±0.20°, about 17.20±0.20°, about 17.67±0.20°, about 18.91±0.20°, about 19.16±0.20°, about 19.97±0.20°, about 20.55±0.20°, about 22.09±0.20°, and about 28.35±0.20°.

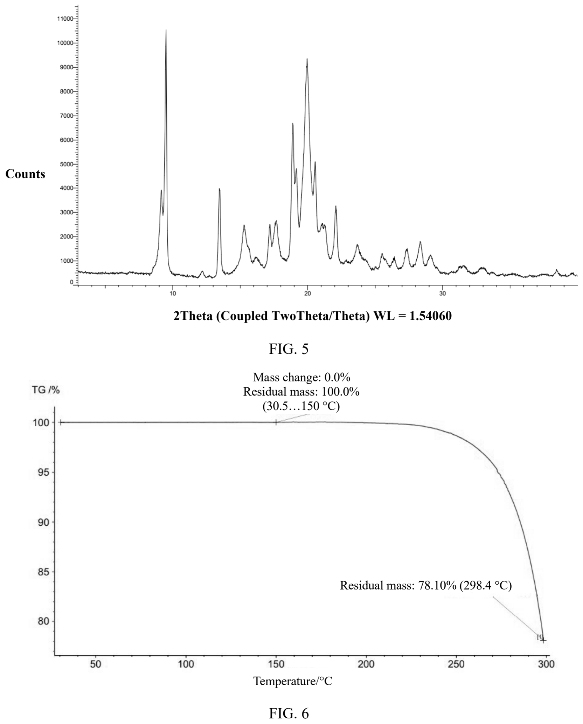

In some embodiments of the present disclosure, the above crystal form C has an XRPD pattern as shown in FIG. 5.

In some embodiments of the present disclosure, the above crystal form C has characteristic diffraction peaks in an XRPD pattern at the following 2θ angles, as shown in Table 3.

**Table 3: XRPD pattern data for crystal form C**

| No. | 2θ(±0.20°) | Relative intensity (%) | No. | 2θ(±0.20°) | Relative intensity (%) |
|---|---|---|---|---|---|
| 1 | 9.16 | 34.5% | 10 | 19.97 | 84.2% |
| 2 | 9.51 | 100.0% | 11 | 20.55 | 42.1% |
| 3 | 13.46 | 35.5% | 12 | 21.08 | 16.7% |
| 4 | 15.30 | 18.7% | 13 | 21.23 | 16.1% |
| 5 | 16.19 | 4.8% | 14 | 22.09 | 24.1% |
| 6 | 17.20 | 17.5% | 15 | 23.67 | 9.3% |
| 7 | 17.67 | 18.5% | 16 | 25.52 | 6.5% |
| 8 | 18.91 | 58.0% | 17 | 27.36 | 8.3% |
| 9 | 19.16 | 39.5% | 18 | 28.35 | 11.8% |

In some embodiments of the present disclosure, the above crystal form C shows almost no weight loss in a thermogravimetric analysis (TGA) curve upon heating to 150 °C.

In some embodiments of the present disclosure, the above crystal form C has a TGA profile as shown in FIG. 6.

In another aspect, the present disclosure provides a preparation method for the above crystal form C, which includes: 1) mixing compound 21-A with acetone; and 2) separating a solid to give the crystal form C.

In some embodiments, in the preparation method for the above crystal form C, compound 21-A is mixed with acetone, and the mixture is heated and/or stirred to dissolve completely. In some embodiments, in the preparation method for the crystal form C, the solid obtained in step 2) is dried (optionally at a temperature of 30-50 °C or 40 °C).

In some embodiments of the present disclosure, in the preparation method for the above crystal form C, the mass-to-volume ratio of compound 21-A to acetone is selected from the group consisting of 1 g:5-50 mL, 1 g:10-40 mL, 1 g:10-30 mL, 1 g:15-25 mL, 1 g:8-12 mL, and 1 g:10 mL.

In some embodiments, in the preparation method for the above crystal form C, compound 21-A is mixed with acetone, and optionally, the mixture is completely dissolved at 20-30 °C, or completely dissolved by heating to 40-70 °C, or completely dissolved by heating to 40-60 °C, or completely dissolved by heating to 45-50 °C, or completely dissolved by heating to 50 °C.

The present disclosure provides a crystal form D of compound 21-A, in an X-ray powder diffraction (XRPD) pattern using Cu Kα radiation, the crystal form D of compound 21-A has diffraction peaks at 2θ angles selected from about 7.87±0.20° and about 23.01±0.20°. In some embodiments, the above crystal form D has diffraction peaks at about 7.87±0.20°, about 8.38±0.20°, about 22.39±0.20°, or about 23.01±0.20°. In some embodiments, the above crystal form D has diffraction peaks at about 7.87+0.20°, about 8.38+0.20°, about 11.50+0.20°, about 22.39+0.20°, about 23.01+0.20°, or about 24.73+0.20°. In some embodiments, the above crystal form D has diffraction peaks at about 7.87+0.20°, about 8.38+0.20°, about 11.50+0.20°, about 12.72+0.20°, about 14.81±0.20°, about 18.23+0.20°, about 22.39+0.20°, about 23.01+0.20°, or about 24.73+0.20°. In some embodiments, the above crystal form D has diffraction peaks at about 7.87+0.20°, about 8.38±0.20°, about 11.50±0.20°, about 12.72±0.20°, about 14.81±0.20°, about 18.23±0.20°, about 19.35±0.20°, about 20.01±0.20°, about 22.39±0.20°, about 23.01±0.20°, or about 24.73±0.20°. In some embodiments, the above crystal form D has diffraction peaks at about 7.87±0.20°, about 8.38±0.20°, about 11.06±0.20°, about 11.50±0.20°, about 12.72±0.20°, about 14.81±0.20°, about 18.23±0.20°, about 19.35±0.20°, about 20.01±0.20°, about 21.64±0.20°, about 22.39±0.20°, about 23.01±0.20°, about 23.77±0.20°, or about 24.73±0.20°.

In some embodiments, the above crystal form D has at least 3, at least 4, at least 5, at least 6, at least 7, at least 8, at least 9, at least 10, or more characteristic diffraction peaks in an X-ray powder diffraction (XRPD) pattern using Cu Kα radiation at 20 values selected from the group consisting of about 7.87±0.20°, about 8.38±0.20°, about 11.06±0.20°, about 11.50±0.20°, about 12.72±0.20°, about 14.81±0.20°, about 18.23±0.20°, about 19.35±0.20°, about 20.01±0.20°, about 21.64±0.20°, about 22.39±0.20°, about 23.01±0.20°, about 23.77±0.20°, and about 24.73±0.20°.

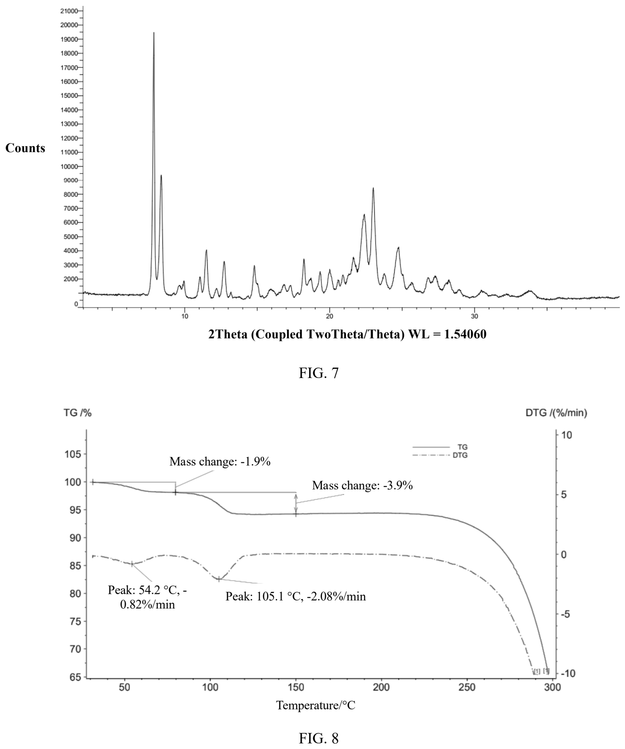

In some embodiments of the present disclosure, the above crystal form D has an XRPD pattern as shown in FIG. 7.

In some embodiments of the present disclosure, the above crystal form D has characteristic diffraction peaks in an XRPD pattern at the following 2θ angles, as shown in Table 4.

**Table 4: XRPD pattern data for crystal form D**

| No. | 2θ (±0.20°) | Relative intensity (%) | No. | 2θ (±0.20°) | Relative intensity (%) |
|---|---|---|---|---|---|
| 1 | 7.87 | 100.00% | 10 | 20.61 | 4.40% |
| 2 | 8.38 | 46.00% | 11 | 20.91 | 6.30% |
| 3 | 9.95 | 6.20% | 12 | 21.64 | 12.90% |
| 4 | 11.06 | 8.00% | 13 | 22.39 | 29.10% |
| 5 | 11.50 | 18.20% | 14 | 23.01 | 39.10% |
| 6 | 12.72 | 14.00% | 15 | 23.77 | 6.50% |
| 7 | 14.81 | 12.40% | 16 | 24.73 | 16.50% |
| 8 | 18.23 | 13.80% | 17 | 25.67 | 3.50% |
| 9 | 19.35 | 8.40% | | | |

In some embodiments of the present disclosure, the above crystal form D shows a weight loss of 1.9% in a thermogravimetric analysis (TGA) curve upon heating to 80 °C and/or a weight loss of 3.9% in the TGA curve upon heating to 150 °C.

In some embodiments of the present disclosure, the above crystal form D has a TGA profile as shown in FIG. 8.

In some embodiments of the present disclosure, the above crystal form D is a crystal form of an isopropanol solvate of compound 21-A.

In some embodiments of the present disclosure, in the above crystal form D, the ratio of the number of compound 21-A molecules to the number of isopropanol molecules (molar ratio) is selected from the group consisting of about 1:0.2-5, about 1:0.2-4, about 1:0.2-3, about 1:0.2-2, and about 1:0.2-1. In some embodiments of the present disclosure, in the above crystal form D, the ratio of the number of compound 21-A molecules to the number of isopropanol molecules (molar ratio) is selected from the group consisting of about 1:0.3, about 1:0.5, about 1:1, about 1:2, and about 1:3, or a range formed by any of the above values. In some embodiments of the present disclosure, in the above crystal form D, the ratio of the number of compound 21-A molecules to the number of isopropanol molecules (molar ratio) is selected from the group consisting of about 1:0.5 and about 1:1.

In some embodiments, the above crystal form D has an isopropanol content of about 1%-20% wt, about 1%-15% wt, about 1%-12% wt, about 1%-10% wt, about 1%-8% wt, about 1%-6% wt, or about 3%-9% wt. In some embodiments, the above crystal form D has an isopropanol content of about 1% wt, about 2% wt, about 3% wt, about 4% wt, about 5% wt, about 6% wt, about 7% wt, about 8% wt, about 9% wt, about 10% wt, about 11% wt, about 12% wt, about 13% wt, about 14% wt, or about 15% wt, or a range formed by any of the above values. In some embodiments, the above crystal form D has an isopropanol content of about 4% wt, about 6% wt, or about 8% wt. In some embodiments, the above crystal form D has an isopropanol content of about 4% wt. In some embodiments, the above crystal form D has an isopropanol content of about 6% wt.

In another aspect, the present disclosure provides a preparation method for the above crystal form D, which includes: 1) mixing compound 21-A with isopropanol; and 2) separating a solid to give the crystal form D.

In some embodiments of the present disclosure, in the preparation method for the above crystal form D, the mass-to-volume ratio of compound 21-A to isopropanol is selected from the group consisting of 1 g:5-50 mL, 1 g:10-40 mL, 1 g:10-30 mL, 1 g:15-25 mL, 1 g:8-12 mL, and 1 g:10 mL.

The present disclosure provides a crystal form E of compound 21-A, in an X-ray powder diffraction (XRPD) pattern using Cu Kα radiation, the crystal form E of compound 21-A has diffraction peaks at 2θ angles selected from about 7.38±0.20° and about 23.40±0.20°. In some embodiments, the above crystal form E has diffraction peaks at about 7.38±0.20°, about 17.45±0.20°, about 23.40±0.20°, or about 25.04±0.20°. In some embodiments, the above crystal form E has diffraction peaks at about 7.38±0.20°, about 11.57±0.20°, about 17.45±0.20°, about 17.87±0.20°, about 23.40±0.20°, or about 25.04±0.20°. In some embodiments, the above crystal form E has diffraction peaks at about 7.38±0.20°, about 11.57±0.20°, about 17.45±0.20°, about 17.87±0.20°, about 19.05±0.20°, about 23.40±0.20°, about 23.63±0.20°, or about 25.04±0.20°. In some embodiments, the above crystal form E has diffraction peaks at about 7.38±0.20°, about 11.57±0.20°, about 17.25±0.20°, about 17.45±0.20°, about 17.87±0.20°, about 18.45±0.20°, about 19.05±0.20°, about 23.40±0.20°, about 23.63±0.20°, or about 25.04±0.20°. In some embodiments, the above crystal form E has diffraction peaks at about 7.38±0.20°, about 11.57±0.20°, about 17.25±0.20°, about 17.45±0.20°, about 17.87±0.20°, about 18.45±0.20°, about 19.05±0.20°, about 19.45±0.20°, about 22.08±0.20°, about 22.53±0.20°, about 23.40±0.20°, about 23.63±0.20°, about 25.04±0.20°, or about 28.62±0.20°.

In some embodiments, the above crystal form E has at least 3, at least 4, at least 5, at least 6, at least 7, at least 8, at least 9, at least 10, or more characteristic diffraction peaks in an X-ray powder diffraction (XRPD) pattern using Cu-Kα radiation at 2θ values selected from the group consisting of about 7.38±0.20°, about 11.57±0.20°, about 17.25±0.20°, about 17.45±0.20°, about 17.87±0.20°, about 18.45±0.20°, about 19.05±0.20°, about 19.45±0.20°, about 22.08±0.20°, about 22.53±0.20°, about 23.40±0.20°, about 23.63±0.20°, about 25.04±0.20°, and about 28.62±0.20°.

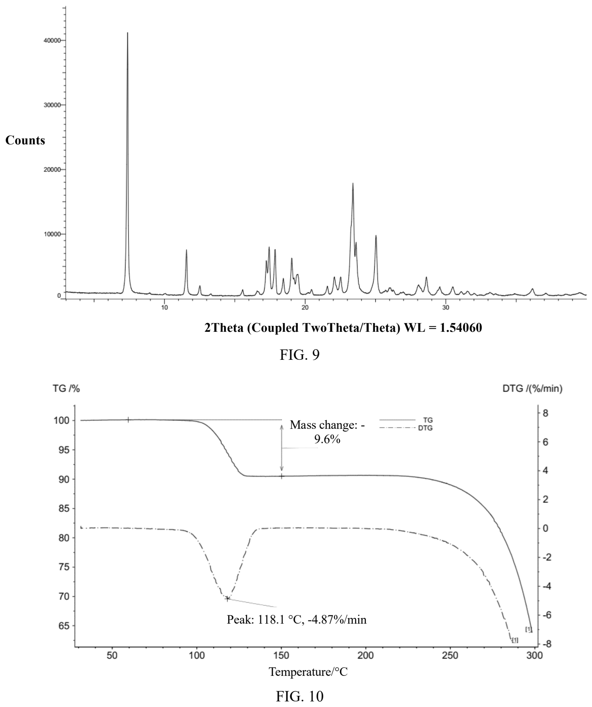

In some embodiments of the present disclosure, the above crystal form E has an XRPD pattern as shown in FIG. 9.

In some embodiments of the present disclosure, the above crystal form E has characteristic diffraction peaks in an XRPD pattern at the following 2θ angles, as shown in Table 5.

**Table 5: XRPD pattern data for crystal form E**

| No. | 2θ (±0.20°) | Relative intensity (%) | No. | 2θ (±0.20°) | Relative intensity (%) |
|---|---|---|---|---|---|
| 1 | 7.38 | 100.00% | 11 | 22.08 | 6.70% |
| 2 | 11.57 | 17.20% | 12 | 22.53 | 6.60% |
| 3 | 12.52 | 3.70% | 13 | 23.40 | 42.50% |
| 4 | 17.25 | 13.00% | 14 | 23.63 | 19.80% |
| 5 | 17.45 | 18.30% | 15 | 25.04 | 22.40% |
| 6 | 17.87 | 17.30% | 16 | 28.06 | 3.80% |
| 7 | 18.45 | 6.20% | 17 | 28.62 | 6.90% |
| 8 | 19.05 | 14.00% | 18 | 29.57 | 3.00% |
| 9 | 19.45 | 7.80% | 19 | 30.51 | 2.90% |
| 10 | 21.58 | 3.20% | | | |

In some embodiments of the present disclosure, the above crystal form E shows a weight loss of 9.6% in a thermogravimetric analysis (TGA) curve upon heating to 150 °C.

In some embodiments of the present disclosure, the above crystal form E has a TGA profile as shown in FIG. 10.

In some embodiments of the present disclosure, the above crystal form E is a crystal form of an ethanol solvate of compound 21-A.

In some embodiments of the present disclosure, in the above crystal form E, the ratio of the number of compound 21-A molecules to ethanol molecules (molar ratio) is selected from the group consisting of about 1:0.5-5, about 1:0.5-4, about 1:0.5-3, about 1:0.5-2, about 1:0.5-1.5, and about 1:0.5-1. In some embodiments of the present disclosure, in the above crystal form E, the ratio of the number of compound 21-A molecules to ethanol molecules (molar ratio) is selected from the group consisting of about 1:0.5, about 1:1, about 1:2, about 1:3, about 1:4, about 1:5, and about 1:6. In some embodiments of the present disclosure, in the above crystal form E, the ratio of the number of compound 21-A molecules to ethanol molecules (molar ratio) is selected from about 1:1.

In some embodiments, the above crystal form E has an ethanol content of about 1%-40% wt, about 1%-30% wt, about 1%-20% wt, about 2%-15% wt, about 5%-10% wt, about 5%-10% wt, or about 7%-10% wt. In some embodiments, the above crystal form E has an ethanol content of about 1% wt, about 2% wt, about 3% wt, about 4% wt, about 5% wt, about 6% wt, about 7% wt, about 8% wt, about 9% wt, about 10% wt, about 11% wt, about 12% wt, about 13% wt, about 14% wt, or about 15% wt, or a range formed by any of the above values. In some embodiments, the above crystal form E has an ethanol content of about 7% wt or 10% wt. In some embodiments, the above crystal form E has an ethanol content of about 7% wt. In some embodiments, the above crystal form E has an ethanol content of about 10% wt.

In another aspect, the present disclosure provides a preparation method for the above crystal form E, which includes: 1) dissolving compound 21-A in a mixed solvent of ethanol and water; and 2) separating a solid to give the crystal form E.

In some embodiments of the present disclosure, in the preparation method for the above crystal form E, the mass-to-volume ratio of compound 21-A to ethanol is selected from the group consisting of 1 g:5-50 mL, 1 g:10-40 mL, 1 g:10-30 mL, 1 g:15-25 mL, 1 g:8-12 mL, and 1 g:10 mL.

In some embodiments of the present disclosure, in the preparation method for the above crystal form E, the mass-to-volume ratio of compound 21-A to water is selected from the group consisting of 1 g:0.1-10 mL, 1 g:0.1-8 mL, 1 g:0.1-5 mL, 1 g:0.1-4 mL, 1 g:0.1-3 mL, 1 g:0.1-2 mL, 1 g:0.1-1.0 mL, 1 g:0.1-0.5 mL, and 1 g:0.2 mL.

The present disclosure provides a crystal form F of compound 21-A, in an X-ray powder diffraction (XRPD) pattern using Cu Kα radiation, the crystal form F of compound 21-A has diffraction peaks at 2θ angles selected from about 7.59±0.20°, about 23.28±0.20°, and about 24.94±0.20°. In some embodiments, the above crystal form F has diffraction peaks at about 7.59±0.20°, about 11.83±0.20°, about 17.77±0.20°, about 23.28±0.20°, or about 24.94±0.20°. In some embodiments, the above crystal form F has diffraction peaks at about 7.59±0.20°, about 11.83±0.20°, about 17.77±0.20°, about 19.44±0.20°, about 23.28±0.20°, about 23.70±0.20°, or about 24.94±0.20°. In some embodiments, the above crystal form F has diffraction peaks at about 7.59±0.20°, about 8.05±0.20°, about 11.83±0.20°, about 17.77±0.20°, about 19.44±0.20°, about 22.60±0.20°, about 23.28+0.20°, about 23.70+0.20°, or about 24.94+0.20°. In some embodiments, the above crystal form F has diffraction peaks at about 7.59+0.20°, about 8.05+0.20°, about 11.83±0.20°, about 17.77+0.20°, about 19.44+0.20°, about 22.60+0.20°, about 23.28+0.20°, about 23.70+0.20°, about 24.20+0.20°, about 24.94+0.20°, or about 28.68+0.20°.

In some embodiments, the above crystal form F has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 7.59±0.20°, about 8.05±0.20°, about 11.83±0.20°, about 17.77±0.20°, about 19.44±0.20°, about 22.60±0.20°, about 23.28±0.20°, about 23.70±0.20°, about 24.20±0.20°, about 24.94±0.20°, and about 28.68±0.20°.

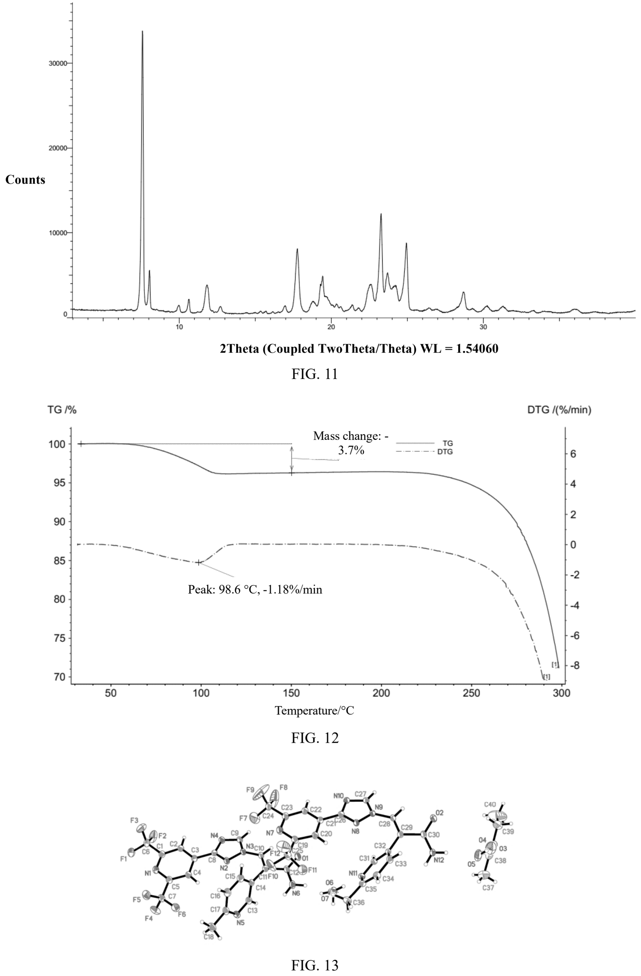

In some embodiments of the present disclosure, the above crystal form F has an XRPD pattern as shown in FIG. 11.

In some embodiments of the present disclosure, the above crystal form F has characteristic diffraction peaks in an XRPD pattern at the following 2θ angles, as shown in Table 6.

**Table 6: XRPD pattern data for crystal form F**

| No. | 2θ (±0.20°) | Relative intensity (%) | No. | 2θ (±0.20°) | Relative intensity (%) |
|---|---|---|---|---|---|
| 1 | 7.59 | 100.00% | 10 | 19.44 | 12.60% |
| 2 | 8.05 | 14.40% | 11 | 19.67 | 5.20% |
| 3 | 10.00 | 2.40% | 12 | 22.60 | 9.60% |
| 4 | 10.63 | 4.80% | 13 | 23.28 | 34.60% |
| 5 | 11.83 | 10.00% | 14 | 23.70 | 13.50% |
| 6 | 12.71 | 2.30% | 15 | 24.20 | 9.10% |
| 7 | 16.96 | 2.50% | 16 | 24.94 | 24.20% |
| 8 | 17.77 | 22.70% | 17 | 28.68 | 7.10% |
| 9 | 18.79 | 3.70% | | | |

In some embodiments of the present disclosure, the above crystal form F shows a weight loss of 3.7% in a thermogravimetric analysis (TGA) curve upon heating to 150+3 °C.

In some embodiments of the present disclosure, the above crystal form F has a TGA profile as shown in FIG. 12.

In some embodiments of the present disclosure, the above crystal form F is a crystal form of a methanol solvate of compound 21-A.

In some embodiments of the present disclosure, in the above crystal form F, the ratio of the number of compound 21-A molecules to methanol molecules (molar ratio) is selected from the group consisting of about 1:0.1-6, about 1:0.1-5, about 1:0.1-4, about 1:0.5-4, about 1:0.5-3, about 1:0.5-2, about 1:0.5-1, and about 1:1. In some embodiments of the present disclosure, in the above crystal form F, the ratio of the number of compound 21-A molecules to methanol molecules (molar ratio) is selected from the group consisting of about 1:0.1, about 1:0.5, about 1:1, about 1:1.5, about 1:2, about 1:3, about 1:4, about 1:5, and about 1:6, or a range formed by any of the above values. In some embodiments of the present disclosure, in the above crystal form F, the ratio of the number of compound 21-A molecules to methanol molecules (molar ratio) is selected from the group consisting of about 1:0.5 and 1:1.

In some embodiments, the above crystal form F has a methanol content of about 1%-20% wt, about 1%-15% wt, about 1%-12% wt, about 1%-10% wt, about 1%-8% wt, about 2%-6% wt, or about 2%-4% wt. In some embodiments, the above crystal form F has a methanol content of about 1% wt, about 2% wt, about 3% wt, about 4% wt, about 5% wt, about 6% wt, about 7% wt, about 8% wt, about 9% wt, about 10% wt, about 11% wt, about 12% wt, about 13% wt, about 14% wt, or about 15% wt, or a range formed by any of the above values. In some embodiments, the above crystal form F has a methanol content of about 2% wt or about 4% wt. In some embodiments, the above crystal form F has a methanol content of about 2% wt. In some embodiments, the above crystal form F has a methanol content of about 4% wt.

In another aspect, the present disclosure provides a preparation method for the above crystal form F, which includes: 1) dissolving compound 21-A in a mixed solvent of isopropyl acetate, methanol, and water; and 2) separating a solid to give the crystal form F.

In some embodiments of the present disclosure, in the preparation method for the above crystal form F, the mass-to-volume ratio of compound 21-A to isopropyl acetate is selected from the group consisting of 1 g:5-50 mL, 1 g:10-40 mL, 1 g:10-30 mL, 1 g:10-25 mL, 1 g:10-20 mL, and 1 g:15 mL.

In some embodiments of the present disclosure, in the preparation method for the above crystal form F, the mass-to-volume ratio of compound 21-A to methanol is selected from the group consisting of 1 g:0.1-10 mL, 1 g:0.2-8 mL, 1 g:0.2-5 mL, 1 g:0.5-4 mL, 1 g:1-3 mL, 1 g:2-3 mL, and 1 g:2.5 mL.

In some embodiments of the present disclosure, in the preparation method for the above crystal form F, the mass-to-volume ratio of compound 21-A to water is selected from the group consisting of 1 g:0.1-10 mL, 1 g:0.2-8 mL, 1 g:0.2-5 mL, 1 g:0.2-4 mL, 1 g:0.2-2 mL, 1 g:0.2-1 mL, and 1 g:0.5 mL.

In one specific embodiment, the present disclosure provides a crystal form G of compound 21-A, wherein the crystalline form is characterized by: a monoclinic crystal system; space group: P-1; unit cell parameters: a = 10.9919(3) Å, b = 14.7579(4) Å, c = 16.0678(4) Å, α = 108.8840(10)°, β = 105.1420(10)°, γ = 98.9680(10)°; Z = 2.

In one specific embodiment, in the crystal form G of compound 21-A, the ratio of the number of molecules (molar ratio) is that compound 21-A:ethyl acetate:water = 2:1:3.

The present disclosure provides a crystal form A of compound 3-A. In an X-ray powder diffraction (XRPD) pattern using Cu Kα radiation, the crystal form A of compound 3-A has diffraction peaks at 2θ angles selected from about 10.48±0.20°, about 19.46±0.20°, about 21.56±0.20°, and about 23.28±0.20°.

In some embodiments, the crystal form A of compound 3-A has diffraction peaks at about 10.48±0.20°, about 12.78±0.20°, about 19.46±0.20°, about 20.74±0.20°, about 21.56±0.20°, about 21.94±0.20°, or about 23.28±0.20°.

In some embodiments, the crystal form A of compound 3-A has diffraction peaks at about 10.48±0.20°, about 12.78±0.20°, about 14.68±0.20°, about 18.49±0.20°, about 19.07±0.20°, about 19.46±0.20°, about 21.02±0.20°, about 20.74±0.20°, about 21.56±0.20°, about 21.94±0.20°, or about 23.28±0.20°.

In some embodiments, the crystal form A of compound 3-A has diffraction peaks at about 10.48±0.20°, about 11.89±0.20°, about 12.78±0.20°, about 14.68±0.20°, about 18.49±0.20°, about 19.07±0.20°, about 19.46±0.20°, about 21.02±0.20°, about 20.74±0.20°, about 21.56±0.20°, about 21.94±0.20°, about 22.33±0.20°, or about 23.28±0.20°.

In some embodiments, the above crystal form A of compound 3-A has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 10.48+0.20°, about 11.89+0.20°, about 12.78+0.20°, about 14.68+0.20°, about 18.49+0.20°, about 19.07+0.20°, about 19.46+0.20°, about 21.02+0.20°, about 20.74+0.20°, about 21.56±0.20°, about 21.94±0.20°, about 22.33±0.20°, and about 23.28±0.20°.

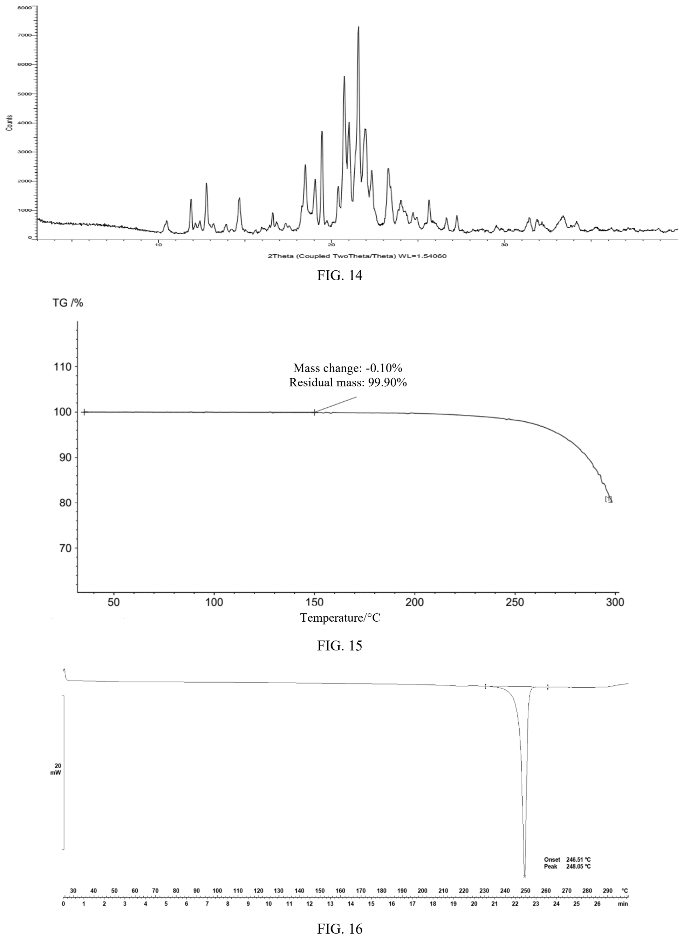

In some embodiments of the present disclosure, the above crystal form A of compound 3-A has an XRPD pattern as shown in FIG. 14.

In some embodiments of the present disclosure, the above crystal form A of compound 3-A has characteristic diffraction peaks in an XRPD pattern at the following 2θ angles, as shown in Table 7.

**Table 7: XRPD pattern data for crystal form A of compound 3-A**

| No. | 2θ (±0.20°) | Relative intensity (%) | No. | 2θ (±0.20°) | Relative intensity (%) |
|---|---|---|---|---|---|
| 1 | 10.48 | 5.90% | 12 | 21.02 | 52.30% |
| 2 | 11.89 | 16.90% | 13 | 21.56 | 100% |
| 3 | 12.14 | 5.1% | 14 | 21.94 | 49.00% |
| 4 | 12.40 | 6.0% | 15 | 22.33 | 27.90% |
| 5 | 12.78 | 24.80% | 16 | 23.28 | 29.10% |
| 6 | 14.68 | 17.40% | 17 | 24.03 | 13.30% |
| 7 | 16.61 | 9.50% | 18 | 24.72 | 7.5% |
| 8 | 18.49 | 32.40% | 19 | 25.64 | 14.20% |
| 9 | 19.07 | 24.30% | 20 | 26.65 | 6.30% |
| 10 | 19.46 | 48.40% | 21 | 27.25 | 7.90% |
| 11 | 20.74 | 75.80% | | | |

In some embodiments of the present disclosure, the above crystal form A of compound 3-A shows a weight loss of 0.10% in a thermogravimetric analysis (TGA) curve upon heating to about 150±3 °C. In some embodiments of the present disclosure, the above crystal form A of compound 3-A has a TGA profile as shown in FIG. 15.

In some embodiments of the present disclosure, the above crystal form A of compound 3-A has an endothermic peak in a differential scanning calorimetry (DSC) curve at 248.05±3 °C.

In some embodiments of the present disclosure, the above crystal form A of compound 3-A has a differential scanning calorimetry (DSC) curve as shown in FIG. 16.

In some embodiments of the present disclosure, the above crystal form A of compound 3-A is an anhydrous crystal form of compound 3-A.

In another aspect, the present disclosure provides a preparation method for the above crystal form A of compound 3-A, which includes: 1) mixing compound 3-3 with pyridine and Boc anhydride in 1,4-dioxane; 2) mixing with ammonium bicarbonate; 3) mixing with isopropyl acetate and n-heptane; 4) mixing with isopropyl acetate; and 5) separating a solid to give the crystal form A of compound **3-A.**

In another aspect, the present disclosure provides a preparation method for the above crystal form A of compound 3-A, which includes: mixing compound 3-A with isopropyl acetate (or n-heptane), and separating a solid to give the crystal form A of compound **3-A.**

In some embodiments, in the preparation method for the crystal form A of compound **3-A,** step 4) further includes after mixing with isopropyl acetate, heating to 60-100 °C or 80 °C and stirring, and then cooling to 10-30 °C or stirring at room temperature.

In some embodiments, in the preparation method for the crystal form A of compound **3-A,** the mass-to-volume ratio of compound **3-3** in step 1) to isopropyl acetate/n-heptane (v/v = 1: 1) in step 3) is 1 g:1-50 mL, 1 g:5-40 mL, 1 g:5-30 mL, 1 g:5-25 mL, 1 g:5-20 mL, 1 g:5-15 mL, 1 g:5-10 mL, or 1 g:10 mL.

In some embodiments, in the preparation method for the crystal form A of compound **3-A,** the mass-to-volume ratio of compound **3-3** in step 1) to isopropyl acetate in step 4) is 1 g:1-50 mL, 1 g:5-40 mL, 1 g:5-30 mL, 1 g:5-25 mL, 1 g:5-20 mL, 1 g:5-15 mL, 1 g:5-10 mL, or 1 g:10 mL.

The present disclosure provides a crystal form B of compound 3-A, in an X-ray powder diffraction (XRPD) pattern using Cu Kα radiation, the crystal form C of compound 3-A has diffraction peaks at 2θ angles selected from about 7.21±0.20°, about 23.03±0.20°, and about 24.18±0.20°.

In some embodiments, the crystal form B of compound 3-A has diffraction peaks at about 7.21±0.20°, about 19.74+0.20°, about 21.31+0.20°, about 21.68+0.20°, about 22.03+0.20°, about 23.03+0.20°, or about 24.18+0.20°.

In some embodiments, the crystal form B of compound 3-A has diffraction peaks at about 7.21±0.20°, about 12.56+0.20°, about 13.47+0.20°, about 13.87+0.20°, about 19.74+0.20°, about 21.31±0.20°, about 21.68±0.20°, about 22.03±0.20°, about 23.03±0.20°, or about 24.18±0.20°.

In some embodiments, the crystal form B of compound 3-A has diffraction peaks at about 7.21±0.20°, about 12.56±0.20°, about 13.47±0.20°, about 13.87±0.20°, about 18.31±0.20°, about 18.60±0.20°, about 19.74±0.20°, about 21.31±0.20°, about 21.68±0.20°, about 22.03±0.20°, about 23.03±0.20°, about 24.18±0.20°, or about 24.95±0.20°.

In some embodiments, the above crystal form B of compound 3-A has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 7.21±0.20°, about 12.56±0.20°, about 13.47±0.20°, about 13.87±0.20°, about 18.31±0.20°, about 18.60±0.20°, about 19.74±0.20°, about 21.31±0.20°, about 21.68±0.20°, about 22.03±0.20°, about 23.03±0.20°, about 24.18±0.20°, and about 24.95±0.20°.

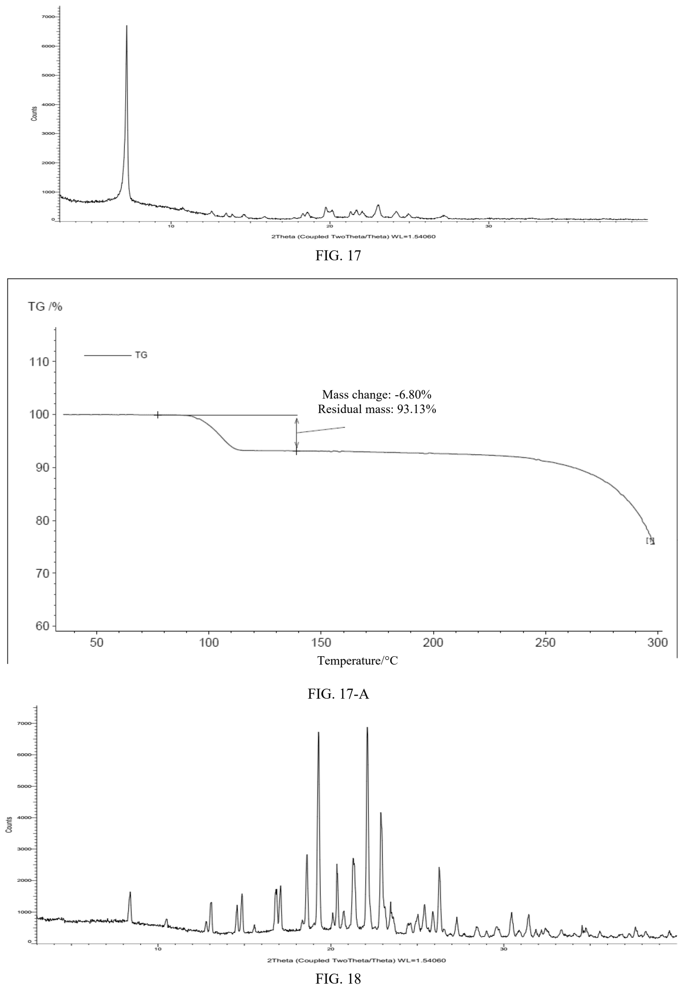

In some embodiments of the present disclosure, the above crystal form B of compound 3-A has an XRPD pattern as shown in FIG. 17.

In some embodiments of the present disclosure, the above crystal form B of compound 3-A has characteristic diffraction peaks in an XRPD pattern at the following 2θ angles, as shown in Table 9.

**Table 9: XRPD pattern data for crystal form B of compound 3-A**

| No. | 2θ (±0.20°) | Relative intensity (%) | No. | 2θ (±0.20°) | Relative intensity (%) |
|---|---|---|---|---|---|
| 1 | 7.21 | 100.00% | 9 | 20.08 | 3.7% |
| 2 | 12.56 | 2.20% | 10 | 21.31 | 3.20% |
| 3 | 13.47 | 2.10% | 11 | 21.68 | 4.10% |
| 4 | 13.87 | 1.30% | 12 | 22.03 | 3.50% |
| 5 | 14.57 | 2.00% | 13 | 23.03 | 6.90% |
| 6 | 18.31 | 2.80% | 14 | 24.18 | 3.40% |
| 7 | 18.60 | 3.10% | 15 | 24.95 | 2.20% |
| 8 | 19.74 | 6.00% | 16 | 27.17 | 1.70% |

In some embodiments of the present disclosure, the above crystal form B of compound 3-A shows a weight loss of 6.8% in a thermogravimetric analysis (TGA) curve during the heating process.

In some embodiments of the present disclosure, the above crystal form B of compound 3-A has a thermogravimetric analysis (TGA) curve as shown in FIG. 17-A.

In some embodiments of the present disclosure, the above crystal form B of compound 3-A is a crystal form of a 1,4-dioxane solvate of compound 3-A.

In some embodiments of the present disclosure, in the above crystal form B of compound 3-A, the molar ratio of compound 3-A to 1,4-dioxane is selected from the group consisting of: about 1:0.2-2, about 1:0.2-1, about 1:0.3-0.8, and about 1:0.5.

In some embodiments, the above crystal form B of compound 3-A has a 1,4-dioxane content of: about 4%-15% wt, about 5%-10% wt, or about 6%-8% wt. In some embodiments, the above crystal form B of compound 3-A has a 1,4-dioxane content of: about 4% wt, about 5% wt, about 6% wt, about 7% wt, about 8% wt, about 9% wt, about 10% wt, about 11% wt, about 12% wt, about 13% wt, about 14% wt, or about 15% wt, or a range formed by any of the above values. In some embodiments, the above crystal form B of compound 3-A has a 1,4-dioxane content of: about 6% wt, about 7% wt, or about 8% wt.

In another aspect, the present disclosure provides a preparation method for the above crystal form B of compound 3-A, which includes: 1) mixing compound 3-A with 1,4-dioxane; 2) adding petroleum ether for crystallization; and 3) separating a solid to give the crystal form B.

In another aspect, the present disclosure provides a preparation method for the above crystal form B of compound 3-A, which includes: 1) mixing the crystal form A of compound 3-A with 1,4-dioxane; 2) adding petroleum ether for crystallization; and 3) separating a solid to give the crystal form B.

In some embodiments, in the preparation method for the above crystal form B of compound 3-A, the crystal form A of compound 3-A is mixed with 1,4-dioxane, and the mixture is further dissolved by heating (optionally at a temperature of 50-80 °C, 50-70 °C, 60-70 °C, or 65 °C) in a water bath. In some embodiments, in the preparation method for the above crystal form B of compound 3-A, step 3) includes centrifuging, removing supernatant, and drying (optionally at a temperature of 30-60 °C, 30-50 °C, or 40 °C) to give the crystal form B.

In some embodiments, in the preparation method for the above crystal form B of compound 3-A, the mass-to-volume ratio of the crystal form A of compound 3-A to 1,4-dioxane is selected from the group consisting of 1 g:50-500 mL, 1 g:50-400 mL, 1 g:50-300 mL, 1 g:50-200 mL, 1 g:100-200 mL, and 1 g:100 mL.

In some embodiments, in the preparation method for the above crystal form B of compound 3-A, the mass-to-volume ratio of the crystal form A of compound 3-A to the petroleum ether is selected from the group consisting of 1 g:1000-5000 mL, 1 g:1000-4000 mL, 1 g:2000-4000 mL, 1 g:2000-3000 mL, and 1 g:2500 mL.

The present disclosure provides a crystal form C of compound 3-A, in an X-ray powder diffraction (XRPD) pattern using Cu Kα radiation, the crystal form D of compound 3-A has diffraction peaks at 2θ angles selected from about 8.40±0.20°, about 19.29±0.20°, about 22.11±0.20°, and about 22.89±0.20°.

In some embodiments, the crystal form C of compound 3-A has diffraction peaks at about 8.40±0.20°, about 18.62±0.20°, about 19.29±0.20°, about 20.35±0.20°, about 21.30±0.20°, about 22.11±0.20°, about 22.89±0.20°, or about 26.27±0.20°.

In some embodiments, the crystal form C of compound 3-A has diffraction peaks at about 8.40±0.20°, about 14.87±0.20°, about 16.83±0.20°, about 17.10±0.20°, about 18.62±0.20°, about 19.29±0.20°, about 20.35±0.20°, about 21.30±0.20°, about 22.11±0.20°, about 22.89±0.20°, or about 26.27±0.20°.

In some embodiments, the crystal form C of compound 3-A has diffraction peaks at about 8.40±0.20°, about 13.08±0.20°, about 14.58±0.20°, about 14.87±0.20°, about 16.83±0.20°, about 17.10±0.20°, about 18.62±0.20°, about 19.29±0.20°, about 20.35±0.20°, about 21.30±0.20°, about 22.11±0.20°, about 22.89±0.20°, about 23.47±0.20°, about 25.43±0.20°, or about 26.27±0.20°.

In some embodiments, the above crystal form C of compound 3-A has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 8.40±0.20°, about 13.08±0.20°, about 14.58±0.20°, about 14.87±0.20°, about 16.83±0.20°, about 17.10±0.20°, about 18.62±0.20°, about 19.29±0.20°, about 20.35±0.20°, about 21.30±0.20°, about 22.11±0.20°, about 22.89±0.20°, about 23.47±0.20°, about 25.43±0.20°, and about 26.27±0.20°.

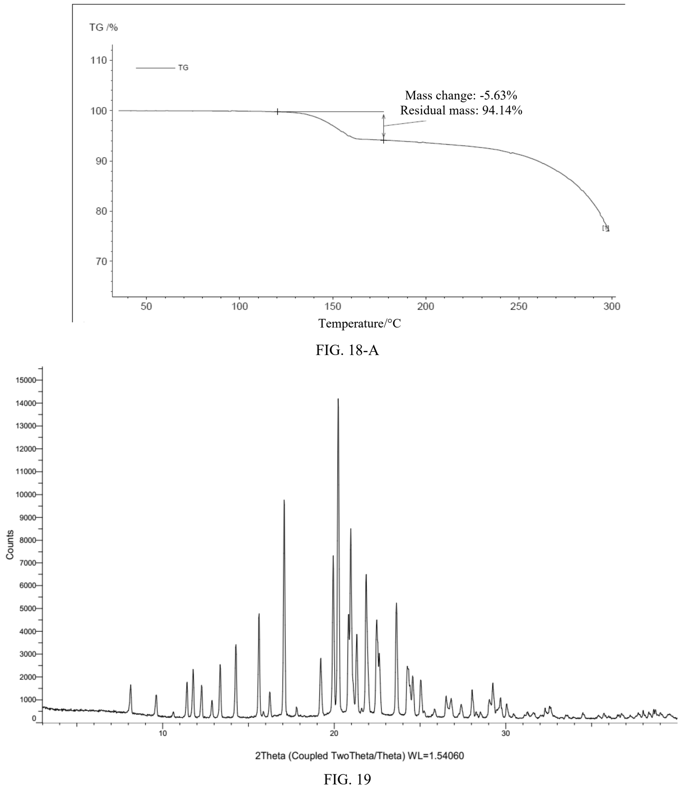

In some embodiments of the present disclosure, the above crystal form C of compound 3-A has an XRPD pattern as shown in FIG. 18.

In some embodiments of the present disclosure, the above crystal form C of compound 3-A shows a weight loss of 5.63% in a thermogravimetric analysis (TGA) curve during the heating process.

In some embodiments of the present disclosure, the above crystal form C of compound 3-A has a thermogravimetric analysis (TGA) curve as shown in FIG. 18-A.

In some embodiments of the present disclosure, the above crystal form C of compound 3-A is a crystal form of a tetrahydrofuran solvate of compound 3-A.

In some embodiments of the present disclosure, in the above crystal form C of compound 3-A, the molar ratio of compound 3-A to tetrahydrofuran is selected from the group consisting of: about 1:0.2-2, about 1:0.2-1, about 1:0.3-0.8, and about 1:0.5.

In some embodiments, the above crystal form C of compound 3-A has a tetrahydrofuran content of: about 4%-15% wt, about 5%-10% wt, or about 5%-8% wt. In some embodiments, the above crystal form C of compound 3-A has a tetrahydrofuran content of: about 4% wt, about 5% wt, about 6% wt, about 7% wt, about 8% wt, about 9% wt, about 10% wt, about 11% wt, about 12% wt, about 13% wt, about 14% wt, or about 15% wt, or a range formed by any of the above values. In some embodiments, the above crystal form C of compound 3-A has a tetrahydrofuran content of: about 5% wt, about 6% wt, or about 7% wt.

In another aspect, the present disclosure provides a preparation method for the above crystal form C of compound 3-A, which includes: 1) mixing compound 3-A with tetrahydrofuran; and 2) heating the mixture to remove the tetrahydrofuran to give the crystal form C.

In another aspect, the present disclosure provides a preparation method for the above crystal form C of compound 3-A, which includes: 1) mixing the crystal form A of compound 3-A with tetrahydrofuran; and 2) heating the mixture to remove the tetrahydrofuran to give the crystal form C.

In some embodiments, in the preparation method for the above crystal form C of compound 3-A, the crystal form A of compound 3-A is mixed with tetrahydrofuran, and then the mixture is dried at 50-80 °C to give the crystal form C.

In some embodiments, in the preparation method for the above crystal form C of compound 3-A, the crystal form A of compound 3-A is mixed with tetrahydrofuran, and then optionally, the mixture is dried at 50-80 °C, 50-70 °C, 60-70 °C, or 65 °C in a water bath to give the crystal form C.

In some embodiments of the present disclosure, in the preparation method for the above crystal form C of compound 3-A, the mass-to-volume ratio of the crystal form A of compound 3-A to tetrahydrofuran is selected from the group consisting of 1 g: 1000-5000 mL, 1 g: 1000-4000 mL, 1 g: 1000-3000 mL, and 1 g:2000 mL.

In some embodiments of the present disclosure, the above crystal form C of compound 3-A has characteristic diffraction peaks in an XRPD pattern at the following 2θ angles, as shown in Table 10.

**Table 10: XRPD pattern data for crystal form C of compound 3-A**

| No. | 2θ (±0.20°) | Relative intensity (%) | No. | 2θ (±0.20°) | Relative intensity (%) |
|---|---|---|---|---|---|
| 1 | 8.40 | 15.50% | 14 | 21.30 | 35.10% |
| 2 | 10.50 | 4.10% | 15 | 22.89 | 58.60% |
| 3 | 12.80 | 5% | 16 | 23.47 | 14.90% |
| 4 | 13.08 | 14.80% | 17 | 24.54 | 4.50% |
| 5 | 14.58 | 13.80% | 18 | 25.04 | 9.80% |
| 6 | 14.87 | 19.20% | 19 | 25.43 | 14.60% |
| 7 | 16.83 | 20.70% | 20 | 25.90 | 11.10% |
| 8 | 17.10 | 22.30% | 21 | 26.27 | 33.10% |
| 9 | 18.62 | 37.00% | 22 | 27.29 | 9.20% |
| 10 | 19.29 | 97.70% | 23 | 28.46 | 4.90% |
| 11 | 20.35 | 32.30% | 24 | 29.59 | 4.90% |
| 12 | 20.77 | 9.10% | 25 | 30.46 | 11.70% |
| 13 | 22.11 | 100.00% | 26 | 31.44 | 10.70% |

The present disclosure provides a crystal form A of compound 15-A, in an X-ray powder diffraction (XRPD) pattern using Cu Kα radiation, the crystal form A of compound 15-A has diffraction peaks at 2θ angles selected from about 17.08±0.20°, about 20.23±0.20°, and about 20.97±0.20°.

In some embodiments, the crystal form A of compound 15-A has diffraction peaks at about 8.13±0.20°, about 15.63±0.20°, about 17.08±0.20°, about 19.94±0.20°, about 20.23±0.20°, about 20.97±0.20°, about 21.87±0.20°, or about 23.63±0.20°.

In some embodiments, the crystal form A of compound 15-A has diffraction peaks at about 8.13±0.20°, about 9.63±0.20°, about 14.27±0.20°, about 15.63±0.20°, about 17.08±0.20°, about 19.22±0.20°, about 19.94±0.20°, about 20.23±0.20°, about 20.97±0.20°, about 21.32±0.20°, about 21.87±0.20°, about 22.48±0.20°, or about 23.63±0.20°.

In some embodiments, the crystal form A of compound 15-A has diffraction peaks at about 8.13±0.20°, about 9.63±0.20°, about 11.78±0.20°, about 13.36±0.20°, about 14.27±0.20°, about 15.63±0.20°, about 17.08±0.20°, about 19.22±0.20°, about 19.94±0.20°, about 20.23±0.20°, about 20.97±0.20°, about 21.32±0.20°, about 21.87±0.20°, about 22.48±0.20°, about 23.63±0.20°, about 24.26±0.20°, or about 25.05±0.20°.

In some embodiments, the above crystal form A of compound 15-A has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 8.13±0.20°, about 9.63±0.20°, about 11.78±0.20°, about 13.36±0.20°, about 14.27±0.20°, about 15.63±0.20°, about 17.08±0.20°, about 19.22±0.20°, about 19.94±0.20°, about 20.23±0.20°, about 20.97±0.20°, about 21.32±0.20°, about 21.87±0.20°, about 22.48±0.20°, about 23.63±0.20°, about 24.26±0.20°, and about 25.05±0.20°.

In some embodiments of the present disclosure, the above crystal form A of compound 15-A has an XRPD pattern as shown in FIG. 19.

In some embodiments of the present disclosure, the above crystal form A of compound 15-A shows a weight loss of 0.4% in a thermogravimetric analysis (TGA) curve upon heating to 150±3 °C.

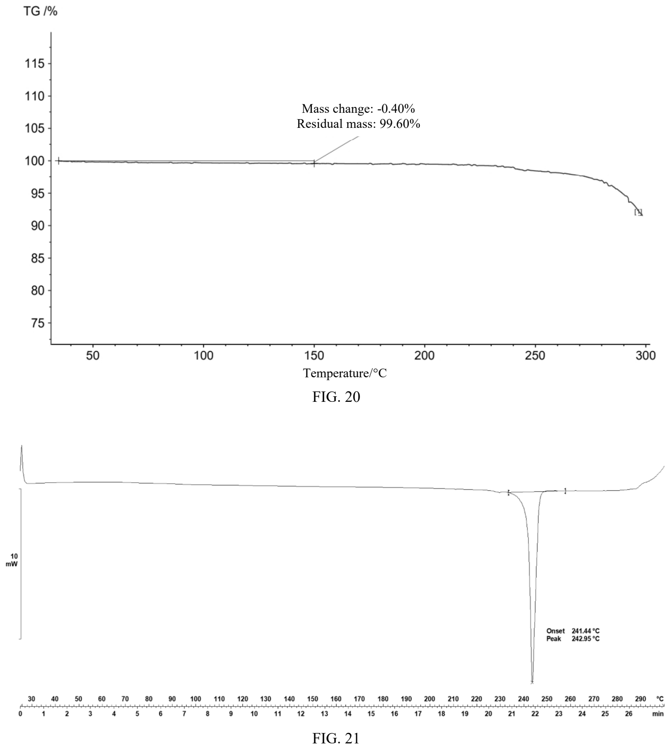

In some embodiments of the present disclosure, the above crystal form A of compound 15-A has a TGA profile as shown in FIG. 20.

In some embodiments of the present disclosure, the above crystal form A of compound 15-A is an anhydrous crystal form of compound 15-A.

In some embodiments of the present disclosure, the above crystal form A of compound 15-A has an endothermic peak in a differential scanning calorimetry (DSC) curve at 242.95±3 °C.

In some embodiments of the present disclosure, the above crystal form A of compound 15-A has a differential scanning calorimetry (DSC) curve as shown in FIG. 21.

In another aspect, the present disclosure provides a preparation method for the above crystal form A of compound 15-A, which includes: 1) mixing compound 15-3 with pyridine and Boc anhydride in 1,4-dioxane; 2) mixing with ammonium bicarbonate; 3) mixing with isopropyl acetate; and 4) separating a solid to give the crystal form A of compound 15-A.

In another aspect, the present disclosure provides a preparation method for the above crystal form A of compound 15-A, which includes: mixing compound 15-A with isopropyl acetate; and separating a solid to give the crystal form A of compound **15-A.**

In the preparation method for the above crystal form A of compound **15-A,** step 1) further includes after mixing, allowing the mixture to react at 10-30 °C (e.g., room temperature).

In some embodiments, in the preparation method for the crystal form A of compound **15-A,** step 2) further includes after mixing, stirring at 10-30 °C (e.g., room temperature), adding water, and stirring to precipitate a product.

In some embodiments, in the preparation method for the crystal form A of compound **15-A,** step 2) further includes filtering the precipitated product under vacuum, and extracting the filter cake with ethyl acetate, followed by washing, drying, and concentrating to give a crude product.

In some embodiments, in the preparation method for the crystal form A of compound **15-A,** step 3) includes mixing with isopropyl acetate and then recrystallizing.

In some embodiments, in the preparation method for the crystal form A of compound **15-A,** the mass-to-volume ratio of compound **15-3** in step 1) to isopropyl acetate in step 3) is 1 g:5-30 mL, 1 g:5-20 mL, 1 g:5-15 mL, 1 g:8-12 mL, 1 g:8-10 mL, or 1 g:9.23 mL.

In some embodiments of the present disclosure, the above crystal form A of compound 15-A has characteristic diffraction peaks in an XRPD pattern at the following 2θ angles, as shown in Table 11.

**Table 11: XRPD pattern data for crystal form A of compound 15-A**

| No. | 2θ (±0.20°) | Relative intensity (%) | No. | 2θ (±0.20°) | Relative intensity (%) |
|---|---|---|---|---|---|
| 1 | 8.13 | 8.90% | 13 | 20.23 | 100.00% |
| 2 | 9.63 | 6.70% | 14 | 20.97 | 58.80% |
| 3 | 11.42 | 11.00% | 15 | 21.32 | 25.30% |
| 4 | 11.78 | 15.30% | 16 | 21.87 | 44.30% |
| 5 | 12.27 | 10.10% | 17 | 22.48 | 29.80% |
| 6 | 16.25 | 7.7% | 18 | 23.63 | 35.30% |
| 7 | 13.36 | 16.60% | 19 | 24.26 | 15.50% |
| 8 | 14.27 | 22.80% | 20 | 24.58 | 12.3% |
| 9 | 15.63 | 32.70% | 21 | 25.05 | 11.40% |
| 10 | 17.08 | 68.60% | 22 | 28.04 | 8.7% |
| 11 | 19.22 | 18.00% | 23 | 29.25 | 10.8% |
| 12 | 19.94 | 50.40% | | | |

The present disclosure provides a crystal form B of compound 15-A, in an X-ray powder diffraction (XRPD) pattern using Cu Kα radiation, the crystal form B of compound 15-A has diffraction peaks at 2θ angles selected from about 6.30±0.20°, about 16.79±0.20°, and about 24.00±0.20°.

In some embodiments, the crystal form B of compound 15-A has diffraction peaks at about 6.30±0.20°, about 7.85±0.20°, about 16.79±0.20°, about 18.43±0.20°, about 22.85±0.20°, or about 24.00±0.20°.

In some embodiments, the crystal form B of compound 15-A has diffraction peaks at about 6.30±0.20°, about 7.85±0.20°, about 10.18±0.20°, about 12.21±0.20°, about 15.69±0.20°, about 16.79±0.20°, about 17.69±0.20°, about 18.43±0.20°, about 18.80±0.20°, about 22.85±0.20°, or about 24.00±0.20°.

In some embodiments, the crystal form B of compound 15-A has diffraction peaks at about 6.30±0.20°, about 7.85±0.20°, about 10.18±0.20°, about 12.21±0.20°, about 14.71±0.20°, about 15.69±0.20°, about 16.79±0.20°, about 17.69±0.20°, about 18.43±0.20°, about 18.80±0.20°, about 22.85±0.20°, about 24.00±0.20°, or about 26.08±0.20°.

In some embodiments, the crystal form B of compound 15-A has diffraction peaks at about 6.30±0.20°, about 7.85±0.20°, about 10.18±0.20°, about 12.21±0.20°, about 14.71±0.20°, about 15.69+0.20°, about 16.79+0.20°, about 17.69+0.20°, about 18.43+0.20°, about 18.80+0.20°, about 21.23+0.20°, about 22.85+0.20°, about 24.00+0.20°, about 26.08+0.20°, or about 26.72+0.20°.

In some embodiments, the above crystal form B of compound 15-A has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 6.30±0.20°, about 7.85±0.20°, about 10.18±0.20°, about 12.21±0.20°, about 14.71±0.20°, about 15.69±0.20°, about 16.79±0.20°, about 17.69±0.20°, about 18.43±0.20°, about 18.80±0.20°, about 21.23±0.20°, about 22.85±0.20°, about 24.00±0.20°, about 26.08±0.20°, and about 26.72±0.20°.

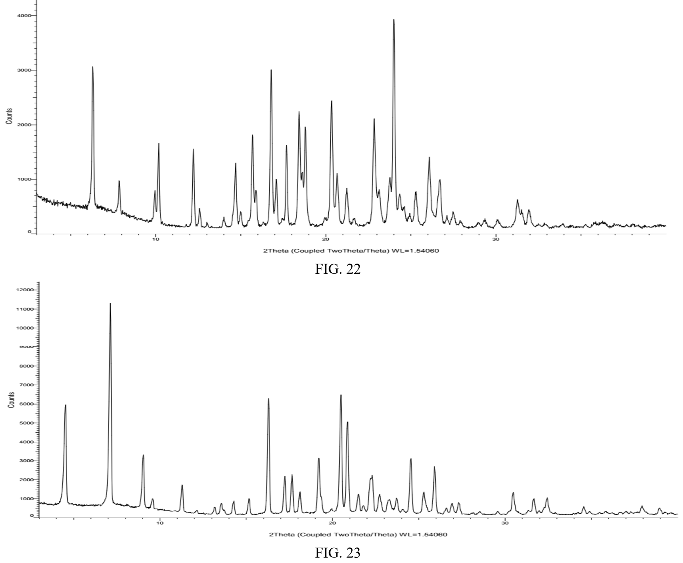

In some embodiments of the present disclosure, the above crystal form B of compound 15-A has an XRPD pattern as shown in FIG. 22.

In some embodiments of the present disclosure, the above crystal form B of compound 15-A shows a weight loss of 6.46% in a thermogravimetric analysis (TGA) curve during the heating process. In some embodiments of the present disclosure, the above crystal form B of compound 15-A is a crystal form of an acetonitrile solvate of compound 15-A.

In some embodiments of the present disclosure, in the above crystal form B of compound 15-A, the molar ratio of compound 15-A to acetonitrile is selected from the group consisting of: about 1:0.2-2, about 1:0.5-1.5, about 1:0.5-1, and about 1:1.

In some embodiments, the above crystal form B of compound 15-A has an acetonitrile content of: about 4%-15% wt, about 5%-10% wt, or about 6%-8% wt. In some embodiments, the above crystal form B of compound 15-A has an acetonitrile content of: about 4% wt, about 5% wt, about 6% wt, about 7% wt, about 8% wt, about 9% wt, about 10% wt, about 11% wt, about 12% wt, about 13% wt, about 14% wt, or about 15% wt, or a range formed by any of the above values. In some embodiments, the above crystal form B of compound 15-A has an acetonitrile content of: about 6% wt, about 7% wt, or about 8% wt.

In another aspect, the present disclosure provides a preparation method for the above crystal form B of compound 15-A, which includes: 1) mixing compound 15-A with acetonitrile; and 2) cooling for crystallization to give the crystal form B.

In another aspect, the present disclosure provides a preparation method for the above crystal form B of compound 15-A, which includes: 1) mixing the crystal form A of compound 15-A with acetonitrile; and 2) cooling for crystallization to give the crystal form B.

In some embodiments, in the preparation method for the above crystal form B of compound 15-A, the crystal form A of compound 15-A is mixed with acetonitrile and then cooled for crystallization, and further optionally, drying is performed at 30-60 °C, 30-50 °C, or 40 °C to give the crystal form B.

In some embodiments of the present disclosure, in the preparation method for the above crystal form B, the mass-to-volume ratio of the crystal form A of compound 15-A to acetonitrile is selected from the group consisting of 1 g:100-500 mL, 1 g:100-400 mL, 1 g:200-400 mL, 1 g:250-350 mL, and 1 g:300 mL.

In some embodiments of the present disclosure, the above crystal form B of compound 15-A has characteristic diffraction peaks in an XRPD pattern at the following 2θ angles, as shown in Table 12.

**Table 12: XRPD pattern data for crystal form B of compound 15-A**

| No. | 2θ (±0.20°) | Relative intensity (%) | No. | 2θ (±0.20°) | Relative intensity (%) |
|---|---|---|---|---|---|
| 1 | 6.30 | 69.50% | 14 | 18.43 | 55.60% |
| 2 | 7.85 | 16.70% | 15 | 18.80 | 48.10% |
| 3 | 9.96 | 15.9% | 16 | 20.34 | 60.80% |
| 4 | 10.18 | 39.40% | 17 | 20.66 | 25.30% |
| 5 | 12.21 | 38.10% | 18 | 21.23 | 18.20% |
| 6 | 12.58 | 9.1% | 19 | 22.85 | 52.20% |
| 7 | 14.71 | 31.20% | 20 | 24.00 | 100.00% |
| 8 | 15.00 | 7% | 21 | 24.34 | 14.7% |
| 9 | 15.69 | 44.70% | 22 | 25.31 | 16.30% |
| 10 | 15.90 | 17.3% | 23 | 26.08 | 33.10% |
| 11 | 16.79 | 76.00% | 24 | 26.72 | 21.80% |
| 12 | 17.09 | 22.30% | 25 | 31.27 | 13.10% |
| 13 | 17.69 | 39.00% | 26 | 31.95 | 8.40% |

The present disclosure provides a crystal form A of compound 25-A. In an X-ray powder diffraction (XRPD) pattern using Cu Kα radiation, the crystal form A of compound 25-A has diffraction peaks at 2θ angles selected from about 4.53±0.20°, about 7.13±0.20°, about 16.30±0.20°, and about 20.50±0.20°.

In some embodiments, the crystal form A of compound 25-A has diffraction peaks at about 4.53±0.20°, about 7.13±0.20°, about 9.04±0.20°, about 16.30±0.20°, about 19.21±0.20°, about 20.50±0.20°, about 20.87±0.20°, about 24.54±0.20°, or about 25.92±0.20°.

In some embodiments, the crystal form A of compound 25-A has diffraction peaks at about 4.53±0.20°, about 7.13±0.20°, about 9.04±0.20°, about 11.29±0.20°, about 16.30±0.20°, about 17.24±0.20°, about 17.65±0.20°, about 19.21±0.20°, about 20.50±0.20°, about 20.87±0.20°, about 22.29±0.20°, about 24.54±0.20°, or about 25.92±0.20°.

In some embodiments, the crystal form A of compound 25-A has diffraction peaks at about 4.53±0.20°, about 7.13±0.20°, about 9.04±0.20°, about 11.29±0.20°, about 16.30±0.20°, about 17.24±0.20°, about 17.65±0.20°, about 18.12±0.20°, about 19.21±0.20°, about 20.50±0.20°, about 20.87±0.20°, about 22.29±0.20°, about 24.54±0.20°, about 25.29±0.20°, about 25.92±0.20°, or about 30.47±0.20°.

In some embodiments, the above crystal form A of compound 25-A has at least 3, at least 4, at least 5, at least 6, at least 7, at least 8, at least 9, at least 10, or more characteristic diffraction peaks in an X-ray powder diffraction (XRPD) pattern using Cu Kα radiation at 2θ values selected from the group consisting of about 4.53±0.20°, about 7.13±0.20°, about 9.04±0.20°, about 11.29±0.20°, about 16.30±0.20°, about 17.24±0.20°, about 17.65±0.20°, about 18.12±0.20°, about 19.21±0.20°, about 20.50±0.20°, about 20.87±0.20°, about 22.29±0.20°, about 24.54±0.20°, about 25.29±0.20°, about 25.92±0.20°, and about 30.47±0.20°.

In some embodiments of the present disclosure, the above crystal form A of compound 25-A has an XRPD pattern as shown in FIG. 23.

In another aspect, the present disclosure provides a preparation method for the above crystal form A of compound 25-A, which includes:
1) mixing compound **25-3** with pyridine and Boc anhydride in 1,4-dioxane; 2) mixing with ammonium bicarbonate; 3) mixing with isopropyl acetate; and 4) separating a solid to give the crystal form A of compound **25-A.**

In another aspect, the present disclosure provides a preparation method for the above crystal form A of compound 25-A, which includes: mixing compound 25-A with isopropyl acetate; and separating a solid.

In the preparation method for the above crystal form A of compound **25-A,** step 1) further includes after mixing, allowing the mixture to react at 10-30 °C (e.g., room temperature).

In some embodiments, in the preparation method for the crystal form A of compound **25-A,** step 2) further includes after mixing, stirring the mixture at 10-30 °C (e.g., room temperature), adding water to the reaction solution, and stirring to precipitate a product.

In some embodiments, in the preparation method for the crystal form A of compound **25-A,** step 2) further includes filtering the precipitated product under vacuum, and extracting the filter cake with ethyl acetate, followed by washing, drying, and concentrating to give a crude product.

In some embodiments, in the preparation method for the crystal form A of compound **25-A,** step 3) includes mixing the crude product obtained in step 2) with isopropyl acetate, heating the mixture for dissolution (optionally at a temperature of 60-100 °C, 70-90 °C, or 80 °C), cooling to 10-30 °C (e.g., room temperature), and stirring.

In some embodiments, in the preparation method for the crystal form A of compound **25-A,** step 4) includes filtering and drying the solid obtained in step 3) to give the crystal form A of compound **25-A.**

In some embodiments, the preparation method for the crystal form A of compound **25-A** includes: 1) mixing compound **25-3** with pyridine and Boc anhydride in 1,4-dioxane, and allowing the mixture to react at 10-30 °C (e.g., room temperature); and 2) mixing with ammonium bicarbonate, stirring at 10-30 °C (e.g., room temperature), then adding water, and stirring to precipitate a product; filtering the product under vacuum, extracting the filter cake with ethyl acetate, followed by washing, drying, and concentrating to give a crude product; 3) adding isopropyl acetate, heating the mixture for dissolution (optionally at a temperature of 60-100 °C, 70-90 °C, or 80 °C), cooling to 10-30 °C (e.g., room temperature), and stirring; and 4) filtering and drying to give the crystal form A of compound **25-A.**

In some embodiments, in the preparation method for the crystal form A of compound **25-A,** the mass-to-volume ratio of compound 25-3 in step 1) to isopropyl acetate in step 3) is 1 g:5-30 mL, 1 g:5-20 mL, 1 g:5-15 mL, 1 g:8-12 mL, 1 g:9-11 mL, or 1 g:10.20 mL.

In some embodiments of the present disclosure, the above crystal form A of compound 25-A has characteristic diffraction peaks in an XRPD pattern at the following 2θ angles, as shown in Table 13.

**Table 13: XRPD pattern data for crystal form A of compound 25-A**

| No. | 2θ (±0.20°) | Relative intensity (%) | No. | 2θ (±0.20°) | Relative intensity (%) |
|---|---|---|---|---|---|
| 1 | 4.53 | 49.70% | 15 | 20.87 | 44.60% |
| 2 | 7.13 | 100.00% | 16 | 21.50 | 8.60% |
| 3 | 9.04 | 26.40% | 17 | 22.29 | 18.20% |
| 4 | 9.58 | 5.1% | 18 | 22.73 | 8.50% |
| 5 | 11.29 | 13.40% | 19 | 23.29 | 6% |
| 6 | 13.57 | 5.4% | 20 | 23.72 | 6.9% |
| 7 | 14.28 | 6.30% | 21 | 24.54 | 26.70% |
| 8 | 15.17 | 7.80% | 22 | 25.29 | 10.30% |
| 9 | 16.30 | 56.90% | 23 | 25.92 | 23.00% |
| 10 | 17.24 | 18.40% | 24 | 26.93 | 5.3% |
| 11 | 17.65 | 19.10% | 25 | 27.32 | 5.5% |
| 12 | 18.12 | 10.40% | 26 | 30.47 | 10.70% |
| 13 | 19.21 | 27.00% | 27 | 31.67 | 7.6% |
| 14 | 20.50 | 58.30% | 28 | 32.44 | 7.9% |

In still another aspect, the present disclosure provides a crystal form composition comprising the crystal forms described above, wherein the crystal form described in the present disclosure accounts for 50% or more, preferably 80% or more, more preferably 90% or more, and most preferably 95% or more of the weight of the crystal form composition.

In still another aspect, the present disclosure provides a pharmaceutical composition comprising a therapeutically effective amount of the compound (crystalline form or amorphous form), the stereoisomer (crystalline form or amorphous form) thereof, the pharmaceutically acceptable salt (crystalline form or amorphous form) of the compound, the solvate (crystalline form or amorphous form) of the compound or the crystal form composition thereof described in the present disclosure. The pharmaceutical composition of the present disclosure may or may not contain a pharmaceutically acceptable excipient. In addition, the pharmaceutical composition of the present disclosure may further comprise one or more additional therapeutic agents.

The present disclosure further provides use of the compound (crystalline form or amorphous form), the stereoisomer (crystalline form or amorphous form) thereof, the pharmaceutically acceptable salt (crystalline form or amorphous form) of the compound, the solvate (crystalline form or amorphous form) of the compound or the crystal form composition thereof, or the pharmaceutical composition thereof of the present disclosure described above in preparing a medicament for preventing or treating various XPO-1-related diseases.

The present disclosure further provides a method for preventing or treating various XPO-1-related diseases. The method includes administering to a mammal, preferably a human, in need of such treatment or prevention a therapeutically effective amount of the compound (crystalline form or amorphous form), the stereoisomer (crystalline form or amorphous form) thereof, the pharmaceutically acceptable salt (crystalline form or amorphous form) of the compound, the solvate (crystalline form or amorphous form) of the compound or the crystal form composition thereof, or the pharmaceutical composition thereof described in the present disclosure.

The present disclosure further provides the compound (crystalline form or amorphous form), the stereoisomer (crystalline form or amorphous form) thereof, the pharmaceutically acceptable salt (crystalline form or amorphous form) of the compound, the solvate (crystalline form or amorphous form) of the compound or the crystal form composition thereof, or the pharmaceutical composition thereof described in the present disclosure, for preventing or treating various XPO-1-related diseases.

The present disclosure further provides use of the compound (crystalline form or amorphous form), the stereoisomer (crystalline form or amorphous form) thereof, the pharmaceutically acceptable salt (crystalline form or amorphous form) of the compound, the solvate (crystalline form or amorphous form) of the compound or the crystal form composition thereof, or the pharmaceutical composition thereof described in the present disclosure in preventing or treating various XPO-1-related diseases. In some embodiments, the prevention or treatment of various XPO-1-related diseases are selected from the group consisting of tumors; in some embodiments, the various XPO-1-related diseases are selected from the group consisting of leukemia and lymphoma.

### Technical Effects

The compound of the present disclosure has cell proliferation inhibitory activity (e.g., against Jurkat cells and/or OCI-LY10 cells); stable *in vitro* liver microsome metabolism and good stability in human whole blood; good *in vivo* pharmacokinetic data (e.g., parameters such as AUC, Cmax, Tmax, or absolute bioavailability), *in vivo* pharmacodynamic data, and *in vivo* safety data (parameters such as brain-to-blood ratio).

The compound of formula I of the present disclosure (specifically, for example, compound 2-A, 3-A, 11-A, 15-A, 21-A, 25-A, 38-A, or 59-A) has one or more of the following properties: stable crystal form (including stability under conditions of high humidity, high temperature, light exposure, acid, or alkali), low sensitivity to light, heat, and humidity, good solubility, and wide druggability prospects. The crystal form demonstrates good pharmacokinetic properties which can be verified by preclinical (e.g., in SD rats and beagle dogs) and clinical trials, and is suitable for use as a medicament. The crystal form of the present disclosure contributes to the solid form of the compound.

### Definitions and Description

Unless otherwise stated, the following terms and phrases used herein are intended to have the following meanings. A certain phrase or term, unless otherwise specifically defined, should not be considered ambiguous or unclear, but construed according to its common meaning. When referring to a trade name, it is intended to refer to its corresponding commercial product or its active ingredient.

It should be noted that in the X-ray powder diffraction pattern, the position and relative intensity of a peak may vary due to measuring instruments, measuring methods/conditions, and other factors. For any particular crystal form, the position of a peak may have an error, and the measurement of 2θ may have an error of ±0.2°. Therefore, this error should be considered when determining each crystal form, and crystal forms within this margin of error are within the scope of the present disclosure.

The phenomenon that lattice planes or edges of some grains in a crystal are predominantly aligned along certain directions and planes is called preferential orientation. In the powder method, the calculation of the relative intensity of the lines requires that the crystals in the powder sample be in a completely random orientation, and if the number of oriented crystals in the sample is increased, the increase in intensity is necessarily caused. This allows a certain correspondence between the intensity of the diffraction lines and the degree of orientation. *(*X-ray Structure Analysis, edited by Qi Jingyu, Tongji University Press, April 2003, 1st edition).

The phenomenon that the grains in the sample have a remarkable tendency toward a certain crystallographic direction is called preferential orientation, and the preferential orientation phenomenon can be easily found by visual inspection for a strongly cleaved substance. For such materials, for example, flaky or needle crystals, preferential orientation readily occurs when test samples are made. Like plate crystals, in a cylindrical sample tube, the finding of flaky crystal faces tends to coincide with the axis of the sample tube. In the flat sample holder of the diffractometer, the normal to the flaky crystal face tends to be perpendicular to the basal plane of the sample holder. The diffraction intensity of the preferentially oriented crystal faces is enhanced abnormally when diffraction data are collected using a standard Bragg-Brentano type diffractometer coupled with θ-2θ. Even though some improvement can be made by making samples many times, it is still difficult to completely overcome the preferential orientation phenomenon *(*Measurement of Crystal Structure by Powder Diffraction Method, edited by Liang Jingkui, Science Press, April 2003, 1st edition).

The preferential orientation influences the measurement result of the crystal structure determined by the powder diffraction method. XRPD measurement results of different batches of the crystal form differ, but this does not prevent those skilled in the art from making a decision as to whether the crystal form is the same.

It should be noted that, for the same crystal form, the position of an endothermic peak in the DSC pattern may vary due to measuring instruments, measuring methods/conditions, and other factors. For any particular crystal form, the position of an endothermic peak may have an error of ±5 °C or ±3 °C. Therefore, this error should be considered when determining each crystal form, and crystal forms within this margin of error are within the scope of the present disclosure.

The term "solvate" refers to a substance formed by combining the compound of formula I of the present disclosure (specifically, for example, 2-A, 3-A, 11-A, 15-A, 21-A, 25-A, 38-A, or 59-A) with a pharmaceutically acceptable solvent. The pharmaceutically acceptable solvent includes water, methanol, ethanol, isopropanol, 1,4-dioxane, tetrahydrofuran, acetic acid, acetonitrile, ethyl acetate, isopropyl acetate, acetone, or the like. The solvate includes both stoichiometric and non-stoichiometric solvates.

When certain structural units or groups in the present application have a covalent bond that is not connected to a specific atom, it means that the covalent bond can be connected to any atom within that structural unit or group, as long as it adheres to the rules of valence bonding.

The term "substituted" means that any one or more hydrogen atoms on a specific atom are substituted with substituents, as long as the valence of the specific atom is normal and the resulting compound is stable. When the substituent is oxo (namely =O), it means that two hydrogen atoms are substituted and oxo is not available on an aromatic group.

The term "optional" or "optionally" means that the subsequently described event or circumstance may, but does not necessarily, occur. The description includes instances where the event or circumstance occurs and instances where it does not. For example, ethyl being "optionally" substituted with halogen means that the ethyl may be unsubstituted (CH₂CH₃), monosubstituted (for example, CH₂CH ₂F), polysubstituted (for example, CHFCH₂F, CH₂CHF₂, and the like), or fully substituted (CF₂CF ₃). It will be appreciated by those skilled in the art that for any groups comprising one or more substituents, any substitutions or substituting patterns that may not exist and/or cannot be synthesized spatially are not introduced.

"One or more" used herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms. For example, C₁₋₃ means that the group may have 1 carbon atom, 2 carbon atoms, or 3 carbon atoms.

When any variable (e.g., R) occurs once or more in the constitution or structure of a compound, the definition of the variable in each case is independent. Therefore, for example, if a group is substituted with 2 R, the definition of each R is independent.

When the number of connecting groups is 0, for example, -(CH₂)₀-, it means that the connecting group is a covalent bond.

When one of the variables is selected from a covalent bond, it means that the two groups connected to it are directly linked. For example, in A-L'-Z, where L' represents a covalent bond, it means that the structure is actually A-Z.

When a bond of a substituent is crosslinked to two atoms on a ring, the substituent can be bonded to any atoms on the ring. For example, structural unit 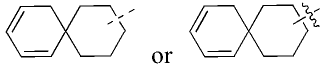 represents that substitution may occur at any one position of cyclohexyl or cyclohexadienyl.

The term "halo-" or "halogen" refers to fluorine, chlorine, bromine, and iodine.

The term "alkyl" refers to hydrocarbyl with a general formula of CₙH₂ₙ₊₁. The alkyl may be linear or branched. For example, the term "C₁-₆ alkyl" refers to alkyl containing 1 to 6 carbon atoms (for example, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). Likewise, the alkyl moieties (namely alkyl) of alkoxy, alkylamino, dialkylamino, alkylsulfonyl and alkylthio have the same definition as those described above. As another example, the term "C₁-₃ alkyl" refers to alkyl containing 1 to 3 carbon atoms (e.g., methyl, ethyl, propyl, and isopropyl). The term "alkoxy" refers to -O-alkyl.

The term "cycloalkyl" refers to a carbon ring that is fully saturated and may exist as a monocyclic, bridged cyclic, or spiro cyclic structure. Unless otherwise indicated, the carbon ring is generally a 3- to 10-membered ring (e.g., a 3-, 4-, 5-, 6-, 7-, 8-, 9-, or 10-membered ring). Non-limiting examples of cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, dicyclo[1.1.1]pentan-1-yl, and the like. For example, C₃₋₄ cycloalkyl includes cyclopropyl and cyclobutyl.

The term "heterocycloalkyl" refers to a fully saturated cyclic group that may exist in the form of a monocyclic, bridged cyclic (including fused ring), or spiro cyclic structure. Unless otherwise indicated, the heterocyclyl is generally a 3- to 7-membered ring (e.g., a 3-, 4-, 5-, 6-, or 7-membered ring) containing 1 to 3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl. Non-limiting examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiapyranyl, morpholinyl, piperazinyl, 1,4-oxathianyl, 1,4-dioxanyl, sulfomorpholinyl, 1,3-dithianyl, and 1,4-dithianyl. Examples of 7-membered heterocycloalkyl include, but are not limited to, azepanyl, oxepanyl, and thiepanyl. Monocyclic heterocycloalkyl having 5 or 6 ring atoms is preferred.

The term "aryl" refers to an all-carbon aromatic monocyclic or fused polycyclic group having a conjugated π-electron system. For example, aryl may have 6-20 carbon atoms, 6-14 carbon atoms, or 6-12 carbon atoms. Non-limiting examples of aryl include, but are not limited to, phenyl, naphthyl, anthryl, 1,2,3,4-tetrahydronaphthalene, and the like.

The term "heteroaryl" or "heteroaromatic ring group" refers to a monocyclic or fused polycyclic system which contains at least one ring atom selected from the group consisting of N, O, and S, with the remaining ring atoms being C, and which has at least one aromatic ring. Preferably, heteroaryl has a single 5- to 8-membered ring (e.g., a 5-, 6-, 7-, or 8-membered ring), or has a plurality of fused rings containing 6 to 14 ring atoms, particularly 6 to 10 ring atoms (e.g., 6-, 7-, 8-, 9-, or 10-membered rings). Non-limiting examples of heteroaryl include, but are not limited to, pyrrolyl, furanyl, thienyl, imidazolyl, oxazolyl, isoxazolyl, pyrazolyl, pyridinyl, pyrimidinyl, pyrazinyl, pyridazinyl, quinolyl, isoquinolyl, tetrazolyl, triazolyl, triazinyl, benzofuranyl, benzothienyl, indolyl, naphthyridinyl, indazolyl, isoindolyl, and the like.

The word "comprise" or variations thereof, such as "comprises" or "comprising" will be understood in an open, non-exclusive sense, i.e., "including but not limited to".

The term "therapeutically effective amount" refers to an amount of the compound disclosed herein for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound disclosed herein composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio. The term "pharmaceutically acceptable excipient" refers to an inert substance administered with active ingredient to facilitate administration of the active ingredient, including, but not limited to, any glidant, sweetener, diluent, preservative, dye/coloring agent, flavor enhancer, surfactant, wetting agent, dispersant, disintegrant, suspending agent, stabilizer, isotonizing agent, solvent or emulsifier acceptable for use in humans or animals (e.g., domesticated animals) as permitted by the National Medical Products Administration, PRC. Non-limiting examples of the excipients include calcium carbonate, calcium phosphate, various sugars and types of starch, cellulose derivatives, gelatin, vegetable oils, and polyethylene glycols.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, water, or the like. The compounds and intermediates disclosed herein may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerization. A specific example of a proton tautomer is an imidazole moiety where a proton can transfer between two ring nitrogens. A valence tautomer includes the interconversion via recombination of some bonding electrons.

The present disclosure also comprises isotopically labeled compounds of the present disclosure which are identical to those recited herein but have one or more atoms replaced by an atom having an atomic mass or mass number different from the atomic mass or mass number generally found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl. Certain isotopically labeled compounds of the present disclosure (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H) may provide certain therapeutic advantages (e.g., increased *in vivo* half-life or reduced dosage) resulting from greater metabolic stability and hence may be preferred in some circumstances in which deuterium substitution may be partial or complete, wherein partial deuterium substitution refers to substitution of at least one hydrogen with at least one deuterium, and all such forms of the compounds are included within the scope of the present disclosure.

The compound of the present disclosure can be asymmetrical, for example, having one or more stereoisomers. Unless otherwise stated, all stereoisomers are included, for example, enantiomers and diastereoisomers. The compound with asymmetrical carbon atoms disclosed herein can be separated in an optically pure form or in a racemic form. The optically pure form can be separated from a racemic mixture or can be synthesized using a chiral raw material or a chiral reagent.

The pharmaceutical composition of the present disclosure can be prepared by combining the compound of the present disclosure with a suitable pharmaceutically acceptable excipient, and can be formulated, for example, into a solid, semisolid, liquid or gaseous formulation such as tablet, pill, capsule, powder, granule, ointment, emulsion, suspension, suppository, injection, inhalant, gel, microsphere, aerosol, and the like.

Typical routes of administration of the crystal form or the pharmaceutical composition thereof described in the present disclosure include, but are not limited to, oral, rectal, local, inhalation, parenteral, sublingual, intravaginal, intranasal, intraocular, intraperitoneal, intramuscular, subcutaneous and intravenous administration.

The pharmaceutical composition of the present disclosure can be manufactured using methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compound of the present disclosure to be formulated into tablets, pills, pastilles, dragees, capsules, liquids, gels, slurries, suspensions, and the like for oral administration to a patient.

Therapeutic dosages of the compound of the present disclosure may be determined by, for example, the specific use of a treatment, the route of administration of the compound, the health and condition of a patient, and the judgment of a prescribing physician. The proportion or concentration of the compound of the present disclosure in a pharmaceutical composition may not be constant and depends on a variety of factors including dosages, chemical properties (e.g., hydrophobicity), and routes of administration.

The term "treat" or "treatment" means administering the compound or formulation described herein to ameliorate or eliminate a disease or one or more symptoms associated with the disease, including:
(i) inhibiting a disease or disease state, i.e., arresting its progression; and
(ii) alleviating a disease or disease state, i.e., causing its regression.

The term "prevent" or "prevention" means administering the compound or formulation described herein to prevent a disease or one or more symptoms associated with the disease, including preventing the occurrence of the disease or disease state in a mammal, particularly when such a mammal is predisposed to the disease state but has not yet been diagnosed with it.

For drugs and pharmacologically active agents, the term "therapeutically effective amount" refers to an amount of a drug or a medicament that is sufficient to provide the desired effect and is non-toxic. The determination of the effective amount varies from person to person. It depends on the age and general condition of a subject, as well as the particular active substance used. The appropriate effective amount in a case may be determined by those skilled in the art in the light of conventional tests.

The therapeutically effective amount of a crystal form of the present disclosure is from about 0.0001 to 20 mg/kg body weight (bw)/day, for example from 0.001 to 10 mg/kg bw/day.

The dosage frequency of the crystal form of the present disclosure depends on needs of an individual patient, e.g., once or twice daily or more times daily. Administration may be intermittent, for example, in a period of several days, the patient receives a daily dose of the crystal forms, and in the following period of several days or more days, the patient does not receive the daily dose of the crystal forms.

The intermediate compounds of the present disclosure can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the specific embodiments of the present disclosure are conducted in a proper solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., amino in the present disclosure). For example, reference may be made to Greene's Protective Groups in Organic Synthesis (4th Ed.) Hoboken, New Jersey: John Wiley & Sons, Inc.

The present disclosure is described in detail below by way of examples, which are not intended to limit the present disclosure in any way.

In some embodiments, the compound disclosed herein may be prepared using the following preparation routes in combination with methods known in the art: 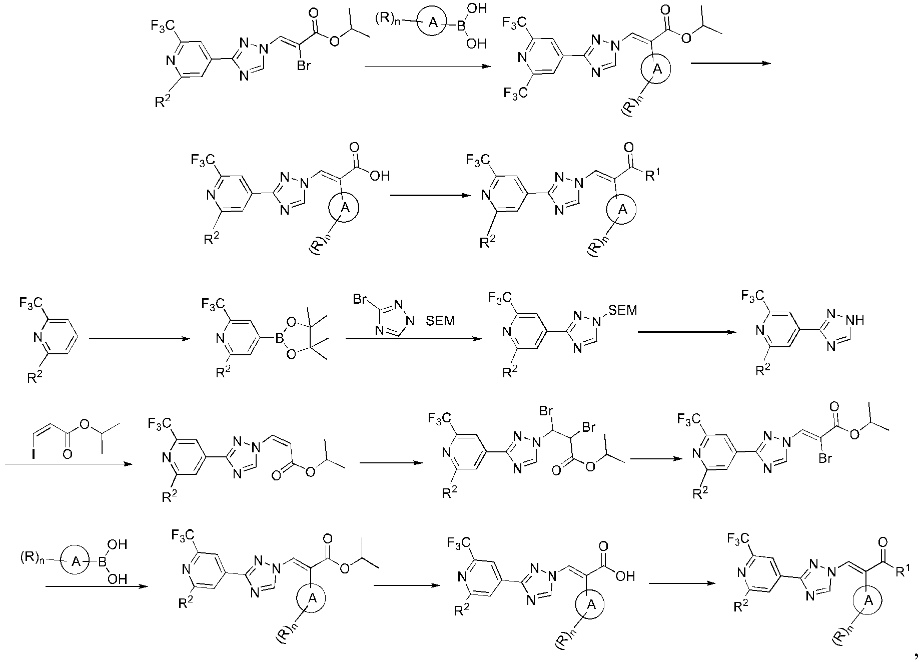 wherein ring A, R, n, R², and R¹ are as defined in the present disclosure.

All the solvents used in the present disclosure are commercially available and can be used without further purification.

### Specific methods of XRPD, DSC and TGA (including instrument model and parameters) X-ray powder diffractometer (XRPD) method of the present disclosure

X-ray powder diffraction (XRPD): Bruker D8 X-ray diffractometer; target tube: Cu. Thermogravimetric analysis (TGA): instrument model: NETZSCH TG 209F3; temperature range: 30-500 °C; heating rate: 10 °C/min.

Differential scanning calorimetry (DSC): instrument model: METTLER TOLEDO DSC3; temperature range: 25-300 °C; heating rate: 10 K/min, N₂ at 50 mL/min.

### Gas chromatography conditions:

| | | |
|---|---|---|
| Instrument | Agilent 7890B gas chromatograph and 7697A headspace sampler | |
| Reagents | Methanol, ethanol, isopropanol, ethyl acetate, isopropyl acetate, and dimethyl sulfoxide, all of chromatographic grade. | |
| Chromatogr aphic column | DB-624 capillary column (60 m × 0.32 mm × 1.8 µm) | |
| Chromatogr aphic conditions | Column temperature: initial temperature of 40 °C, hold for 8 min, increase to 200 °C at a rate of 10 °C/min, then increase to 220 °C at a rate of 20 °C/min and hold for 3 min. | |
| | Injection port temperature: 250 °C | Detector (FID) temperature: 260 °C |
| | Headspace vial equilibration temperature: 90 °C | Headspace vial equilibration time: 30 min |
| | Transfer line temperature: 100 °C | Quantitative loop temperature: 110 °C |
| | Column flow rate: 1.5 mL/min | Split ratio: 20:1 |
| | Carrier gas: nitrogen | Injection volume: 1 mL |

| | | |
|---|---|---|
| (1) Blank solvent: dimethyl sulfoxide. (2) Test sample solution: About 0.1 g of the test sample is accurately weighed out, and placed into a headspace vial; 2 mL of dimethyl sulfoxide is accurately added, and the mixture is sealed, and mixed well to obtain the test sample solution. (3) Reference solution 1: Appropriate amounts of methanol, ethanol, isopropanol, and ethyl acetate are accurately weighed out and dissolved in dimethyl sulfoxide, and the mixture is quantitatively diluted to prepare a mixed solution containing about 150 µg of methanol, 250 µg of ethanol, 250 µg of isopropanol, and 250 µg of ethyl acetate per 1 mL. 2 mL of the mixed solution is accurately measured out, placed into a headspace vial, and sealed to obtain the reference solution. | | |

Reference solution 2: An appropriate amount of isopropyl acetate is accurately weighed out and dissolved in dimethyl sulfoxide, and the mixture is quantitatively diluted to prepare a solution containing about 250 µg of isopropyl acetate per 1 mL. 2 mL of the mixed solution is accurately measured out, placed into a headspace vial, and sealed to obtain the reference solution.

### Single crystal detection method

Diffraction intensity data is collected using a Bruker D8 venture diffractometer with CuKα radiation as the light source and φ/ω scanning as the scanning mode.

### BRIEF DESCRIPTION OF THE DRAWINGS

FIG. 1 is an XRPD pattern of a crystal form A of compound 21-A;
FIG. 2 is a TGA profile of a crystal form A of compound 21-A;
FIG. 3 is an XRPD pattern of a crystal form B of compound 21-A;
FIG. 4 is a TGA profile of a crystal form B of compound 21-A;
FIG. 5 is an XRPD pattern of a crystal form C of compound 21-A;
FIG. 6 is a TGA profile of a crystal form C of compound 21-A;
FIG. 7 is an XRPD pattern of a crystal form D of compound 21-A;
FIG. 8 is a TGA profile of a crystal form D of compound 21-A;
FIG. 9 is an XRPD pattern of a crystal form E of compound 21-A;
FIG. 10 is a TGA profile of a crystal form E of compound 21-A;
FIG. 11 is an XRPD pattern of a crystal form F of compound 21-A;
FIG. 12 is a TGA profile of a crystal form F of compound 21-A;
FIG. 13 is a projection of a three-dimensional molecular structure of a single crystal G of compound 21-A;
FIG. 14 is an XRPD pattern of a crystal form A of compound 3-A;
FIG. 15 is a TGA profile of a crystal form A of compound 3-A;
FIG. 16 is a DSC curve of a crystal form A of compound 3-A;
FIG. 17 is an XRPD pattern of a crystal form B of compound 3-A;
FIG. 17-A is a TGA profile of a crystal form B of compound 3-A;
FIG. 18 is an XRPD pattern of a crystal form C of compound 3-A;
FIG. 18-A is a TGA profile of a crystal form C of compound 3-A;
FIG. 19 is an XRPD pattern of a crystal form A of compound 15-A;
FIG. 20 is a TGA profile of a crystal form A of compound 15-A;
FIG. 21 is a DSC curve of a crystal form A of compound 15-A;
FIG. 22 is an XRPD pattern of a crystal form B of compound 15-A; and
FIG. 23 is an XRPD pattern of a crystal form A of compound 25-A.

The following abbreviations are used in the present disclosure:
Pd(dppf)Cl₂ represents [1,1'-bis(diphenylphosphino)ferrocene]palladium dichloride; DMF represents N,N-dimethylformamide; DABCO represents triethylenediamine; THF represents tetrahydrofuran; SEM represents trimethylsilylethoxymethyl.

For clarity, the present disclosure is further described with the following examples, which are, however, not intended to limit the scope of the present disclosure. All reagents used in the present disclosure are commercially available and can be used without further purification.

### DETAILED DESCRIPTION

### Example 1

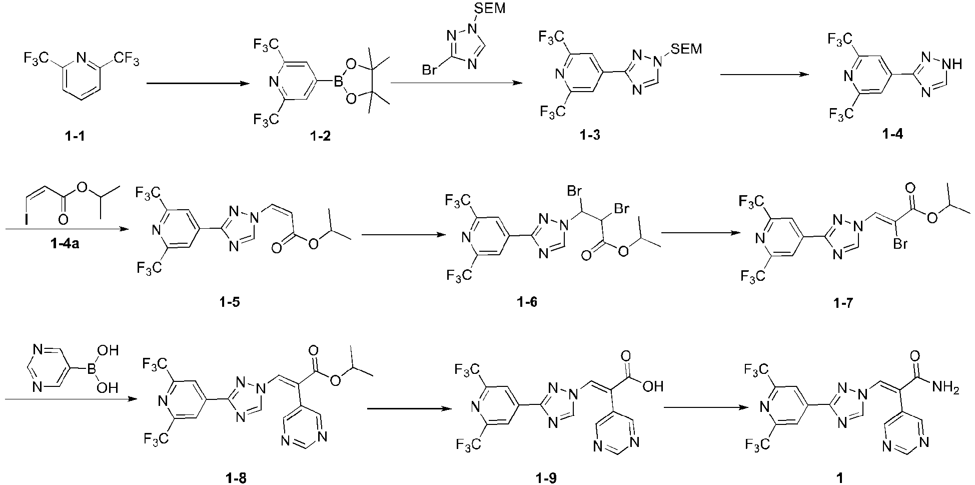

### (1) Preparation of compound 1-2

Bis(pinacolato)diboron (2.13 g), 4,4'-di-tert-butyl-2,2'-bipyridine (18.7 mg), and (1,5-cyclooctadiene)(methoxy)iridium(I) dimer (23.1 mg) were added to cyclohexane (30 mL). Under nitrogen atmosphere, the mixture was stirred for 10 min, and 3,5-bis(trifluoromethyl)pyridine (compound **1-1,** 3.0 g) was added. The resulting mixture was stirred at 55 °C under nitrogen atmosphere. After the reaction was completed, the reaction solution was quenched with ice water (20 mL) and extracted with ethyl acetate (30 mL × 3). The organic phase was collected, dried over anhydrous sodium sulfate, and filtered, and the filtrate was evaporated to dryness under reduced pressure to give compound **1-2,** which was directly used in the next step.

### (2) Preparation of compound 1-3

Compound **1-2** (4.7 g) was added to ethylene glycol dimethyl ether (50 mL), and 3-bromo-1-((2-(trimethylsilyl)ethoxy)methyl)-1H-1,2,4-triazole (3.9 g) and an aqueous solution (10 mL) of potassium carbonate (5.8 g) were added. The mixture was stirred at room temperature under nitrogen atmosphere. After 1 h, tetrakis(triphenylphosphine)palladium (805.8 mg) was added, and the resulting mixture was stirred at 90 °C under nitrogen atmosphere. After the reaction was completed, the reaction solution was quenched with ice water (40 mL) and extracted with ethyl acetate (100 mL). The organic phase was collected and washed with brine (50 mL × 2). The organic layer was dried over anhydrous sodium sulfate and filtered, and the filtrate was evaporated to dryness under reduced pressure to give compound **1-3,** which was directly used in the next step.

### (3) Preparation of compound 1-4

Compound **1-3** (5.7 g) was dispersed in 4 M HCl/dioxane (50 mL), and the mixture was stirred at 60 °C. After the reaction was completed, the reaction solution was slowly poured into an ice saturated sodium bicarbonate solution. The resulting mixture was adjusted to pH = 6-7 with sodium bicarbonate and extracted with ethyl acetate (100 mL × 3). The organic phase was washed with saturated brine (100 mL), dried over anhydrous sodium sulfate, concentrated under reduced pressure until no liquid flowed out, and purified by column chromatography to give compound **1-4.**

### (4) Preparation of compound 1-5

Compound **1-4** (900 mg) was dispersed in DMF (20 mL), and DABCO (716 mg) was added. The mixture was stirred at 20-25 °C for 30 min and then cooled to 0-10 °C, and compound **1-4a** (1.15 g) was added dropwise. After addition, the resulting mixture was stirred at 20-25 °C for 1.5 h. After the reaction was completed, the reaction solution was poured into water (50 mL), and the resulting mixture was extracted with ethyl acetate (50 mL × 2). The organic phases were combined, dried over anhydrous sodium sulfate, and purified by column chromatography to give compound 1-5 (750 mg).

### (5) Preparation of compound 1-6

Compound **1-5** (750 mg) was added to dichloromethane (20 mL), and the mixture was cooled to 0 °C. Liquid bromine (605 mg) was added dropwise within 15 min. After addition, the mixture was slowly heated to room temperature and stirred for 4 h. After the reaction was completed, the reaction mixture was poured into ice water (30 mL), and the resulting mixture was extracted with dichloromethane (30 mL × 3). The organic phases were combined, washed with a saturated sodium bisulfite solution (30 mL), dried over anhydrous sodium sulfate, concentrated under reduced pressure until no liquid flowed out, and purified by column chromatography to give compound **1-6.**

### (6) Preparation of compound 1-7

Compound **1-6** (1.05 g) was added to THF (20 mL), and the mixture was cooled to 0 °C. Triethylamine (383.5 mg) was added dropwise. After addition, the mixture was slowly heated to room temperature and reacted for 12 h. After the reaction was completed, the reaction solution was diluted with water (20 mL) and extracted with ethyl acetate (30 mL × 3). The organic phases were combined, dried over anhydrous sodium sulfate, and purified by column chromatography to give compound **1-7.**

### (7) Preparation of compound 1-8

Compound 1-7 (700 mg), 5-pyrimidineboronic acid (220 mg), potassium acetate (435.7 mg), and Pd(dppf)Cl₂ (54 mg) were added to dioxane (20 mL) and water (2 mL). The mixture was heated to 90 °C and reacted under nitrogen atmosphere. After the reaction was completed, the reaction solution was diluted with water (20 mL) and extracted with ethyl acetate (30 mL × 3). The organic phase was collected, dried over anhydrous sodium sulfate, and filtered, and the filtrate was evaporated to dryness under reduced pressure and purified by column chromatography to give compound **1-8** (200 mg).

### (8) Preparation of compound 1-9

Compound **1-8** (200 mg) was dissolved in tetrahydrofuran (15 mL), a solution of sodium hydroxide (33.9 mg) and water (15 mL) was added dropwise in an ice-water bath, and the mixture was reacted at room temperature overnight. The reaction solution was adjusted to acidity (pH 3-5) with hydrochloric acid, extracted with ethyl acetate (30 mL × 3), dried over anhydrous sodium sulfate, and filtered, and the filtrate was evaporated to dryness under reduced pressure to give compound **1-9,** which was directly used in the next step.

### (9) Preparation of Example 1

Compound **1-9** (182 mg) was added to tetrahydrofuran (15 mL), N-methylmorpholine (85.6 mg) and isobutyl chloroformate (115.5 mg) were added in an ice-water bath, and the mixture was stirred for 30 min. Ammonium hydroxide (88.8 mg) was added dropwise, and the mixture was reacted in an ice-water bath for 10 min. Water was added to quench the reaction, and the resulting mixture was extracted with ethyl acetate (30 mL × 3), dried over anhydrous sodium sulfate, concentrated until no liquid flowed out, and purified by preparative liquid chromatography to give Example 1 (13 mg). ESI-MS: m/z = 430.26 [M+H] ⁺. ¹H NMR (500 MHz, DMSO-*d*₆) δ 9.22 (d, *J* = 9.8 Hz, 2H), 8.74 (s, 2H), 8.45 (s, 1H), 8.07 (s, 2H), 7.68 (s, 1H), 7.44 (s, 1H).

### Example 2

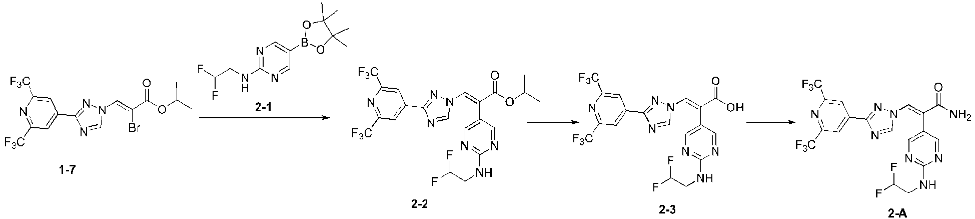

### (1) Preparation of compound 2-2:

Compounds **1-7** (394 mg) and **2-1** (285 mg) were dispersed in 1,4-dioxane (8 mL), and potassium acetate (244 mg), Pd(dppf)Cl₂ (29 mg), and water (0.8 mL) were added. The mixture was heated to 90 °C and reacted under nitrogen atmosphere. After the reaction was completed, the reaction solution was concentrated under reduced pressure and purified by column chromatography to give compound **2-2.**

### (2) Preparation of compound 2-3:

Compound **2-2** (100 mg) was dissolved in tetrahydrofuran (5 mL), a solution of lithium hydroxide hydrate (38.1 mg) and water (5 mL) was added dropwise in an ice-water bath, and the mixture was reacted at room temperature for 2 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **2-3,** which was directly used in the next step.

### (3) Preparation of compound 2-A:

Compound **2-3** (91 mg) was dispersed in tetrahydrofuran (5 mL), N-methylmorpholine (36 mg) and isobutyl chloroformate (49 mg) were added in an ice-water bath, and the mixture was stirred for 30 min. Ammonium hydroxide (38 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **2-A** (41 mg). ESI-MS: m/z =509.32 [M+H] ⁺.

¹H NMR (500 MHz, DMSO-*d*₆) δ 9.14 (s, 1H), 8.25 (d, *J* = 10.4 Hz, 5H), 7.76 (t, *J* = 6.2 Hz, 1H), 7.58 (s, 1H), 7.43 (s, 1H), 6.09 (tt, *J =* 56.5, 4.2 Hz, 1H), 3.74 (dddd, *J =* 19.0, 14.9, 8.2, 5.2 Hz, 2H).

### Example 3

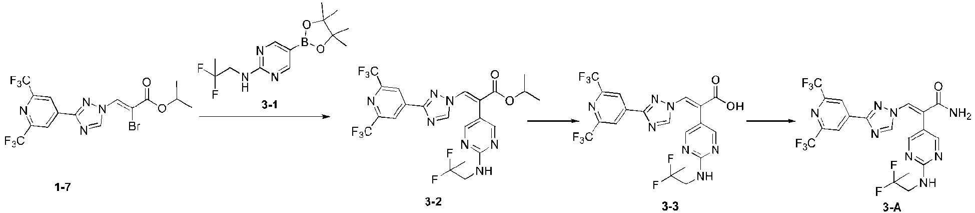

### (1) Preparation of compound 3-2:

Compounds **1-7** (394 mg) and **3-1** (299 mg) were dispersed in 1,4-dioxane (8 mL), and potassium acetate (244 mg), Pd(dppf)Cl₂ (29 mg), and water (0.8 mL) were added. The mixture was heated to 90 °C and reacted under nitrogen atmosphere. After the reaction was completed, the reaction solution was concentrated under reduced pressure and purified by column chromatography to give compound **3-2.**

### (2) Preparation of compound 3-3:

Compound **3-2** (360 mg) was dissolved in tetrahydrofuran (5 mL), a solution of lithium hydroxide hydrate (133.6 mg) and water (5 mL) was added dropwise in an ice-water bath, and the mixture was reacted at room temperature for 2 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **3-3,** which was directly used in the next step.

### (3) Preparation of compound 3-A:

Compound **3-3** (335 mg) was dispersed in tetrahydrofuran (10 mL), N-methylmorpholine (129 mg) and isobutyl chloroformate (175 mg) were added in an ice-water bath, and the mixture was stirred for 30 min. Ammonium hydroxide (134 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **3-A** (90 mg). ESI-MS: m/z =523.37 [M+H] ⁺.

¹H NMR (500 MHz, DMSO-*d*₆) δ 9.14 (s, 1H), 8.26 (s, 2H), 8.21 (d, *J =* 5.5 Hz, 3H), 7.76 (t, *J* = 6.6 Hz, 1H), 7.57 (s, 1H), 7.45 (s, 1H), 3.83 (td, *J* = 13.6, 6.6 Hz, 2H), 1.61 (t, *J* = 19.0 Hz, 3H).

### Example 4

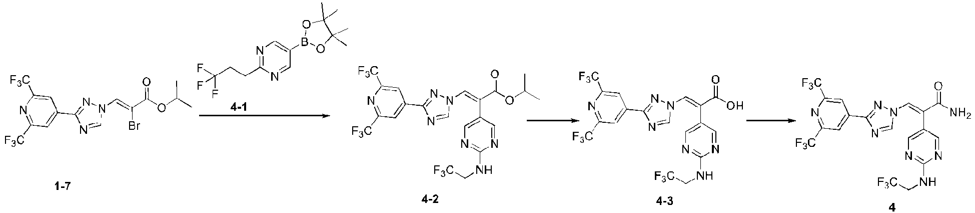

### (1) Preparation of compound 4-2:

Compounds **1-7** (300 mg) and **4-1** (230 mg) were dispersed in 1,4-dioxane (8 mL), and potassium acetate (186.6 mg), Pd(dppf)Cl₂ (46 mg), and water (0.8 mL) were added. The mixture was heated to 90 °C and reacted under nitrogen atmosphere. After the reaction was completed, the reaction solution was concentrated under reduced pressure and purified by column chromatography to give compound **4-2.**

### (2) Preparation of compound 4-3:

Compound **4-2** (302 mg) was dissolved in tetrahydrofuran (10 mL), a solution of lithium hydroxide hydrate (44.6 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **4-3,** which was directly used in the next step.

### (3) Preparation of compound 4:

Compound **4-3** (280 mg) was dispersed in tetrahydrofuran (15 mL), N-methylmorpholine (107 mg) and isobutyl chloroformate (145 mg) were added in an ice-water bath, and the mixture was stirred for 30 min. Ammonium hydroxide (111 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **4** (30 mg). ESI-MS: m/z =527.17 [M+H] ⁺.

¹H NMR (500 MHz, DMSO-*d*₆) δ 9.16 (s, 1H), 8.24 (d, *J* = 4.6 Hz, 5H), 7.99 (t, *J* = 6.7 Hz, 1H), 7.57 (s, 1H), 7.45 (s, 1H), 4.18 (q, *J* = 8.7, 8.3 Hz, 2H).

### Example 5

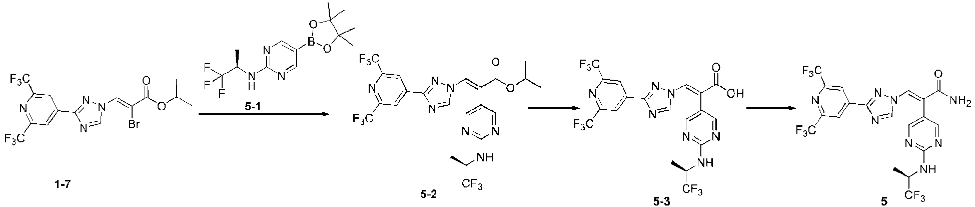

### (1) Preparation of compound 5-2:

Compounds **1-7** (300 mg) and **5-1** (241 mg) were dispersed in 1,4-dioxane (8 mL), and potassium acetate (187 mg), Pd(dppf)Cl₂ (23 mg), and water (0.8 mL) were added. The mixture was heated to 90 °C and reacted under nitrogen atmosphere. After the reaction was completed, the reaction solution was concentrated under reduced pressure and purified by column chromatography to give compound **5-2.**

### (2) Preparation of compound 5-3:

Compound **5-2** (200 mg) was dissolved in tetrahydrofuran (10 mL), a solution of lithium hydroxide hydrate (29 mg) and water (10 mL) was added dropwise in an ice-water bath, and the mixture was reacted at 0-10 °C for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **5-3,** which was directly used in the next step.

### (3) Preparation of compound 5:

Compound **5-3** (185 mg) was dispersed in tetrahydrofuran (15 mL), N-methylmorpholine (69 mg) and isobutyl chloroformate (94 mg) were added in an ice-water bath, and the mixture was stirred for 30 min. Ammonium hydroxide (72 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **5** (50 mg). ESI-MS: m/z =541.25 [M+H] ⁺.

¹H NMR (500 MHz, DMSO-*d*₆) δ 9.17 (d, *J* = 3.5 Hz, 1H), 8.25 (q, *J* = 3.8 Hz, 5H), 7.96 (dd, *J =* 9.3, 3.2 Hz, 1H), 7.58 (s, 1H), 7.47 (s, 1H), 5.02 - 4.86 (m, 1H), 1.36 (dd, *J =* 7.3, 3.2 Hz, 3H).

### Example 6

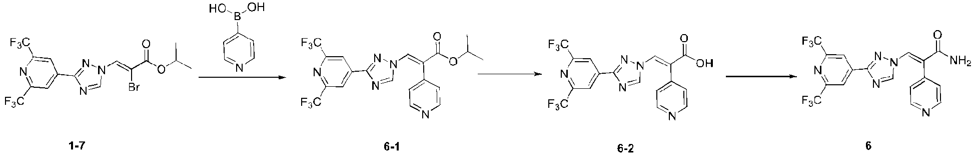

### (1) Preparation of compound 6-1:

Compound **1-7** (200 mg) and pyridine-4-boronic acid (62.35 mg) were dispersed in 1,4-dioxane (10 mL), and potassium acetate (124.4 mg), Pd(dppf)Cl₂ (15.5 mg), and water (1 mL) were added. The mixture was heated to 90 °C and reacted under nitrogen atmosphere. After the reaction was completed, the reaction solution was concentrated under reduced pressure and purified by column chromatography to give compound **6-1.**

### (2) Preparation of compound 6-2:

Compound **6-1** (150 mg) was dissolved in tetrahydrofuran (10 mL), a solution of lithium hydroxide hydrate (67.14 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **6-2,** which was directly used in the next step.

### (3) Preparation of compound 6:

Compound **6-2** (137.4 mg) was dispersed in tetrahydrofuran (10 mL), N-methylmorpholine (64.74 mg) and isobutyl chloroformate (87.41 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **6** (10 mg). ESI-MS: m/z =429.19 [M+H] ⁺.

¹H NMR (500 MHz, DMSO-*d*₆) δ 9.02 (s, 1H), 8.65 (d, *J* = 5.0 Hz, 2H), 8.26 (s, 1H), 8.10 (s, 2H), 7.66 (s, 1H), 7.32 (d, *J =* 5.0 Hz, 2H), 7.23 (s, 1H).

### Example 7

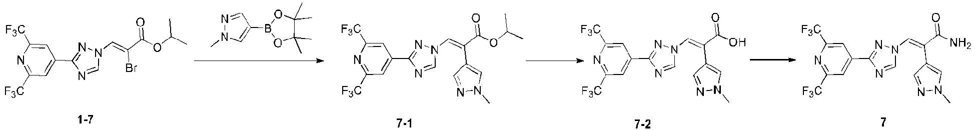

### (1) Preparation of compound 7-1:

Compound **1-7** (300 mg) and 1-methylpyrazole-4-boronic acid pinacol ester (158.3 mg) were dispersed in 1,4-dioxane (8 mL), and potassium acetate (186.6 mg), Pd(dppf)Cl₂ (46 mg), and water (0.8 mL) were added. The mixture was heated to 90 °C and reacted under nitrogen atmosphere. After the reaction was completed, the reaction solution was concentrated under reduced pressure to give compound **7-1,** which was directly used in the next step.

### (2) Preparation of compound 7-2:

Compound **7-1** (298 mg) was dissolved in tetrahydrofuran (10 mL), a solution of lithium hydroxide hydrate (52.87 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **7-2,** which was directly used in the next step.

### (3) Preparation of compound 7:

Compound 7-2 (270 mg) was dispersed in tetrahydrofuran (15 mL), N-methylmorpholine (127.45 mg) and isobutyl chloroformate (154.41 mg) were added in an ice-water bath, and the mixture was stirred for 30 min. Ammonium hydroxide (132.3 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **7** (77 mg). ESI-MS: m/z =432.18 [M+H] ⁺.

¹H NMR (500 MHz, DMSO-*d*₆) δ 8.87 (s, 1H), 8.48 (s, 2H), 7.84 (s, 1H), 7.73 (s, 1H), 7.58 (s, 1H), 7.54 (s, 1H), 7.26 (d, *J =* 0.9 Hz, 1H), 3.85 (s, 3H).

### Example 8

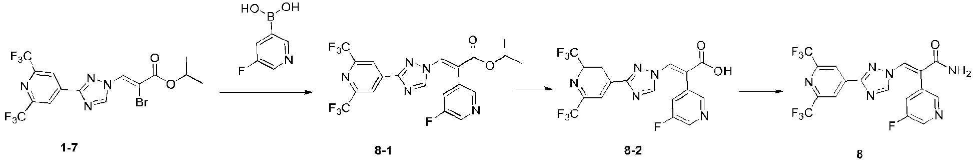

### (1) Preparation of compound 8-1:

Compound **1-7** (200 mg) and 5-fluoro-3-pyridineboronic acid (75.03 mg) were dispersed in 1,4-dioxane (10 mL), and potassium acetate (124.4 mg), Pd(dppf)Cl₂ (15.5 mg), and water (1 mL) were added. The mixture was heated to 90 °C and reacted under nitrogen atmosphere. After the reaction was completed, the reaction solution was concentrated under reduced pressure and purified by column chromatography to give compound **8-1.**

### (2) Preparation of compound 8-2:

Compound **8-1** (150 mg) was dissolved in tetrahydrofuran (10 mL), a solution of lithium hydroxide hydrate (65.1 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **8-2,** which was directly used in the next step.

### (3) Preparation of compound 8:

Compound **8-2** (138.65 mg) was dispersed in tetrahydrofuran (10 mL), N-methylmorpholine (62.71 mg) and isobutyl chloroformate (84.68 mg) were added in an ice-water bath, and the mixture was stirred for 30 min. Ammonium hydroxide (65.1 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **8** (43 mg). ESI-MS: m/z =447.14 [M+H] ⁺.

¹H NMR (500 MHz, DMSO-*d*₆) δ 9.15 (s, 1H), 8.63 (d, *J* = 2.8 Hz, 1H), 8.39 (s, 1H), 8.34 (d, *J =* 1.8 Hz, 1H), 8.09 (s, 2H), 7.76 (dt, *J =* 10.0, 2.2 Hz, 1H), 7.67 (s, 1H), 7.28 (s, 1H).

### Example 9

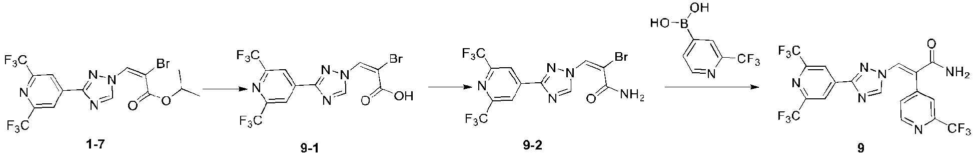

### (1) Preparation of compound 9-1:

Compound **1-7** (500 mg) was dissolved in tetrahydrofuran (12 mL), a solution of sodium hydroxide (84.5 mg) and water (12 mL) was added dropwise in an ice-water bath, and the mixture was reacted at room temperature overnight. The reaction solution was adjusted to pH 3-5 with hydrochloric acid, extracted with ethyl acetate (30 mL × 2), dried over anhydrous sodium sulfate, and filtered, and the filtrate was evaporated to dryness under reduced pressure to give compound **9-1,** which was directly used in the next step.

### (2) Preparation of compound 9-2:

Compound **9-1** (455 mg) was dispersed in tetrahydrofuran (15 mL), N-methylmorpholine (213.7 mg) and isobutyl chloroformate (288.6 mg) were added in an ice-water bath, and the mixture was stirred for 30 min. Ammonium hydroxide (222 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, and concentrated until no liquid flowed out to give compound **9-2.** ESI-MS: m/z =430.04/432.06 [M+H] ⁺.

### (3) Preparation of compound 9:

Compound **9-2** (180 mg) and 4-boronic acid-2-trifluoromethylpyridine (95.9 mg) were dispersed in 1,4-dioxane (6 mL), and potassium acetate (123 mg), Pd(dppf)Cl₂ (15.3 mg), and water (0.6 mL) were added. The mixture was heated to 90 °C and reacted under nitrogen atmosphere. After the reaction was completed, the reaction solution was concentrated under reduced pressure and purified by preparative liquid chromatography to give compound **9** (18 mg). ESI-MS: m/z = 497.09[M+H] ⁺. ¹H NMR (500 MHz, DMSO-*d*₆) δ 9.21 (s, 1H), 8.83 (d, *J* = 4.9 Hz, 1H), 8.39 (s, 1H), 7.99 (s, 2H), 7.89 (s, 1H), 7.69 (s, 1H), 7.62 (d, *J* = 4.9 Hz, 1H), 7.29 (s, 1H).

### Example 10

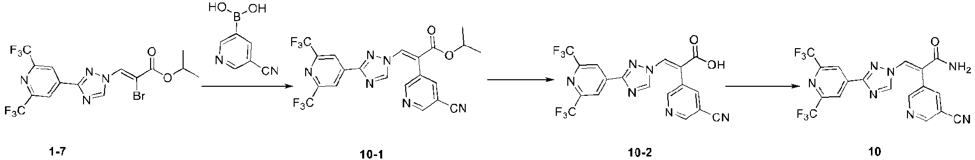

### (1) Preparation of compound 10-1:

Compound **1-7** (200 mg) and 5-cyano-3-pyridinylboronic acid (75.03 mg) were dispersed in 1,4-dioxane (10 mL), and potassium acetate (124.4 mg), Pd(dppf)Cl₂ (15.5 mg), and water (1 mL) were added. The mixture was heated to 90 °C and reacted under nitrogen atmosphere. After the reaction was completed, the reaction solution was concentrated under reduced pressure and purified by column chromatography to give compound **10-1.**

### (2) Preparation of compound 10-2:

Compound **10-1** (173 mg) was dissolved in tetrahydrofuran (10 mL), a solution of lithium hydroxide hydrate (73.12 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **10-2,** which was directly used in the next step.

### (3) Preparation of compound 10:

Compound **10-2** (159 mg) was dispersed in tetrahydrofuran (10 mL), N-methylmorpholine (70.8 mg) and isobutyl chloroformate (95.6 mg) were added in an ice-water bath, and the mixture was stirred for 30 min. Ammonium hydroxide (73.5 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **10** (23 mg). ESI-MS: m/z = 454.20 [M+H] ⁺.

¹H NMR (500 MHz, DMSO-*d*₆) δ 9.21 (s, 1H), 9.09 (d, *J* = 2.0 Hz, 1H), 8.76 (d, *J* = 2.1 Hz, 1H), 8.45 (s, 1H), 8.36 (t, *J =* 2.1 Hz, 1H), 8.05 (s, 2H), 7.70 (s, 1H), 7.30 (s, 1H).

### Example 11

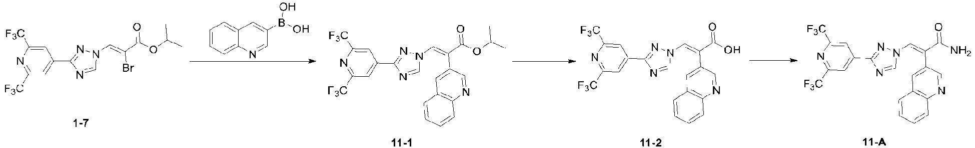

### (1) Preparation of compound 11-1:

Compound 1-7 (300 mg) and 3-quinolineboronic acid (131.61 mg) were dispersed in 1,4-dioxane (8 mL), and potassium acetate (186.6 mg), Pd(dppf)Cl₂ (46 mg), and water (0.8 mL) were added. The mixture was heated to 90 °C and reacted under nitrogen atmosphere. After the reaction was completed, the reaction solution was concentrated under reduced pressure to give compound **11-1,** which was directly used in the next step.

### (2) Preparation of compound 11-2:

Compound **11-1** (328 mg) was dissolved in tetrahydrofuran (10 mL), a solution of lithium hydroxide hydrate (52.87 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **11-2,** which was directly used in the next step.

### (3) Preparation of compound 11-A:

Compound **11-2** (301 mg) was dispersed in tetrahydrofuran (15 mL), N-methylmorpholine (127.45 mg) and isobutyl chloroformate (154.41 mg) were added in an ice-water bath, and the mixture was stirred for 30 min. Ammonium hydroxide (132.3 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **11-A** (30 mg); ESI-MS: m/z =479.31 [M+H] ⁺.

¹H NMR (500 MHz, DMSO-*d*₆) δ 9.17 (s, 1H), 8.76 (d, *J* = 2.2 Hz, 1H), 8.43 (s, 1H), 8.30 (d, *J =* 2.2 Hz, 1H), 8.08 (d, *J* = 8.4 Hz, 1H), 8.01 (dd, *J* = 8.1, 1.4 Hz, 1H), 7.88 (s, 2H), 7.84 (ddd, *J =* 8.5, 6.9, 1.5 Hz, 1H), 7.70 - 7.62 (m, 2H), 7.36 (s, 1H).

### Example 12

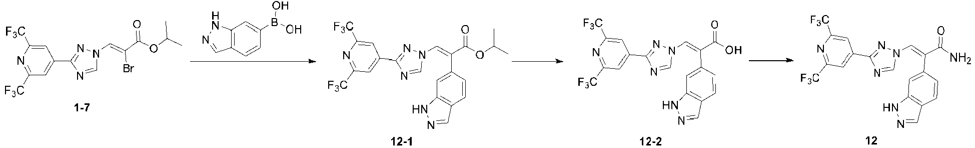

### (1) Preparation of compound 12-1:

Compound **1-7** (300 mg) and indazole-6-boronic acid (123.22 mg) were dispersed in 1,4-dioxane (8 mL), and potassium acetate (186.6 mg), Pd(dppf)Cl₂ (46 mg), and water (0.8 mL) were added. The mixture was heated to 90 °C and reacted under nitrogen atmosphere. After the reaction was completed, the reaction solution was concentrated under reduced pressure to give compound **12-1,** which was directly used in the next step.

### (2) Preparation of compound 12-2:

Compound **12-1** (321 mg) was dissolved in tetrahydrofuran (10 mL), a solution of lithium hydroxide hydrate (52.87 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **12-2,** which was directly used in the next step.

### (3) Preparation of compound 12:

Compound **12-2** (295 mg) was dispersed in tetrahydrofuran (15 mL), N-methylmorpholine (127.45 mg) and isobutyl chloroformate (154.41 mg) were added in an ice-water bath, and the mixture was stirred for 30 min. Ammonium hydroxide (132.3 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **12** (40 mg). ESI-MS: m/z =468.22 [M+H] ⁺.

¹H NMR (500 MHz, DMSO-*d*₆) δ 13.11 (s, 1H), 8.63 (s, 1H), 8.19 (s, 1H), 8.13 (t, *J* = 1.3 Hz, 1H), 8.09 (s, 2H), 7.82 (d, *J* = 8.2 Hz, 1H), 7.62 (s, 1H), 7.47 - 7.43 (m, 1H), 7.11 (s, 1H), 6.95 (dd, *J* = 8.3, 1.4 Hz, 1H).

### Example 13

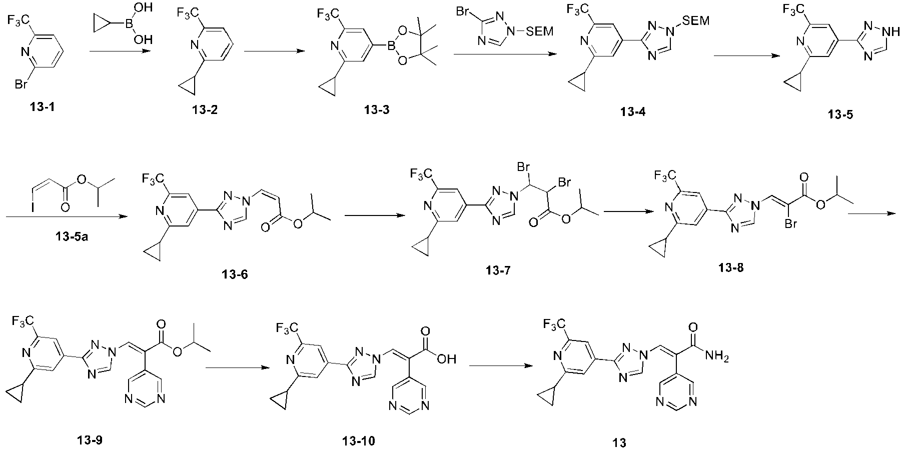

### (1) Preparation of compound 13-2:

Compound **13-1** (15 g), cyclopropylboronic acid (11.4 g), potassium carbonate (27.5 g), and Pd(dppf)Cl₂ (2.4 g) were dispersed in dioxane (150 mL) and water (15 mL). The mixture was heated to 90 °C and reacted under nitrogen atmosphere. After the reaction was completed, the reaction solution was diluted with water (1000 mL) and extracted with ethyl acetate (100 mL × 3). The organic phase was collected, dried over anhydrous sodium sulfate, and filtered, and the filtrate was evaporated to dryness under reduced pressure and purified by column chromatography to give compound **13-2.**

### (2) Preparation of compound 13-3:

Bis(pinacolato)diboron (6.51 g), 4,4'-di-tert-butyl-2,2'-bipyridine (76.16 mg), and (1,5-cyclooctadiene)(methoxy)iridium(I) dimer (70.82 mg) were dispersed in cyclohexane (80 mL). The mixture was stirred for 10 min under nitrogen atmosphere, and compound **13-2** (8.0 g) was added. The resulting mixture was stirred at 55 °C under nitrogen atmosphere. After the reaction was completed, the reaction solution was quenched with ice water (80 mL) and extracted with ethyl acetate (80 mL × 3). The organic phase was collected, dried over anhydrous sodium sulfate, and filtered, and the filtrate was evaporated to dryness under reduced pressure to give compound **13-3,** which was directly used in the next step.

### (3) Preparation of compound 13-4:

Compound **13-3** (10.7 g) was dispersed in ethylene glycol dimethyl ether (80 mL), and 3-bromo-1-((2-(trimethylsilyl)ethoxy)methyl)-1H-1,2,4-triazole (10.5 g) and an aqueous solution (16 mL) of potassium carbonate (14.18 g) were added. The mixture was stirred at room temperature under nitrogen atmosphere. After 1 h, tetrakis(triphenylphosphine)palladium (1.97 g) was added, and the resulting mixture was stirred at 90 °C under nitrogen atmosphere. After the reaction was completed, the reaction solution was quenched with ice water (60 mL) and extracted with ethyl acetate (100 mL × 3). The organic phase was collected and washed with brine (60 mL × 2). The organic layer was dried over anhydrous sodium sulfate and filtered, and the filtrate was evaporated to dryness under reduced pressure to give compound **13-4,** which was directly used in the next step.

### (4) Preparation of compound 13-5:

Compound **13-4** (6.0 g) was dispersed in a solution of hydrogen chloride in 1,4-dioxane (4.0 M, 50 mL), and the mixture was stirred at 60 °C until the reaction was completed. The reaction solution was slowly poured into an ice saturated sodium bicarbonate solution. The resulting mixture was adjusted to pH = 7-8 with sodium bicarbonate and extracted with ethyl acetate (100 mL × 3). The organic phase was washed with saturated brine (100 mL), dried over anhydrous sodium sulfate, concentrated under reduced pressure until no liquid flowed out, and purified by column chromatography to give compound **13-5.**

### (5) Preparation of compound 13-6:

Compound **13-5** (4 g) was dispersed in DMF (20 mL) and dioxane (20 mL), and DABCO (3.5 g) was added. The mixture was stirred at 20-25 °C for 30 min and then cooled to 0-10 °C, and compound **13-5a** (5.6 g) was added dropwise. After addition, the resulting mixture was stirred at 20-25 °C for 1.5 h. After the reaction was completed, the reaction solution was poured into water (100 mL), and the resulting mixture was extracted with ethyl acetate (100 mL × 2). The organic phases were combined, dried over anhydrous sodium sulfate, and purified by column chromatography to give compound **13-6** (3.0 g).

### (6) Preparation of compound 13-7:

Compound **13-6** (3 g) was dispersed in dichloromethane (30 mL), and the mixture was cooled to 0 °C. Liquid bromine (2.6 g) was added dropwise within 15 min. After addition, the mixture was slowly heated to room temperature and stirred for 6 h. After the reaction was completed, the reaction mixture was poured into ice water (40 mL), and the resulting mixture was extracted with dichloromethane (40 mL × 3). The organic phases were combined, washed with a saturated sodium bisulfite solution (30 mL), dried over anhydrous sodium sulfate, concentrated under reduced pressure until no liquid flowed out, and purified by column chromatography to give compound **13-7.**

### (7) Preparation of compound 13-8:

Compound **13-7** (4.3 g) was dispersed in tetrahydrofuran (40 mL), and the mixture was cooled to 0 °C. Triethylamine (1.65 g) was added dropwise. After addition, the mixture was slowly heated to room temperature and reacted for 6 h. After the reaction was completed, the reaction solution was diluted with water (40 mL) and extracted with ethyl acetate (40 mL × 3). The organic phases were combined, dried over anhydrous sodium sulfate, and purified by column chromatography to give compound **13-8** (2 g).

### (8) Preparation of compound 13-9:

Compound **13-8** (300 mg), 5-pyrimidineboronic acid (100 mg), potassium acetate (198 mg), and Pd(dppf)Cl₂ (24.6 mg) were dispersed in dioxane (8 mL) and water (0.8 mL). The mixture was heated to 90 °C and reacted under nitrogen atmosphere. After the reaction was completed, the reaction solution was diluted with water (15 mL) and extracted with ethyl acetate (20 mL × 3). The organic phase was collected, dried over anhydrous sodium sulfate, and filtered, and the filtrate was evaporated to dryness under reduced pressure and purified by column chromatography to give compound **13-9** (200 mg).

### (9) Preparation of compound 13-10:

Compound **13-9** (200 mg) was dissolved in tetrahydrofuran (8 mL), a solution of sodium hydroxide (36 mg) and water (8 mL) was added dropwise in an ice-water bath, and the mixture was reacted at room temperature overnight. The reaction solution was adjusted to pH 3-5 with hydrochloric acid, extracted with ethyl acetate (20 mL × 3), dried over anhydrous sodium sulfate, and filtered, and the filtrate was evaporated to dryness under reduced pressure to give compound **13-10,** which was directly used in the next step.

### (10) Preparation of compound 13:

Compound **13-10** (181 mg) was dispersed in tetrahydrofuran (10 mL), N-methylmorpholine (91 mg) and isobutyl chloroformate (123 mg) were added in an ice-water bath, and the mixture was stirred for 30 min. Ammonium hydroxide (95 mg) was added dropwise, and the mixture was reacted in an ice-water bath for 10 min. Water was added to quench the reaction, and the resulting mixture was extracted with ethyl acetate (20 mL × 3), dried over anhydrous sodium sulfate, concentrated until no liquid flowed out, and purified by preparative liquid chromatography to give compound 13 (57 mg). ESI-MS: m/z = 402.27 [M+H] ⁺.

¹H NMR (500 MHz, DMSO-*d*₆) δ 9.24 (s, 1H), 9.13 (s, 1H), 8.73 (s, 2H), 8.42 (s, 1H), 7.69 (s, 1H), 7.65 (s, 1H), 7.49 (s, 1H), 7.41 (s, 1H), 2.28 (tt, *J=* 8.3, 4.7 Hz, 1H), 1.10 (dq, *J* = 6.6, 3.8 Hz, 2H), 0.99 - 0.91 (m, 2H).

### Example 14

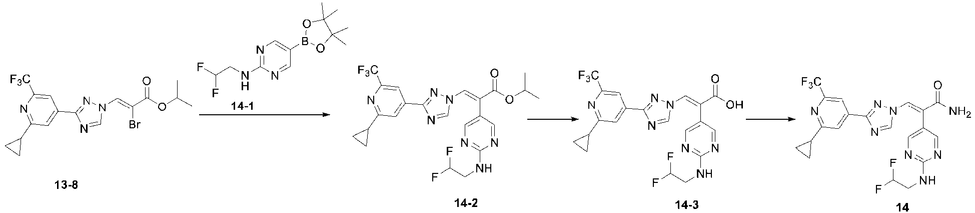

### (1) Preparation of compound 14-2:

Compounds **13-8** (300 mg) and **14-1** (228 mg) were dispersed in 1,4-dioxane (15 mL), and potassium acetate (196 mg), Pd(dppf)Cl₂ (22 mg), and water (1.5 mL) were added. The mixture was heated to 90 °C and reacted under nitrogen atmosphere. After the reaction was completed, the reaction solution was concentrated under reduced pressure and purified by column chromatography to give compound **14-2.**

### (2) Preparation of compound 14-3:

Compound **14-2** (100 mg) was dissolved in tetrahydrofuran (5 mL), a solution of lithium hydroxide hydrate (40.1 mg) and water (5 mL) was added dropwise in an ice-water bath, and the mixture was reacted at room temperature for 2 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **14-3,** which was directly used in the next step.

### (3) Preparation of compound 14:

Compound **14-3** (60 mg) was dispersed in tetrahydrofuran (5 mL), N-methylmorpholine (26 mg) and isobutyl chloroformate (35 mg) were added in an ice-water bath, and the mixture was stirred for 30 min. Ammonium hydroxide (27 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound 14 (10 mg). ESI-MS: m/z =481.3 [M+H] ⁺.

¹H NMR (500 MHz, DMSO-*d*₆) δ 9.05 (s, 1H), 8.21 (s, 3H), 7.84 (d, *J =* 1.3 Hz, 1H), 7.76 (t, *J =* 6.2 Hz, 1H), 7.71 (d, *J =* 1.3 Hz, 1H), 7.55 (s, 1H), 7.40 (s, 1H), 6.10 (tt, *J =* 56.5, 4.2 Hz, 1H), 3.74 (tdd, *J* = 15.0, 6.2, 4.1 Hz, 2H), 2.30 (tt, *J* = 8.1, 4.7 Hz, 1H), 1.08 (dt, *J =* 8.1, 3.2 Hz, 2H), 0.99 - 0.95 (m, 2H).

### Example 15

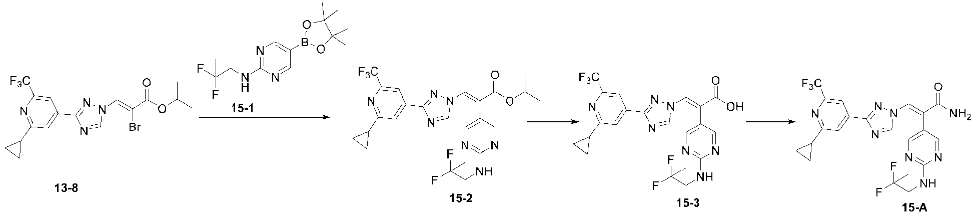

### (1) Preparation of compound 15-2:

Compounds **13-8** (371 mg) and **15-1** (299 mg) were dispersed in 1,4-dioxane (55 mL), and potassium acetate (244 mg), Pd(dppf)Cl₂ (30 mg), and water (1.5 mL) were added. The mixture was heated to 90 °C and reacted under nitrogen atmosphere. After the reaction was completed, the reaction solution was concentrated under reduced pressure and purified by column chromatography to give compound **15-2.**

### (2) Preparation of compound 15-3:

Compound **15-2** (340 mg) was dissolved in tetrahydrofuran (5 mL), a solution of lithium hydroxide hydrate (132.7 mg) and water (5 mL) was added dropwise in an ice-water bath, and the mixture was reacted at room temperature for 2 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **15-3,** which was directly used in the next step.

### (3) Preparation of compound 15-A:

Compound **15-3** (312 mg) was dispersed in tetrahydrofuran (5 mL), N-methylmorpholine (127 mg) and isobutyl chloroformate (172 mg) were added in an ice-water bath, and the mixture was stirred for 30 min. Ammonium hydroxide (132 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **15-A** (70 mg). ESI-MS: m/z =495.39 [M+H] ⁺.

¹H NMR (500 MHz, DMSO-*d*₆) δ 9.05 (s, 1H), 8.20 (d, *J* = 3.2 Hz, 3H), 7.84 (d, *J* = 1.2 Hz, 1H), 7.77 (t, *J* = 6.6 Hz, 1H), 7.73 (d, *J =* 1.3 Hz, 1H), 7.54 (s, 1H), 7.42 (s, 1H), 3.83 (td, *J* = 13.6, 6.6 Hz, 2H), 2.29 (tt, *J =* 8.3, 4.7 Hz, 1H), 1.61 (t, *J* = 19.0 Hz, 3H), 1.08 (dt, *J =* 8.0, 3.2 Hz, 2H), 1.00 - 0.94 (m, 2H).

### Example 16

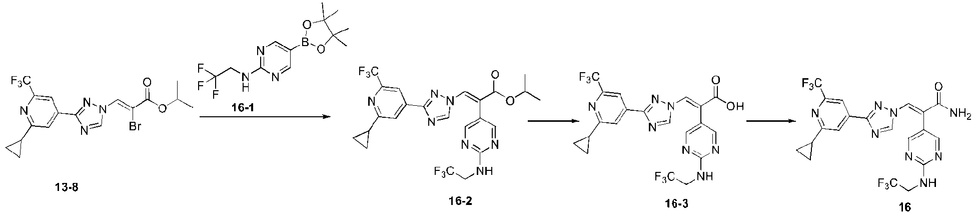

### (1) Preparation of compound 16-2:

Compounds **13-8** (400 mg) and **16-1** (327 mg) were dispersed in 1,4-dioxane (15 mL), and potassium acetate (264 mg), Pd(dppf)Cl₂ (65.7 mg), and water (1.5 mL) were added. The mixture was heated to 90 °C and reacted under nitrogen atmosphere. After the reaction was completed, the reaction solution was concentrated under reduced pressure and purified by column chromatography to give compound **16-2.**

### (2) Preparation of compound 16-3:

Compound **16-2** (300 mg) was dissolved in tetrahydrofuran (10 mL), a solution of lithium hydroxide hydrate (46.5 mg) and water (10 mL) was added dropwise in an ice-water bath, and the mixture was reacted at room temperature for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **16-3,** which was directly used in the next step.

### (3) Preparation of compound 16:

Compound **16-3** (276 mg) was dispersed in tetrahydrofuran (15 mL), N-methylmorpholine (112 mg) and isobutyl chloroformate (151 mg) were added in an ice-water bath, and the mixture was stirred for 30 min. Ammonium hydroxide (116 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **16** (58.4 mg). ESI-MS: m/z = 499.28[M+H] ⁺.

¹H NMR (500 MHz, DMSO-*d*₆) δ 9.07 (s, 1H), 8.23 (d, *J* = 6.1 Hz, 3H), 8.00 (t, *J* = 6.7 Hz, 1H), 7.83 (d, *J* = 1.3 Hz, 1H), 7.70 (d, *J =* 1.3 Hz, 1H), 7.55 (s, 1H), 7.42 (s, 1H), 4.18 (qd, *J* = 9.5, 6.6 Hz, 2H), 2.28 (tt, *J* = 8.1, 4.7 Hz, 1H), 1.08 (dt, *J* = 8.0, 3.2 Hz, 2H), 0.99 - 0.94 (m, 2H).

### Example 17

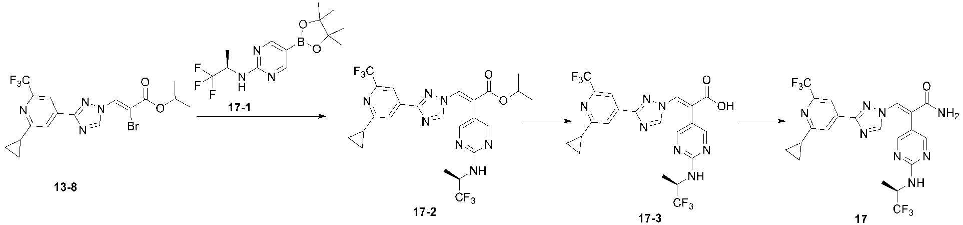

### (1) Preparation of compound 17-2:

Compounds **13-8** (400 mg) and **17-1** (342 mg) were dispersed in 1,4-dioxane (15 mL), and potassium acetate (264 mg), Pd(dppf)Cl₂ (65.7 mg), and water (1.5 mL) were added. The mixture was heated to 90 °C and reacted under nitrogen atmosphere. After the reaction was completed, the reaction solution was concentrated under reduced pressure and purified by column chromatography to give compound **17-2.**

### (2) Preparation of compound 17-3:

Compound **17-2** (400 mg) was dissolved in tetrahydrofuran (10 mL), and a solution of lithium hydroxide hydrate (91 mg) and water (10 mL) was added dropwise in an ice-water bath. The temperature was controlled at 0-10 °C, and the mixture was reacted for 3 h. The reaction solution was adjusted to pH 3-5 with hydrochloric acid, extracted with ethyl acetate (20 mL × 2), dried over anhydrous sodium sulfate, and filtered, and the filtrate was evaporated to dryness under reduced pressure to give compound **17-3,** which was directly used in the next step.

### (3) Preparation of compound 17:

Compound **17-3** (370 mg) was dispersed in tetrahydrofuran (15 mL), N-methylmorpholine (146 mg) and isobutyl chloroformate (197 mg) were added in an ice-water bath, and the mixture was stirred for 30 min. Ammonium hydroxide (151 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound 17 (49 mg). ESI-MS: m/z = 513.33[M+H] ⁺.

¹H NMR (500 MHz, DMSO-*d*₆) δ 9.07 (s, 1H), 8.23 (d, *J* = 7.2 Hz, 3H), 7.96 (d, *J* = 9.1 Hz, 1H), 7.85 (s, 1H), 7.71 (d, *J =* 1.3 Hz, 1H), 7.54 (s, 1H), 7.43 (s, 1H), 4.95 (h, *J* = 7.2 Hz, 1H), 2.29 (tt, *J* = 8.3, 4.7 Hz, 1H), 1.35 (d, *J=* 7.0 Hz, 3H), 1.07 (dt, *J* = 8.3, 3.1 Hz, 2H), 0.98 (dq, *J* = 7.0, 3.4 Hz, 2H).

### Example 18

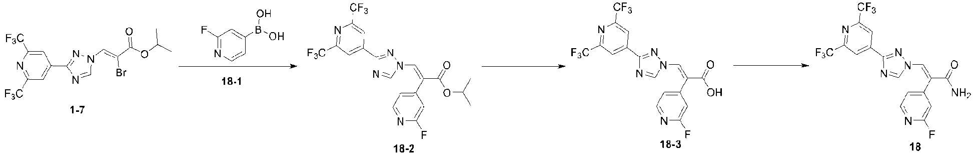

### (1) Preparation of compound 18-2:

Compound **1-7** (300 mg) and 2-fluoropyridine-4-boronic acid (**18-1,** 107 mg) were dispersed in 1,4-dioxane (8 mL), and potassium acetate (186.6 mg), Pd(dppf)Cl₂ (23.2 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **18-2.**

### (2) Preparation of compound 18-3:

Compound **18-2** (240 mg) was dissolved in tetrahydrofuran (10 mL), a solution of lithium hydroxide hydrate (61.7 mg) and water (10 mL) was added dropwise in an ice-water bath, and the mixture was reacted at 0-5 °C for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **18-3,** which was directly used in the next step.

### (3) Preparation of compound 18:

Compound **18-3** (219 mg) was dispersed in tetrahydrofuran (15 mL), N-methylmorpholine (99 mg) and isobutyl chloroformate (133.8 mg) were added in an ice-water bath, and the mixture was stirred for 30 min. Ammonium hydroxide (102.9 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **18** (57 mg). ESI-MS: m/z =447.17 [M+H] ⁺.

¹H NMR (500 MHz, DMSO-*d*₆) δ 9.13 (s, 1H), 8.34 (s, 1H), 8.30 (d, *J* = 5.1 Hz, 1H), 8.09 (s, 2H), 7.68 (s, 1H), 7.26 (dt, *J* = 5.1, 1.7 Hz, 1H), 7.21 (s, 1H), 7.19 (d, *J* = 1.6 Hz, 1H).

### Example 19

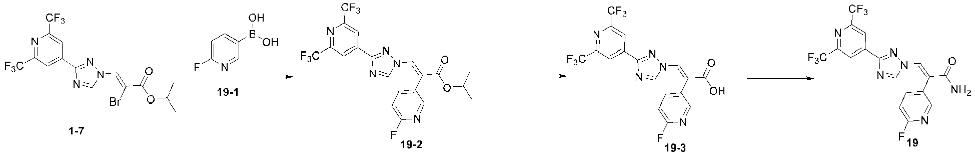

### (1) Preparation of compound 19-2:

Compound **1-7** (200 mg) and 2-fluoropyridine-5-boronic acid (**19-1,** 71.5 mg) were dispersed in 1,4-dioxane (10 mL), and potassium acetate (124.4 mg), Pd(dppf)Cl₂ (15.5 mg), and water (1 mL) were added. The mixture was heated to 90 °C and reacted under nitrogen atmosphere. After the starting materials were consumed completely as detected by TLC, the reaction solution was concentrated under reduced pressure and purified by column chromatography to give compound **19-2.**

### (2) Preparation of compound 19-3:

Compound **19-2** (205 mg) was dissolved in tetrahydrofuran (10 mL), a solution of lithium hydroxide hydrate (35.4 mg) and water (10 mL) was added dropwise in an ice-water bath, and the mixture was reacted at room temperature for 3 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **19-3,** which was directly used in the next step.

### (3) Preparation of compound 19:

Compound **19-3** (137.4 mg) was dispersed in tetrahydrofuran (10 mL), N-methylmorpholine (64.74 mg) and isobutyl chloroformate (87.41 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **19** (70 mg). ESI-MS: m/z =447.15 [M+H] ⁺.

¹H NMR (500 MHz, DMSO-*d*₆) δ 9.14 (s, 1H), 8.37 (s, 1H), 8.14 (d, *J* = 2.4 Hz, 1H), 8.11 (s, 2H), 7.89 (td, *J* = 8.1, 2.5 Hz, 1H), 7.64 (s, 1H), 7.27 (dd, *J* = 8.4, 2.7 Hz, 2H).

### Example 20

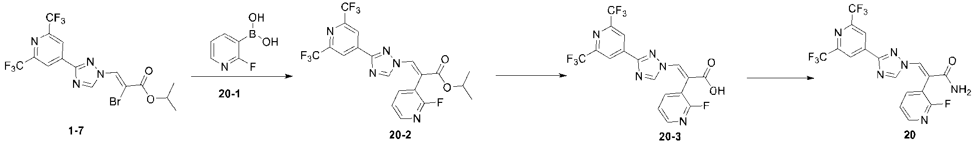

### (1) Preparation of compound 20-2:

Compound **1-7** (200 mg) and 2-fluoropyridine-3-boronic acid (**20-1,** 71.5 mg) were dispersed in 1,4-dioxane (10 mL), and potassium acetate (124.4 mg), Pd(dppf)Cl₂ (15.5 mg), and water (1 mL) were added. The mixture was heated to 90 °C and reacted under nitrogen atmosphere. After the starting materials were consumed completely as detected by TLC, the reaction solution was concentrated under reduced pressure and purified by column chromatography to give compound **20-2.**

### (2) Preparation of compound 20-3:

Compound **20-2** (205 mg) was dissolved in tetrahydrofuran (10 mL), a solution of lithium hydroxide hydrate (35.4 mg) and water (10 mL) was added dropwise in an ice-water bath, and the mixture was reacted at room temperature for 3 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **20-3,** which was directly used in the next step.

### (3) Preparation of compound 20:

Compound **20-3** (137.4 mg) was dispersed in tetrahydrofuran (10 mL), N-methylmorpholine (64.74 mg) and isobutyl chloroformate (87.41 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **20** (15 mg). ESI-MS: m/z =447.16 [M+H] ⁺.

### Example 21

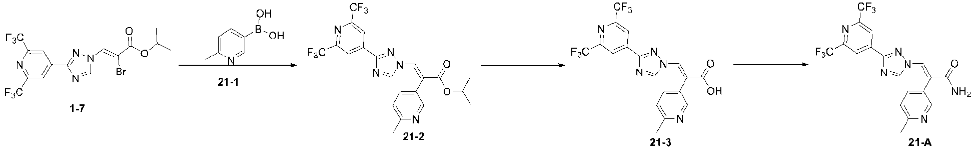

### (1) Preparation of compound 21-2:

Compound 1-7 (300 mg) and 6-methylpyridine-3-boronic acid (**21-1,** 104 mg) were dispersed in 1,4-dioxane (8 mL), and potassium acetate (186.6 mg), Pd(dppf)Cl₂ (23.2 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **21-2.**

### (2) Preparation of compound 21-3:

Compound **21-2** (120 mg) was dissolved in tetrahydrofuran (10 mL), a solution of lithium hydroxide hydrate (20.7 mg) and water (10 mL) was added dropwise in an ice-water bath, and the mixture was reacted at 0-5 °C for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **21-3,** which was directly used in the next step.

### (3) Preparation of compound 21-A:

Compound **21-3** (109 mg) was dispersed in tetrahydrofuran (15 mL), N-methylmorpholine (50 mg) and isobutyl chloroformate (67.5 mg) were added in an ice-water bath, and the mixture was stirred for 30 min. Ammonium hydroxide (51.9 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **21-A** (42 mg). ESI-MS: m/z =443.17[M+H] ⁺.

¹H NMR (500 MHz, DMSO-*d*₆) δ 9.05 (s, 1H), 8.32 (d, *J* = 2.1 Hz, 1H), 8.29 (s, 1H), 8.11 (s, 2H), 7.61 (s, 1H), 7.57 (dd, *J* = 7.9, 2.3 Hz, 1H), 7.34 (d, *J =* 7.9 Hz, 1H), 7.23 (s, 1H), 2.54 (s, 3H).

### Example 22

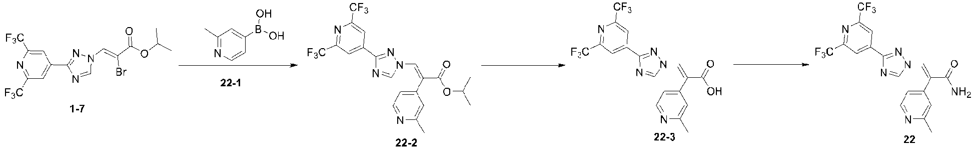

### (1) Preparation of compound 22-2:

Compound **1-7** (300 mg) and 2-methylpyridine-4-boronic acid (**22-1,** 104 mg) were dispersed in 1,4-dioxane (8 mL), and potassium acetate (186.6 mg), Pd(dppf)Cl₂ (23.2 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **22-2.**

### (2) Preparation of compound 22-3:

Compound **22-2** (170 mg) was dissolved in tetrahydrofuran (10 mL), a solution of lithium hydroxide hydrate (29 mg) and water (10 mL) was added dropwise in an ice-water bath, and the mixture was reacted at 0-5 °C for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **22-3,** which was directly used in the next step.

### (3) Preparation of compound 22

Compound **22-3** (155 mg) was dispersed in tetrahydrofuran (15 mL), N-methylmorpholine (70.8 mg) and isobutyl chloroformate (95.6 mg) were added in an ice-water bath, and the mixture was stirred for 30 min. Ammonium hydroxide (73.5 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **22** (57 mg). ESI-MS: m/z =443.16 [M+H] ⁺.

¹H NMR (500 MHz, DMSO-*d*₆) δ 8.99 (s, 1H), 8.49 (dd, *J* = 5.0, 0.8 Hz, 1H), 8.23 (s, 1H), 8.12 (s, 2H), 7.64 (s, 1H), 7.21 - 7.19 (m, 1H), 7.17 (s, 1H), 7.06 (dd, *J* = 5.1, 1.6 Hz, 1H), 2.49 (s, 3H).

### Example 23

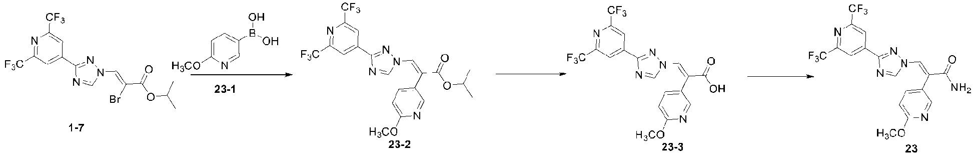

### (1) Preparation of compound 23-2

Compound **1-7** (300 mg) and 6-methoxy-3-pyridineboronic acid (**23-1**, 116.23 mg) were dispersed in 1,4-dioxane (10 mL), and potassium acetate (186.7 mg), Pd(dppf)Cl₂ (23.2 mg), and water (1 mL) were added. The mixture was heated to 90 °C and reacted under nitrogen atmosphere. After the starting materials were consumed completely as detected by TLC, the reaction solution was concentrated under reduced pressure and purified by column chromatography to give compound **23-2.**

### (2) Preparation of compound 23-3

Compound **23-2** (306 mg) was dissolved in tetrahydrofuran (10 mL), a solution of lithium hydroxide hydrate (53.2 mg) and water (10 mL) was added dropwise in an ice-water bath, and the mixture was reacted at room temperature for 3 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **23-3,** which was directly used in the next step.

### (3) Preparation of compound 23

Compound **23-3** (289 mg) was dispersed in tetrahydrofuran (10 mL), N-methylmorpholine (128.3 mg) and isobutyl chloroformate (173.2 mg) were added in an ice-water bath, and the mixture was stirred for 30 min. Ammonium hydroxide (133.1 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound 23 (100 mg). ESI-MS: m/z =459.22 [M+H] ⁺.

¹H NMR (500 MHz, DMSO-*d*₆) δ 9.04 (s, 1H), 8.25 (s, 1H), 8.17 (s, 2H), 8.07 - 8.04 (m, 1H), 7.58 (dd, *J =* 8.5, 2.5 Hz, 2H), 7.23 (s, 1H), 6.90 (dd, *J =* 8.6, 0.7 Hz, 1H), 3.91 (s, 3H).

### Example 24

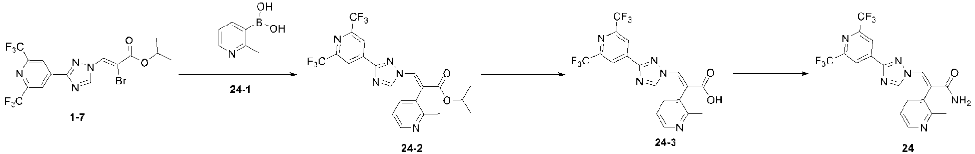

### (1) Preparation of compound 24-2

Compound **1-7** (300 mg) and 2-methylpyridine-3-boronic acid (**24**-**1**, 104 mg) were dispersed in 1,4-dioxane (8 mL), and potassium acetate (186.6 mg), Pd(dppf)Cl₂ (23.2 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **24-2.**

### (1) Preparation of compound 24-3

Compound **24-2** (150 mg) was dissolved in tetrahydrofuran (10 mL), a solution of lithium hydroxide hydrate (38.9 mg) and water (10 mL) was added dropwise in an ice-water bath, and the mixture was reacted at 0-25 °C for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **24-3,** which was directly used in the next step.

### (3) Preparation of compound 24

Compound **24-3** (137 mg) was dispersed in tetrahydrofuran (15 mL), N-methylmorpholine (62.5 mg) and isobutyl chloroformate (84.4 mg) were added in an ice-water bath, and the mixture was stirred for 30 min. Ammonium hydroxide (64.9 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **24** (82 mg). ESI-MS: m/z = 443.28[M+H] ⁺.

¹H NMR (500 MHz, DMSO-*d*₆) δ 9.07 (s, 1H), 8.51 (dd, *J* = 4.9, 1.7 Hz, 1H), 8.34 (s, 1H), 8.05 (s, 2H), 7.59 (s, 1H), 7.51 (dd, *J =* 7.6, 1.7 Hz, 1H), 7.29 (dd, *J =* 7.6, 4.9 Hz, 1H), 7.18 (s, 1H), 2.28 (s, 3H).

### Example 25

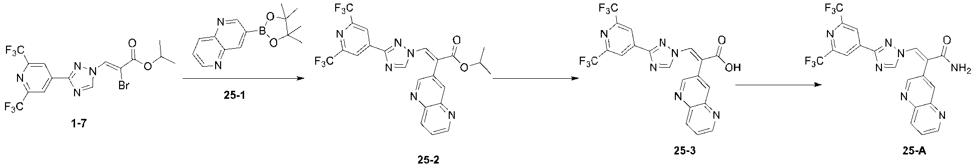

### (1) Preparation of compound 25-2:

Compounds **1-7** (300 mg) and **25-1** (195 mg) were dispersed in 1,4-dioxane (8 mL), and potassium acetate (186.6 mg), Pd(dppf)Cl₂ (46 mg), and water (0.8 mL) were added. The mixture was heated to 90 °C and reacted under nitrogen atmosphere. After the starting materials were consumed completely as detected by TLC, the reaction solution was concentrated under reduced pressure to give compound **25-2.**

### (2) Preparation of compound 25-3:

Compound **25-2** (240 mg) was dissolved in tetrahydrofuran (10 mL), a solution of lithium hydroxide hydrate (57.8 mg) and water (10 mL) was added dropwise in an ice-water bath, and the mixture was reacted at room temperature for 3 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **25-3,** which was directly used in the next step.

### (3) Preparation of compound 25-A:

Compound **25-3** (220 mg) was dispersed in tetrahydrofuran (15 mL), N-methylmorpholine (93 mg) and isobutyl chloroformate (125 mg) were added in an ice-water bath, and the mixture was stirred for 30 min. Ammonium hydroxide (96 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **25-A** (86 mg). ESI-MS: m/z = 480.24 [M+H] ⁺.

¹H NMR (500 MHz, DMSO-*d*₆) δ 9.22 (s, 1H), 9.04 (dd, *J =* 4.2, 1.7 Hz, 1H), 8.89 (d, *J =* 2.1 Hz, 1H), 8.53 - 8.45 (m, 2H), 8.34 (d, *J* = 2.1 Hz, 1H), 7.88 (dd, *J =* 8.5, 4.2 Hz, 1H), 7.82 (s, 2H), 7.70 (s, 1H), 7.38 (s, 1H).

### Example 26

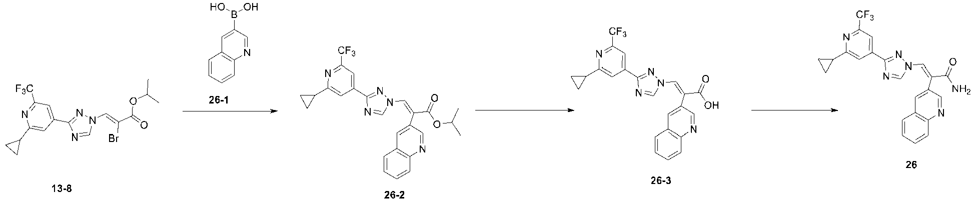

### (1) Preparation of compound 26-2:

Compound **13-8** (200 mg) and 3-quinolineboronic acid (**26-1,** 76.6 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **26-2.**

### (2) Preparation of compound 26-3:

Compound **26-2** (120 mg) was dissolved in tetrahydrofuran (4 mL), a solution of lithium hydroxide hydrate (30.6 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **26-3,** which was directly used in the next step.

### (3) Preparation of compound 26:

Compound **26-3** (100 mg) was dispersed in tetrahydrofuran (5 mL), N-methylmorpholine (44.5 mg) and isobutyl chloroformate (60.1 mg) were added in an ice-water bath, and the mixture was stirred for 30 min. Ammonium hydroxide (46.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **26** (40 mg); ESI-MS: m/z =451.20[M+H] ⁺.

¹H NMR (500 MHz, DMSO-*d*₆) δ 9.07 (s, 1H), 8.74 (d, *J =* 2.2 Hz, 1H), 8.40 (s, 1H), 8.29 (d, *J =* 2.2 Hz, 1H), 8.09 (dd, *J =* 8.4, 1.1 Hz, 1H), 8.01 (dd, *J =* 8.1, 1.5 Hz, 1H), 7.82 (ddd, *J =* 8.5, 6.9, 1.5 Hz, 1H), 7.64 (ddd, *J =* 8.1, 6.9, 1.2 Hz, 2H), 7.48 (d, *J =* 1.4 Hz, 1H), 7.33 (s, 1H), 7.29 (d, *J* = 1.3 Hz, 1H), 2.07 (tt, *J =* 8.1, 4.7 Hz, 1H), 1.06 - 0.97 (m, 2H), 0.83 - 0.75 (m, 2H).

### Example 27

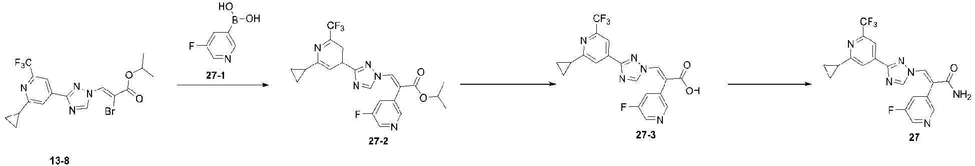

### (1) Preparation of compound 27-2:

Compound **13-8** (200 mg) and 5-fluoro-3-pyridineboronic acid (**27-1,** 76.1 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **27-2.**

### (2) Preparation of compound 27-3:

Compound **27-2** (120 mg) was dissolved in tetrahydrofuran (4 mL), a solution of lithium hydroxide hydrate (32.8 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **27-3,** which was directly used in the next step.

### (3) Preparation of compound 27:

Compound **27-3** (100 mg) was dispersed in tetrahydrofuran (5 mL), N-methylmorpholine (48.6 mg) and isobutyl chloroformate (65.6 mg) were added in an ice-water bath, and the mixture was stirred for 30 min. Ammonium hydroxide (50.4 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **27** (40 mg). ESI-MS: m/z =419.22 [M+H] ⁺.

¹H NMR (500 MHz, DMSO-*d*₆) δ 9.05 (s, 1H), 8.63 (d, *J =* 2.8 Hz, 1H), 8.34 (d, *J =* 19.3 Hz, 2H), 7.79 - 7.69 (m, 2H), 7.64 (s, 1H), 7.51 (s, 1H), 7.25 (s, 1H), 2.28 (tt, *J=* 8.4, 4.7 Hz, 1H), 1.10 (dd, *J =* 8.0, 3.0 Hz, 2H), 0.93 (dd, *J =* 4.7, 2.6 Hz, 2H).

### Example 28

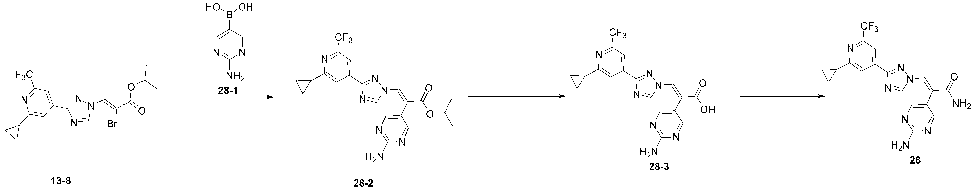

### (1) Preparation of compound 28-2:

Compound **13-8** (200 mg) and 2-aminopyrimidine-5-boronic acid (**28-1,** 75 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **28-2.**

### (2) Preparation of compound 28-3:

Compound **28-2** (102 mg) was dissolved in tetrahydrofuran (4 mL), a solution of lithium hydroxide hydrate (27.7 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **28-3,** which was directly used in the next step.

### (3) Preparation of compound 28:

Compound **28-3** (100 mg) was dispersed in tetrahydrofuran (5 mL), N-methylmorpholine (48.6 mg) and isobutyl chloroformate (65.6 mg) were added in an ice-water bath, and the mixture was stirred for 30 min. Ammonium hydroxide (50.4 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **28** (45 mg); ESI-MS: m/z =417.22[M+H] ⁺.

¹H NMR (500 MHz, DMSO-*d*₆) δ 9.00 (s, 1H), 8.15 (s, 1H), 8.08 (s, 2H), 7.85 (d, *J =* 1.2 Hz, 1H), 7.78 (d, *J =* 1.3 Hz, 1H), 7.53 (s, 1H), 7.41 (s, 1H), 6.84 (s, 2H), 2.36 - 2.28 (m, 1H), 1.09 (dt, *J =* 8.0, 3.2 Hz, 2H), 0.98 (dq, *J =* 6.9, 3.9 Hz, 2H).

### Example 29

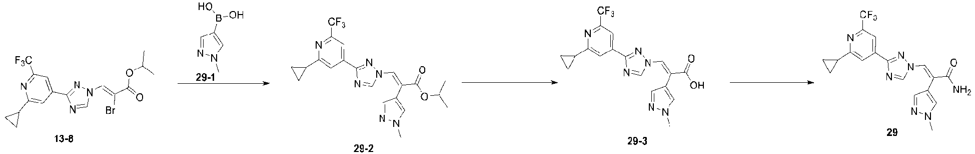

### (1) Preparation of compound 29-2:

Compound **13-8** (200 mg) and 1-methyl-1H-pyrazole-4-boronic acid (**29-1,** 68 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **29-2.**

### (2) Preparation of compound 29-3:

Compound **29-2** (100 mg) was dissolved in tetrahydrofuran (4 mL), a solution of lithium hydroxide hydrate (27.7 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **29-3,** which was directly used in the next step.

### (3) Preparation of compound 29:

Compound **29-3** (88.96 mg) was dispersed in tetrahydrofuran (5 mL), N-methylmorpholine (44.5 mg) and isobutyl chloroformate (60.09 mg) were added in an ice-water bath, and the mixture was stirred for 30 min. Ammonium hydroxide (46.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **29** (30 mg). ESI-MS: m/z =404.24[M+H] ⁺.

¹H NMR (500 MHz, DMSO-*d*₆) δ 8.75 (s, 1H), 8.08 - 8.03 (m, 1H), 7.94 (d, *J =* 1.3 Hz, 1H), 7.82 (s, 1H), 7.72 (s, 1H), 7.55 (s, 1H), 7.49 (s, 1H), 7.25 (s, 1H), 3.85 (s, 3H), 2.37 (tt, *J* = 8.1, 4.7 Hz, 1H), 1.10 (dt, *J =* 8.1, 3.2 Hz, 2H), 1.00 (dq, *J =* 7.0, 4.3, 3.9 Hz, 2H).

### Example 30

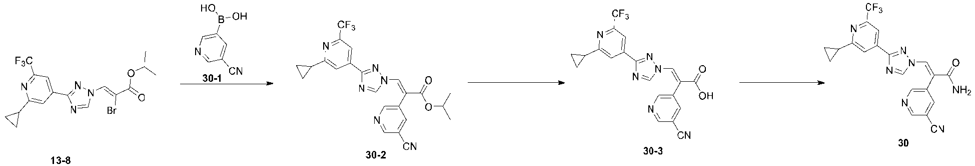

### (1) Preparation of compound 30-2:

Compound **13-8** (200 mg) and 5-cyano-pyridine-3-boronic acid (**30-1,** 79.8 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **30-2.**

### (2) Preparation of compound 30-3:

Compound **30-2** (150 mg) was dissolved in tetrahydrofuran (4 mL), a solution of lithium hydroxide hydrate (40.28 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **30-3,** which was directly used in the next step.

### (3) Preparation of compound 30:

Compound **30-3** (136.44 mg) was dispersed in tetrahydrofuran (5 mL), N-methylmorpholine (64.74 mg) and isobutyl chloroformate (87.4 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **30** (45 mg). ESI-MS: m/z =426.21 [M+H] ⁺.

¹H NMR (500 MHz, DMSO-*d*₆) δ 9.12 (s, 1H), 9.09 (d, *J =* 2.0 Hz, 1H), 8.75 (d, *J =* 2.1 Hz, 1H), 8.42 (s, 1H), 8.35 (t, *J* = 2.1 Hz, 1H), 7.70 (d, *J =* 1.3 Hz, 1H), 7.66 (s, 1H), 7.45 (d, *J =* 1.3 Hz, 1H), 7.28 (s, 1H), 2.29 (tt, *J* = 8.1, 4.7 Hz, 1H), 1.10 (dt, *J =* 8.1, 3.3 Hz, 2H), 0.97 - 0.89 (m, 2H).

### Example 31

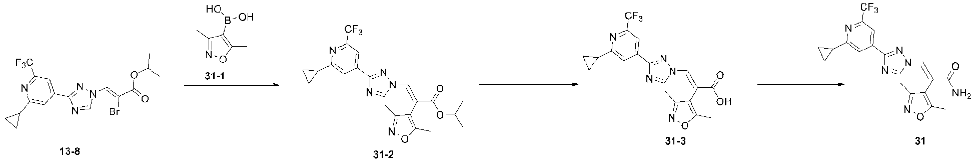

### (1) Preparation of compound 31-2:

Compound **13-8** (200 mg) and 3,5-dimethylisoxazole-4-boronic acid (**31-1,** 76.1 mg) were dispersed in 1,4-dioxane (5 mL), and potassium carbonate (186.6 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **31-2.**

### (2) Preparation of compound 31-3:

Compound **31-2** (150 mg) was dissolved in tetrahydrofuran (4 mL), a solution of lithium hydroxide hydrate (41.54 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **31-3,** which was directly used in the next step.

### (3) Preparation of compound 31:

Compound **31-3** (134 mg) was dispersed in tetrahydrofuran (5 mL), N-methylmorpholine (64.74 mg) and isobutyl chloroformate (87.4 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **31** (30 mg). ESI-MS: m/z =419.23[M+H] ⁺.

¹H NMR (500 MHz, DMSO-*d*₆) δ 9.07 (s, 1H), 8.38 (s, 1H), 7.86 (d, *J =* 1.3 Hz, 1H), 7.78 (d, *J =* 1.3 Hz, 1H), 7.52 (s, 1H), 7.37 (s, 1H), 2.31 (tt, *J =* 8.1, 4.7 Hz, 1H), 2.17 (s, 3H), 1.96 (s, 3H), 1.14 - 1.08 (m, 2H), 1.01 - 0.95 (m, 2H).

### Example 32

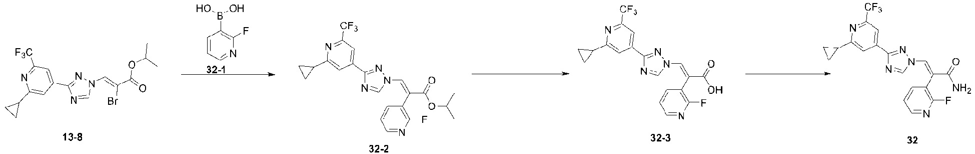

### (1) Preparation of compound 32-2:

Compound **13-8** (200 mg) and 2-fluoro-pyridine-3-boronic acid (**32-1,** 76.1 mg) were dispersed in 1,4-dioxane (5 mL), and potassium carbonate (186.6 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **32-2.**

### (2) Preparation of compound 32-3:

Compound **32-2** (70 mg) was dissolved in tetrahydrofuran (4 mL), a solution of lithium hydroxide hydrate (18.9 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **32-3,** which was directly used in the next step.

### (3) Preparation of compound 32:

Compound **32-3** (50 mg) was dispersed in tetrahydrofuran (5 mL), N-methylmorpholine (24.3 mg) and isobutyl chloroformate (32.7 mg) were added in an ice-water bath, and the mixture was stirred for 30 min. Ammonium hydroxide (25.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **32** (10 mg). ESI-MS: m/z =419.21[M+H]⁺.

¹H NMR (500 MHz, DMSO-*d*₆) δ 9.13 (s, 1H), 8.39 (s, 1H), 8.32 (dd, *J* = 5.0, 1.9 Hz, 1H), 7.86 (ddd, *J* = 9.5, 7.3, 1.9 Hz, 1H), 7.69 (d, *J =* 1.3 Hz, 1H), 7.59 (s, 1H), 7.50 (d, *J =* 1.3 Hz, 1H), 7.44 (ddd, *J =* 7.1, 4.9, 1.9 Hz, 1H), 7.40 (s, 1H), 2.27 (tt, *J =* 8.2, 4.7 Hz, 1H), 1.10 (dt, *J =* 8.1, 3.3 Hz, 2H), 0.97 - 0.90 (m, 2H).

### Example 33

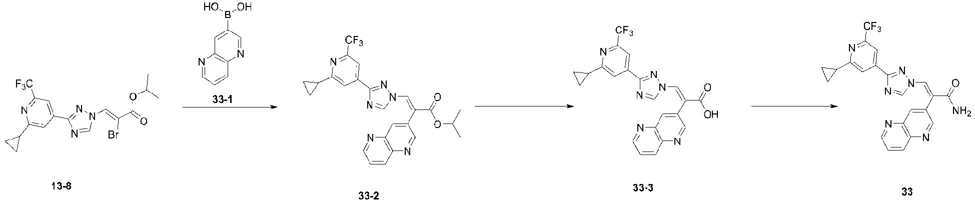

### (1) Preparation of compound 33-2:

Compound **13-8** (200 mg) and [1,5]naphthyridine-3-boronic acid (**33-1,** 138.3 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **33-2.**

### (2) Preparation of compound 33-3:

Compound **33-2** (160 mg) was dissolved in tetrahydrofuran (4 mL), a solution of lithium hydroxide hydrate (40.28 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **33-3,** which was directly used in the next step.

### (3) Preparation of compound 33:

Compound **33-3** (144.7 mg) was dispersed in tetrahydrofuran (5 mL), N-methylmorpholine (64.74 mg) and isobutyl chloroformate (87.4 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound 33 (20 mg). ESI-MS: m/z =452.21 [M+H] ⁺.

¹H NMR (500 MHz, DMSO-*d*₆) δ 9.12 (s, 1H), 9.03 (dd, *J =* 4.2, 1.7 Hz, 1H), 8.87 (d, *J =* 2.2 Hz, 1H), 8.51 (d, *J =* 8.5 Hz, 1H), 8.46 (s, 1H), 8.31 (d, *J* = 2.2 Hz, 1H), 7.86 (dd, *J =* 8.5, 4.2 Hz, 1H), 7.65 (s, 1H), 7.51 (s, 1H), 7.34 (s, 1H), 7.20 - 7.14 (m, 1H), 2.10 (tt, *J =* 8.4, 4.7 Hz, 1H), 1.01 (dt, *J =* 6.4, 3.3 Hz, 2H), 0.79 (dq, *J =* 6.9, 4.0 Hz, 2H).

### Example 34

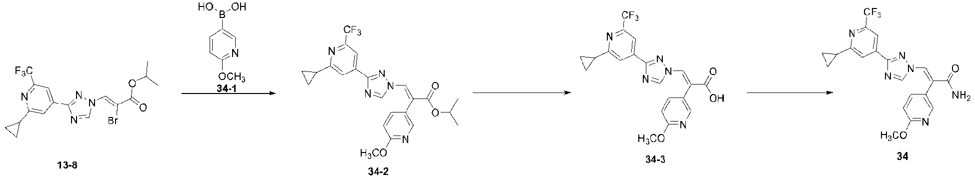

### (1) Preparation of compound 34-2:

Compound **13-8** (200 mg) and 2-methoxypyridine-5-boronic acid (**34-1,** 82.6 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **34-2.**

### (2) Preparation of compound 34-3:

Compound **34-2** (180 mg) was dissolved in tetrahydrofuran (4 mL), a solution of lithium hydroxide hydrate (47.9 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **34-3,** which was directly used in the next step.

### (3) Preparation of compound 34:

Compound **34-3** (163.9 mg) was dispersed in tetrahydrofuran (5 mL), N-methylmorpholine (76.8 mg) and isobutyl chloroformate (103.8 mg) were added in an ice-water bath, and the mixture was stirred for 30 min. Ammonium hydroxide (79.8 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound 34 (80 mg). ESI-MS: m/z =431.19[M+H] ⁺.

¹H NMR (500 MHz, DMSO-*d*₆) δ 8.91 (s, 1H), 8.21 (s, 1H), 8.04 (d, *J =* 2.4 Hz, 1H), 7.81 (d, *J =* 1.3 Hz, 1H), 7.61 (d, *J* = 1.3 Hz, 1H), 7.58 (d, *J =* 2.4 Hz, 1H), 7.56 (d, *J =* 2.5 Hz, 1H), 7.55 (s, 1H), 7.20 (s, 1H), 6.89 (d, *J =* 8.5 Hz, 1H), 3.91 (s, 3H), 2.29 (tt, *J =* 8.1, 4.8 Hz, 1H), 1.12 - 1.05 (m, 2H), 0.98 - 0.93 (m, 2H).

### Example 35

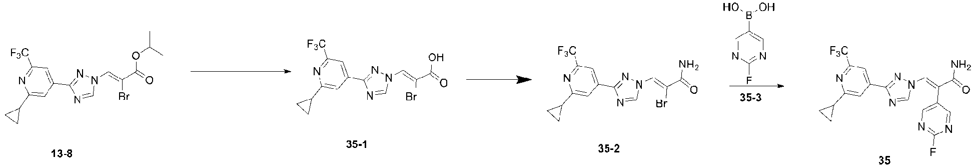

### (1) Preparation of compound 35-1:

Compound **13-8** (200 mg) was dissolved in tetrahydrofuran (4 mL), a solution of lithium hydroxide hydrate (56.65 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **35-1,** which was directly used in the next step.

### (2) Preparation of compound 35-2:

Compound **35-1** (181.4 mg) was dispersed in tetrahydrofuran (5 mL), N-methylmorpholine (91.04 mg) and isobutyl chloroformate (122.9 mg) were added in an ice-water bath, and the mixture was stirred for 30 min. Ammonium hydroxide (94.5 mg) was added dropwise, and the mixture was reacted in an ice-water bath for 10 min. Water was added to quench the reaction, and the resulting mixture was extracted with ethyl acetate, dried over anhydrous sodium sulfate, and concentrated until no liquid flowed out to give compound **35-2,** which was directly used in the next step.

### (2) Preparation of compound 35:

Compound **35-2** (200 mg) and 2-fluoro-pyrimidine-5-boronic acid (**35-3,** 134.4 mg) were dispersed in 1,4-dioxane (5 mL), and potassium acetate (147.21 mg), Pd(dppf)Cl₂ (18.3 mg), and water (0.5 mL) were added. The mixture was heated to 90 °C and reacted under nitrogen atmosphere. After the starting materials were consumed completely as detected by TLC, the reaction solution was concentrated under reduced pressure and purified by preparative liquid chromatography to give compound 35 (30 mg). ESI-MS: m/z =420.19[M+H] ⁺.

¹H NMR (500 MHz, DMSO-*d*₆) δ 9.18 (s, 1H), 8.74 (s, 2H), 8.47 (s, 1H), 7.74 (s, 1H), 7.66 (s, 1H), 7.49 (s, 1H), 7.35 (s, 1H), 2.28 (tt, *J =* 8.4, 4.7 Hz, 1H), 1.11 (dq, *J =* 6.7, 3.9 Hz, 2H), 1.00 - 0.89 (m, 2H).

### Example 36

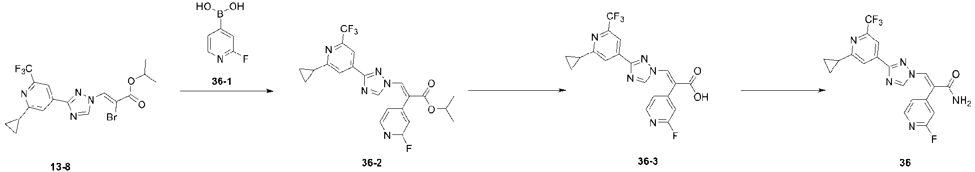

### (1) Preparation of compound 36-2:

Compound **13-8** (200 mg) and 2-fluoro-pyridine-4-boronic acid (**36-1,** 76.09 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **36-2.**

### (2) Preparation of compound 36-3:

Compound **36-2** (150 mg) was dissolved in tetrahydrofuran (4 mL), a solution of lithium hydroxide hydrate (40.3 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **36-3,** which was directly used in the next step.

### (3) Preparation of compound 36:

Compound **36-3** (100 mg) was dispersed in tetrahydrofuran (5 mL), N-methylmorpholine (48.5 mg) and isobutyl chloroformate (65.5 mg) were added in an ice-water bath, and the mixture was stirred for 30 min. Ammonium hydroxide (50.4 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **36** (30 mg). ESI-MS: m/z =419.19[M+H] ⁺.

¹H NMR (500 MHz, DMSO-*d*₆) δ 9.03 (s, 1H), 8.29 (d, *J =* 5.3 Hz, 2H), 7.68 (s, 1H), 7.63 (s, 1H), 7.53 (s, 1H), 7.25 (d, *J =* 5.0 Hz, 1H), 7.18 (d, *J =* 6.6 Hz, 2H), 2.26 (tt, *J =* 8.3, 4.7 Hz, 1H), 1.10 (dq, *J=* 6.8, 3.9 Hz, 2H), 0.94 (dq, *J =* 6.7, 4.0 Hz, 2H).

### Example 37

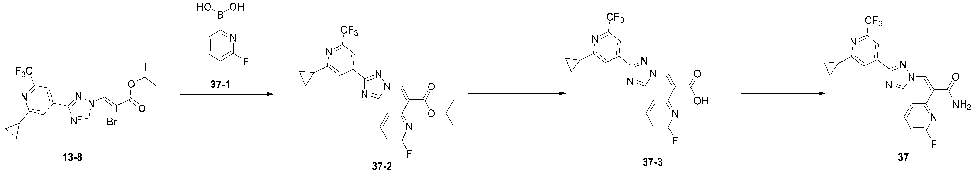

### (1) Preparation of compound 37-2:

Compound **13-8** (200 mg) and 6-fluoro-pyridine-2-boronic acid (37-1, 76.09 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **37-2.**

### (2) Preparation of compound 37-3:

Compound **37-2** (170 mg) was dissolved in tetrahydrofuran (4 mL), a solution of lithium hydroxide hydrate (46.4 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **37-3,** which was directly used in the next step.

### (3) Preparation of compound 37:

Compound **37-3** (155.2 mg) was dispersed in tetrahydrofuran (5 mL), N-methylmorpholine (74.8 mg) and isobutyl chloroformate (101.1 mg) were added in an ice-water bath, and the mixture was stirred for 30 min. Ammonium hydroxide (77.7 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **37** (40 mg). ESI-MS: m/z =419.19[M+H] ⁺.

¹H NMR (500 MHz, DMSO-*d*₆) δ 9.01 (d, *J =* 1.4 Hz, 1H), 8.27 (d, *J =* 1.4 Hz, 1H), 8.05 (q, *J =* 8.1 Hz, 1H), 7.73 (s, 1H), 7.60 (s, 1H), 7.51 (d, *J =* 1.4 Hz, 1H), 7.34 (dd, *J =* 7.4, 2.3 Hz, 1H), 7.23 (dd, *J =* 8.5, 2.3 Hz, 2H), 2.29 (tt, *J* = 8.4, 4.7 Hz, 1H), 1.09 (dq, *J =* 6.5, 3.8 Hz, 2H), 0.94 (dt, *J =* 5.0, 3.3 Hz, 2H).

### Example 38

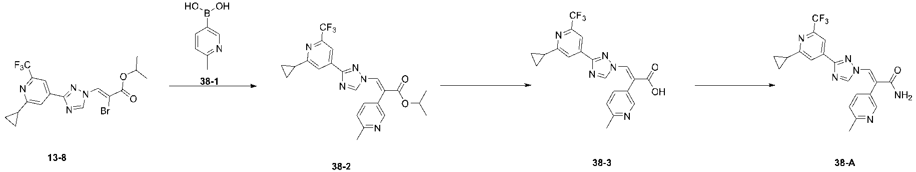

### (1) Preparation of compound 38-2:

Compound **13-8** (200 mg) and 2-methyl-pyridine-5-boronic acid (**38-1,** 73.95 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **38-2.**

### (2) Preparation of compound 38-3:

Compound **38-2** (105 mg) was dissolved in tetrahydrofuran (4 mL), a solution of lithium hydroxide hydrate (28.95 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **38-3,** which was directly used in the next step.

### (3) Preparation of compound 38-A:

Compound **38-3** (95.5 mg) was dispersed in tetrahydrofuran (5 mL), N-methylmorpholine (46.5 mg) and isobutyl chloroformate (62.8 mg) were added in an ice-water bath, and the mixture was stirred for 30 min. Ammonium hydroxide (48.3 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **38-A** (30 mg). ESI-MS: m/z =415.24[M+H]⁺.

¹H NMR (500 MHz, DMSO-*d*₆) δ 8.93 (s, 1H), 8.30 (d, *J =* 2.3 Hz, 1H), 8.24 (s, 1H), 7.78 (s, 1H), 7.56 (dd, *J =* 8.0, 2.4 Hz, 2H), 7.53 (d, *J =* 1.3 Hz, 1H), 7.33 (d, *J =* 7.9 Hz, 1H), 7.20 (s, 1H), 2.54 (s, 3H), 2.29 (tt, *J =* 8.3, 4.7 Hz, 1H), 1.09 (dt, *J =* 8.1, 3.3 Hz, 2H), 1.00 - 0.89 (m, 2H).

### Example 39

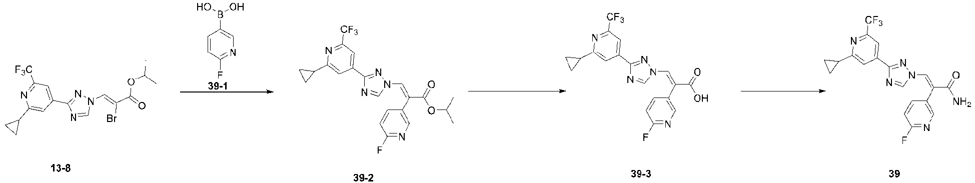

### (1) Preparation of compound 39-2:

Compound **13-8** (200 mg) and 2-fluoro-pyridine-5-boronic acid (**39-1,** 76.09 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **39-2.**

### (2) Preparation of compound 39-3:

Compound **39-2** (180 mg) was dissolved in tetrahydrofuran (4 mL), a solution of lithium hydroxide hydrate (49.1 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **39-3,** which was directly used in the next step.

### (3) Preparation of compound 39:

Compound **39-3** (163 mg) was dispersed in tetrahydrofuran (5 mL), N-methylmorpholine (78.9 mg) and isobutyl chloroformate (106.5 mg) were added in an ice-water bath, and the mixture was stirred for 30 min. Ammonium hydroxide (81.9 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **39** (40 mg). ESI-MS: m/z =419.20[M+H] ⁺.

¹H NMR (500 MHz, DMSO-*d*₆) δ 9.04 (s, 1H), 8.33 (s, 1H), 8.13 (d, *J =* 2.4 Hz, 1H), 7.88 (td, *J =* 8.2, 2.5 Hz, 1H), 7.75 (d, *J* = 1.2 Hz, 1H), 7.61 (s, 1H), 7.52 (d, *J =* 1.3 Hz, 1H), 7.27 (dd, *J* = 8.4, 2.7 Hz, 1H), 7.24 (s, 1H), 2.28 (tt, *J =* 8.0, 4.7 Hz, 1H), 1.09 (dt, *J =* 8.0, 3.3 Hz, 2H), 0.99 - 0.89 (m, 2H).

### Example 40

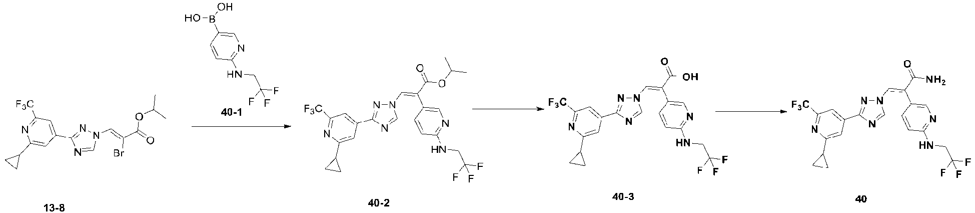

### (1) Preparation of compound 40-2:

Compound **13-8** (200 mg, 0.45 mmol, 1.0 eq) and 2-trifluoroethylaminopyridine-5-boronic acid (**40-1,** 118.08 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **40-2.**

### (2) Preparation of compound 40-3:

Compound **40-2** (130 mg) was dissolved in tetrahydrofuran (4 mL), a solution of lithium hydroxide hydrate (30.3 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **40-3,** which was directly used in the next step.

### (3) Preparation of compound 40:

Compound **40-3** (119.6 mg) was dispersed in tetrahydrofuran (5 mL), N-methylmorpholine (48.6 mg) and isobutyl chloroformate (65.6 mg) were added in an ice-water bath, and the mixture was stirred for 30 min. Ammonium hydroxide (50.4 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **40** (50 mg). ESI-MS: m/z =498.29[M+H] ⁺.

¹H NMR (500 MHz, DMSO-*d*₆) δ 8.78 (s, 1H), 8.05 (s, 1H), 7.88 (d, *J =* 2.0 Hz, 2H), 7.75 (d, *J* = 1.3 Hz, 1H), 7.56 - 7.49 (m, 1H), 7.39 (t, *J =* 6.6 Hz, 1H), 7.32 (dd, *J =* 8.5, 2.4 Hz, 1H), 7.24 (s, 1H), 6.69 (d, *J =* 8.5 Hz, 1H), 4.19 (qd, *J* = 9.7, 6.5 Hz, 2H), 2.30 (tt, *J =* 8.2, 4.7 Hz, 1H), 1.08 (dt, *J =* 8.0, 3.2 Hz, 2H), 1.02 - 0.91 (m, 2H).

### Example 41

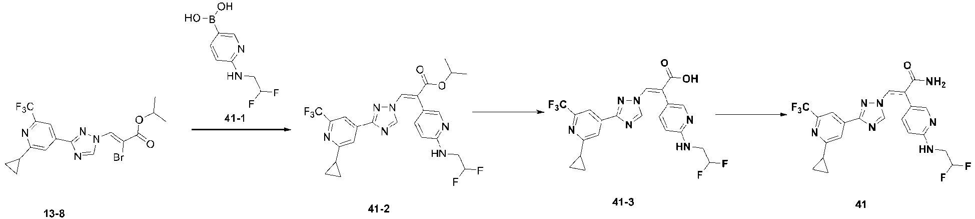

### (1) Preparation of compound 41-2:

Compound **13-8** (200 mg) and 6-(2,2-difluoroethylamino)pyridine-3-boronic acid (**41-1,** 108.86 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **41-2.**

### (2) Preparation of compound 41-3:

Compound **41-2** (120 mg) was dissolved in tetrahydrofuran (4 mL), a solution of lithium hydroxide hydrate (28.9 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **41-3,** which was directly used in the next step.

### (3) Preparation of compound 41:

Compound **41-3** (110.5 mg) was dispersed in tetrahydrofuran (5 mL), N-methylmorpholine (46.5 mg) and isobutyl chloroformate (62.8 mg) were added in an ice-water bath, and the mixture was stirred for 30 min. Ammonium hydroxide (48.3 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **41** (40 mg). ESI-MS: m/z =480.31 [M+H] ⁺.

¹H NMR (500 MHz, DMSO-*d*₆) δ 8.74 (s, 1H), 8.03 (s, 1H), 7.90 (d, *J =* 1.3 Hz, 1H), 7.87 (d, *J* = 2.4 Hz, 1H), 7.77 (d, *J =* 1.2 Hz, 1H), 7.53 (s, 1H), 7.27 (dd, *J =* 8.6, 2.4 Hz, 1H), 7.25 - 7.18 (m, 2H), 6.64 (d, *J =* 8.6 Hz, 1H), 6.09 (tt, *J =* 56.6, 4.1 Hz, 1H), 3.73 (tdd, *J =* 15.4, 6.1, 4.1 Hz, 2H), 2.31 (tt, *J =* 8.1, 4.7 Hz, 1H), 1.08 (dt, *J =* 8.0, 3.2 Hz, 2H), 1.00 - 0.94 (m, 2H).

### Example 42

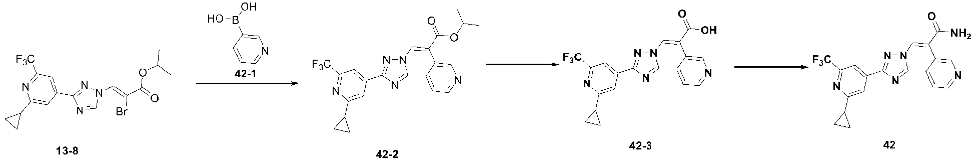

### (1) Preparation of compound 42-2:

Compound **13-8** (200 mg) and pyridine-3-boronic acid (**42-1,** 66.37 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **42-2.**

### (2) Preparation of compound 42-3:

Compound **42-2** (85 mg) was dissolved in tetrahydrofuran (4 mL), a solution of lithium hydroxide hydrate (24.1 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **42-3,** which was directly used in the next step.

### (3) Preparation of compound 42:

Compound **42-3** (76.28 mg) was dispersed in tetrahydrofuran (5 mL), N-methylmorpholine (38.4 mg) and isobutyl chloroformate (51.9 mg) were added in an ice-water bath, and the mixture was stirred for 30 min. Ammonium hydroxide (39.9 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **42** (20 mg). ESI-MS: m/z =401.32[M+H] ⁺.

### Example 43

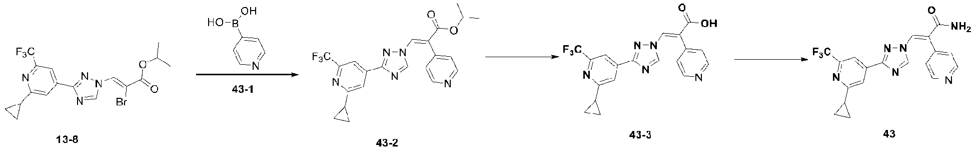

### (1) Preparation of compound 43-2:

Compound **13-8** (200 mg) and 4-pyridineboronic acid (**43-1**, 66.37 mg) were dispersed in 1,4-dioxane (5 mL), and potassium acetate (132.5 mg), Pd(dppf)Cl₂ (14.6 mg), and water (0.5 mL) were added. The mixture was heated to 90 °C and reacted under nitrogen atmosphere. After the starting materials were consumed completely as detected by TLC, the reaction solution was concentrated under reduced pressure and purified by column chromatography to give compound **43-2.**

### (2) Preparation of compound 43-3:

Compound **43-2** (120 mg) was dissolved in tetrahydrofuran (4 mL), a solution of lithium hydroxide hydrate (34.06 mg) and water (4 mL) was added dropwise in an ice-water bath, and the mixture was reacted at room temperature for 4 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **43-3,** which was directly used in the next step.

### (3) Preparation of compound 43:

Compound **43-3** (108.4 mg) was dispersed in tetrahydrofuran (5 mL), N-methylmorpholine (54.6 mg) and isobutyl chloroformate (73.7 mg) were added in an ice-water bath, and the mixture was stirred for 30 min. Ammonium hydroxide (56.7 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **43** (60 mg). ESI-MS: m/z =401.24[M+H] ⁺.

### Example 44

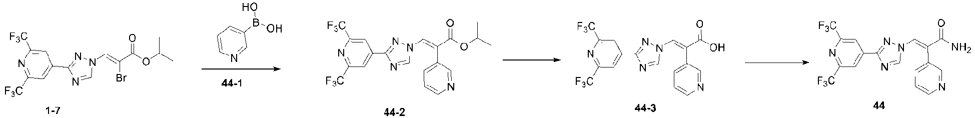

### (1) Preparation of compound 44-2:

Compound **1-7** (200 mg) and pyridine-3-boronic acid (**44-1,** 62.4 mg) were dispersed in 1,4-dioxane (8 mL), and potassium carbonate (175 mg), Pd(dppf)Cl₂ (15.5 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **44-2.**

### (2) Preparation of compound 44-3:

Compound **44-2** (100 mg) was dissolved in tetrahydrofuran (5 mL), a solution of lithium hydroxide hydrate (290 mg) and water (5 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **44-3,** which was directly used in the next step.

### (3) Preparation of compound 44:

Compound **44-3** (91 mg) was dispersed in tetrahydrofuran (10 mL), N-methylmorpholine (42.9 mg) and isobutyl chloroformate (58 mg) were added in an ice-water bath, and the mixture was stirred for 30 min. Ammonium hydroxide (44.5 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **44** (60 mg). ESI-MS: m/z = 429.29M+H] ⁺.

¹H NMR (500 MHz, DMSO-*d*₆) δ 9.05 (s, 1H), 8.62 (d, *J =* 4.9 Hz, 1H), 8.46 (d, *J =* 2.2 Hz, 1H), 8.31 (s, 1H), 8.10 (s, 2H), 7.70 (dt, *J =* 7.9, 1.9 Hz, 1H), 7.64 (s, 1H), 7.48 (dd, *J =* 7.8, 4.8 Hz, 1H), 7.28 (s, 1H).

### Example 45

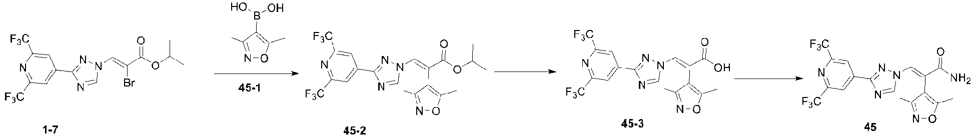

### (1) Preparation of compound 45-2:

Compound **1-7** (200 mg) and 3,5-dimethylisoxazole-4-boronic acid (**45-1,** 71.6 mg) were dispersed in 1,4-dioxane (8 mL), and potassium carbonate (175 mg), Pd(dppf)Cl₂ (15.5 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **45-2.**

### (2) Preparation of compound 45-3:

Compound **45-2** (159 mg) was dissolved in tetrahydrofuran (10 mL), a solution of lithium hydroxide hydrate (44.7 mg) and water (10 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **45-3,** which was directly used in the next step.

### (3) Preparation of compound 45:

Compound **45-3** (145 mg) was dispersed in tetrahydrofuran (10 mL), N-methylmorpholine (65.7 mg) and isobutyl chloroformate (88.7 mg) were added in an ice-water bath, and the mixture was stirred for 30 min. Ammonium hydroxide (68 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **45** (66 mg). ESI-MS: m/z = 447.29[M+H] ⁺.

¹H NMR (500 MHz, DMSO-*d*₆) δ 9.16 (s, 1H), 8.41 (s, 1H), 8.31 (s, 2H), 7.57 (s, 1H), 7.42 (s, 1H), 2.18 (s, 3H), 1.97 (s, 3H).

### Example 46

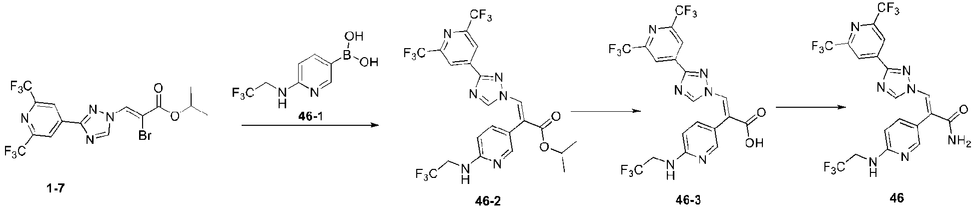

### (1) Preparation of compound 46-2:

Compound **1-7** (250 mg) and 6-(2,2,2-trifluoroethylamino)pyridine-3-boronic acid (**46-1,** 140 mg) were dispersed in 1,4-dioxane (8 mL), and potassium carbonate (219 mg), Pd(dppf)Cl₂ (19.3 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **46-2.**

### (2) Preparation of compound 46-3:

Compound **46-2** (220 mg) was dissolved in tetrahydrofuran (10 mL), a solution of lithium hydroxide hydrate (48.7 mg) and water (10 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **46-3,** which was directly used in the next step.

### (3) Preparation of compound 46:

Compound **46-3** (203 mg) was dispersed in tetrahydrofuran (10 mL), N-methylmorpholine (78 mg) and isobutyl chloroformate (106 mg) were added in an ice-water bath, and the mixture was stirred for 30 min. Ammonium hydroxide (81 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **46** (30 mg). ESI-MS: m/z = 526.33[M+H] ⁺.

¹H NMR (500 MHz, DMSO-*d*₆) δ 8.91 (s, 1H), 8.28 (s, 2H), 8.08 (s, 1H), 7.89 (d, *J =* 2.3 Hz, 1H), 7.55 (s, 1H), 7.40 (t, *J =* 6.5 Hz, 1H), 7.33 (dd, *J =* 8.6, 2.4 Hz, 1H), 7.27 (s, 1H), 6.68 (d, *J =* 8.6 Hz, 1H), 4.18 (qd, *J =* 9.7, 6.4 Hz, 2H).

### Example 47

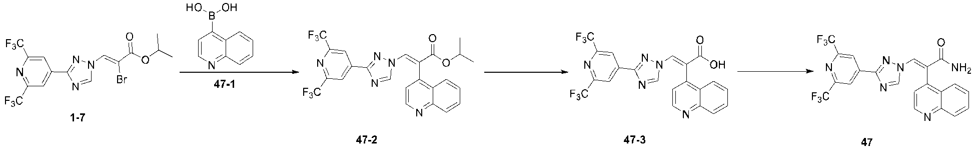

### (1) Preparation of compound 47-2:

Compound **1-7** (200 mg) and 4-quinolineboronic acid (**47-1,** 87.7 mg) were dispersed in 1,4-dioxane (8 mL), and potassium acetate (124 mg), Pd(dppf)Cl₂ (15.5 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **47-2.**

### (2) Preparation of compound 47-3:

Compound **47-2** (200 mg) was dissolved in tetrahydrofuran (10 mL), a solution of lithium hydroxide hydrate (48 mg) and water (10 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **47-3,** which was directly used in the next step.

### (3) Preparation of compound 47:

Compound **47-3** (184 mg) was dispersed in tetrahydrofuran (10 mL), N-methylmorpholine (77.7 mg) and isobutyl chloroformate (105 mg) were added in an ice-water bath, and the mixture was stirred for 30 min. Ammonium hydroxide (80 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **47** (90 mg). ESI-MS: m/z = 479.22 [M+H] ⁺.

¹H NMR (500 MHz, DMSO-*d*₆) δ 9.10 (s, 1H), 8.95 (d, *J =* 4.4 Hz, 1H), 8.57 (s, 1H), 8.12 (d, *J =* 8.4 Hz, 1H), 7.77 (dd, *J =* 8.4, 1.4 Hz, 1H), 7.74 (ddd, *J =* 8.3, 6.8, 1.4 Hz, 1H), 7.63 (s, 1H), 7.61 (s, 2H), 7.52 (ddd, *J =* 8.2, 6.8, 1.2 Hz, 1H), 7.42 (d, *J =* 4.4 Hz, 1H), 7.22 (s, 1H).

### Example 48

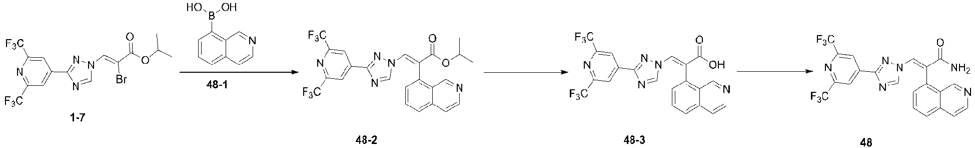

### (1) Preparation of compound 48-2:

Compound **1-7** (200 mg) and 8-isoquinolineboronic acid (**48-1,** 87.7 mg) were dispersed in 1,4-dioxane (8 mL), and potassium acetate (124 mg), Pd(dppf)Cl₂ (15.5 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **48-2.**

### (2) Preparation of compound 48-3:

Compound **48-2** (170 mg) was dissolved in tetrahydrofuran (10 mL), a solution of lithium hydroxide hydrate (41 mg) and water (10 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **48-3,** which was directly used in the next step.

### (3) Preparation of compound 48:

Compound **48-3** (156 mg) was dispersed in tetrahydrofuran (10 mL), N-methylmorpholine (66 mg) and isobutyl chloroformate (89 mg) were added in an ice-water bath, and the mixture was stirred for 30 min. Ammonium hydroxide (68 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **48** (45 mg). ESI-MS: m/z = 479.24 [M+H] ⁺.

¹H NMR (500 MHz, DMSO-*d*₆) δ 9.10 (d, *J* = 43.7 Hz, 2H), 8.54 (d, *J =* 37.6 Hz, 2H), 8.08 (d, *J =* 8.1 Hz, 1H), 7.99 - 7.81 (m, 2H), 7.66 (s, 3H), 7.55 (d, *J =* 7.0 Hz, 1H), 7.28 (s, 1H).

### Example 49

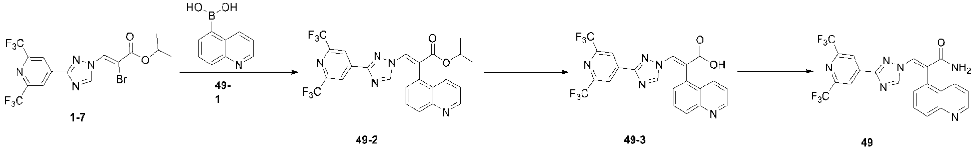

### (1) Preparation of compound 49-2:

Compound **1-7** (200 mg) and 5-quinolineboronic acid (**49-1,** 87.7 mg) were dispersed in 1,4-dioxane (8 mL), and potassium carbonate (175 mg), Pd(dppf)Cl₂ (15.5 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **49-2.**

### (2) Preparation of compound 49-3:

Compound **49-2** (200 mg) was dissolved in tetrahydrofuran (10 mL), a solution of lithium hydroxide hydrate (48 mg) and water (10 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **49-3,** which was directly used in the next step.

### (3) Preparation of compound 49:

Compound **49-3** (184 mg) was dispersed in tetrahydrofuran (10 mL), N-methylmorpholine (77.7 mg) and isobutyl chloroformate (105 mg) were added in an ice-water bath, and the mixture was stirred for 30 min. Ammonium hydroxide (80 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **49** (62 mg). ESI-MS: m/z = 479.25 [M+H] ⁺.

¹H NMR (500 MHz, DMSO-*d*₆) δ 8.98 (s, 1H), 8.93 (d, *J =* 4.4 Hz, 1H), 8.56 (s, 1H), 8.18 (dd, *J =* 18.0, 8.5 Hz, 2H), 7.88 (t, *J =* 7.8 Hz, 1H), 7.72 (s, 2H), 7.63 (s, 1H), 7.59 - 7.43 (m, 2H), 7.15 (s, 1H).

### Example 50

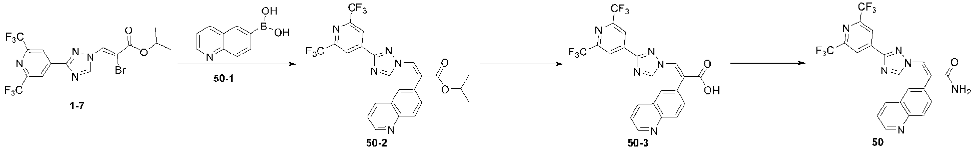

### (1) Preparation of compound 50-2:

Compound **1-7** (200 mg) and 6-quinolineboronic acid **(50-1,** 87.7 mg) were dispersed in 1,4-dioxane (8 mL), and potassium carbonate (175 mg), Pd(dppf)Cl₂ (15.5 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **50-2.**

### (2) Preparation of compound 50-3:

Compound **50-2** (150 mg) was dissolved in tetrahydrofuran (10 mL), a solution of lithium hydroxide hydrate (36 mg) and water (10 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **50-3,** which was directly used in the next step.

### (3) Preparation of compound 50:

Compound **50-3** (138 mg) was dispersed in tetrahydrofuran (10 mL), N-methylmorpholine (58 mg) and isobutyl chloroformate (78.7 mg) were added in an ice-water bath, and the mixture was stirred for 30 min. Ammonium hydroxide (60.5 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **50** (87 mg). ESI-MS: m/z = 479.22 [M+H] ⁺.

¹H NMR (500 MHz, DMSO-*d*₆) δ 9.00 (dd, *J =* 4.3, 1.7 Hz, 1H), 8.95 (s, 1H), 8.45 (dd, *J* = 8.4, 1.8 Hz, 1H), 8.32 (s, 1H), 8.09 (d, *J* = 8.6 Hz, 1H), 7.98 (d, *J =* 1.9 Hz, 1H), 7.92 (s, 2H), 7.67 (s, 1H), 7.65 - 7.62 (m, 1H), 7.62 - 7.58 (m, 1H), 7.19 (s, 1H).

### Example 51

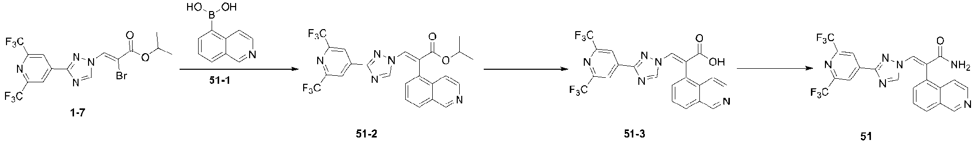

### (1) Preparation of compound 51-2:

Compound **1-7** (200 mg) and 5-quinolineboronic acid (**51-1,** 87.7 mg) were dispersed in 1,4-dioxane (8 mL), and potassium carbonate (175 mg), Pd(dppf)Cl₂ (15.5 mg), and water (0.8 mL) were added. The mixture was heated to 90 °C and reacted under nitrogen atmosphere. After the starting materials were consumed completely as detected by TLC, the reaction solution was concentrated under reduced pressure and purified by column chromatography to give compound **51-2.**

### (2) Preparation of compound 51-3:

Compound **51-2** (200 mg) was dissolved in tetrahydrofuran (10 mL), a solution of lithium hydroxide hydrate (48 mg) and water (10 mL) was added dropwise in an ice-water bath, and the mixture was reacted at room temperature for 2 h. The reaction solution was adjusted to pH 6-7 with hydrochloric acid, extracted with ethyl acetate, dried over anhydrous sodium sulfate, and filtered, and the filtrate was evaporated to dryness under reduced pressure to give compound **51-3,** which was directly used in the next step.

### (3) Preparation of compound 51:

Compound **51-3** (184 mg) was dispersed in tetrahydrofuran (10 mL), N-methylmorpholine (77.7 mg) and isobutyl chloroformate (105 mg) were added in an ice-water bath, and the mixture was stirred for 30 min. Ammonium hydroxide (80 mg) was added dropwise, and the mixture was reacted in an ice-water bath for 10 min. Water was added to quench the reaction, and the resulting mixture was extracted with ethyl acetate, dried over anhydrous sodium sulfate, concentrated until no liquid flowed out, and purified by preparative liquid chromatography to give compound **51** (29 mg). ESI-MS: m/z = 479.23 [M+H] ⁺.

¹H NMR (500 MHz, DMSO-*d*₆) δ 9.39 (s, 1H), 9.02 (s, 1H), 8.56 (s, 1H), 8.42 (d, *J =* 5.8 Hz, 1H), 8.24 (d, *J=* 8.2 Hz, 1H), 7.90 - 7.49 (m, 6H), 7.16 (s, 1H).

### Example 52

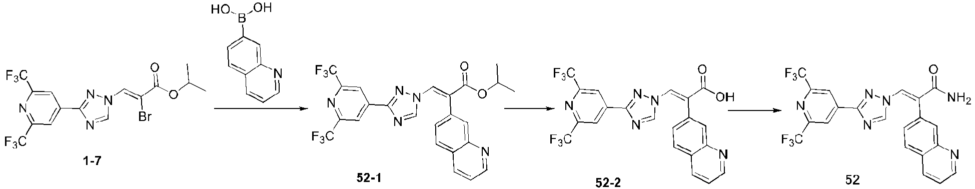

### (1) Preparation of compound 52-1:

Compound **1-7** (300 mg) and 5-quinolineboronic acid (131.6 mg) were dispersed in 1,4-dioxane (8 mL), and potassium carbonate (263 mg), Pd(dppf)Cl₂ (23.2 mg), and water (0.8 mL) were added. The mixture was heated to 90 °C and reacted under nitrogen atmosphere. After the reaction was completed, the reaction solution was concentrated under reduced pressure and purified by column chromatography to give compound **52-1.**

### (2) Preparation of compound 52-2:

Compound **52-1** (200 mg) was dissolved in tetrahydrofuran (10 mL), a solution of lithium hydroxide hydrate (48 mg) and water (10 mL) was added dropwise in an ice-water bath, and the mixture was reacted at room temperature for 2 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **52-2,** which was directly used in the next step.

### (3) Preparation of compound 52:

Compound **52-2** (184 mg) was dispersed in tetrahydrofuran (10 mL), N-methylmorpholine (77.7 mg) and isobutyl chloroformate (105 mg) were added in an ice-water bath, and the mixture was stirred for 30 min. Ammonium hydroxide (80 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **52** (90 mg in total). ESI-MS: m/z = 479.14 [M+H] ⁺.

¹H NMR (500 MHz, DMSO-*d*₆) δ 8.96 (s, 1H), 8.92 (dd, *J =* 4.2, 1.7 Hz, 1H), 8.44 (dd, *J* = 8.5, 1.8 Hz, 1H), 8.31 (s, 1H), 8.04 (d, *J =* 8.4 Hz, 1H), 7.94 (d, *J =* 1.6 Hz, 1H), 7.91 (s, 2H), 7.65 (s, 1H), 7.61 (dd, *J =* 8.3, 4.2 Hz, 1H), 7.47 (dd, *J =* 8.3, 1.7 Hz, 1H), 7.19 (s, 1H).

### Example 53

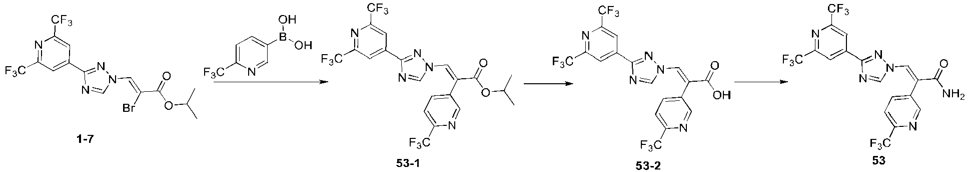

### (1) Preparation of compound 53-1:

Compound **1-7** (300 mg) and 6-trifluoromethyl-3-pyridineboronic acid (145 mg) were dispersed in 1,4-dioxane (8 mL), and potassium carbonate (138.2 mg), Pd(dppf)Cl₂ (23.2 mg), and water (0.8 mL) were added. The mixture was heated to 90 °C and reacted under nitrogen atmosphere. After the reaction was completed, the reaction solution was concentrated under reduced pressure and purified by column chromatography to give compound **53-1.**

### (2) Preparation of compound 53-2:

Compound **53-1** (300 mg) was dissolved in tetrahydrofuran (10 mL), a solution of lithium hydroxide hydrate (70 mg) and water (10 mL) was added dropwise in an ice-water bath, and the mixture was reacted at room temperature for 3 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **53-2,** which was directly used in the next step.

### (3) Preparation of compound 53:

Compound **53-2** (276 mg) was dispersed in tetrahydrofuran (10 mL), N-methylmorpholine (112 mg) and isobutyl chloroformate (152 mg) were added in an ice-water bath, and the mixture was stirred for 30 min. Ammonium hydroxide (117 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **53** (150 mg in total). ESI-MS: m/z = 497.05 [M+H] ⁺.

¹H NMR (500 MHz, DMSO-*d*₆) δ 9.22 (s, 1H), 8.68 (d, *J =* 2.0 Hz, 1H), 8.44 (s, 1H), 8.01 (d, *J =* 4.4 Hz, 3H), 7.98 (d, *J =* 8.0 Hz, 1H), 7.67 (s, 1H), 7.33 (s, 1H).

### Example 54

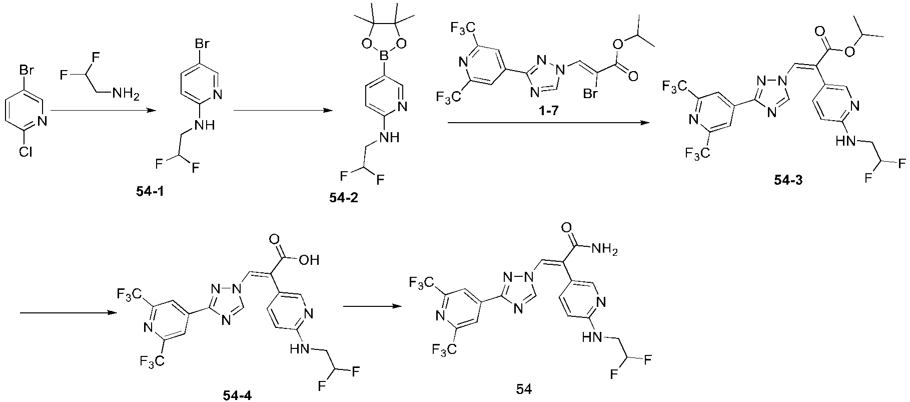

### (1) Preparation of compound 54-1:

Compounds 5-bromo-2-chloropyridine (2.4 g) and 2,2-difluoroethylamine (1.76 g) were dispersed in 1,4-dioxane (30 mL), and bis(dibenzylideneacetone)palladium (420 mg), 1,1'-bis(di-tert-butylphosphino)ferrocene (710 mg), and potassium tert-butoxide (5.6 g) were added. The mixture was heated to 90 °C and reacted under nitrogen atmosphere. After the reaction was completed, the reaction solution was concentrated under reduced pressure and purified by column chromatography to give compound **54-1.**

### (2) Preparation of compound 54-2:

Compound **54-1** (200 mg) was dispersed in 1,4-dioxane (10 mL), and bis(pinacolato)diboron (236 mg), potassium acetate (331 mg), and Pd(dppf)Cl₂ (62 mg) were added. The mixture was reacted at 90 °C for 3 h under nitrogen atmosphere. The reaction solution was diluted with water (10 mL), extracted with ethyl acetate (20 mL × 2), dried over anhydrous sodium sulfate, and concentrated until no liquid flowed out to give compound **54-2.**

### (3) Preparation of compound 54-3:

Compounds **1-7** (332 mg) and **54-2** (240 mg) were dispersed in 1,4-dioxane (10 mL), and potassium acetate (207 mg), Pd(dppf)Cl₂ (25.7 mg), and water (1 mL) were added. The mixture was heated to 90 °C and reacted under nitrogen atmosphere. After the reaction was completed, the reaction solution was concentrated under reduced pressure and purified by column chromatography to give compound **54-3.**

### (4) Preparation of compound 54-4:

Compound **54-3** (180 mg) was dissolved in tetrahydrofuran (8 mL), a solution of lithium hydroxide hydrate (41 mg) and water (8 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **54-4,** which was directly used in the next step.

### (5) Preparation of compound 54:

Compound **54-4** (166 mg) was dispersed in tetrahydrofuran (10 mL), N-methylmorpholine (66 mg) and isobutyl chloroformate (89 mg) were added in an ice-water bath, and the mixture was stirred for 30 min. Ammonium hydroxide (69 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **54** (73 mg). ESI-MS: m/z =508.16 [M+H] ⁺.

¹H NMR (500 MHz, DMSO-*d*₆) δ 8.87 (s, 1H), 8.31 (s, 2H), 8.06 (s, 1H), 7.88 (d, *J =* 2.4 Hz, 1H), 7.55 (s, 1H), 7.31 - 7.19 (m, 3H), 6.64 (dd, *J =* 8.6, 0.8 Hz, 1H), 6.08 (tt, *J* = 56.6, 4.1 Hz, 1H), 3.73 (tdd, *J =* 15.4, 6.1, 4.2 Hz, 2H).

### Example 55

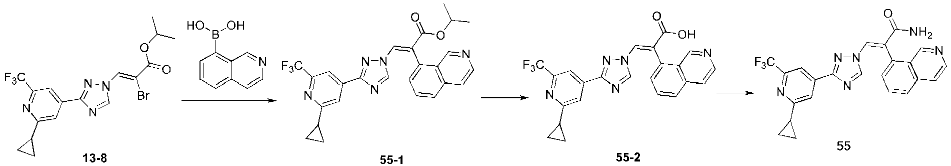

### (1) Preparation of compound 55-1:

Compound **13-8** (200 mg) and isoquinoline-8-boronic acid (93.4 mg) were dispersed in 1,4-dioxane (5 mL), and potassium carbonate (186.57 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **55-1.**

### (2) Preparation of compound 55-2:

Compound **55-1** (170 mg) was dissolved in tetrahydrofuran (4 mL), a solution of lithium hydroxide hydrate (43.4 mg) and water (4 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **55-2,** which was directly used in the next step.

### (3) Preparation of compound 55:

Compound **55-2** (171.5 mg) was dispersed in tetrahydrofuran (5 mL), N-methylmorpholine (76.8 mg) and isobutyl chloroformate (103.8 mg) were added in an ice-water bath, and the mixture was stirred for 30 min. Ammonium hydroxide (79.8 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **55** (30 mg). ESI-MS: m/z =451.23 [M+H] ⁺.

¹H NMR (500 MHz, DMSO-*d*₆) δ 9.12 (s, 1H), 8.93 (s, 1H), 8.51 (d, *J =* 12.6 Hz, 2H), 8.07 (d, *J =* 8.2 Hz, 1H), 8.01 - 7.76 (m, 2H), 7.71 - 7.47 (m, 2H), 7.38 (s, 1H), 7.22 (s, 1H), 7.03 (s, 1H), 2.18 (s, 1H), 1.05 (d, *J =* 8.3 Hz, 2H), 0.84 (s, 2H).

### Example 56

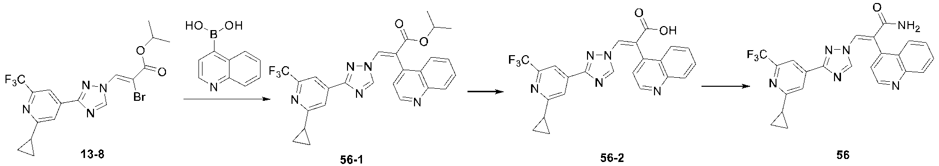

### (1) Preparation of compound 56-1:

Compound **13-8** (200 mg) and quinoline-4-boronic acid (93.4 mg) were dispersed in 1,4-dioxane (5 mL), and potassium carbonate (186.57 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **56-1.**

### (2) Preparation of compound 56-2:

Compound **56-1** (160 mg) was dissolved in tetrahydrofuran (4 mL), a solution of lithium hydroxide hydrate (40.8 mg) and water (4 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **56-2,** which was directly used in the next step.

### (3) Preparation of compound 56:

Compound **56-2** (144.4 mg) was dispersed in tetrahydrofuran (5 mL), N-methylmorpholine (64.7 mg) and isobutyl chloroformate (87.4 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **56** (60 mg). ESI-MS: m/z =451.26[M+H] ⁺.

¹H NMR (500 MHz, DMSO-*d*₆) δ 9.00 (s, 1H), 8.95 (d, *J =* 4.3 Hz, 1H), 8.54 (s, 1H), 8.13 (d, *J =* 8.4 Hz, 1H), 7.82 - 7.70 (m, 2H), 7.59 (s, 1H), 7.52 (t, *J =* 7.6 Hz, 1H), 7.42 (d, *J =* 4.3 Hz, 1H), 7.23 (s, 1H), 7.18 (s, 1H), 7.05 (s, 1H), 2.14 (tt, *J =* 8.3, 4.7 Hz, 1H), 1.06 (dd, *J =* 8.1, 3.0 Hz, 2H), 0.90 - 0.75 (m, 2H).

### Example 57

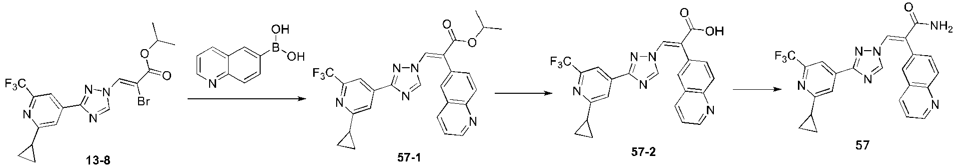

### (1) Preparation of compound 57-1:

Compound **13-8** (200 mg) and quinoline-6-boronic acid (93.4 mg) were dispersed in 1,4-dioxane (5 mL), and potassium carbonate (186.57 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **57-1.**

### (2) Preparation of compound 57-2:

Compound **57-1** (150 mg) was dissolved in tetrahydrofuran (4 mL), a solution of lithium hydroxide hydrate (38.3 mg) and water (4 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **57-2,** which was directly used in the next step.

### (3) Preparation of compound 57:

Compound **57-2** (135.4 mg) was dispersed in tetrahydrofuran (5 mL), N-methylmorpholine (60.7 mg) and isobutyl chloroformate (81.9 mg) were added in an ice-water bath, and the mixture was stirred for 30 min. Ammonium hydroxide (63 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound 57 (30 mg). ESI-MS: m/z =451.21 [M+H] ⁺.

¹H NMR (500 MHz, DMSO-*d*₆) δ 8.96 (dd, *J* = 4.2, 1.7 Hz, 1H), 8.81 (s, 1H), 8.39 (dd, *J* = 8.6, 1.8 Hz, 1H), 8.27 (s, 1H), 8.07 (d, *J =* 8.6 Hz, 1H), 7.93 (d, *J =* 1.9 Hz, 1H), 7.65 - 7.53 (m, 4H), 7.37 (d, *J* = 1.3 Hz, 1H), 7.15 (s, 1H), 2.10 (ddd, *J =* 8.2, 6.5, 4.1 Hz, 1H), 1.03 - 0.98 (m, 2H), 0.82 - 0.77 (m, 2H).

### Example 58

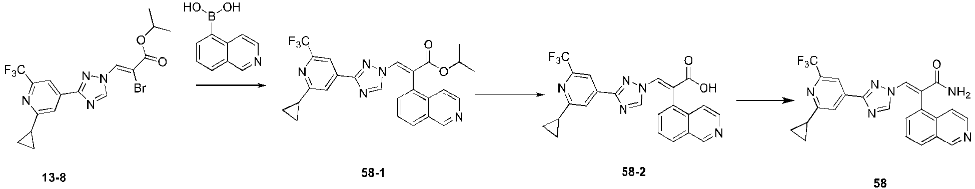

### (1) Preparation of compound 58-1:

Compound **13-8** (200 mg) and isoquinoline-5-boronic acid (93.4 mg) were dispersed in 1,4-dioxane (5 mL), and potassium carbonate (186.57 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **58-1.**

### (2) Preparation of compound 58-2:

Compound **58-1** (160 mg) was dissolved in tetrahydrofuran (4 mL), a solution of lithium hydroxide hydrate (40.8 mg) and water (4 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **58-2,** which was directly used in the next step.

### (3) Preparation of compound 58:

Compound **58-2** (144.4 mg) was dispersed in tetrahydrofuran (5 mL), N-methylmorpholine (64.7 mg) and isobutyl chloroformate (87.4 mg) were added in an ice-water bath, and the mixture was stirred for 30 min. Ammonium hydroxide (67.2 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound 58 (40 mg). ESI-MS: m/z =451.20[M+H] ⁺.

¹H NMR (500 MHz, DMSO-*d*₆) δ 9.39 (s, 1H), 8.89 (s, 1H), 8.51 (s, 1H), 8.41 (d, *J =* 5.9 Hz, 1H), 8.23 (d, *J* = 8.2 Hz, 1H), 7.76 (dd, *J =* 8.2, 7.1 Hz, 1H), 7.66 (dd, *J =* 7.0, 1.2 Hz, 1H), 7.56 (s, 1H), 7.54 (d, *J =* 5.9 Hz, 1H), 7.37 (d, *J =* 1.2 Hz, 1H), 7.11 (s, 1H), 7.04 (d, *J =* 1.2 Hz, 1H), 2.18 (tt, *J* = 8.1, 4.7 Hz, 1H), 1.05 (dd, *J =* 8.2, 2.9 Hz, 2H), 0.85 (d, *J =* 7.0 Hz, 2H).

### Example 59

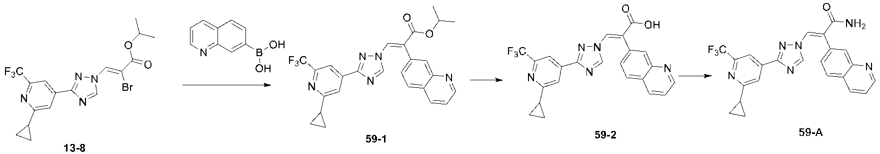

### (1) Preparation of compound 59-1:

Compound **13-8** (200 mg) and quinoline-7-boronic acid (93.4 mg) were dispersed in 1,4-dioxane (5 mL), and potassium carbonate (186.57 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **59-1.**

### (2) Preparation of compound 59-2:

Compound **59-1** (145 mg) was dissolved in tetrahydrofuran (4 mL), a solution of lithium hydroxide hydrate (37 mg) and water (4 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **59-2,** which was directly used in the next step.

### (3) Preparation of compound 59-A:

Compound **59-2** (135.4 mg) was dispersed in tetrahydrofuran (5 mL), N-methylmorpholine (60.6 mg) and isobutyl chloroformate (81.9 mg) were added in an ice-water bath, and the mixture was stirred for 30 min. Ammonium hydroxide (63 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **59-A** (40 mg). ESI-MS: m/z =451.22[M+H] ⁺.

¹H NMR (500 MHz, DMSO-*d*₆) δ 8.93 (dd, *J* = 4.2, 1.8 Hz, 1H), 8.83 (s, 1H), 8.44 (dd, *J =* 8.4, 1.8 Hz, 1H), 8.27 (s, 1H), 8.04 (d, *J =* 8.4 Hz, 1H), 7.93 (d, *J =* 1.6 Hz, 1H), 7.66 - 7.55 (m, 3H), 7.46 (dd, *J* = 8.4, 1.7 Hz, 1H), 7.32 (d, *J* = 1.3 Hz, 1H), 7.17 (s, 1H), 2.12 (tt, *J =* 8.4, 4.7 Hz, 1H), 1.01 (dt, *J =* 8.1, 3.3 Hz, 2H), 0.83 - 0.77 (m, 2H).

### Example 60

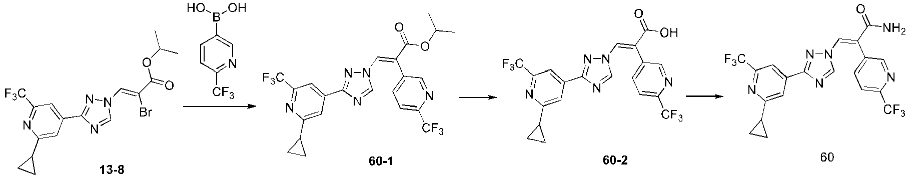

### (1) Preparation of compound 60-1:

Compound **13-8** (200 mg) and 6-trifluoromethylpyridine-3-boronic acid (103.1 mg) were dispersed in 1,4-dioxane (5 mL), and potassium carbonate (186.57 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **60-1.**

### (2) Preparation of compound 60-2:

Compound **60-1** (130 mg) was dissolved in tetrahydrofuran (4 mL), a solution of lithium hydroxide hydrate (32 mg) and water (4 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **60-2,** which was directly used in the next step.

### (3) Preparation of compound 60:

Compound **60-2** (117.3 mg) was dispersed in tetrahydrofuran (5 mL), N-methylmorpholine (50.6 mg) and isobutyl chloroformate (68.3 mg) were added in an ice-water bath, and the mixture was stirred for 30 min. Ammonium hydroxide (52.5 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **60** (45 mg). ESI-MS: m/z =469.19[M+H] ⁺.

¹H NMR (500 MHz, DMSO-*d*₆) δ 9.13 (s, 1H), 8.66 (d, *J =* 2.0 Hz, 1H), 8.41 (s, 1H), 8.04 - 7.94 (m, 2H), 7.74 (s, 1H), 7.64 (s, 1H), 7.38 (s, 1H), 7.30 (s, 1H), 2.24 (tt, *J =* 8.3, 4.7 Hz, 1H), 1.06 (dt, *J =* 8.0, 3.3 Hz, 2H), 0.99 - 0.86 (m, 2H).

### Example 61

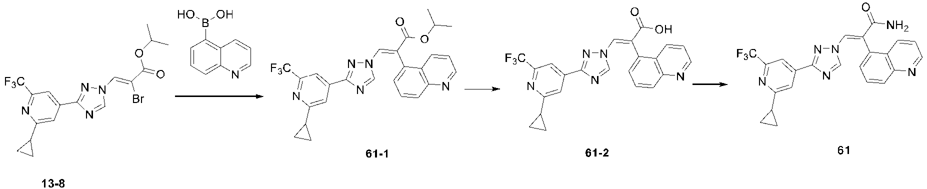

### (1) Preparation of compound 61-1:

Compound **13-8** (200 mg) and quinoline-5-boronic acid (93.4 mg) were dispersed in 1,4-dioxane (5 mL), and potassium carbonate (186.57 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **61-1.**

### (2) Preparation of compound 61-2:

Compound **61-1** (220 mg) was dissolved in tetrahydrofuran (4 mL), a solution of lithium hydroxide hydrate (56.6 mg) and water (4 mL) was added dropwise in an ice-water bath, and the mixture was reacted at room temperature for 4 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **61-2,** which was directly used in the next step.

### (3) Preparation of compound 61:

Compound **61-2** (203.1 mg) was dispersed in tetrahydrofuran (5 mL), N-methylmorpholine (91 mg) and isobutyl chloroformate (123 mg) were added in an ice-water bath, and the mixture was stirred for 30 min. Ammonium hydroxide (94 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **61** (70 mg). ESI-MS: m/z =451.21 [M+H] ⁺.

¹H NMR (500 MHz, DMSO-*d*₆) δ 8.89 (dd, *J =* 4.1, 1.7 Hz, 1H), 8.83 (s, 1H), 8.51 (s, 1H), 8.18 - 8.08 (m, 2H), 7.84 (dd, *J =* 8.5, 7.1 Hz, 1H), 7.57 (s, 1H), 7.50 (dd, *J =* 7.0, 1.2 Hz, 1H), 7.45 (dd, *J* = 8.5, 4.1 Hz, 1H), 7.32 (d, *J =* 1.3 Hz, 1H), 7.17 (d, *J =* 1.4 Hz, 1H), 7.09 (s, 1H), 2.16 (tt, *J =* 8.0, 4.7 Hz, 1H), 1.06 (dd, *J =* 8.2, 2.8 Hz, 2H), 0.85 (q, *J =* 4.2 Hz, 2H).

### Example 62

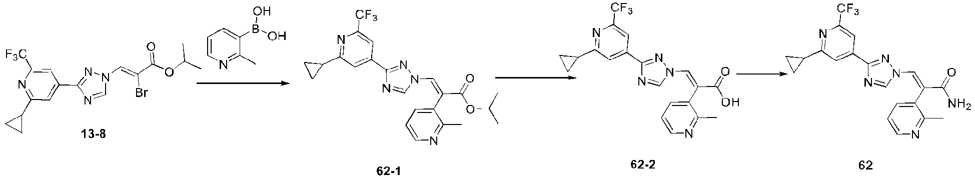

### (1) Preparation of compound 62-1:

Compound **13-8** (200 mg) and 2-methylpyridine-3-boronic acid (73.95 mg) were dispersed in 1,4-dioxane (5 mL), and potassium carbonate (186.6 mg), Pd(dppf)Cl₂ (14.6 mg), and water (0.5 mL) were added. The mixture was heated to 90 °C and reacted under nitrogen atmosphere. After the reaction was completed, the reaction solution was concentrated under reduced pressure and purified by column chromatography to give compound **62-1.**

### (2) Preparation of compound 62-2:

Compound **62-1** (140 mg) was dissolved in tetrahydrofuran (5 mL), a solution of lithium hydroxide hydrate (38.5 mg) and water (5 mL) was added dropwise in an ice-water bath, and the mixture was reacted at room temperature for 2 h. The reaction solution was adjusted to pH 6-7 with hydrochloric acid, extracted with ethyl acetate (20 mL × 2), dried over anhydrous sodium sulfate, and filtered, and the filtrate was evaporated to dryness under reduced pressure to give compound **62-2,** which was directly used in the next step.

### (3) Preparation of compound 62:

Compound **62-2** (124.6 mg) was dispersed in tetrahydrofuran (5 mL), N-methylmorpholine (60.7 mg) and isobutyl chloroformate (81.9 mg) were added in an ice-water bath, and the mixture was stirred for 30 min. Ammonium hydroxide (63 mg) was added dropwise, and the mixture was reacted in an ice-water bath for 10 min. Water was added to quench the reaction, and the resulting mixture was extracted with ethyl acetate (20 mL × 2), dried over anhydrous sodium sulfate, concentrated until no liquid flowed out, and purified by preparative liquid chromatography to give compound **62** (72 mg). ESI-MS: m/z = 415.27[M+H] ⁺.

¹H NMR (500 MHz, DMSO-*d*₆) δ 8.95 (s, 1H), 8.52 (dd, *J =* 4.9, 1.8 Hz, 1H), 8.31 (s, 1H), 7.66 (d, *J* = 1.3 Hz, 1H), 7.57 - 7.52 (m, 1H), 7.50 (dt, *J =* 4.4, 2.4 Hz, 2H), 7.29 (dd, *J =* 7.6, 4.9 Hz, 1H), 7.14 (s, 1H), 2.28 (s, 3H), 2.27 - 2.23 (m, 1H), 1.09 (dt, *J =* 8.3, 3.2 Hz, 2H), 0.96 - 0.90 (m, 2H).

### Example 63

Preparation of crystal form A of compound 21-A: 5 g of compound 21-A sample was weighed out, and 40 mL of isopropyl acetate was added. The mixture was heated to 90 °C under reflux to dissolve completely, and then gradiently cooled. When the temperature reached 0-10 °C, the mixture was stirred for 6 h, and filtered under vacuum. The filter cake was dried in vacuum at 60 °C for 6 h to give the crystal form A, which has an XRPD pattern as shown in FIG. 1, and a TGA profile as shown in FIG. 2.

### Example 64

Preparation of crystal form B of compound 21-A: 5 g of compound 21-A sample was weighed out, and a mixed solvent of 100 mL of isopropyl acetate and 1 mL of water was added. The resulting mixture was heated to 90 °C under reflux to dissolve completely, and then gradiently cooled. When the temperature reached 0-10 °C, the mixture was stirred for 6 h, and filtered under vacuum. 50 mL of water was added to the filter cake, the mixture was heated to 50 °C, and stirred for 3 h; the mixture was then cooled to 20-30 °C, stirred for 12 h, and filtered under vacuum. The filter cake was dried by air blasting at 40 °C for 6 h to give the crystal form B with a water content of about 4 wt% (instrument model: METTLER TOLEDO V20S; method: the product was taken and anhydrous methanol and N,N-dimethylformamide (1:1) were used as solvents; the molar ratio of compound 21-A to water was about 1:1 as determined by the moisture determination method (Method 1, General Chapter 0832, Chinese Pharmacopoeia, Volume IV, 2020 Edition). For the crystal form B, the XRPD pattern is shown in FIG. 3, and the TGA profile is shown in FIG. 4.

### Example 65

Preparation Example I of crystal form C of compound 21-A: 2 g of compound 21-A sample was weighed out, and 20 mL of acetone was added. The mixture was heated to 50 °C to dissolve completely and then stirred for 10 min. The mixture was slowly cooled, stirred at 20-30 °C for 4 h, and filtered under vacuum. The filter cake was dried in vacuum at 40 °C for 6 h to give the crystal form C, which has an XRPD pattern as shown in FIG. 5.

Preparation Example II of crystal form C of compound 21-A: 5 g of compound 21-A sample was weighed out, and 50 mL of acetone was added. The mixture was stirred at 20-30 °C for 6 h and filtered under vacuum, and the filter cake was dried in vacuum at 40 °C for 6 h to give the crystal form C, which has a TGA profile as shown in FIG. 6.

### Example 66

Preparation of crystal form D of compound 21-A: 5 g of compound 21-A sample was weighed out, and 50 mL of isopropanol was added. The mixture was stirred at 20-30 °C for 12 h and filtered under vacuum, and the filter cake was dried in vacuum at 60 °C for 6 h to give the crystal form D, which has an XRPD pattern as shown in FIG. 7, and a TGA profile as shown in FIG. 8. The isopropanol content was (about) 4 wt% based on the analysis of gas phase content.

### Example 67

Preparation of crystal form E of compound 21-A: 5 g of compound 21-A sample was weighed out, and a mixed solvent of 50 mL of ethanol and 1 mL of water was added. The mixture was stirred at 20-30 °C for 12 h and filtered under vacuum, and the filter cake was dried in vacuum at 60 °C for 6 h to give the crystal form E, which has an XRPD pattern as shown in FIG. 9, and a TGA profile as shown in FIG. 10. The ethanol content was (about) 7 wt% based on the analysis of gas phase content.

### Example 68

Preparation of crystal form F of compound 21-A: 5 g of compound 21-A sample was weighed out, and 75 mL of isopropyl acetate, 12.5 mL of methanol, and 2.5 mL of water were added. The mixture was heated under reflux and stirred for 10 min, then slowly cooled, stirred at 20-30 °C for 12 h, and filtered under vacuum. The filter cake was dried in vacuum at 60 °C for 6 h to give the crystal form F, which has an XRPD pattern as shown in FIG. 11, and a TGA profile as shown in FIG. 12. The methanol content was (about) 2 wt% based on the analysis of gas phase content.

### Example 69

Culture and preparation of single crystal G: 15 mg of compound 21-A sample was weighed out and dissolved in 1.5 mL of ethyl acetate, and then the mixture was added to a large glass vial containing 3 mL of n-hexane (antisolvent). The vial was sealed, and n-hexane was allowed to diffuse gradually into the ethyl acetate solution by using the vial-in-vial method. After about 9 days, block-shaped single crystals were grown. The single crystals were isolated and submitted for testing, and the single crystal result was obtained by analysis. The projection of the three-dimensional molecular structure of the single crystal is shown in FIG. 13.

The unit cell parameters, crystallographic data and atomic coordinates, and the like of the single crystal G are shown in Tables 14 and 15 below.

**Table 14. Crystallographic data and structure refinement**

| Experimental molecular formula | 2(C₁₈H₁₂F₆N₆O) ·C₄H₈O₂·3(H₂O) |
|---|---|
| Molecular weight | 1026.82 |
| Temperature | 173(2)K |
| Wavelength | 1.54178A |
| Crystal system | Triclinic |
| Space group | P-1 |
| Unit cell parameters | a = 10.9919(3)Å |
| | b =14.7579(4)Å |
| | c = 16.0678(4) Å |
| | α=108.8840(10)° |
| | β= 105.1420(10)° |
| | γ=98.9680(10)° |
| Volume of unit cell | 2296.60(11) Å³ |
| Z | 2 |
| Calculated density | 1.485 Mg/m³ |
| Absorption correction parameter | 1.202 mm⁻¹ |
| F(000) | 1052 |
| Size of crystal | 0.180 x 0.160 x 0.120 mm |
| Angle range for data collection | 3.083 to 68.362° |
| Collection range for hkl | -13≤h≤13, -17≤k≤17, -19≤l≤19 |
| Reflection data collection/independence | 40884 / 8412 [R(int) = 0.0406] |
| Data integrity for theta = 66.66 | 99.7 % |
| Absorption correction | Semi-empirical from equivalents |
| Maximum and minimum transmission | 0.7531 and 0.6081 |
| Refinement method | Full-matrix least-squares on F₂ |
| Number of data/number of usage restrictions/number of parameters | 8412 / 1 / 674 |
| Degree of fitting of F₂ | 1.053 |
| **Final** R indices [I > 2sigma(I)] | R1 = 0.0526, wR2 = 0.1380 |
| R indices (all data) | R1 = 0.0625, wR2 = 0.1464 |
| Extinction coefficient | n/a |
| Maximum difference (peak top and valley) | 1.139 and -0.706 e.A⁻³ |

**Table 15. Atomic coordinate (×10⁴) and equivalent isotropic displacement parameters (Å2 × 10³)**

| | x | y | z | U(eq) |
|---|---|---|---|---|
| F(3) | 10146(2) | -5649(1) | 545(1) | 58(1) |
| O(3) | 8453(3) | 11109(2) | 8153(2) | 82(1) |
| N(3) | 9083(2) | -982(1) | 3414(1) | 28(1) |
| C(3) | 8632(2) | -3534(2) | 2273(2) | 27(1) |
| F(4) | 5203(2) | -6199(1) | 1402(1) | 53(1) |
| O(4) | 9921(2) | 11456(2) | 9529(2) | 59(1) |
| N(4) | 10378(2) | -1974(1) | 3354(1) | 31(1) |
| C(4) | 7291(2) | -3990(2) | 1913(2) | 29(1) |
| F(5) | 5041(2) | -6048(1) | 93(1) | 52(1) |
| O(5) | 6607(2) | 10189(2) | 6387(2) | 70(1) |
| N(5) | 4453(2) | -2104(1) | 2765(1) | 31(1) |
| C(5) | 6876(2) | -4996(2) | 1391(2) | 30(1) |
| F(6) | 4682(1) | -4938(1) | 1178(1) | 45(1) |
| O(6) | 5771(2) | 2951(1) | 4727(1) | 35(1) |
| N(6) | 6063(2) | 1074(2) | 3691(2) | 41(1) |
| C(6) | 9823(2) | -5766(2) | 1252(2) | 35(1) |
| F(7) | 9595(2) | 336(2) | 2129(3) | 119(1) |
| O(7) | 7305(2) | 2806(2) | 6357(2) | 57(1) |
| N(7) | 7924(2) | 1464(1) | 1892(1) | 32(1) |
| C(7) | 5449(2) | -5547(2) | 1014(2) | 34(1) |
| F(8) | 11191(2) | 1538(2) | 2862(2) | 119(1) |
| N(8) | 8532(2) | 5128(1) | 3697(1) | 25(1) |
| C(8) | 9102(2) | -2464(2) | 2830(2) | 27(1) |
| F(9) | 10337(3) | 1133(3) | 1458(2) | 138(1) |
| N(9) | 9358(2) | 6031(1) | 4278(1) | 24(1) |
| C(9) | 10323(2) | -1049(2) | 3709(2) | 31(1) |
| F(10) | 5360(2) | 606(1) | 1347(2) | 66(1) |
| N(10) | 10630(2) | 5025(1) | 4104(1) | 28(1) |
| C(10) | 8652(2) | -116(2) | 3658(2) | 30(1) |
| F(11) | 4903(1) | 1995(1) | 1823(1) | 60(1) |
| N(11) | 5576(2) | 4543(1) | 4176(1) | 28(1) |
| C(11) | 7432(2) | -45(2) | 3413(2) | 28(1) |
| F(12) | 5427(2) | 1559(2) | 596(1) | 72(1) |
| N(12) | 6632(2) | 8153(1) | 5272(1) | 30(1) |
| F(1) | 9278(2) | -6723(1) | 979(1) | 58(1) |
| C(12) | 7278(2) | 996(2) | 3791(2) | 30(1) |
| C(13) | 5544(2) | -1358(2) | 3240(2) | 29(1) |
| C(14) | 6244(2) | -883(2) | 2830(2) | 26(1) |
| C(15) | 5775(2) | -1209(2) | 1862(2) | 34(1) |
| C(16) | 4655(2) | -1981(2) | 1364(2) | 36(1) |
| C(17) | 4011(2) | -2415(2) | 1833(2) | 32(1) |
| C(18) | 2782(3) | -3248(2) | 1322(2) | 47(1) |
| C(19) | 7117(2) | 2047(2) | 2021(2) | 30(1) |
| C(20) | 7515(2) | 3044(2) | 2585(2) | 28(1) |
| C(21) | 8849(2) | 3482(2) | 3050(1) | 25(1) |
| C(22) | 9704(2) | 2887(2) | 2949(2) | 28(1) |
| C(23) | 9188(2) | 1892(2) | 2366(2) | 31(1) |
| C(24) | 10075(2) | 1220(2) | 2199(2) | 38(1) |
| C(25) | 5700(2) | 1543(2) | 1447(2) | 40(1) |
| C(26) | 9335(2) | 4552(2) | 3618(1) | 24(1) |
| C(27) | 10602(2) | 5949(2) | 4504(2) | 27(1) |
| C(28) | 8962(2) | 6904(2) | 4622(1) | 25(1) |
| C(29) | 7749(2) | 6975(1) | 4585(1) | 24(1) |
| C(30) | 7666(2) | 8033(2) | 5004(1) | 26(1) |
| C(31) | 6567(2) | 5348(2) | 4517(1) | 25(1) |
| C(32) | 6570(2) | 6136(1) | 4219(1) | 24(1) |
| C(33) | 5433(2) | 6083(2) | 3555(2) | 29(1) |
| C(34) | 4386(2) | 5252(2) | 3206(2) | 32(1) |
| C(35) | 4490(2) | 4483(2) | 3509(2) | 29(1) |
| C(36) | 3411(2) | 3545(2) | 3118(2) | 41(1) |
| C(37) | 7860(4) | 10467(4) | 9222(4) | 100(2) |
| C(38) | 8750(3) | 11037(2) | 8905(3) | 60(1) |
| C(39) | 10848(4) | 12007(3) | 9270(3) | 69(1) |
| C(40) | 12128(5) | 12279(6) | 9989(4) | 149(3) |
| O(1) | 8226(2) | 1725(1) | 4159(1) | 41(1) |
| N(1) | 7670(2) | -5572(1) | 1185(1) | 31(1) |
| C(1) | 8941(2) | -5119(2) | 1530(2) | 30(1) |
| F(2) | 10920(2) | -5544(1) | 1953(1) | 67(1) |
| O(2) | 8523(2) | 8737(1) | 5094(1) | 35(1) |
| N(2) | 8279(2) | -1898(1) | 2844(1) | 29(1) |
| C(2) | 9477(2) | -4117(2) | 2083(2) | 29(1) |

### Example 70

### Preparation of crystal form A of compound 3-A:

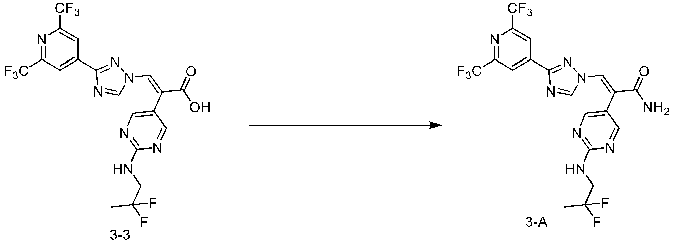

Compound **3-3** (24 g) was dispersed in 1,4-dioxane (240 mL), and pyridine (11.52 g) and Boc anhydride (27.12 g) were added. The mixture was reacted at room temperature for 30 min, and ammonium bicarbonate (9.84 g) was added. The mixture was stirred at room temperature for 12 h. Water (500 mL) was added to the reaction solution, and the mixture was stirred to precipitate a product and filtered under vacuum. The filter cake was dispersed in ethyl acetate (2.0 L), washed with an aqueous sodium bicarbonate solution and an aqueous sodium chloride solution in sequence, dried over anhydrous sodium sulfate, and then filtered and concentrated. 240 mL of isopropyl acetate/n-heptane (v/v = 1:1) was added to the concentrate, and the mixture was stirred at room temperature for 30 min and filtered to give a filter cake. The filter cake was dried, and then heated to 80 °C with 240 mL of isopropyl acetate. The mixture was stirred for 30 min, slowly cooled to room temperature and stirred for 4 h, filtered, and dried to give the crystal form A of compound **3-A** (9 g), which has an XRPD pattern as shown in FIG. 14, a TGA profile as shown in FIG. 15, and a DSC curve as shown in FIG. 16.

### Example 71

Preparation of crystal form B of compound 3-A: 50 mg of the crystal form A of compound 3-A prepared in Example 70 was weighed out and dissolved in 5 mL of 1,4-dioxane in a water bath at 65 °C. 25 mL of petroleum ether was added for crystallization. The mixture was centrifuged, and the supernatant was removed. The residue was dried at 40 °C to give the crystal form B, which has an XRPD pattern as shown in FIG. 17, and a TGA profile as shown in FIG. 17-A. Based on the analysis of gas phase content or calculation of TGA result data, the molar ratio of compound 3-A to 1,4-dioxane in the crystal form B of compound 3-A was (about) 1:0.5.

### Example 72

Preparation of crystal form C of compound 3-A: 50 mg of the crystal form A of compound 3-A prepared in Example 70 was weighed out and dissolved in 10 mL of tetrahydrofuran. The mixture was then dried in a water bath at 65 °C to give the crystal form C, which has an XRPD pattern as shown in FIG. 18, and a TGA profile as shown in FIG. 18-A. Based on the analysis of gas phase content or calculation of TGA result data, the molar ratio of compound 3-A to tetrahydrofuran in the crystal form C of compound 3-A was (about) 1:0.5.

### Example 73

### Preparation of crystal form A of compound 15-A:

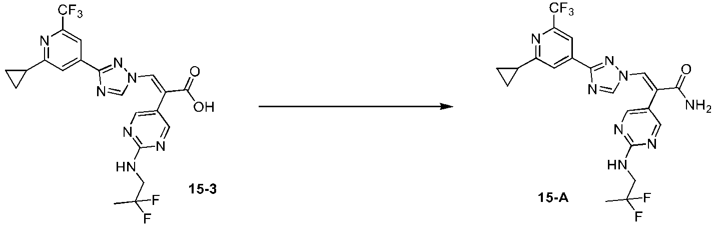

Compound 15-3 (32.5 g) was dispersed in 1,4-dioxane (300 mL), and pyridine (15.42 g) and Boc anhydride (36 g) were added. The mixture was reacted at room temperature for 30 min, and ammonium bicarbonate (13.05 g) was added. The mixture was stirred at room temperature for 12 h. Water (600 mL) was added to the reaction solution, and the mixture was stirred to precipitate a solid and filtered under vacuum. The filter cake was dispersed in ethyl acetate (2.4 L), washed with an aqueous sodium bicarbonate solution and an aqueous sodium chloride solution in sequence, dried over anhydrous sodium sulfate, and then filtered and concentrated. The concentrate was recrystallized in isopropyl acetate (300 mL), and the resulting product was filtered, and dried to give crystal form A of compound 15-A (9 g), which has an XRPD pattern as shown in FIG. 19, a TGA profile as shown in FIG. 20, and a DSC curve as shown in FIG. 21.

### Example 74

Preparation of crystal form B of compound 15-A: 100 mg of the crystal from A of compound 15-A prepared in Example 73 was weighed out and dissolved in 30 mL of acetonitrile. The mixture was then cooled for crystallization, and the resulting product was dried at 40 °C to give the crystal form B of compound 15-A, which has an XRPD pattern as shown in FIG. 22. Based on the analysis of gas phase content or calculation of TGA result data, the molar ratio of compound 15-A to acetonitrile in the crystal form B of compound 15-A was (about) 1:1.

### Example 75

### Preparation of crystal form A of compound 25-A:

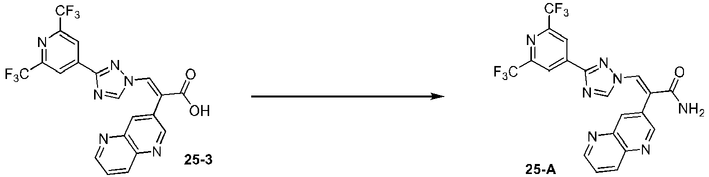

Compound 25-3 (14.7 g) was dispersed in 1,4-dioxane (150 mL), and pyridine (7.74 g) and Boc anhydride (18 g) were added. The mixture was reacted at room temperature for 30 min, and ammonium bicarbonate (6.53 g) was added. The mixture was stirred at room temperature for 12 h. Water (300 mL) was added to the reaction solution, and the mixture was stirred to precipitate a solid and filtered under vacuum. The filter cake was dispersed in ethyl acetate (1.2 L), washed with an aqueous sodium bicarbonate solution and an aqueous sodium chloride solution in sequence, dried over anhydrous sodium sulfate, and then filtered and concentrated. The concentrate was dispersed in 150 mL of isopropyl acetate. The mixture was heated to 80 °C and stirred for 30 min, then slowly cooled to room temperature and stirred for 4 h, filtered, and dried to give crystal form A of compound 25-A (6 g), which has an XRPD pattern as shown in FIG. 23.

### Test Example 1. Inhibitory Activity on In Vitro Cell Proliferation

### 1.1 Assay for inhibitory activity on Jurkat cell proliferation

Jurkat cells in a good growth state were collected into a centrifuge tube, adjusted to a cell density of 5 × 10⁴ cells/mL, and seeded in a 96-well plate (100 µL/well). The cells were incubated overnight in a cell incubator. Compounds were added using a nanoliter pipettor such that the final concentrations of the compounds were 1000 nM-0.46 nM (the addition was performed in duplicate). Meanwhile, a control was set. After another 72 h of incubation in the cell incubator, the assay reagent CCK-8 (manufacturer: Dojindo Laboratories, Beijing; 10 µL/well) was added. After 2 h of incubation in the cell incubator, absorbance was measured at 450 nm on an Envision microplate reader. A four-parameter analysis was performed, a dose-response curve was fit, and IC₅₀ was calculated.

### 1.2 Assay for inhibitory activity on OCI-LY10 cell proliferation

OCI-LY10 cells in a good growth state were collected into a centrifuge tube, adjusted to a cell density of 9 × 10⁴ cells/mL, and seeded in a 96-well plate (100 µL/well). The cells were incubated overnight in a cell incubator. Compounds were added using a nanoliter pipettor such that the final concentrations of the compounds were 1000 nM-0.46 nM (the addition was performed in duplicate). Meanwhile, a control was set. After another 72 h of incubation in the cell incubator, the assay reagent CCK-8 (manufacturer: Dojindo Laboratories, Beijing; 10 µL/well) was added. After 2 h of incubation in the cell incubator, absorbance was measured at 450 nm on an Envision microplate reader. A four-parameter analysis was performed, a dose-response curve was fit, and IC₅₀ was calculated.

### 1.3 Assay for inhibitory activity on RPMI-8226 cell proliferation

RPMI-8226 cells in a good growth state were collected into a centrifuge tube. The cell density was adjusted, and the cells were seeded in a 96-well plate and incubated overnight in a cell incubator. Compounds were added using a nanoliter pipettor, and a control was set. The plate was placed in the cell incubator for further incubation, and the assay reagent was added. After the cells were incubated in the cell incubator for a period of time, absorbance was measured on an Envision microplate reader. A four-parameter analysis was performed, a dose-response curve was fit, and IC₅₀ was calculated.

The specific results are shown in Table 16. For the inhibitory activity on Jurkat cell proliferation, A represents IC₅₀ ≤ 20 nM; For the inhibitory activity on OCI-LY10 cell proliferation, + represents IC₅₀ ≤ 40 nM.

**Table 16. Activity assay results for compounds**

| Compound/Ex ample No. | Inhibitory activity on Jurkat cell proliferation (IC₅₀, nM) | Inhibitory activity on OCI-LY10 cell proliferation (IC₅₀, nM) |
|---|---|---|
| 1 | A | + |
| 2(2-A) | A | + |
| 3(2-A) | A | + |
| 4 | A | + |
| 5 | A | + |
| 10 | A | + |
| 11(11-A) | A | + |
| 13 | A | + |
| 14 | A | + |
| 15(15-A) | A | + |
| 16 | A | + |
| 17 | A | + |
| 25(25-A) | A | + |
| 26 | A | + |
| 30 | A | + |
| 32 | A | + |
| 33 | A | + |
| 36 | A | + |
| 37 | A | + |
| 38(38-A) | A | + |
| 39 | A | + |
| 40 | A | + |
| 41 | A | + |
| 43 | A | + |
| 46 | A | + |
| 53 | A | + |
| 54 | A | + |
| 57 | A | + |
| 59(59-A) | A | + |
| 60 | A | + |

The compounds of the present disclosure showed good results in the *in vitro* cell proliferation inhibitory activity assay.

### Test Example 2. In Vitro Liver Microsomal Stability

Liver microsome incubation samples were prepared by mixing a PBS buffer (pH = 7.4), a liver microsome solution (0.5 mg/mL), a test compound, and an NADPH + MgCl₂ solution, and incubated at 37 °C and 300 rpm for 1 h. Zero-hour samples were prepared by mixing a PBS buffer (pH = 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The assay results are shown in Table 17.

**Table 17. In vitro metabolic stability of related compounds in liver microsomes of various species**

| Compound/Example No. | Mouse liver microsome | Human liver microsome |
|---|---|---|
| | Residuals at 60 min (%) | |
| 1 | 97.97% | 101.38% |
| 2(2-A) | 87.82% | 92.23% |
| 3(3-A) | 94.26% | 95.65% |
| 4 | 93.27% | 100.33% |
| 5 | 99.03% | 97.64% |
| 8 | 98.98% | 101.7% |
| 10 | 93.99% | 95.75% |
| 11(11-A) | 89.63% | 86.18% |
| 13 | 91.15% | 93.23% |
| 14 | 83.14% | 80.48% |
| 15(15-A) | 88.38% | 87.55% |
| 16 | 90.60% | 88.75% |
| 17 | 95.11% | 93.92% |
| 21(21-A) | 99.28% | 94.26% |
| 25(25-A) | 94.66% | 110.48% |
| 26 | 90.27% | 96.64% |
| 27 | 94.24% | 102.54% |
| 30 | 89.94% | 98.75% |
| 32 | 95.08% | 100.32% |
| 33 | 98.21% | 104.79% |
| 34 | 87.79% | 101.21% |
| 38(38-A) | 83.25% | 102.40% |
| 39 | 89.18% | 106.91% |

The compounds of the present disclosure showed good properties in the liver microsomal stability assay.

### Test Example 3. In Vivo Pharmacokinetics

### 3.1 Mouse pharmacokinetics

ICR mice weighing 18-22 g were randomized into groups of 9 mice per group after 3-5 days of acclimatization and then administered intragastrically at a dose of 3 mg/kg.

Plasma samples to be tested were prepared by taking blood from the orbit at time points of 15 min, 30 min, 1 h, 2 h, 4 h, 6 h, 8 h, 10 h, and 24 h.

20 µL of each plasma sample to be tested and a standard curve sample were taken, and an acetonitrile solution containing an internal standard was added. A supernatant was obtained by protein precipitation, diluted, and then assayed by LC/MS/MS.

Pharmacokinetic parameters were fitted using a non-compartmental model. The assay results are shown in Table 18.

**Table 18**

| Compound/ Example No. | Mice administered by single intravenous injection 1 mpk | | | | Mice administered intragastrically 3 mpk | | | | | |
|---|---|---|---|---|---|---|---|---|---|---|
| | AUC (0-24) (h*ng/mL) | AUC (0-∞) h*ng/mL | t1/2 (h) | CL (L/h/kg) | AUC (0-24) (h*ng/mL) | AUC (0-∞) (h*ng/mL) | Tmax(h) | t1/2 (h) | Cmax (ng/mL) | Absolute bioavailability F (%) |
| 2(2-A) | 874 | 879 | 1.26 | 1.14 | 2726 | 2731 | 3.0 | 2.57 | 483 | 103.93% |
| 3(3-A) | 1544 | 1574 | 1.66 | 0.635 | 5277 | 5296 | 3.0 | 2.80 | 681 | 113.9% |
| 15(15-A) | 1619 | 1629 | 1.34 | 0.614 | 4604 | 4654 | 3.0 | 3.48 | 599 | 94.77% |
| 21(21-A) | 1629 | 1633 | 3.02 | 0.61 | 4754 | 4759 | 1.0 | 2.35 | 871 | 97.31% |
| 38(38-A) | 1037 | 1044 | 1.41 | 0.958 | 3485 | 3804 | 1.0 | 2.55 | 874 | 112.06% |

The compounds of the present disclosure showed good properties in the pharmacokinetic assay, including but not limited to, good bioavailability, AUC, and the like.

### 3.2 Mouse brain distribution

ICR mice weighing 20-26 g were randomized into groups of 3 mice per group after 3-5 days of acclimatization and then administered intragastrically at a dose of 5 mg/kg.

Plasma samples to be tested were prepared by taking blood from the orbit at a time point of 3 h. Meanwhile, brain tissue at each time point was collected, and the homogenate was weighed.

20 µL of each plasma to be tested and tissue homogenate sample and a standard curve sample were taken, and an acetonitrile solution was added. A supernatant was obtained by protein precipitation, diluted, and then assayed by LC/MS/MS. The brain-to-blood ratio was calculated.

The brain-to-blood ratios of the compounds of the present disclosure were less than 1, showing a low brain uptake amount.

### Test Example 4. In Vivo Pharmacodynamics

Efficacy evaluation in OCI-LY10 human diffuse large B-cell lymphoma NOD-SCID mouse subcutaneous xenograft tumor model
(1) SPF female NOD-SCID mice (source: Jiangsu Huachuang Xinnuo Pharmaceutical Technology Co., Ltd.) were inoculated subcutaneously at the right side armpit with OCI-LY10 human diffuse large B-cell lymphoma cells at 1 × 10⁷ cells/mouse. When the mean tumor volume reached about 200 mm³, the animals were divided into a control group (vehicle control group) and treatment groups (compound groups).

The administration dose included 1 mg/kg, 2 mg/kg, and 4 mg/kg, and the administration frequency was once daily. The day of grouping was defined as day 0, and intragastric administration was started on day 0 for 21 consecutive days.

Alternatively, the administration dose was 4 mg/kg, and the administration frequency was 5 times per week. The day of grouping was defined as day 0, and intragastric administration was started on day 0 five times per week for two consecutive weeks.

The tumor volume was measured once every 3 days, and meanwhile, the mice were weighed and the data were recorded; the general behavior of the mice was observed and recorded every day. After the experiment was completed, the tumors were removed, weighed, and photographed.

The detection index and the calculation formula are as follows:
Tumor volume TV (mm³) = 1/2 × (a × b²), wherein a is the long diameter of the tumor, and b is the short diameter of the tumor;
Relative tumor volume RTV = TVₜ/TV₀, wherein TV₀ is the tumor volume on day 0, and TVₜ is the tumor volume at each measurement;
Relative tumor proliferation rate T/C (%) = (T_{RTV}/C_{RTV}) × 100%, wherein T_{RTV} is RTV of the treatment group, and C_{RTV} is RTV of the vehicle control group;
Tumor growth inhibition rate TGI (%) = (1 - TW/TW₀) × 100%; wherein TW is the tumor weight of the treatment group, and TW₀ is the tumor weight of the vehicle control group;
Weight change rate WCR (%) = (Wtₜ - Wt₀)/Wt₀ × 100%, wherein Wt₀ is the body weight of the mouse on day 0, and Wtₜ is the body weight of the mouse at each measurement.

The specific assay results are shown in Table 19 and Table 20.

**Table 19. Effect on OCI-LY10 cell (NOD-SCID) mouse subcutaneous xenograft tumor**

| Compound/Ex ample No. | Dose | Day 21 of assay | | | Day 23 of assay | |
|---|---|---|---|---|---|---|
| | | TV(mm³) (mean±SD) | RTV (mean±SD) | T/C(%) | Weight change rate (%) (mean ± SD) | TGI(%) |
| Control group | N/A | 2260±850 | 10.8±4.3 | ---- | 9.9±4.7 | ---- |
| 21(21-A) | 2mg/kg | 520±178 | 2.4±0.4 | 22.2% | -2.0±6.5 | 89.3%* |
| 25(25-A) | 4mg/kg | 360±105 | 1.8±0.6 | 16.7% | 1.7±5.3 | 91.1%* |

| | | | | | | |
|---|---|---|---|---|---|---|
| Note: comparison with the control group, where * denotes p < 0.05 and ** denotes p < 0.01. | | | | | | |

**Table 20. Effect on OCI-LY10 cell (NOD-SCID) mouse subcutaneous xenograft tumor**

| Compound/ Example No. | Dose | Day 18 of assay | | | Day 21 of assay | |
|---|---|---|---|---|---|---|
| | | TV(mm³) (mean±SD) | RTV (mean±SD) | T/C(%) | Weight change rate (%) (mean ± SD) | TGI(%) |
| Control group | N/A | 1525±586 | 7.8±3.5 | ---- | 7.9±1.0 | ---- |
| 11(11-A) | 4mg/kg | 326±52 | 1.7±0.2 | 21.8% | 0.6±8.1 | 89.7%** |

| | | | | | | |
|---|---|---|---|---|---|---|
| Note: comparison with the control group, where ** denotes p < 0.01. | | | | | | |

The compounds of the present disclosure had no significant toxicity in the assay, and the results showed that the compounds had good tumor growth inhibition rates.

### Test Example 5: Study on Stability of Crystal Forms

Crystalline forms of the test samples were taken for (conventional) stability studies or influencing factor (including high temperature, high humidity, and light exposure) tests.

The crystalline forms of the test samples were placed in an open, suitable clean flat weighing bottle and left to stand at 60 °C; or at 92.5% RH/25 °C (or 75% RH/40 °C); high humidity (75% RH/25 °C) or high temperature and high humidity (60 °C ± 2 °C and 75% RH); or at room temperature (25 °C ± 2 °C) for 7 days (10 days, 30 days, 2 months; or 3 months); or in a pharmaceutical strong light exposure test chamber (temperature: 25 °C, illuminance: about 5000 Lux, near-ultraviolet energy: 85.0 µW/cm ²). The crystal form and chemical purity (appearance, purity, total impurity, and maximum single impurity content) of the samples were detected.

### Determination method for related substances:

**Test sample solution:** An appropriate amount of the test sample was accurately weighed out and dissolved in a solvent. The mixture was diluted to prepare a solution containing about 0.5 mg of the test sample per 1 mL.

**Chromatographic conditions:** Octadecylsilane chemically bonded to silica gel was used as a filler. Linear gradient elution was performed using 0.1% aqueous ammonia solution (adjusted to pH 5.0 with formic acid) as mobile phase A and acetonitrile as mobile phase B according to the following table. The flow rate was 1.0 mL/min, the column temperature was 40 °C, the detection wavelength was 276 nm, and the injection volume was 10 µL. Linear elution was performed.

After placement under light exposure (without packaging) for a period of time, the total impurity increase of the crystalline forms of the compound of formula (I) or the solvate thereof of the present application (e.g., compound 21-A, 3-A, 15-A, or 25-A) was ≤ 0.2% (for some crystal forms, the change was almost negligible, e.g., ≤ 0.02%), and the maximum single impurity increase was ≤ 0.1% (for some crystal forms, the change was almost negligible), for example, after placement for 7 days or longer.

Under high temperature (60 °C) and/or high humidity (75% RH) conditions, the total impurity and maximum single impurity content of the crystalline forms of the compound of formula (I) or the solvate thereof of the present application (e.g., compound 21-A, 3-A, 15-A, or 25-A) was almost no increase, for example, after placement for 7 days or longer.

Under conventional stability study conditions (25 °C, pharmaceutical low-density polyethylene bags), the total impurity and maximum single impurity content of the crystalline forms of the present application (e.g., compound 21-A, 3-A, 15-A, or 25-A) was almost no increase, for example, after placement for 7 days or longer.

The test results demonstrated that the crystal forms of the compound of formula (I) or the solvate thereof of the present disclosure (e.g., compound 21-A, 3-A, 15-A, or 25-A) exhibited good stability, and their appearance, related substances, and purity were not significantly changed under the conditions of high temperature, high humidity, and light exposure.

### Test Example 6: Hygroscopicity Study

Hygroscopicity was measured with reference to the Guidelines for Hygroscopicity Test of Drugs, Chapter 9103, Chinese Pharmacopoeia, Volume IV, 2020 Edition. A dynamic vapor sorption (DVS) analyzer was used to measure the hygroscopicity of the crystalline active pharmaceutical ingredient of the compound of formula (I) of the present application over a relative humidity range of 0%-90%.

The results showed that in terms of hygroscopicity, the crystal forms of the compound of formula (I) or the solvate thereof (e.g., compound 21-A, 3-A, 15-A, or 25-A) of the present application had a hygroscopic weight gain of less than 0.2%, exhibited almost no hygroscopicity, and had good stability.

### Test Example 7: Solubility

Appropriate amounts of 0.1 mol/L hydrochloric acid solution, purified water, acetate buffer (pH 4.5), and phosphate buffer (pH 6.8) were prepared, and appropriate amounts of different crystalline forms of the test samples were added to each solution, respectively. The mixtures were placed in a thermostatic shaker at 37 °C and taken out at 2 h and 24 h, and the apparent solubility and saturated solubility of the products were determined.

### Test Example 8: Intrinsic Dissolution Rate

About 100 mg of each of the different crystalline forms of the test samples was taken. Dissolution media of hydrochloric acid solution (0.1 mol/L), acetate buffer (pH 4.5), and phosphate buffer (pH 6.8) (700 mL each) were used to determine the intrinsic dissolution rate of the drugs, respectively.

### Test Example 9: Degradation Study

Acidic degradation: Different crystalline forms of the test samples were weighed out, and an aqueous HCl solution was added. The resulting mixture was left to stand at room temperature for 24 h (or in a water bath at 80 °C for 4 h), and then neutralized with an aqueous NaOH solution. The solution was diluted with a diluent (acetonitrile and/or water) to the required volume, and an appropriate amount of the solution was taken for analysis.

The test results showed that the crystalline forms of the compound of formula (I) or the solvate thereof of the present application (e.g., compound 21-A, 3-A, 15-A, or 25-A) were stable under acidic conditions, and fewer degradation impurities were produced in the test sample solution after the acid degradation test.

Alkali degradation: Different crystalline forms of the test samples were weighed out, and an aqueous NaOH solution was added. The resulting mixture was left to stand at room temperature for 24 h (or in a water bath at 80 °C for 4 h), and then neutralized with an aqueous HCl solution. The solution was diluted with a diluent (acetonitrile and/or water) to the required volume, and an appropriate amount of the solution was taken for analysis.

The test results showed that after the crystalline forms of the present application were subjected to the alkaline degradation test, the degradation impurity content in the test sample solution was < 3%.

### Test Example 10: Crystal Habit (Scanning Electron Microscopy)

The morphology of the products of different crystal forms was observed using a scanning electron microscope.

### Test Example 11: Pharmacokinetic Study

Mouse study: ICR mice were randomly grouped and subjected to intragastric administration.

The test animals were fasted for 12 h before administration and fed 4 h after administration, and were given free access to water before, after, and during the study. Plasma samples to be tested were prepared by taking blood from the orbit at time points of 0.25 h (15 min), 0.5 h (30 min), 1 h, 2 h, 4 h, 6 h, 8 h, 10 h, and 24 h after administration. Each plasma sample to be tested and a standard curve sample were taken, and an acetonitrile solution containing an internal standard was added. A supernatant was obtained by protein precipitation, diluted, and then assayed by LC/MS/MS. Chromatograms were recorded.

Rat study: Male SD rats were grouped and subjected to intragastric administration.

The test animals were fasted for 12 h before administration and fed 4 h after administration, and were given free access to water before, after, and during the study. Blood samples were collected at 0.25 h (15 min), 0.5 h (30 min), 1 h, 2 h, 4 h, 6 h, 8 h, 10 h, and 24 h after administration. Blood samples were collected via the retro-orbital venous plexus and placed into EDTA-K2 anticoagulation tubes. The samples were centrifuged, and the plasma was transferred to a centrifuge tube and stored at -80 °C until analysis. The plasma samples were extracted using the protein precipitation method. The extracts were analyzed by LC/MS/MS, and chromatograms were recorded.

Beagle dog study: After a period of acclimatization, male beagle dogs were randomly grouped and subjected to intragastric administration.

The test animals (male beagle dogs) were fasted for 12 h before administration and fed 4 h after administration, and were given free access to water before, after and during the study. After administration, blood samples were collected from the forelimb vein at 0.25 h (15 min), 0.5 h (30 min), 1 h, 2 h, 4 h, 6 h, 8 h, 10 h, 24 h, 30 h, 48 h, and 72 h and placed into EDTA-K2 anticoagulation vacutainers, and plasma was separated by centrifugation. All the plasma samples were collected and immediately stored at -80 °C for testing. Each plasma sample to be tested and a standard curve sample were taken, and an acetonitrile solution containing an internal standard (diazepam) was added. The mixture was oscillated and mixed well, and centrifuged. The supernatant was taken, diluted with ultrapure water, and mixed well for LC/MS/MS determination. Chromatograms were recorded.

Oral exposure of different crystal forms of the compound of the present application was evaluated by *in vivo* pharmacokinetic studies in mice, rats, or beagle dogs.

## Claims

1. A compound of formula I, a stereoisomer thereof, or a pharmaceutically acceptable salt thereof, 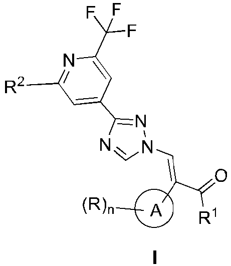 wherein,
R¹ is selected from the group consisting of -NH₂, C₁₋₆ alkyl-O-, C₁₋₆ alkyl-NH-, and (C₁₋₆ alkyl)₂N-;
ring A is selected from the group consisting of a C₆₋₁₀ aromatic ring group and a 5- to 10-membered heteroaromatic ring group;
R is selected from the group consisting of halogen, CN, OH, NH₂, C₁₋₆ alkyl, C₁₋₆ alkyl-O-, C₁₋₆ alkyl-S-, C₁₋₆ haloalkyl-O-, C₁₋₆ haloalkyl-S-, C₁₋₆ haloalkyl, C₁₋₆ alkyl substituted with 3- to 10-membered heterocycloalkyl, R^{a}NH-, and (R^{a})₂N-;
R^{a} is selected from the group consisting of C₁₋₆ alkyl, C₃₋₁₀ cycloalkyl, and 3- to 10-membered heterocycloalkyl, and R^{a} is optionally substituted with one or more groups selected from the group consisting of halogen, CN, OH, NH₂, C₁₋₆ alkyl, C₁₋₆ alkyl-O-, and 5- to 6-membered heterocycloalkyl;
n is selected from the group consisting of 0, 1, 2, 3, and 4;
R² is selected from the group consisting of cyclopropyl and trifluoromethyl.

2. The compound of formula I, the stereoisomer thereof, or the pharmaceutically acceptable salt thereof according to claim 1, being selected from the group consisting of a crystalline form or an amorphous form of the compound of formula I, a crystalline form or an amorphous form of the stereoisomer thereof, a crystalline form or an amorphous form of the pharmaceutically acceptable salt thereof, and a solvate of the compound of formula I.

3. A crystalline form or an amorphous form of a compound of formula I, or a solvate of a compound of formula I, wherein the compound of formula I is as shown in claim 1.

4. A crystalline form of a compound of formula I, or a solvate of a compound of formula I, wherein the compound of formula I is as shown in claim 1.

5. The compound, the crystalline form or the amorphous form thereof, the stereoisomer thereof, the pharmaceutically acceptable salt thereof, or the solvate thereof according to any one of claims 2 to 4, wherein the solvate of the compound of formula I is selected from a crystalline form or an amorphous form of the solvate of the compound of formula I.

6. The compound, the crystalline form or the amorphous form thereof, the stereoisomer thereof, the pharmaceutically acceptable salt thereof, or the solvate thereof according to any one of claims 2 to 5, wherein the solvate of the compound of formula I has a solvent content of: 0.5%-50% wt, 0.5%-40% wt, 0.5%-30% wt, 0.5%-20% wt, 1%-15% wt, 1.5%-12% wt, 2%-10% wt, or 2%-7% wt;
alternatively, the solvate of the compound of formula I has a solvent content of: 1% wt, 2% wt, 3% wt, 4% wt, 5% wt, 6% wt, 7% wt, 8% wt, 9% wt, 10% wt, 11% wt, 12% wt, 13% wt, 14% wt, 15% wt, 16% wt, 17% wt, 18% wt, 19% wt, or 20% wt, or a range formed by any of the above values.

7. The compound, the crystalline form or the amorphous form thereof, the stereoisomer thereof, the pharmaceutically acceptable salt thereof, or the solvate thereof according to any one of claims 2 to 6, wherein in the solvate of the compound of formula I, a molar ratio of the compound of formula I to the solvent is selected from the group consisting of 1:0.1-50, 1:0.1-30, 1:0.1-20, 1:0.1-15, 1:0.1-10, 1:0.15-10, 1:0.2-10, 1:0.2-8, 1:0.2-8, 1:0.2-6, 1:0.2-5, 1:0.2-4, 1:0.2-3, 1:0.2-2, 1:0.2-1, and 1:0.3-1;
alternatively, in the solvate of the compound of formula I, a molar ratio of the compound of formula I to the solvent is 1:0.2, 1:0.3, 1:0.4, 1:0.5, 1:1, 1:2, 1:3, 1:4, 1:5, 1:6, 1:7, 1:8, 1:9, or 1:10, or a range formed by any of the above values.

8. The compound, the crystalline form or the amorphous form thereof, the stereoisomer thereof, the pharmaceutically acceptable salt thereof, or the solvate thereof according to any one of claims 2 to 7, wherein the solvent in the solvate of the compound of formula I is selected from one or more of the group consisting of water, hydrocarbons, alcohols, ethers, esters, amides, sulfoxides, ketones, and nitriles;
alternatively, the solvent in the solvate of the compound of formula I is selected from one or more of the group consisting of water, C₅₋₁₀ hydrocarbons, C₁₋₁₀ alcohols, C₂₋₁₀ ethers, C₂₋₁₀ esters, C₂₋₁₀ amides, C₂₋₁₀ sulfoxides, C₂₋₁₀ ketones, and C₂₋₁₀ nitriles;
alternatively, the solvent in the solvate of the compound of formula I is selected from one or more of the group consisting of water, C₁₋₃ alcohols, C₂₋₆ ethers, C₄₋₆ esters, C₃₋₄ ketones, and C₂₋₄ nitriles;
alternatively, the solvent in the solvate of the compound of formula I is selected from one or more of the group consisting of water, methanol, ethanol, isopropanol, 1,4-dioxane, tetrahydrofuran, ethyl acetate, isopropyl acetate, acetone, and acetonitrile.

9. The compound of formula I, the crystalline form or the amorphous form thereof, the stereoisomer thereof, the pharmaceutically acceptable salt thereof, or the solvate thereof according to any one of claims 1 to 8, wherein R¹ is selected from the group consisting of -NH₂, C₁₋₄ alkyl-O-, C₁₋₄ alkyl-NH-, and (C₁₋₄ alkyl)₂N-;
alternatively, R¹ is selected from the group consisting of -NH₂, isopropyl-O-, and methyl-NH-.

10. The compound of formula I, the crystalline form or the amorphous form thereof, the stereoisomer thereof, the pharmaceutically acceptable salt thereof, or the solvate thereof according to any one of claims 1 to 9, wherein ring A is selected from the group consisting of phenyl and a 5- to 10-membered heteroaromatic ring group;
alternatively, ring A is selected from the group consisting of 5-, 6-, 7-, 8-, 9-, and 10-membered heteroaromatic ring groups;
alternatively, ring A is selected from the group consisting of 6- and 10-membered heteroaromatic ring groups.

11. The compound of formula I, the crystalline form or the amorphous form thereof, the stereoisomer thereof, the pharmaceutically acceptable salt thereof, or the solvate thereof according to any one of claims 1 to 10, wherein R is selected from the group consisting of halogen, CN, OH, NH₂, C₁₋₄ alkyl, C₁₋₄ alkyl-O-, C₁₋₄ alkyl-S-, C₁₋₄ haloalkyl-O-, C₁₋₄ haloalkyl-S-, C₁₋₄ haloalkyl, R^{a}NH-, and (R^{a})₂N-;
alternatively, R is selected from the group consisting of halogen, CN, OH, NH₂, C₁₋₃ alkyl, C₁₋₃ alkyl-O-, C₁₋₃ alkyl-S-, C₁₋₃ haloalkyl-O-, C₁₋₃ haloalkyl-S-, C₁₋₃ haloalkyl, R^{a}NH-, and (R^{a})₂N-; alternatively, R is selected from the group consisting of fluorine, CN, NH₂, methyl, methoxy, trifluoromethyl, and R^{a}NH-;
optionally, R^{a} is selected from the group consisting of C₁₋₄ alkyl, C₃₋₆ cycloalkyl, and 3- to 6-membered heterocycloalkyl, and R^{a} is optionally substituted with one or more groups selected from the group consisting of halogen, CN, OH, NH₂, and 5- to 6-membered heterocycloalkyl;
alternatively, R^{a} is selected from the group consisting of C₁₋₃ alkyl and 6-membered heterocycloalkyl, and R^{a} is optionally substituted with one or more groups selected from the group consisting of fluorine, chlorine, bromine, CN, OH, NH₂, and 6-membered heterocycloalkyl;
alternatively, R^{a} is selected from the group consisting of FCH₂CH₂-, F₂CHCH₂-, F₃CCH₂-, CF₃CH(CH₃)-, CH₃CF₂CH₂-, tetrahydropyranyl, and dioxane-CH₂-;
optionally, n is selected from the group consisting of 0, 1, 2, and 3;
alternatively, n is selected from the group consisting of 0, 1, and 2;
alternatively, n is selected from the group consisting of 0 and 1.

12. The compound of formula I, the crystalline form or the amorphous form thereof, the stereoisomer thereof, the pharmaceutically acceptable salt thereof, or the solvate thereof according to any one of claims 1 to 11, wherein the compound of formula I is selected from the group consisting of a compound of formula I-1 and a compound of I-2, 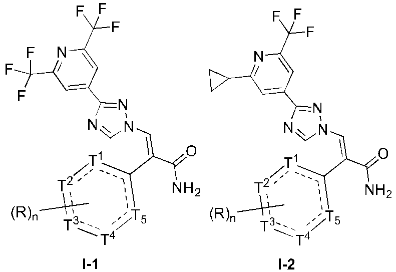 wherein T¹, T², T³, T⁴, and T⁵ are each independently selected from the group consisting of a bond, O, S, N, and CH, no more than one of which is selected from a bond and at least one of which is selected from N; 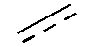 represents a single bond or a double bond.

13. The compound of formula I, the crystalline form or the amorphous form thereof, the stereoisomer thereof, the pharmaceutically acceptable salt thereof, or the solvate thereof according to any one of claims 1 to 12, wherein the compound of formula I is selected from the group consisting of the following compounds: 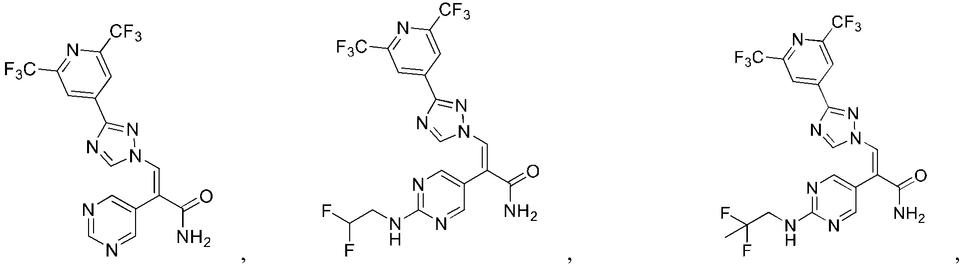 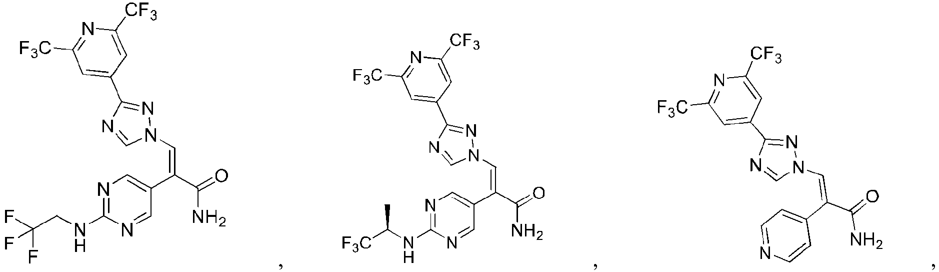 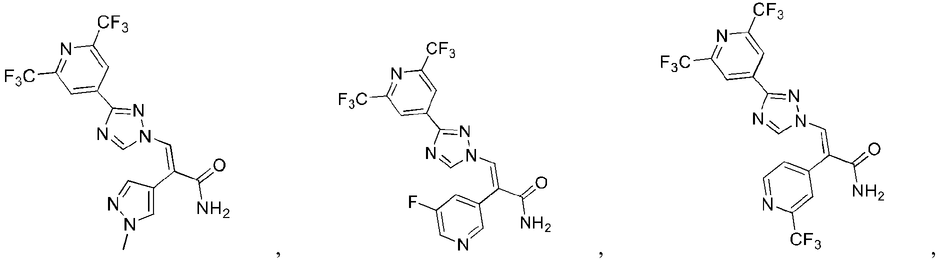 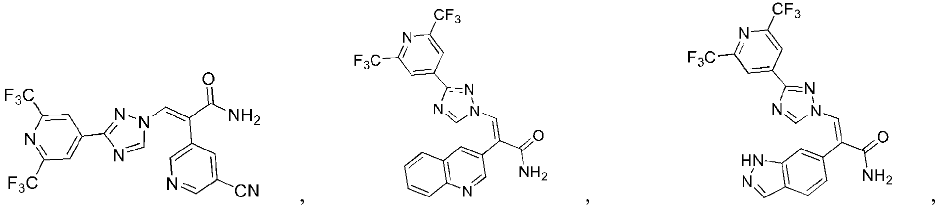 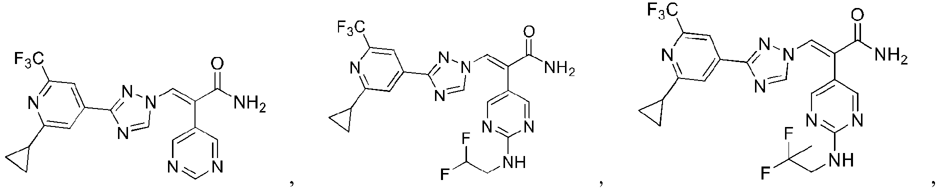 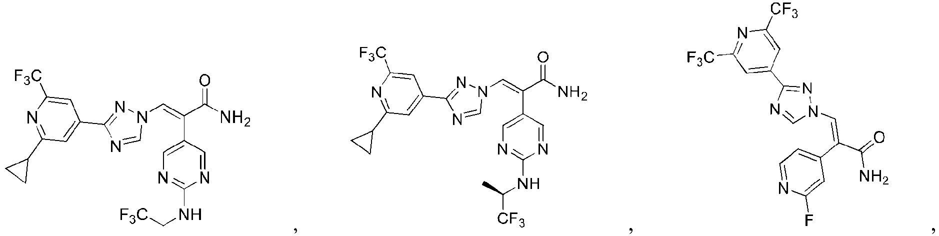 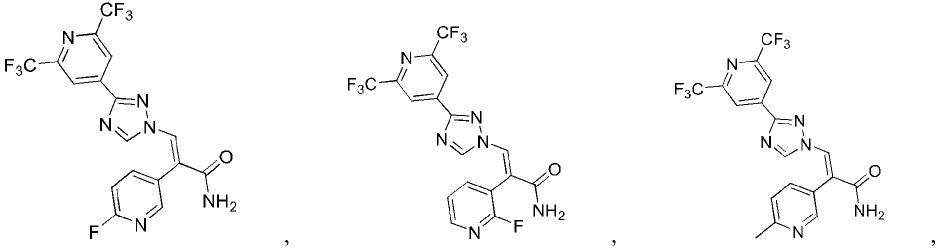 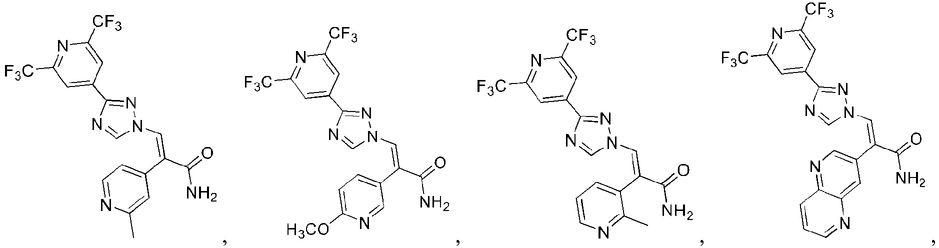 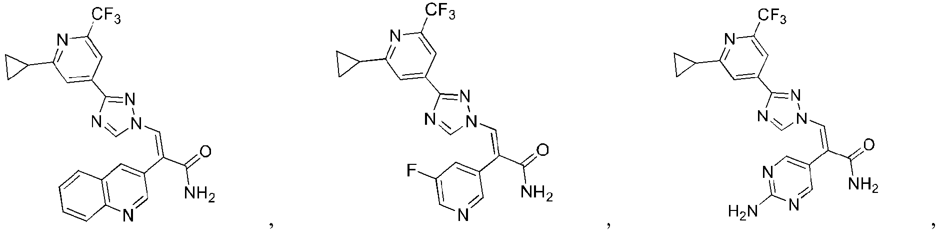 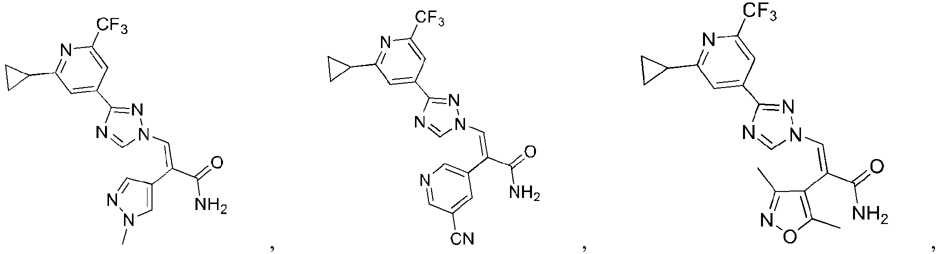 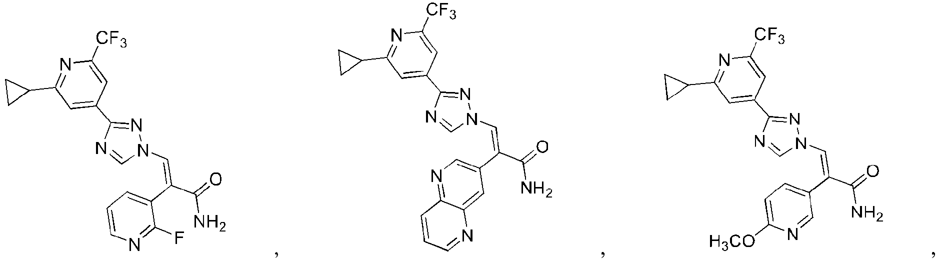 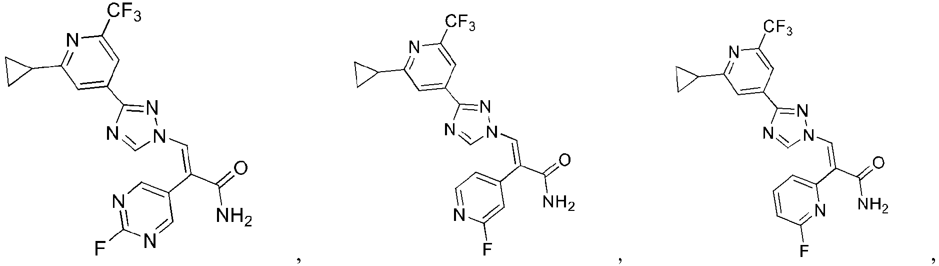 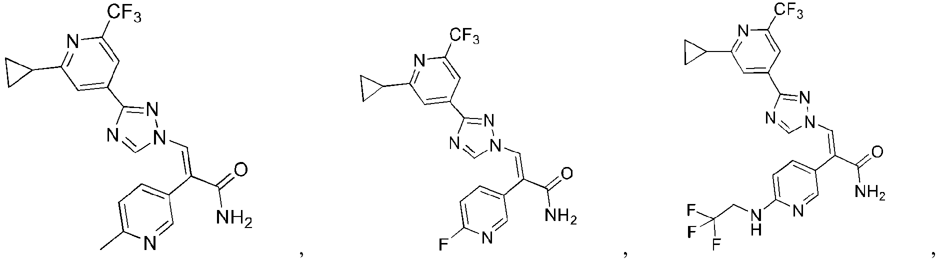 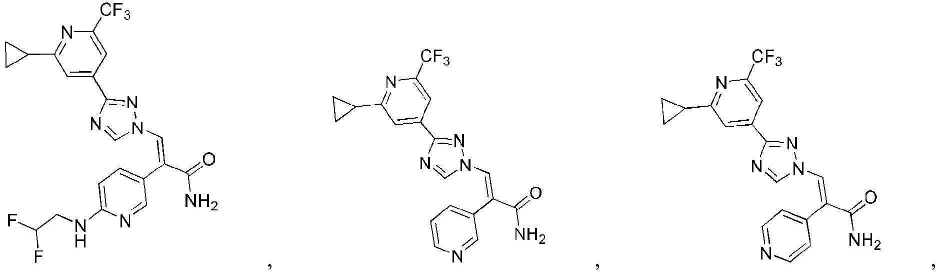 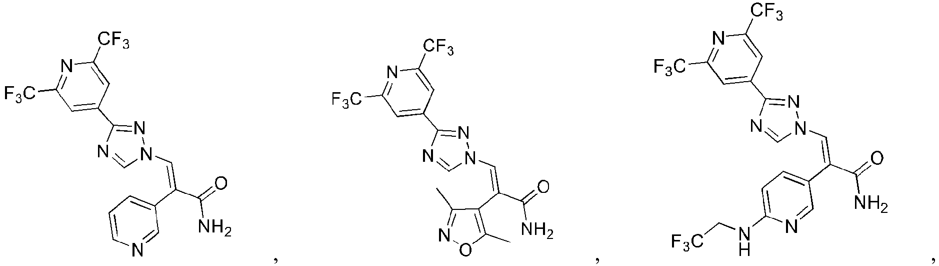 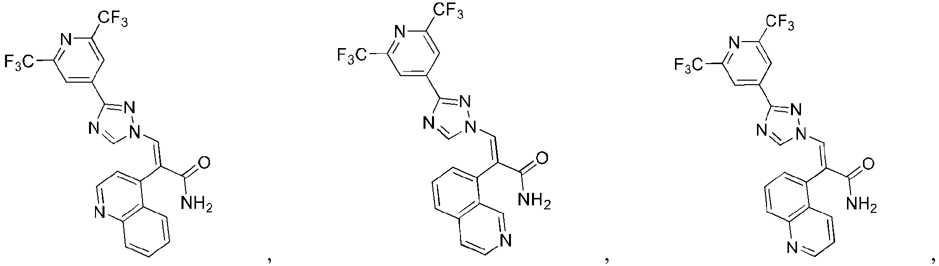 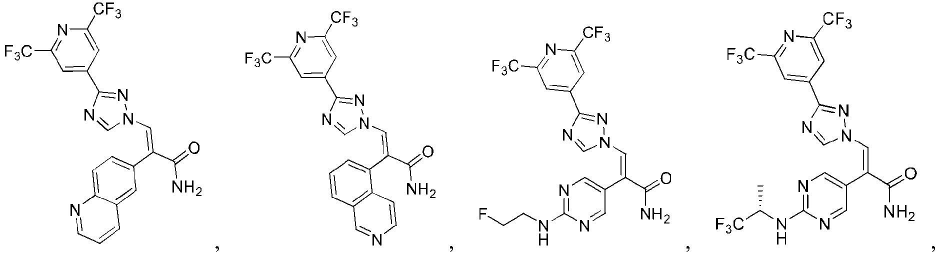 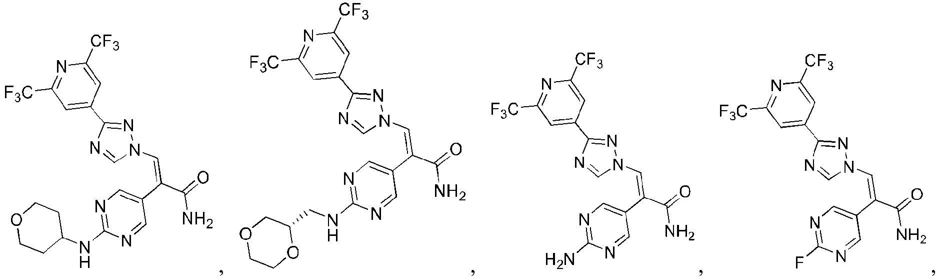 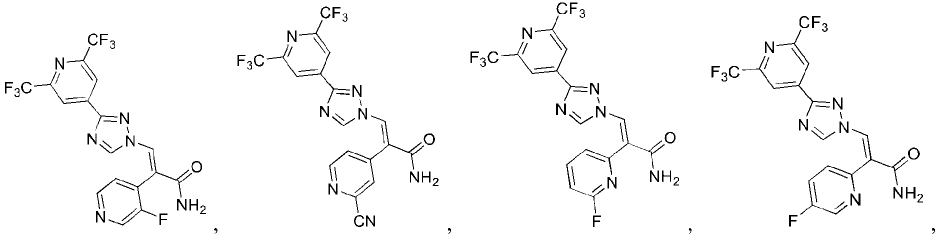 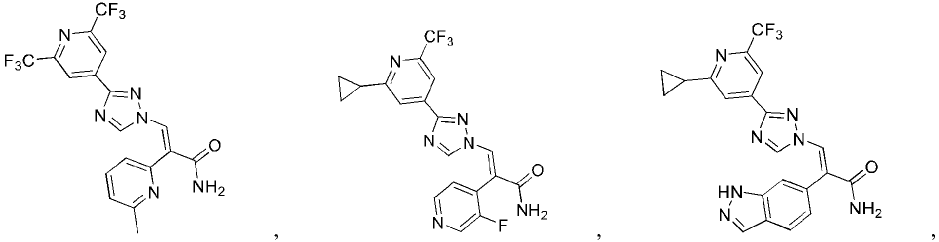 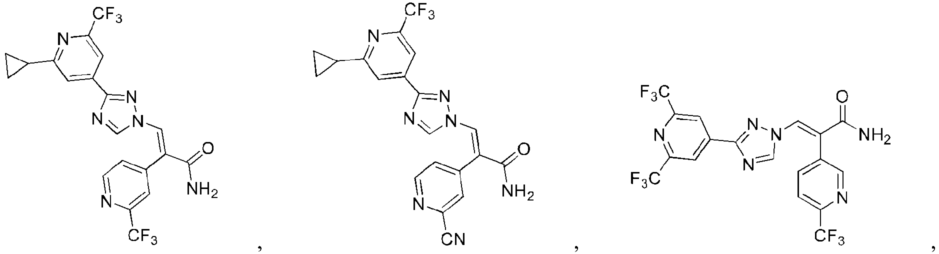 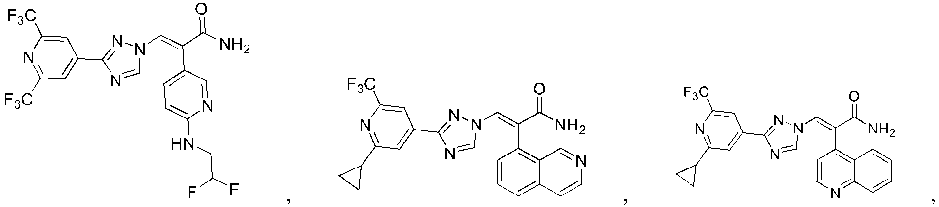 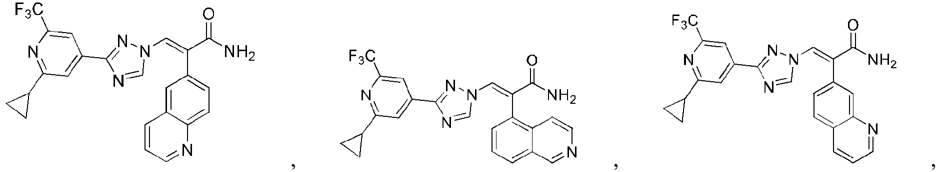 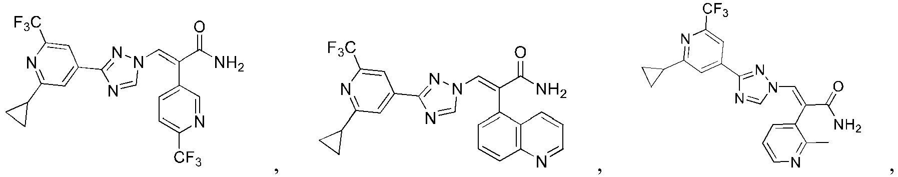 and

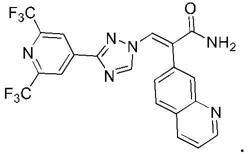

14. The compound of formula I, the crystalline form or the amorphous form thereof, the stereoisomer thereof, the pharmaceutically acceptable salt thereof, or the solvate thereof according to any one of claims 1 to 13, wherein the compound of formula I is selected from the group consisting of the following compounds:

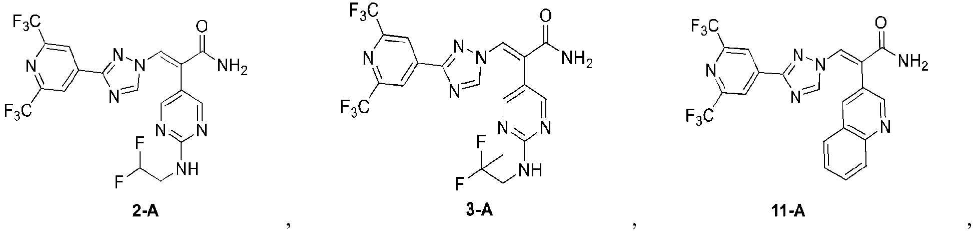

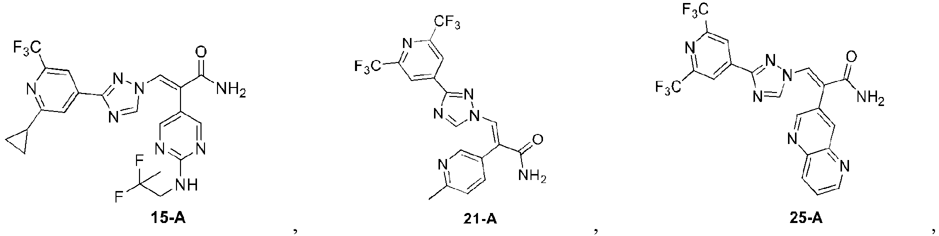

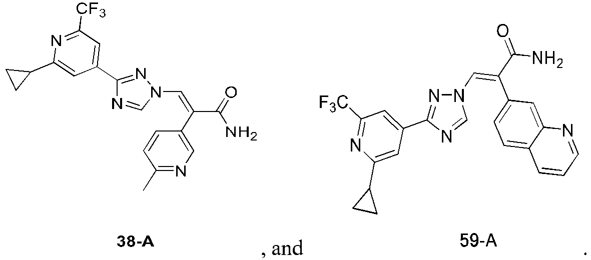

15. The compound of formula I, the crystalline form or the amorphous form thereof, the stereoisomer thereof, the pharmaceutically acceptable salt thereof, or the solvate thereof according to any one of claims 1 to 14, being selected from the group consisting of a crystalline form, a crystalline form of a stereoisomer, and a solvate of the compounds selected from 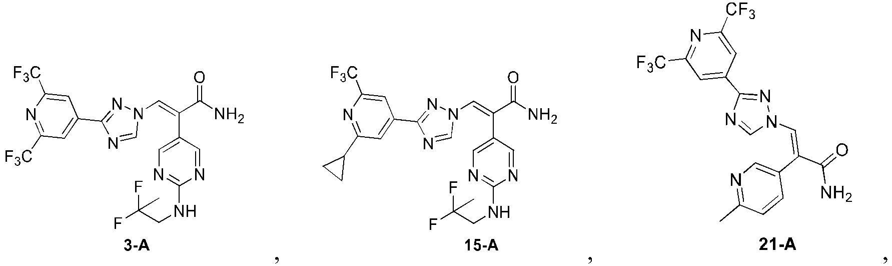 and 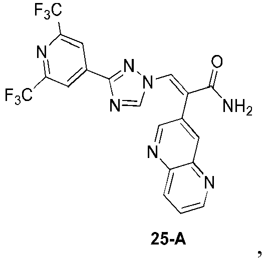 wherein optionally, the solvate is selected from a crystalline form of the solvate;
alternatively, being selected from the group consisting of a crystalline form and a solvate of the compounds selected from 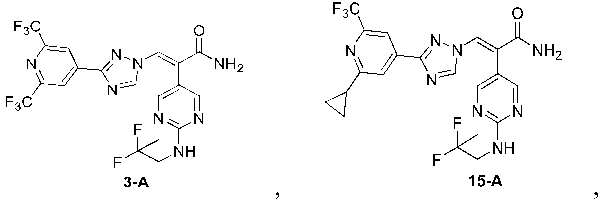 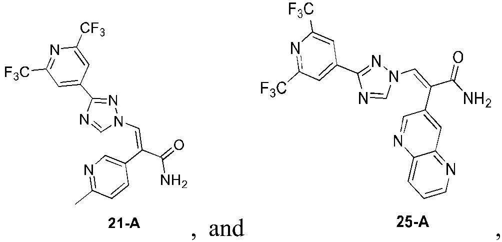 wherein optionally, the solvate is selected from the group consisting of a crystalline form and an amorphous form of the solvate.

16. A crystalline form of compound 21-A or a solvate thereof

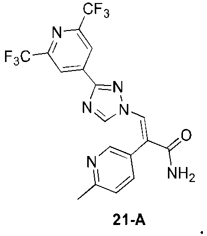

17. The crystalline form of compound 21-A or the solvate thereof according to claim 16, wherein the crystalline form has characteristic diffraction peaks in an X-ray powder diffraction pattern at 2θ angles selected from: 6.44±0.20°, 8.19±0.20°, and 12.84±0.20°;
typically, the crystalline form has characteristic diffraction peaks in an X-ray powder diffraction pattern at 2θ angles selected from: 6.44 ±0.20°, 8.19±0.20°, 12.84±0.20°, 15.49±0.20°, and 21.82±0.20°;
typically, the crystalline form has characteristic diffraction peaks in an X-ray powder diffraction pattern at 2θ angles selected from: 6.44±0.20°, 8.19±0.20°, 12.84±0.20°, 15.49±0.20°, 18.20±0.20°, 21.82±0.20°, and 25.86±0.20°;
typically, the crystalline form has characteristic diffraction peaks in an X-ray powder diffraction pattern at 2θ angles selected from: 6.44±0.20°, 8.19±0.20°, 8.65±0.20°, 12.84±0.20°, 15.49±0.20°, 18.20±0.20°, 21.82±0.20°, and 25.86±0.20°;
typically, the crystalline form has characteristic diffraction peaks in an X-ray powder diffraction pattern at 2θ angles selected from: 6.44±0.20°, 8.19±0.20°, 8.65 ±0.20°, 11.49±0.20°, 12.84±0.20°, 15.49±0.20°, 16.84±0.20°, 18.20±0.20°, 21.08±0.20°, 21.82±0.20°, 22.75±0.20°, 23.05±0.20°, and 25.86±0.20°;
optionally, the crystalline form has at least 3, at least 4, at least 5, at least 6, at least 7, at least 8, at least 9, at least 10, or more characteristic diffraction peaks in an X-ray powder diffraction pattern at 2θ values selected from 6.44±0.20°, 8.19±0.20°, 8.65 ±0.20°, 11.49±0.20°, 12.84+0.20°, 15.49+0.20°, 16.84+0.20°, 18.20+0.20°, 21.08+0.20°, 21.82+0.20°, 22.75+0.20°, 23.05+0.20°, and 25.86+0.20°.

18. The crystalline form of compound 21-A or the solvate thereof according to claim 16, wherein the crystalline form has characteristic diffraction peaks in an X-ray powder diffraction pattern at 2θ angles selected from: 7.15±0.20°, 9.71±0.20°, and 21.78±0.20°;
typically, the crystalline form has characteristic diffraction peaks in an X-ray powder diffraction pattern at 2θ angles selected from: 7.15±0.20°, 9.71±0.20°, 14.35±0.20°, 21.78±0.20°, and 27.99±0.20°;
typically, the crystalline form has characteristic diffraction peaks in an X-ray powder diffraction pattern at 2θ angles selected from: 7.15±0.20°, 9.71±0.20°, 14.35±0.20°, 18.73±0.20°, 21.78±0.20°, 25.09±0.20°, and 27.99±0.20°;
typically, the crystalline form has characteristic diffraction peaks in an X-ray powder diffraction pattern at 2θ angles selected from: 7.15±0.20°, 9.71±0.20°, 14.35±0.20°, 16.55±0.20°, 18.43±0.20°, 21.78±0.20°, 22.93±0.20°, 25.09±0.20°, and 27.99±0.20°;
typically, the crystalline form has characteristic diffraction peaks in an X-ray powder diffraction pattern at 2θ angles selected from: 7.15±0.20°, 9.71±0.20°, 10.05±0.20°, 14.35±0.20°, 16.55±0.20°, 17.32±0.20°, 18.73±0.20°, 21.78±0.20°, 22.93±0.20°, 25.09±0.20°, and 27.99±0.20°;
typically, the crystalline form has characteristic diffraction peaks in an X-ray powder diffraction pattern at 2θ angles selected from: 7.15±0.20°, 9.71±0.20°, 10.05±0.20°, 14.35±0.20°, 16.55±0.20°, 17.32±0.20°, 18.73±0.20°, 19.74±0.20°, 21.78±0.20°, 22.93±0.20°, 25.09±0.20°, 27.99±0.20°, and 28.94±0.20°;
typically, the crystalline form has characteristic diffraction peaks in an X-ray powder diffraction pattern at 2θ angles selected from: 7.15±0.20°, 9.71±0.20°, 10.05±0.20°, 14.35±0.20°, 16.07±0.20°, 16.55±0.20°, 17.32±0.20°, 18.73±0.20°, 19.74±0.20°, 21.78±0.20°, 22.51±0.20°, 22.93±0.20°, 25.09±0.20°, 27.99±0.20°, and 28.94±0.20°;
optionally, the crystalline form has at least 3, at least 4, at least 5, at least 6, at least 7, at least 8, at least 9, at least 10, or more characteristic diffraction peaks in an X-ray powder diffraction pattern at 2θ values selected from 7.15±0.20°, 9.71±0.20°, 10.05±0.20°, 14.35±0.20°, 16.07 ±0.20°, 16.55±0.20°, 17.32±0.20°, 18.73±0.20°, 19.74±0.20°, 21.78±0.20°, 22.51 ±0.20°, 22.93±0.20°, 25.09±0.20°, 27.99±0.20°, and 28.94+0.20°.

19. The crystalline form of compound 21-A or the solvate thereof according to claim 18, wherein the crystalline form is a hydrate of compound 21-A;
optionally, a molar ratio of compound 21-A to water is selected from the group consisting of 1:0.1-10, 1:0.5-10, 1:0.5-8, 1:0.5-7, 1:0.5-6, 1:0.5-5, 1:0.5-4, 1:0.5-3, 1:0.5-2, and 1:0.5-1; alternatively, a molar ratio of compound 21-A to water is selected from the group consisting of 1:0.5, 1:1, 1:2, 1:3, and 1:4, or a range formed by any of the above values;
alternatively, a molar ratio of compound 21-A to water is selected from 1:1;
optionally, a hydrate of compound 21-A has a water content of 0.5%-20% wt, 1%-15% wt, 1%-12% wt, 1%-10% wt, 1%-8% wt, 1%-6% wt, 1%-4% wt, 2%-4% wt, or 3%-4% wt;
alternatively, a hydrate of compound 21-A has a water content of 1% wt, 2% wt, 3% wt, 4% wt, 5% wt, 6% wt, 7% wt, 8% wt, 9% wt, 10% wt, 11% wt, 12% wt, 13% wt, 14% wt, or 15% wt, or a range formed by any of the above values;
alternatively, a hydrate of compound 21-A has a water content of 4 wt%.

20. The crystalline form of compound 21-A or the solvate thereof according to claim 16, wherein the crystalline form has characteristic diffraction peaks in an X-ray powder diffraction pattern at 2θ angles selected from: 9.51±0.20°, 18.91±0.20°, and 19.97±0.20°;
typically, the crystalline form has characteristic diffraction peaks in an X-ray powder diffraction pattern at 2θ angles selected from: 9.51±0.20°, 13.46±0.20°, 18.91±0.20°, 19.97±0.20°, and 20.55±0.20°;
typically, the crystalline form has characteristic diffraction peaks in an X-ray powder diffraction pattern at 2θ angles selected from: 9.51±0.20°, 13.46±0.20°, 15.30±0.20°, 18.91±0.20°, 19.97±0.20°, 20.55±0.20°, and 22.09±0.20°;
typically, the crystalline form has characteristic diffraction peaks in an X-ray powder diffraction pattern at 2θ angles selected from: 9.16±0.20°, 9.51±0.20°, 13.46±0.20°, 15.30±0.20°, 17.67±0.20°, 18.91±0.20°, 19.97±0.20°, 20.55±0.20°, and 22.09±0.20°;
typically, the crystalline form has characteristic diffraction peaks in an X-ray powder diffraction pattern at 2θ angles selected from: 9.16±0.20°, 9.51±0.20°, 13.46±0.20°, 15.30±0.20°, 17.20±0.20°, 17.67±0.20°, 18.91±0.20°, 19.16±0.20°, 19.97±0.20°, 20.55±0.20°, 22.09±0.20°, and 28.35±0.20°;
optionally, the crystalline form has at least 3, at least 4, at least 5, at least 6, at least 7, at least 8, at least 9, at least 10, or more characteristic diffraction peaks in an X-ray powder diffraction pattern at 2θ values selected from 9.16±0.20°, 9.51±0.20°, 13.46±0.20°, 15.30±0.20°, 17.20±0.20°, 17.67±0.20°, 18.91±0.20°, 19.16±0.20°, 19.97±0.20°, 20.55±0.20°, 22.09±0.20°, and 28.35±0.20°.

21. The crystalline form of compound 21-A or the solvate thereof according to claim 16, wherein the crystalline form has characteristic diffraction peaks in an X-ray powder diffraction pattern at 2θ angles selected from: 7.87±0.20° and 23.01±0.20°;
typically, the crystalline form has characteristic diffraction peaks in an X-ray powder diffraction pattern at 2θ angles selected from: 7.87±0.20°, 8.38±0.20°, 22.39±0.20°, and 23.01±0.20°;
typically, the crystalline form has characteristic diffraction peaks in an X-ray powder diffraction pattern at 2θ angles selected from: 7.87±0.20°, 8.38±0.20°, 11.50±0.20°, 22.39±0.20°, 23.01±0.20°, and 24.73±0.20°;
typically, the crystalline form has characteristic diffraction peaks in an X-ray powder diffraction pattern at 2θ angles selected from: 7.87±0.20°, 8.38±0.20°, 11.50±0.20°, 12.72±0.20°, 14.81±0.20°, 18.23±0.20°, 22.39±0.20°, 23.01±0.20°, and 24.73±0.20°;
typically, the crystalline form has characteristic diffraction peaks in an X-ray powder diffraction pattern at 2θ angles selected from: 7.87±0.20°, 8.38±0.20°, 11.50±0.20°, 12.72±0.20°, 14.81±0.20°, 18.23±0.20°, 19.35±0.20°, 20.01±0.20°, 22.39±0.20°, 23.01±0.20°, and 24.73±0.20°;
typically, the crystalline form has characteristic diffraction peaks in an X-ray powder diffraction pattern at 2θ angles selected from: 7.87±0.20°, 8.38±0.20°, 11.06±0.20°, 11.50±0.20°, 12.72±0.20°, 14.81±0.20°, 18.23±0.20°, 19.35±0.20°, 20.01±0.20°, 21.64±0.20°, 22.39±0.20°, 23.01±0.20°, 23.77±0.20°, and 24.73±0.20°;
optionally, the crystalline form has at least 3, at least 4, at least 5, at least 6, at least 7, at least 8, at least 9, at least 10, or more characteristic diffraction peaks in an X-ray powder diffraction pattern at 2θ values selected from 7.87±0.20°, 8.38±0.20°, 11.06±0.20°, 11.50±0.20°, 12.72 ±0.20°, 14.81±0.20°, 18.23±0.20°, 19.35±0.20°, 20.01±0.20°, 21.64±0.20°, 22.39±0.20°, 23.01±0.20°, 23.77±0.20°, and 24.73±0.20°.

22. The crystalline form of compound 21-A or the solvate thereof according to claim 21, wherein the crystalline form is an isopropanol solvate of compound 21-A;
optionally, a molar ratio of compound 21-A to isopropanol is selected from the group consisting of 1:0.2-5, 1:0.2-4, 1:0.2-3, 1:0.2-2, and 1:0.2-1;
alternatively, a molar ratio of compound 21-A to isopropanol is selected from the group consisting of 1:0.3, 1:0.5, 1:1, 1:2, and 1:3, or a range formed by any of the above values; alternatively, a molar ratio of compound 21-A to isopropanol is selected from the group consisting of 1:0.5 and 1:1;
optionally, the isopropanol solvate of compound 21-A has an isopropanol content of 1%-20% wt, 19%-15% wt, 1%-12% wt, 1%-10% wt, 1%-8% wt, 1%-6% wt, or 3%-9% wt;
alternatively, the isopropanol solvate of compound 21-A has an isopropanol content of 1% wt, 2% wt, 3% wt, 4% wt, 5% wt, 6% wt, 7% wt, 8% wt, 9% wt, 10% wt, 11% wt, 12% wt, 13% wt, 14% wt, or 15% wt, or a range formed by any of the above values;
alternatively, the isopropanol solvate of compound 21-A has an isopropanol content of 4% wt, 6% wt, or 8% wt.

23. The crystalline form of compound 21-A or the solvate thereof according to claim 16, wherein the crystalline form has characteristic diffraction peaks in an X-ray powder diffraction pattern at 2θ angles selected from: 7.38±0.20° and 23.40±0.20°;
typically, the crystalline form has characteristic diffraction peaks in an X-ray powder diffraction pattern at 2θ angles selected from: 7.38±0.20°, 17.45±0.20°, 23.40±0.20°, and 25.04±0.20°;
typically, the crystalline form has characteristic diffraction peaks in an X-ray powder diffraction pattern at 2θ angles selected from: 7.38±0.20°, 11.57±0.20°, 17.45±0.20°, 17.87±0.20°, 23.40±0.20°, and 25.04±0.20°;
typically, the crystalline form has characteristic diffraction peaks in an X-ray powder diffraction pattern at 2θ angles selected from: 7.38±0.20°, 11.57±0.20°, 17.45±0.20°, 17.87±0.20°, 19.05±0.20°, 23.40±0.20°, 23.63±0.20°, and 25.04±0.20°;
typically, the crystalline form has characteristic diffraction peaks in an X-ray powder diffraction pattern at 2θ angles selected from: 7.38±0.20°, 11.57±0.20°, 17.25±0.20°, 17.45±0.20°, 17.87±0.20°, 18.45±0.20°, 19.05±0.20°, 23.40±0.20°, 23.63±0.20°, and 25.04±0.20°;
typically, the crystalline form has characteristic diffraction peaks in an X-ray powder diffraction pattern at 2θ angles selected from: 7.38±0.20°, 11.57±0.20°, 17.25±0.20°, 17.45+0.20°, 17.87+0.20°, 18.45+0.20°, 19.05+0.20°, 19.45+0.20°, 22.08+0.20°, 22.53+0.20°, 23.40+0.20°, 23.63+0.20°, 25.04+0.20°, and 28.62+0.20°;
optionally, the crystalline form has at least 3, at least 4, at least 5, at least 6, at least 7, at least 8, at least 9, at least 10, or more characteristic diffraction peaks in an X-ray powder diffraction pattern at 2θ values selected from 7.38±0.20°, 11.57±0.20°, 17.25±0.20°, 17.45±0.20°, 17.87±0.20°, 18.45±0.20°, 19.05±0.20°, 19.45±0.20°, 22.08±0.20°, 22.53±0.20°, 23.40±0.20°, 23.63±0.20°, 25.04±0.20°, and 28.62±0.20°.

24. The crystalline form of compound 21-A or the solvate thereof according to claim 23, wherein the crystalline form is an ethanol solvate of compound 21-A;
optionally, a molar ratio of compound 21-A to ethanol is selected from the group consisting of 1:0.5-5, 1:0.5-4, 1:0.5-3, 1:0.5-2, and 1:0.5-1.5;
alternatively, a molar ratio of compound 21-A to ethanol is selected from the group consisting of 1:0.5, 1:1, 1:2; 1:3, 1:4, 1:5, and 1:6; alternatively, a molar ratio of compound 21-A to ethanol is selected from 1:1;
optionally, the ethanol solvate of compound 21-A has an ethanol content of 1%-40% wt, 1%-30% wt, 1%-20% wt, 2%-15% wt, 5%-10% wt, 5%-10% wt, or 7%-10% wt;
alternatively, the ethanol solvate of compound 21-A has an ethanol content of 1% wt, 2% wt, 3% wt, 4% wt, 5% wt, 6% wt, 7% wt, 8% wt, 9% wt, 10% wt, 11% wt, 12% wt, 13% wt, 14% wt, or 15% wt, or a range formed by any of the above values;
alternatively, the ethanol solvate of compound 21-A has an ethanol content of 7% wt or 10% wt.

25. The crystalline form of compound 21-A or the solvate thereof according to claim 16, wherein the crystalline form has characteristic diffraction peaks in an X-ray powder diffraction pattern at 2θ angles selected from: 7.59±0.20°, 23.28±0.20°, and 24.94±0.20;
typically, the crystalline form has characteristic diffraction peaks in an X-ray powder diffraction pattern at 2θ angles selected from: 7.59±0.20°, 11.83±0.20°, 17.77±0.20°, 23.28±0.20°, and 24.94±0.20°;
typically, the crystalline form has characteristic diffraction peaks in an X-ray powder diffraction pattern at 2θ angles selected from: 7.59±0.20°, 11.83±0.20°, 17.77±0.20°, 19.44±0.20°, 23.28±0.20°, 23.70±0.20°, and 24.94±0.20°;
typically, the crystalline form has characteristic diffraction peaks in an X-ray powder diffraction pattern at 2θ angles selected from: 7.59±0.20°, 8.05±0.20°, 11.83±0.20°, 17.77±0.20°, 19.44±0.20°, 22.60±0.20°, 23.28±0.20°, 23.70±0.20°, and 24.94±0.20°;
typically, the crystalline form has characteristic diffraction peaks in an X-ray powder diffraction pattern at 2θ angles selected from: 7.59±0.20°, 8.05±0.20°, 11.83±0.20°, 17.77±0.20°, 19.44±0.20°, 22.60±0.20°, 23.28±0.20°, 23.70±0.20°, 24.20±0.20°, 24.94±0.20°, and 28.68±0.20°;
optionally, the crystalline form has at least 3, at least 4, at least 5, at least 6, at least 7, at least 8, at least 9, at least 10, or more characteristic diffraction peaks in an X-ray powder diffraction pattern at 2θ values selected from 7.59±0.20°, 8.05±0.20°, 11.83±0.20°, 17.77±0.20°, 19.44±0.20°, 22.60±0.20°, 23.28±0.20°, 23.70±0.20°, 24.20±0.20°, 24.94±0.20°, and 28.68±0.20°.

26. The crystalline form of compound 21-A or the solvate thereof according to claim 25, wherein the crystalline form is a methanol solvate of compound 21-A;
optionally, a molar ratio of compound 21-A to methanol is selected from the group consisting of 1:0.1-6, 1:0.1-5, 1:0.1-4, 1:0.5-4, 1:0.5-3, 1:0.5-2, 1:0.5-1, and 1:1;
alternatively, a molar ratio of compound 21-A to methanol is selected from the group consisting of 1:0.1, 1:0.5, 1:1, 1:1.5, 1:2, 1:3, 1:4, 1:5, and 1:6, or a range formed by any of the above values;
alternatively, a molar ratio of compound 21-A to methanol is selected from the group consisting of 1:0.5 and 1:1;
optionally, the methanol solvate of compound 21-A has a methanol content of 1%-20% wt, 1%-15% wt, 1%-12% wt, 1%-10% wt, 1%-8% wt, 2%-6% wt, and 2%-4% wt;
alternatively, the methanol solvate of compound 21-A has a methanol content of 1% wt, 2% wt, 3% wt, 4% wt, 5% wt, 6% wt, 7% wt, 8% wt, 9% wt, 10% wt, 11% wt, 12% wt, 13% wt, 14% wt, or 15% wt, or a range formed by any of the above values;
alternatively, the methanol solvate of compound 21-A has a methanol content of 2% wt or 4% wt.

27. The crystalline form of compound 21-A or the solvate thereof according to claim 16, wherein the crystalline form is **characterized by**: a monoclinic crystal system; space group: P-1; unit cell parameters: a = 10.9919(3) Å, b = 14.7579(4) Å, c = 16.0678(4) Å, α = 108.8840(10)°, β = 105.1420(10)°, γ = 98.9680(10)°; Z = 2.

28. The compound of formula I, the stereoisomer thereof, or the pharmaceutically acceptable salt thereof according to claim 15, being selected from a crystalline form of compound 3-A, wherein the crystalline form of compound 3-A has diffraction peaks in an X-ray powder diffraction (XRPD) pattern using Cu Kα radiation at 2θ angles selected from 10.48±0.20°, 19.46±0.20°, 21.56±0.20°, and 23.28±0.20°;
alternatively, the crystalline form has diffraction peaks at 10.48±0.20°, 12.78±0.20°, 19.46±0.20°, 20.74±0.20°, 21.56±0.20°, 21.94±0.20°, or 23.28±0.20°;
alternatively, the crystalline form has diffraction peaks at 10.48±0.20°, 12.78±0.20°, 14.68±0.20°, 18.49±0.20°, 19.07±0.20°, 19.46±0.20°, 21.02±0.20°, 20.74±0.20°, 21.56±0.20°, 21.94±0.20°, or 23.28±0.20°;
alternatively, the crystalline form has diffraction peaks at 10.48±0.20°, 11.89±0.20°, 12.78±0.20°, 14.68±0.20°, 18.49±0.20°, 19.07±0.20°, 19.46±0.20°, 21.02±0.20°, 20.74±0.20°, 21.56±0.20°, 21.94±0.20°, 22.33±0.20°, or 23.28±0.20°;
alternatively, the crystalline form has at least 3, at least 4, at least 5, at least 6, at least 7, at least 8, at least 9, at least 10, or more characteristic diffraction peaks at 2θ values selected from the group consisting of 10.48±0.20°, 11.89±0.20°, 12.78±0.20°, 14.68±0.20°, 18.49±0.20°, 19.07±0.20°, 19.46±0.20°, 21.02±0.20°, 20.74±0.20°, 21.56±0.20°, 21.94±0.20°, 22.33±0.20°, and 23.28±0.20°.

29. The compound of formula I, the stereoisomer thereof, or the pharmaceutically acceptable salt thereof according to claim 15, being selected from a crystalline form of a solvate of compound 3-A, wherein the crystalline form of the solvate of compound 3-A has diffraction peaks in an X-ray powder diffraction (XRPD) pattern using Cu Kα radiation at 2θ angles selected from 7.21±0.20°, 23.03±0.20°, and 24.18±0.20°;
alternatively, the crystalline form has diffraction peaks at 7.21±0.20°, 19.74±0.20°, 21.31±0.20°, 21.68±0.20°, 22.03±0.20°, 23.03±0.20°, or 24.18±0.20°;
alternatively, the crystalline form has diffraction peaks at 7.21+0.20°, 12.56+0.20°, 13.47+0.20°, 13.87+0.20°, 19.74+0.20°, 21.31+0.20°, 21.68+0.20°, 22.03+0.20°, 23.03±0.20°, or 24.18+0.20°;
alternatively, the crystalline form has diffraction peaks at 7.21±0.20°, 12.56+0.20°, 13.47+0.20°, 13.87+0.20°, 18.31+0.20°, 18.60+0.20°, 19.74+0.20°, 21.31+0.20°, 21.68+0.20°, 22.03±0.20°, 23.03±0.20°, 24.18±0.20°, or 24.95±0.20°;
alternatively, the crystalline form has at least 3, at least 4, at least 5, at least 6, at least 7, at least 8, at least 9, at least 10, or more characteristic diffraction peaks at 2θ values selected from the group consisting of 7.21±0.20°, 12.56±0.20°, 13.47±0.20°, 13.87±0.20°, 18.31±0.20°, 18.60±0.20°, 19.74±0.20°, 21.31±0.20°, 21.68±0.20°, 22.03±0.20°, 23.03±0.20°, 24.18±0.20°, and 24.95±0.20°;
optionally, the crystalline form of the solvate of compound 3-A is a crystalline form of a 1,4-dioxane solvate of compound 3-A;
optionally, in the crystalline form of the 1,4-dioxane solvate of compound 3-A, a molar ratio of compound 3-A to 1,4-dioxane is selected from the group consisting of about 1:0.2-2, about 1:0.2-1, about 1:0.3-0.8, and about 1:0.5.

30. The compound of formula I, the stereoisomer thereof, or the pharmaceutically acceptable salt thereof according to claim 15, being selected from a crystalline form of a solvate of compound 3-A, wherein the crystalline form of the solvate of compound 3-A has diffraction peaks in an X-ray powder diffraction (XRPD) pattern using Cu Kα radiation at 2θ angles selected from 8.40±0.20°, 19.29±0.20°, 22.11±0.20°, and 22.89±0.20°;
alternatively, the crystalline form has diffraction peaks at 8.40±0.20°, 18.62±0.20°, 19.29±0.20°, 20.35±0.20°, 21.30±0.20°, 22.11±0.20°, 22.89±0.20°, or 26.27±0.20°;
alternatively, the crystalline form has diffraction peaks at 8.40±0.20°, 14.87±0.20°, 16.83±0.20°, 17.10±0.20°, 18.62±0.20°, 19.29±0.20°, 20.35±0.20°, 21.30±0.20°, 22.11±0.20°, 22.89±0.20°, or 26.27±0.20°;
alternatively, the crystalline form has diffraction peaks at 8.40±0.20°, 13.08±0.20°, 14.58±0.20°, 14.87±0.20°, 16.83±0.20°, 17.10±0.20°, 18.62±0.20°, 19.29±0.20°, 20.35±0.20°, 21.30±0.20°, 22.11±0.20°, 22.89±0.20°, 23.47±0.20°, 25.43±0.20°, or 26.27±0.20°;
alternatively, the crystalline form has at least 3, at least 4, at least 5, at least 6, at least 7, at least 8, at least 9, at least 10, or more characteristic diffraction peaks at 2θ values selected from the group consisting of 8.40+0.20°, 13.08±0.20°, 14.58+0.20°, 14.87+0.20°, 16.83+0.20°, 17.10+0.20°, 18.62+0.20°, 19.29+0.20°, 20.35+0.20°, 21.30+0.20°, 22.11+0.20°, 22.89+0.20°, 23.47+0.20°, 25.43+0.20°, and 26.27+0.20°;
optionally, the crystalline form of the solvate of compound 3-A is a crystalline form of a tetrahydrofuran solvate of compound 3-A;
optionally, in the crystalline form of the tetrahydrofuran solvate of compound 3-A, a molar ratio of compound 3-A to tetrahydrofuran is selected from the group consisting of: about 1:0.2-2, about 1:0.2-1, about 1:0.3-0.8, and about 1:0.5.

31. The compound of formula I, the stereoisomer thereof, or the pharmaceutically acceptable salt thereof according to claim 15, being selected from a crystalline form of compound 15-A, wherein the crystalline form of compound 15-A has diffraction peaks in an X-ray powder diffraction (XRPD) pattern using Cu Kα radiation at 2θ angles selected from 8.13±0.20°, 17.08±0.20°, 20.23±0.20°, and 20.97±0.20°;
alternatively, the crystalline form has diffraction peaks at 8.13±0.20°, 15.63±0.20°, 17.08±0.20°, 19.94±0.20°, 20.23±0.20°, 20.97±0.20°, 21.87±0.20°, or 23.63±0.20°;
alternatively, the crystalline form has diffraction peaks at 8.13±0.20°, 9.63±0.20°, 14.27±0.20°, 15.63±0.20°, 17.08±0.20°, 19.22±0.20°, 19.94±0.20°, 20.23±0.20°, 20.97±0.20°, 21.32±0.20°, 21.87±0.20°, 22.48±0.20°, or 23.63±0.20°;
alternatively, the crystalline form has diffraction peaks at 8.13±0.20°, 9.63±0.20°, 11.78±0.20°, 13.36±0.20°, 14.27±0.20°, 15.63±0.20°, 17.08±0.20°, 19.22±0.20°, 19.94±0.20°, 20.23±0.20°, 20.97±0.20°, 21.32±0.20°, 21.87±0.20°, 22.48±0.20°, 23.63±0.20°, 24.26±0.20°, or 25.05±0.20°;
alternatively, the crystalline form has at least 3, at least 4, at least 5, at least 6, at least 7, at least 8, at least 9, at least 10, or more characteristic diffraction peaks at 20 values selected from the group consisting of 8.13±0.20°, 9.63±0.20°, 11.78±0.20°, 13.36±0.20°, 14.27±0.20°, 15.63±0.20°, 17.08±0.20°, 19.22±0.20°, 19.94±0.20°, 20.23±0.20°, 20.97±0.20°, 21.32±0.20°, 21.87±0.20°, 22.48±0.20°, 23.63±0.20°, 24.26±0.20°, and 25.05±0.20°.

32. The compound of formula I, the stereoisomer thereof, or the pharmaceutically acceptable salt thereof according to claim 15, being selected from a crystalline form of a solvate of compound 15-A, wherein the crystalline form of the solvate of compound 15-A has diffraction peaks in an X-ray powder diffraction (XRPD) pattern using Cu Kα radiation at 2θ angles selected from 6.30+0.20°, 16.79+0.20°, and 24.00+0.20°;
alternatively, the crystalline form has diffraction peaks at 6.30+0.20°, 7.85+0.20°, 16.79+0.20°, 18.43+0.20°, 22.85+0.20°, or 24.00+0.20°;
alternatively, the crystalline form has diffraction peaks at 6.30±0.20°, 7.85±0.20°, 10.18±0.20°, 12.21±0.20°, 15.69±0.20°, 16.79±0.20°, 17.69±0.20°, 18.43±0.20°, 18.80±0.20°, 22.85±0.20°, or 24.00±0.20°;
alternatively, the crystalline form has diffraction peaks at 6.30±0.20°, 7.85±0.20°, 10.18±0.20°, 12.21±0.20°, 14.71±0.20°, 15.69±0.20°, 16.79±0.20°, 17.69±0.20°, 18.43±0.20°, 18.80±0.20°, 22.85±0.20°, 24.00±0.20°, or 26.08±0.20°;
alternatively, the crystalline form has diffraction peaks at 6.30±0.20°, 7.85±0.20°, 10.18±0.20°, 12.21±0.20°, 14.71±0.20°, 15.69±0.20°, 16.79±0.20°, 17.69±0.20°, 18.43±0.20°, 18.80±0.20°, 21.23±0.20°, 22.85±0.20°, 24.00±0.20°, 26.08±0.20°, or 26.72±0.20°;
alternatively, the crystalline form has at least 3, at least 4, at least 5, at least 6, at least 7, at least 8, at least 9, at least 10, or more characteristic diffraction peaks at 2θ values selected from the group consisting of 6.30±0.20°, 7.85±0.20°, 10.18±0.20°, 12.21±0.20°, 14.71±0.20°, 15.69±0.20°, 16.79±0.20°, 17.69±0.20°, 18.43±0.20°, 18.80±0.20°, 21.23±0.20°, 22.85±0.20°, 24.00±0.20°, 26.08±0.20°, and 26.72±0.20°;
optionally, the crystalline form of the solvate of compound 15-A is a crystalline form of an acetonitrile solvate of compound 15-A;
optionally, in the crystalline form of the acetonitrile solvate of compound 15-A, a molar ratio of compound 15-A to acetonitrile is selected from the group consisting of: 1:0.2-2, 1:0.5-1.5, 1:0.5-1, and 1:1.

33. The compound of formula I, the stereoisomer thereof, or the pharmaceutically acceptable salt thereof according to claim 15, being selected from a crystalline form of compound 25-A, wherein the crystalline form of compound 25-A has diffraction peaks in an X-ray powder diffraction (XRPD) pattern using Cu Kα radiation at 2θ angles selected from 4.53±0.20°, 7.13±0.20°, 16.30±0.20°, and 20.50±0.20°;
alternatively, the crystalline form has diffraction peaks at 4.53±0.20°, 7.13±0.20°, 9.04±0.20°, 16.30+0.20°, 19.21+0.20°, 20.50+0.20°, 20.87+0.20°, 24.54+0.20°, or 25.92+0.20°;
alternatively, the crystalline form has diffraction peaks at 4.53+0.20°, 7.13+0.20°, 9.04+0.20°, 11.29+0.20°, 16.30+0.20°, 17.24+0.20°, 17.65+0.20°, 19.21+0.20°, 20.50+0.20°, 20.87+0.20°, 22.29+0.20°, 24.54+0.20°, or 25.92+0.20°;
alternatively, the crystalline form has diffraction peaks at 4.53+0.20°, 7.13+0.20°, 9.04+0.20°, 11.29±0.20°, 16.30±0.20°, 17.24±0.20°, 17.65±0.20°, 18.12±0.20°, 19.21±0.20°, 20.50±0.20°, 20.87±0.20°, 22.29±0.20°, 24.54±0.20°, 25.29±0.20°, 25.92±0.20°, or 30.47±0.20°;
alternatively, the crystalline form has at least 3, at least 4, at least 5, at least 6, at least 7, at least 8, at least 9, at least 10, or more characteristic diffraction peaks at 2θ values selected from the group consisting of 4.53±0.20°, 7.13±0.20°, 9.04±0.20°, 11.29+0.20°, 16.30±0.20°, 17.24±0.20°, 17.65±0.20°, 18.12±0.20°, 19.21±0.20°, 20.50±0.20°, 20.87±0.20°, 22.29±0.20°, 24.54±0.20°, 25.29±0.20°, 25.92±0.20°, and 30.47±0.20°.

34. A crystal form composition, comprising the compound, the crystalline form or the amorphous form thereof, the stereoisomer thereof, the pharmaceutically acceptable salt thereof, or the solvate thereof according to any one of claims 1 to 33, wherein the compound, the crystalline form or the amorphous form thereof, the stereoisomer thereof, the pharmaceutically acceptable salt thereof, or the solvate thereof accounts for 50% or more, preferably 80% or more, more preferably 90% or more, and most preferably 95% or more of a weight of the crystal form composition.

35. A pharmaceutical composition, comprising a therapeutically effective amount of the compound, the crystalline form or the amorphous form thereof, the stereoisomer thereof, the pharmaceutically acceptable salt thereof, or the solvate thereof according to any one of claims 1 to 33, or the crystal form composition according to claim 34.

36. Use of the compound, the crystalline form or the amorphous form thereof, the stereoisomer thereof, the pharmaceutically acceptable salt thereof, or the solvate thereof according to any one of claims 1 to 33, the crystal form composition according to claim 34, or the pharmaceutical composition according to claim 35 in preparing a medicament for preventing or treating a related disease.

37. The use according to claim 36, wherein the related disease is selected from the group consisting of tumors; optionally, the related disease is selected from the group consisting of leukemia and lymphoma.
